(19) Europäisches Patentamt
European Patent Office
Office européen des brevets

(11) EP 2 920 163 B1

(12) # EUROPÄISCHE PATENTSCHRIFT

(45) Veröffentlichungstag und Bekanntmachung des Hinweises auf die Patenterteilung:
**02.05.2018 Patentblatt 2018/18**

(21) Anmeldenummer: **13788999.4**

(22) Anmeldetag: **08.11.2013**

(51) Int Cl.:
***C07D 401/12*** (2006.01) ***C07D 405/12*** (2006.01)
***C07D 409/12*** (2006.01)

(86) Internationale Anmeldenummer:
**PCT/EP2013/073424**

(87) Internationale Veröffentlichungsnummer:
**WO 2014/076015 (22.05.2014 Gazette 2014/21)**

(54) **PYRIDYLOXYALKYLCARBOXAMIDE UND DEREN VERWENDUNG ALS ENDOPARASITIZIDE UND NEMATIZIDE**

PYRIDYLOXYAL ALKYL CARBOXAMIDES AND THEIR USE AS ENDOPARASITICIDES AND NEMATICIDE

PYRIDYLOXYALKYLCARBOXAMIDE ET SON UTILISATION COMME ENDOPARASITICIDE ET NÉMATICIDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.11.2012 EP 12192446**

(43) Veröffentlichungstag der Anmeldung:
**23.09.2015 Patentblatt 2015/39**

(73) Patentinhaber: **Bayer CropScience AG**
**40789 Monheim am Rhein (DE)**

(72) Erfinder:
- **GREUL, Jörg Nico**
**51381 Leverkusen (DE)**
- **SCHWARZ, Hans-Georg**
**46282 Dorsten (DE)**
- **ALIG, Bernd**
**53639 Königswinter (DE)**
- **BECKER, Angela**
**40235 Düsseldorf (DE)**
- **PORTZ, Daniela**
**52391 Vettweiß (DE)**
- **ILG, Kerstin**
**50670 Köln (DE)**
- **GÖRGENS, Ulrich**
**40882 Ratingen (DE)**
- **WELZ, Claudia**
**40597 Düsseldorf (DE)**

(74) Vertreter: **BIP Patents**
**c/o Bayer Intellectual Property GmbH**
**Alfred-Nobel-Straße 10**
**40789 Monheim am Rhein (DE)**

(56) Entgegenhaltungen:
**EP-A1- 1 574 511** **EP-A1- 1 997 800**
**EP-A1- 2 132 987** **WO-A1-2008/101976**
**WO-A1-2009/012998** **WO-A1-2011/151370**
**DE-A1- 10 307 845**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 920 163 B1

**Beschreibung**

Die vorliegende Anmeldung betrifft neue Pyridyloxyalkylcarboxamide und ihre Verwendung als Endoparasitizide gegen Endoparasiten in Tieren oder Menschen, sowie ihre Verwendung als Nematizide zur Bekämpfung phytopathogener Nematoden, weiterhin Endoparasitizide und Nematizide, die Pyridyloxyalkylcarboxamide enthalten.

Im Bereich der Tiermedizin stellt das Auftreten von Resistenzen gegen alle kommerziell verfügbaren Anthelmintika ein zunehmendes Problem dar, demzufolge Endoparasitizide mit neuen molekularen Wirkmechanismen benötigt werden. Solche Verbindungen sollten exzellente Effektivität gegen ein breites Helminthen- bzw. Nematodenspektrum zeigen und zugleich bei den behandelten Tieren keine toxischen Effekte hervorrufen.

Auch im Bereich Pflanzenschutz können Nematoden erhebliche Ernteverluste verursachen und werden daher auch mit chemischen Wirkstoffen mit nematizider Aktivität bekämpft. Hierfür sollten geeignete Wirkstoffe von hoher Aktivität sein, ein breites Wirkspektrum gegen verschiedene Nematoden-Arten haben und gleichzeitig toxikologisch unkritisch für nicht-Target-Organismen sein.

Bestimmte Phenacylbenzamide werden in der WO-A 2001/060783 für die orale Anwendung als Anthelmintika für die Veterinärmedizin beansprucht.

Isothiazolcarboxamide sind aus der WO-A 1999/24413, Heterocyclylethylcarboxamid-Derivate aus der WO-A 2006/108791, Heterocyclylethylbenzamid-Derivate aus der WO-A 2006/108792, N-(1-Methyl-2-phenylethyl)-benzamide aus der WO-A 2007/060162, N-(1-Methyl-2-phenylethyl)-carboxamide aus der WO-A 2007/060164, N-Phenethylcarboxamid-Derivative aus der WO-A 2007/060166, N-(3-Phenylpropyl)-carboxamide aus der WO-A 2008/101976, Pyrazolcarboxamide aus der WO-A 2008/148570 und WO-A 2010/063700, Pyrazinylcarboxamide aus der WO-A 2011/128989, verschiedene 2-Pyridylethylcarboxamid-Derivate aus der WO-A 2004/016088, WO-A 2004/074280, WO-A 2005/014545, WO-A 2005/058828, WO-A 2005/058833 und WO-A 2005/085238 sowie Pyridyloxyalkylcarboxamide aus der WO-A 2009/012998 als agrochemische Fungizide bekannt. WO-A 2011/151370 beschreibt N-[(Het)arylalkyl)] pyrazolecarboxamide oder -thiocarboxamide als Fungizide. Ferner sind N-2-(Hetero)arylethyl-carboxamid-Derivate in WO-A 2007/108483/EP-A 1 997800 als Fungizide und Nematizide beschrieben. Die Verwendung von 2-Pyridylethylcarboxamid-Derivaten wird in der WO-A 2008/126922 explizit zur Anwendung gegen Nematoden im Pflanzenanbau beansprucht. Die WO-A 2012/118139 umfasst auch Phenyl-(oxy)ethylcarboxamide und 2-Pyridyl-(oxy)ethylcarboxamide als Endoparasitizide, die aber nur als Benzamide ausgeführt werden. DE 103 07 845 A1 offenbart heterocyclische Amide als Schaedlingsbekeampfungsmittel, wobei die Verbindungen 1009 und 1011 Bestandteil der hier offenbarten Verbindungsklasse sind und daher vom Schutzbereich ausgenommen werden.

Weitere nematizide und/oder anthelmintische Patentanmeldungen wurden nach dem Prioritätsdatum dieser vorliegenden Anmeldung publiziert: WO-A 2013/064460 und WO-A 2013/064461 beschreiben Pyridylethylcarboxamide und deren Verwendung als Nematizide. WO-A2013/076230 beansprucht auch Phenyloxyethylcarboxamide und deren Verwendung als Arzneimittel zur Bekämpfung von Endoparasiten bei Tieren oder Menschen.

Die Anwendung der bisher vorbekannten Pyridyloxyalkylcarboxamide als Endoparasitizide im Bereich der Veterinärmedizin und als Nematizide im Pflanzenanbau ist bislang nicht beschrieben.

Es gibt in der Literatur Hinweise darauf, dass die Einbindung von Sauerstoff in Nachbarschaft zum heteroaromatischen System die Nachteile einer metabolischen Instabilität einer benzylischen $CH_2$-Gruppe überwinden kann. Somit werden neue Pyridyloxyalkylcarboxamide und ihre Verwendung als Endoparasitizide gegen Endoparasiten in Tieren oder Menschen, sowie ihre Verwendung als Nematizide zur Bekämpfung phytopathogener Nematoden, weiterhin Endoparasitizide und Nematizide, die Pyridyloxyalkylcarboxamide enthalten, in dieser Anmeldung beschrieben.

**Erfindung**

Die Erfindung wird nun erläutert durch die folgenden Passagen. Definiert wird die Erfindung allerdings ausschließlich durch die angehängten Ansprüche.

Es wurden Verbindungen der Formel (I)

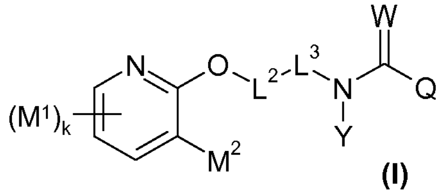

**(I)**

gefunden, in welcher

Q für folgende Strukturelemente steht, wobei n für jedes Q jeweils wie unten angegeben definiert ist:

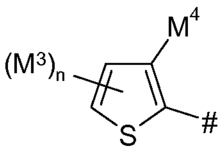

**$Q^{1}$**
n = 1-2

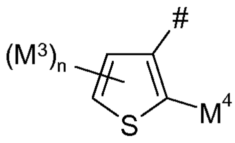

**$Q^{2}$**
n =1-2

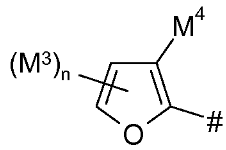

**$Q^{3}$**
n = 1-2

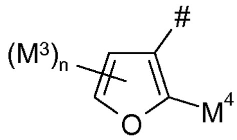

**$Q^{4}$**
n =1-2

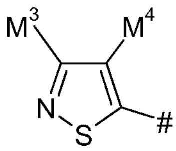

**$Q^{5}$**

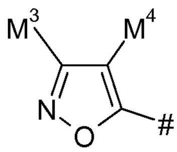

**$Q^{6}$**

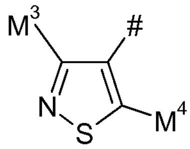

**$Q^{7}$**

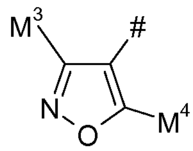

**$Q^{8}$**

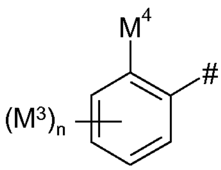

**$Q^{9}$**
n =1-4

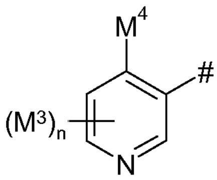

**$Q^{10}$**
n =1-3

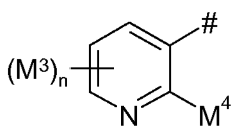

**$Q^{11}$**
n = 1-3

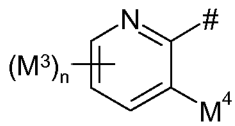

**$Q^{12}$**
n = 1-3

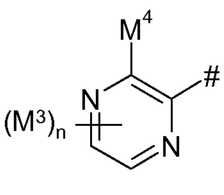

**$Q^{13}$**
n = 1-2

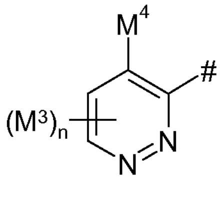

**$Q^{14}$**
n = 1-2

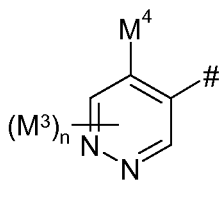

**$Q^{15}$**
n = 1-2

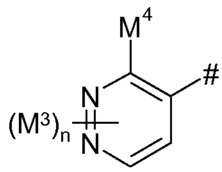

**$Q^{16}$**
n = 1-2

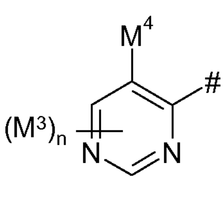

**$Q^{17}$**
n = 1-2

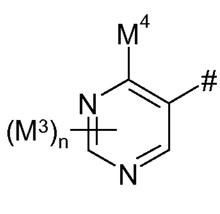

**$Q^{18}$**
n = 1-2

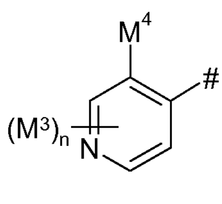

**$Q^{19}$**
n = 1-3

Y für Wasserstoff oder für gegebenenfalls ein- oder mehrfach durch $M^2$ substituiertes ($C_1$-$C_{10}$)-Alkyl,

$(C_2-C_{10})$-Alkenyl, $(C_2-C_{10})$-Alkinyl, $(C_1-C_{10})$-Halogenalkyl, $(C_2-C_{10})$-Halogenalkenyl, $(C_2-C_{10})$-Halogenalkinyl, $(C_1-C_{10})$-Alkoxy, $(C_2-C_{10})$-Alkenyloxy, $(C_3-C_{10})$-Alkinyloxy, $(C_3-C_{14})$-Cycloalkyl-(Ci-Cio)-alkyl oder für eine gegebenenfalls ein- oder mehrfach durch $M^2$ substituierte 3- bis 14-gliedrige cyclische Gruppe steht;

W für Sauerstoff oder Schwefel steht;

$L^2$ für -C($R^{21}$, $R^{22}$)- steht;

$L^3$ für -C($R^{31}$, $R^{32}$)- steht;

$M^1$, $M^2$ und $M^3$ jeweils unabhängig voneinander für Wasserstoff, Halogen, Cyano, Nitro, OH, $(C_1-C_{10})$-Alkyl, $(C_1-C_{10})$-Halogenalkyl, $(C_1-C_{10})$-Alkoxy, $(C_1-C_{10})$-Halogenalkoxy, $(C_1-C_{10})$-Alkylthio, $(C_1-C_{10})$-Halogenalkylthio, $(C_1-C_{10})$-Alkylsulfonyl, $(C_1-C_{10})$-Halogenalkylsulfonyl, $(C_1-C_{10})$-Alkylsulfanyl, $(C_1-C_{10})$-Halogenalkylsulfanyl, oder (3- bis 14-gliedrige cyclische Gruppe)-O-stehen;

$M^4$ für Wasserstoff, Halogen, Cyano, Nitro, OH, $(C_1-C_{10})$-Alkyl, $(C_1-C_{10})$-Halogenalkyl, $(C_1-C_{10})$-Alkoxy, $(C_1-C_{10})$-Halogenalkoxy, $(C_1-C_{10})$-Alkylthio, $(C_1-C_{10})$-Halogenalkylthio, $(C_1-C_{10})$-Alkylsulfonyl, $(C_1-C_{10})$-Halogenalkylsulfonyl, $(C_1-C_{10})$-Alkylsulfanyl, $(C_1-C_{10})$-Halogenalkylsulfanyl, oder (3- bis 14-gliedrige cyclische Gruppe)-O- steht;

k für 1, 2 oder 3 steht;

$R^{21}$, $R^{22}$ für jeweils unabhängig voneinander Wasserstoff, Halogen oder gegebenenfalls ein- oder mehrfach durch $M^2$ substituiertes $(C_1-C_{10})$-Alkyl, $(C_2-C_{10})$-Alkenyl, $(C_2-C_{10})$-Alkinyl, $(C_1-C_{10})$-Halogenalkyl, $(C_2-C_{10})$-Halogenalkenyl, $(C_2-C_{10})$-Halogenalkinyl, $(C_1-C_{10})$-Alkoxy, $(C_1-C_{10})$-Halogenalkoxy, $(C_2-C_{10})$-Alkenyloxy, $(C_3-C_{10})$-Alkinyloxy, $(C_3-C_{14})$-Cycloalkyl-$(C_1-C_{10})$-alkyl oder für eine gegebenenfalls ein- oder mehrfach durch $M^2$ substituierte 3- bis 14-gliedrige cyclische Gruppe steht;

$R^{21}$, $R^{22}$ zusammen für eine jeweils gegebenenfalls ein- oder mehrfach durch $M^2$ substituierte spiroverknüpfte 3- bis 14-gliedrige carbo- oder 3- bis 10-gliedrige heterocyclische Gruppe stehen;

$R^{31}$, $R^{32}$ für jeweils unabhängig voneinander Wasserstoff, Halogen oder gegebenenfalls ein- oder mehrfach durch $M^2$ substituiertes $(C_1-C_{10})$-Alkyl, $(C_2-C_{10})$-Alkenyl, $(C_2-C_{10})$-Alkinyl, $(C_1-C_{10})$-Halogenalkyl, $(C_2-C_{10})$-Halogenalkenyl, $(C_2-C_{10})$-Halogenalkinyl, $(C_3-C_{14})$-Cycloalkyl-$(C_1-C_{10})$-alkyl oder für eine gegebenenfalls ein- oder mehrfach durch $M^2$ substituierte 3- bis 14-gliedrige cyclische Gruppe steht;

$R^{31}$, $R^{32}$ zusammen für eine jeweils gegebenenfalls ein- oder mehrfach durch $M^5$ substituierte spiroverknüpfte 3- bis 14-gliedrige carbo- oder 3- bis 10-gliedrige heterocyclische Gruppe stehen;

$M^5$ jeweils unabhängig voneinander für Halogen, Formyl, Cyano, Nitro, $(C_1-C_{10})$-Alkyl, $(C_1-C_{10})$-Halogenalkyl, $(C_2-C_{10})$-Alkenyl, $(C_2-C_{10})$-Halogenalkenyl, $(C_2-C_{10})$-Alkinyl, $(C_2-C_{10})$-Halogenalkinyl, $(C_1-C_{10})$-Alkoxy, $(C_1-C_{10})$-Halogenalkoxy, $(C_2-C_{10})$-Alkenyloxy, $(C_2-C_{10})$-Halogenalkenoxy, $(C_3-C_{10})$-Alkinyloxy, $(C_3-C_{10})$-Halogenalkinoxy, $(C_1-C_{10})$-Alkylthio, $(C_1-C_{10})$-Halogenalkylthio, $(C_2-C_{10})$-Alkenylthio, $(C_2-C_{10}$-Halogenalkenylthio, $(C_3-C_{10})$-Alkinylthio, $(C_3-C_{10})$-Halogenalkinylthio, $(C_1-C_{10})$-Alkylsulfonyl, $(C_1-C_{10})$-Halogenalkylsulfonyl, $(C_2-C_{10})$-Alkenylsulfonyl, $(C_2-C_{10}$-Halogenalkenylsulfonyl, $(C_3-C_{10})$-Alkinylsulfonyl, $(C_3-C_{10})$-Halogenalkinylsulfonyl, $(C_1-C_{10})$-Alkylsulfanyl, $(C_1-C_{10})$-Halogenalkylsulfanyl, $(C_2-C_{10})$-Alkenylsulfanyl, $(C_2-C_{10})$-Halogenalkenylsulfanyl, $(C_3-C_{10})$-Alkinylsulfanyl, $(C_3-C_{10})$-Halogenalkinylsulfanyl, $(C_1-C_{10})$- Alkylcarbonyl, $(C_1-C_{10})$-Halogenalkylcarbonyl, $(C_2-C_{10})$-Alkenylcarbonyl, $(C_2-C_{10})$-Halogenalkenylcarbonyl, $(C_2-C_{10})$-Alkinylcarbonyl, $(C_2-C_{10})$-Halogenalkinylcarbonyl, $(C_1-C_{10})$-Alkoxycarbonyl, $(C_1-C_{10})$-Halogenalkoxycarbonyl, $(C_2-C_{10})$-Alkenoxycarbonyl, $(C_2-C_{10})$-Halogenalkenoxycarbonyl, $(C_3-C_{10})$-Alkinoxycarbonyl, $(C_3-C_{10})$-Halogenalkinoxycarbonyl, $(C_1-C_{10})$-Alkylcarbonyloxy, $(C_1-C_{10})$-Halogenalkylcarbonyloxy, $(C_2-C_{10})$-Alkenylcarbonyloxy, $(C_2-C_{10})$-Halogenalkenylcarbonyloxy, $(C_2-C_{10})$-Alkinylcarbonyloxy, $(C_2-C_{10})$-Halogenalkinylcarbonyloxy, 3 bis 14-gliedrige cyclische Gruppe steht;

sowie Salze, N-Oxide und tautomere Formen der Verbindungen der Formel (I). In einer weiteren Ausfuehrungsform wurden Verbindungen der Formel (I)

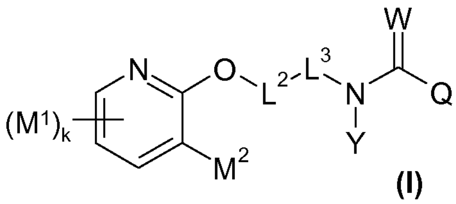

**(I)**

gefunden, in welcher

Q für folgende Strukturelemente steht, wobei n für jedes Q jeweils wie unten angegeben definiert ist:

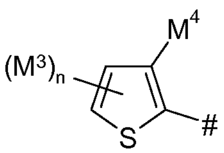

**Q[1]**
n = 1-2

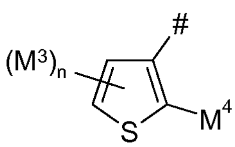

**Q[2]**
n =1-2

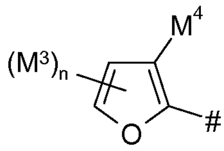

**Q[3]**
n = 1-2

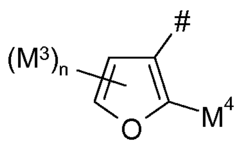

**Q[4]**
n =1-2

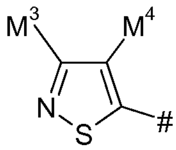

**Q[5]**

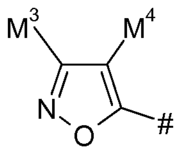

**Q[6]**

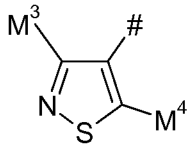

**Q[7]**

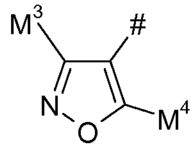

**Q[8]**

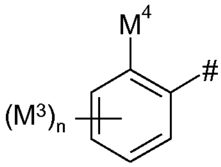

**Q[9]**
n =1-4

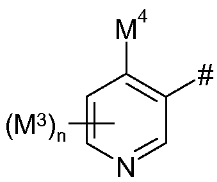

**Q[10]**
n =1-3

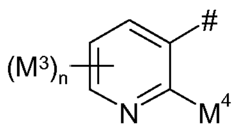

**Q[11]**
n = 1-3

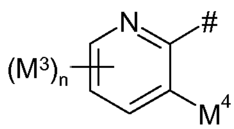

**Q[12]**
n = 1-3

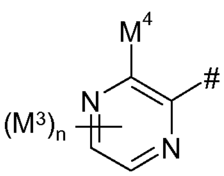

**Q[13]**
n = 1-2

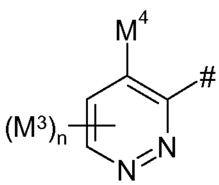

**Q[14]**
n = 1-2

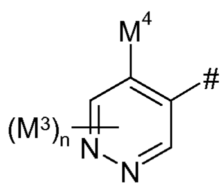

**Q[15]**
n = 1-2

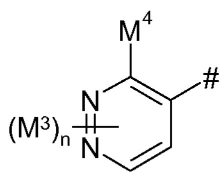

**Q[16]**
n = 1-2

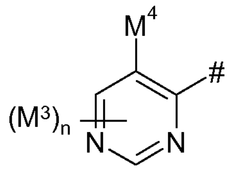 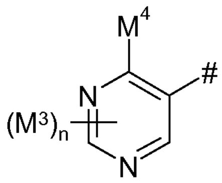 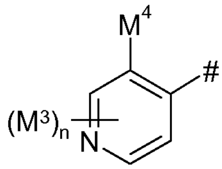

**Q17** n = 1-2 | **Q18** n = 1-2 | **Q19** n = 1-3

Y — für Wasserstoff oder für gegebenenfalls ein- oder mehrfach durch $M^2$ substituiertes ($C_1$-$C_{10}$)-Alkyl, ($C_2$-$C_{10}$)-Alkenyl, ($C_2$-$C_{10}$)-Alkinyl, ($C_1$-$C_{10}$)-Halogenalkyl, ($C_2$-$C_{10}$)-Halogenalkenyl, ($C_2$-$C_{10}$)-Halogenalkinyl, ($C_1$-$C_{10}$)-Alkoxy, ($C_2$-$C_{10}$)-Alkenyloxy, ($C_3$-$C_{10}$)-Alkinyloxy, ($C_3$-$C_{14}$)-Cycloalkyl-(Ci-Cio)-alkyl oder für eine gegebenenfalls ein- oder mehrfach durch $M^2$ substituierte 3- bis 14-gliedrige cyclische Gruppe steht;

W — für Sauerstoff oder Schwefel steht;

$L^2$ — für -C($R^{21}$, $R^{22}$)- steht;

$L^3$ — für -C($R^{31}$, $R^{32}$)- steht;

$M^1$, $M^2$ und $M^3$ — jeweils unabhängig voneinander für Wasserstoff, Halogen, Cyano, Nitro, OH, ($C_1$-$C_{10}$)-Alkyl, ($C_1$-$C_{10}$)-Halogenalkyl, ($C_1$-$C_{10}$)-Alkoxy, ($C_1$-$C_{10}$)-Halogenalkoxy, ($C_1$-$C_{10}$)-Alkylthio, ($C_1$-$C_{10}$)-Halogenalkylthio, ($C_1$-$C_{10}$)-Alkylsulfonyl, ($C_1$-$C_{10}$)-Halogenalkylsulfonyl, ($C_1$-$C_{10}$)-Alkylsulfanyl, ($C_1$-$C_{10}$)-Halogenalkylsulfanyl, oder (3- bis 14-gliedrige cyclische Gruppe)-O-stehen;

$M^4$ — für Wasserstoff, Halogen, Cyano, Nitro, OH, ($C_1$-$C_{10}$)-Alkyl, ($C_1$-$C_{10}$)-Halogenalkyl, ($C_1$-$C_{10}$)-Alkoxy, ($C_1$-$C_{10}$)-Halogenalkoxy, ($C_1$-$C_{10}$)-Alkylthio, ($C_1$-$C_{10}$)-Halogenalkylthio, ($C_1$-$C_{10}$)-Alkylsulfonyl, ($C_1$-$C_{10}$)-Halogenalkylsulfonyl, ($C_1$-$C_{10}$)-Alkylsulfanyl, ($C_1$-$C_{10}$-Halogenalkylsulfanyl, oder (3- bis 14-gliedrige cyclische Gruppe)-O- steht;

k — für 1, 2 oder 3 steht;

$R^{21}$, $R^{22}$ — für jeweils unabhängig voneinander Wasserstoff, Halogen oder gegebenenfalls ein- oder mehrfach durch $M^2$ substituiertes ($C_1$-$C_{10}$)-Alkyl, ($C_2$-$C_{10}$)-Alkenyl, ($C_2$-$C_{10}$)-Alkinyl, ($C_1$-$C_{10}$)-Halogenalkyl, ($C_2$-$C_{10}$)-Halogenalkenyl, ($C_2$-$C_{10}$)-Halogenalkinyl, ($C_1$-$C_{10}$)-Alkoxy, ($C_1$-$C_{10}$)-Halogenalkoxy, ($C_2$-$C_{10}$)-Alkenyloxy, ($C_3$-$C_{10}$)-Alkinyloxy, ($C_3$-$C_{14}$)-Cycloalkyl-($C_1$-$C_{10}$)-alkyl oder für eine gegebenenfalls ein- oder mehrfach durch $M^2$ substituierte 3- bis 14-gliedrige cyclische Gruppe steht;

$R^{21}$, $R^{22}$ — zusammen für eine jeweils gegebenenfalls ein- oder mehrfach durch $M^2$ substituierte spiroverknüpfte 3- bis 14-gliedrige carbo- oder 3- bis 10-gliedrige heterocyclische Gruppe stehen;

$R^{31}$, $R^{32}$ — für jeweils unabhängig voneinander Wasserstoff, Halogen oder gegebenenfalls ein- oder mehrfach durch $M^2$ substituiertes ($C_1$-$C_{10}$)-Alkyl, ($C_2$-$C_{10}$)-Alkenyl, ($C_2$-$C_{10}$)-Alkinyl, ($C_1$-$C_{10}$)-Halogenalkyl, ($C_2$-$C_{10}$)-Halogenalkenyl, ($C_2$-$C_{10}$)-Halogenalkinyl, ($C_3$-$C_{14}$)-Cycloalkyl-($C_1$-$C_{10}$)-alkyl oder für eine gegebenenfalls ein- oder mehrfach durch $M^2$ substituierte 3- bis 14-gliedrige cyclische Gruppe steht;

$R^{31}$, $R^{32}$ — zusammen für eine jeweils gegebenenfalls ein- oder mehrfach durch $M^5$ substituierte spiroverknüpfte 3- bis 14-gliedrige carbo- oder 3- bis 10-gliedrige heterocyclische Gruppe stehen;

$M^5$ — jeweils unabhängig voneinander für Halogen, Formyl, Cyano, Nitro, ($C_1$-$C_{10}$)-Alkyl, ($C_1$-$C_{10}$)-Halogenalkyl, ($C_2$-$C_{10}$)-Alkenyl, ($C_2$-$C_{10}$)-Halogenalkenyl, ($C_2$-$C_{10}$)-Alkinyl, ($C_2$-$C_{10}$)-Halogenalkinyl, ($C_1$-$C_{10}$)-Alkoxy, ($C_1$-$C_{10}$)-Halogenalkoxy, ($C_2$-$C_{10}$)-Alkenyloxy, ($C_2$-$C_{10}$)-Halogenalkenoxy, ($C_3$-$C_{10}$)-Alkinyloxy, ($C_3$-$C_{10}$)-Halogenalkinoxy, ($C_1$-$C_{10}$)-Alkylthio, ($C_1$-$C_{10}$)-Halogenalkylthio, ($C_2$-$C_{10}$)-Alkenylthio, ($C_2$-$C_{10}$)-Halogenalkenylthio, ($C_3$-$C_{10}$)-Alkinylthio, ($C_3$-$C_{10}$)-Halogenalkinylthio,

($C_1$-$C_{10}$)-Alkylsulfonyl, ($C_1$-$C_{10}$)-Halogenalkylsulfonyl, ($C_2$-$C_{10}$)-Alkenylsulfonyl, ($C_2$-$C_{10}$)-Halogenalkenylsulfonyl, ($C_3$-$C_{10}$)-Alkinylsulfonyl, ($C_3$-$C_{10}$)-Halogenalkinylsulfonyl, ($C_1$-$C_{10}$)-Alkylsulfanyl, ($C_1$-$C_{10}$)-Halogenalkylsulfanyl, ($C_2$-$C_{10}$)-Alkenylsulfanyl, ($C_2$-$C_{10}$)-Halogenalkenylsulfanyl, ($C_3$-$C_{10}$)-Alkinylsulfanyl, ($C_3$-$C_{10}$)-Halogenalkinylsulfanyl, ($C_1$-$C_{10}$)- Alkylcarbonyl, ($C_1$-$C_{10}$)-Halogenalkylcarbonyl, ($C_2$-$C_{10}$)-Alkenylcarbonyl, ($C_2$-$C_{10}$)-Halogenalkenylcarbonyl, ($C_2$-$C_{10}$)-Alkinylcarbonyl, ($C_2$-$C_{10}$)-Halogenalkinylcarbonyl, ($C_1$-$C_{10}$)-Alkoxycarbonyl, ($C_1$-$C_{10}$)-Halogenalkoxycarbonyl, ($C_2$-$C_{10}$)-Alkenoxycarbonyl, ($C_2$-$C_{10}$)-Halogenalkenoxycarbonyl, ($C_3$-$C_{10}$)-Alkinoxycarbonyl, ($C_3$-$C_{10}$)-Halogenalkinoxycarbonyl, ($C_1$-$C_{10}$)-Alkylcarbonyloxy, ($C_1$-$C_{10}$)-Halogenalkylcarbonyloxy, ($C_2$-$C_{10}$)-Alkenylcarbonyloxy, ($C_2$-$C_{10}$)-Halogenalkenylcarbonyloxy, ($C_2$-$C_{10}$)-Alkinylcarbonyloxy, ($C_2$-$C_{10}$)-Halogenalkinylcarbonyloxy, 3 bis 14-gliedrige cyclische Gruppe steht;

sowie Salze, N-Oxide und tautomere Formen der Verbindungen der Formel (I).

Die neuen Verbindungen gemäß Formel (I) gemäß den angehängten Ansprüchen weisen endoparasitizide Wirkung in Tieren und Menschen auf und können als Arzneimittel in Tieren oder Menschen, insbesondere gegen Endoparasiten verwendet werden.

Die erfindungsgemäßen Verbindungen gemäß Formel (I) weisen nematizide Wirkung gegenüber phytopathogenen Nematoden auf und können zur Bekämpfung dieser Nematoden in Land- und Forstwirtschaft eingesetzt werden. Die Verbindungen der Formel (I) können gegebenenfalls in Abhängigkeit von der Art der Substituenten als geometrische und/oder als optisch aktive Isomere oder entsprechende Isomerengemische in unterschiedlicher Zusammensetzung vorliegen. Die Erfindung betrifft sowohl die reinen Isomere als auch die Isomerengemische.
Die erfindungsgemäßen Verbindungen können auch als Metallkomplexe vorliegen.

## Definitionen

Ein Nematizid im Pflanzenschutz, wie hier beschrieben, bedeutet die Fähigkeit des Wirkstoffes, Nematoden zu kontrollieren.
"Nematoden kontrollieren" im Sinne der Verwendung der vorliegenden Erfindung bedeutet das Abtöten der Nematoden oder das Verhindern ihrer Entwicklung bzw. Wachstums. Dabei wird die Wirksamkeit der Verbindungen bei einem Vergleich in Bezug auf Mortalitäten, Gallenbildung, Zystenbildung, Nematodendichte pro Bodenvolumen, Nematodendichte pro Wurzel, Anzahl von Nematodeneier pro Bodenvolumen, Mobilität (Beweglichkeit) der Nematoden zwischen einer mit der erfindungsgemäßen Verbindung behandelten Pflanze, Pflanzenteil oder dem behandelten Boden und einer unbehandelten Pflanze, Pflanzenteil oder unbehandelten Boden (100 %) verglichen. Dabei wird vorzugsweise eine Verringerung um 25-50 % im Vergleich mit einer unbehandelten Pflanze, Pflanzenteil oder unbehandelten Boden, besonders bevorzugt eine Verringerung um 40 - 79 % und ganz besonders bevorzugt das vollständige Abtöten oder die vollständige Verhinderung von Entwicklung und Wachstum der Nematoden durch eine Verringerung um 70 bis 100 % erreicht. Die Kontrolle von Nematoden, wie hier beschreiben, beinhaltet ebenso die Kontrolle der Nematoden-Vermehrung (Entwicklung von Zysten und/oder Eier). Wirkstoffe, wie hier beschrieben, können ebenso verwendet werden, um die Organismen gesund zu erhalten und können kurativ, präventiv oder systemisch zur Nematoden-Kontrolle eingesetzt werden.
Dem Fachmann sind Methoden bekannt, wie Mortalitäten, Gallenbildung, Zystenbildung, Nematodendichte pro Bodenvolumen, Nematodendichte pro Wurzel, Anzahl von Nematodeneier pro Bodenvolumen, Mobilität (Beweglichkeit) der Nematoden bestimmt werden.

Der oben beschriebene "Organismus" kann eine Pflanze sein. Die Verwendung eines Wirkstoffes, wie hier beschrieben, kann die Pflanze gesund erhalten und beinhaltet ebenso eine Reduktion der von Nematoden hervorgerufenen Schäden sowie eine Erhöhung der Erntemenge.

Der Begriff Tiere umfasst nicht den Menschen.

Der Begriff "ein- oder mehrfach" bedeutet bevorzugt ein- bis sechsfach, besonders bevorzugt ein- bis vierfach, ganz besonders bevorzugt ein- bis dreifach und insbesonders ganz bevorzugt ein- oder zweifach.

Der Fachmann ist sich bewusst, dass die Ausdrücke "ein", "eine" oder "eines" wie in dieser Anmeldung genutzt je nach Situation "ein/eine/eines (1)", "ein/eine/eines (1) oder mehr" oder "mindestens ein/eine/eines (1)" bedeuten kann.

Für alle bisher beschriebenen Ringsysteme gilt, dass benachbarte Atome nicht -O-O- oder -O-S- sein dürfen.

Strukturen mit einer variablen Anzahl an möglichen Kohlenstoffatomen (C-Atomen) werden zur Vereinfachung in der vorliegenden Anmeldung als $C_1$-$C_{10}$-Strukturen bezeichnet ($C_1$-$C_{10}$). Beispiel: eine Alkylgruppe aus 1 bis 10 C-Atomen entspricht ($C_1$-$C_{10}$)-Alkyl. Ringstrukturen aus C-Atomen und Heteroatomen werden als "3 bis 14-gliedrige" Strukturen bezeichnet.

Steht ein Sammelbegriff für einen Substituenten, z. B. ($C_1$-$C_{10}$)-Alkyl, am Ende eines zusammengesetzten Substituenten wie z.B. ($C_3$-$C_{14}$)-Cycloalkyl-($C_1$-$C_{10}$)-alkyl, so kann der am Ende stehende Bestandteil des zusammengesetzten Substituenten, z.B. das ($C_1$-$C_{10}$)-Alkyl, ein- bzw. mehrfach, gleich oder verschieden und unabhängig voneinander mit dem am Anfang stehenden Substituenten, z. B. ($C_3$-$C_{14}$)-Cycloalkyl, substituiert sein.

Die Definition für Sammelbegriffe solange nicht anders definiert gilt auch für diese Sammelbegriffe in zusammengesetzten Substituenten. Beispiel: Die Definition für ($C_1$-$C_{10}$)-Alkyl gilt auch für ($C_1$-$C_{10}$)-Alkyl als Bestandteil eines zusammengesetzten Substituenten wie z.B. ($C_3$-$C_{14}$)-Cycloalkyl-($C_1$-$C_{10}$)-alkyl.

Dem Fachmann ist klar, dass in dieser Anmeldung genannte Beispiele nicht als beschränkend anzusehen sind sondern lediglich einige Ausführungsformen näher beschreiben.

Bei den in den vorstehenden Formeln angegebenen Definitionen der Symbole wurden Sammelbegriffe verwendet, die allgemein repräsentativ für die folgenden Substituenten stehen:

## Sammelbegriffe

**Halogen,** soweit nicht anders definiert: Elemente der 7. Hauptgruppe, bevorzugt ist Fluor, Chlor, Brom bzw. Iod.

**($C_1$-$C_{10}$)-Alkyl,** soweit nicht an anderer Stelle anders definiert: gesättigte, geradkettige oder verzweigte Kohlenwasserstoffreste bevorzugt mit ($C_1$-$C_6$)-, besonders bevorzugt ($C_1$-$C_4$)- Kohlenstoffatomen. Beispiele: Methyl, Ethyl, iso-Propyl, n-Propyl, 1-Methylethyl, Butyl, tert. Butyl, etc.

**($C_2$-$C_{10}$)-Alkenyl,** soweit nicht an anderer Stelle anders definiert: ungesättigte, geradkettige oder verzweigte Kohlenwasserstoffreste mit einer Doppelbindung. Bevorzugt ist ($C_2$-$C_6$)- bzw. ($C_2$-$C_4$)-Alkenyl. Beispiele: Ethenyl, 1-Propenyl, 3-Butenyl, etc.

**($C_2$-$C_{10}$)-Alkinyl,** soweit nicht an anderer Stelle anders definiert: ungesättigte, geradkettige oder verzweigte Kohlenwasserstoffreste mit einer Dreifachbindung. Bevorzugt ist ($C_2$-$C_6$)- bzw. ($C_2$-$C_4$)-Alkinyl. Beispiele: Ethinyl, 1-Propinyl, etc.

**($C_1$-$C_{10}$)-Alkoxy** (Alkylrest-O-), soweit nicht an anderer Stelle anders definiert: ein Alkylrest, der über ein Sauerstoffatom (-O-) an das Gerüst gebunden ist. Bevorzugt ist ($C_1$-$C_6$)- bzw. ($C_1$-$C_4$)-Alkoxy. Beispiele: Methoxy, Ethoxy, Propoxy, 1-Methylethoxy, etc.

Analog sind **($C_2$-$C_{10}$)-Alkenoxy** und **($C_3$-$C_{10}$)-Alkinoxy,** soweit nicht an anderer Stelle anders definiert Alkenylreste bzw. Alkinylreste, die über -O- an das Gerüst gebunden sind. Bevorzugt ist ($C_2$-$C_6$)- bzw. ($C_2$-$C_4$)-Alkenoxy. Bevorzugt ($C_3$-$C_6$)- bzw. ($C_3$-$C_4$)-Alkinoxy.

**($C_1$-$C_{10}$)-Alkylcarbonyl** (Alkylrest-C(=O)-), soweit nicht an anderer Stelle anders definiert: bevorzugt ist ($C_1$-$C_6$)- bzw. ($C_1$-$C_4$)-Alkylcarbonyl. Die Anzahl der C-Atome bezieht sich dabei auf den Alkylrest in der Alkylcarbonylgruppe.

Analog sind **($C_2$-$C_{10}$)-Alkenylcarbonyl** und **($C_2$-$C_{10}$)-Alkinylcarbonyl,** soweit nicht an anderer Stelle anders definiert: Alkenylreste bzw. Alkinylreste, die über -C(=O)- an das Gerüst gebunden sind. Bevorzugt ist ($C_2$-$C_6$)- bzw. ($C_2$-$C_4$)-Alkenylcarbonyl. Bevorzugt ist ($C_2$-$C_6$)- bzw. ($C_2$-$C_4$)-Alkinylcarbonyl.

**($C_1$-$C_{10}$)-Alkoxycarbonyl** (Alkylrest-O-C(=O)-), soweit nicht an anderer Stelle anders definiert: bevorzugt ist ($C_1$-$C_6$)- bzw. ($C_1$-$C_4$)-Alkoxycarbonyl. Die Anzahl der C-Atome bezieht sich dabei auf den Alkylrest in der Alkoxycarbonylgruppe.

Analog sind **($C_2$-$C_{10}$)-Alkenoxycarbonyl** und **($C_3$-$C_{10}$)-Alkinoxycarbonyl,** soweit nicht an anderer Stelle anders definiert: Alkenylreste bzw. Alkinylreste, die über -O-C(=O)- an das Gerüst gebunden sind. Bevorzugt ist ($C_2$-$C_6$)- bzw. ($C_2$-$C_4$)-Alkenoxycarbonyl. Bevorzugt ist ($C_3$-$C_6$)- bzw. ($C_3$-$C_4$)-Alkinoxycarbonyl.

**($C_1$-$C_{10}$)-Alkylcarbonyloxy** (Alkylrest-C(=O)-O-), soweit nicht an anderer Stelle anders definiert: ein Alkylrest, der über eine Carbonyloxygruppe (-C(=O)-O-) mit dem Sauerstoff an das Gerüst gebunden ist. Bevorzugt ist ($C_1$-$C_6$)- bzw. ($C_1$-$C_4$)-Alkylcarbonyloxy.

Analog sind **($C_2$-$C_{10}$)-Alkenylcarbonyloxy** und **($C_2$-$C_{10}$)-Alkinylcarbonyloxy,** soweit nicht an anderer Stelle anders definiert: Alkenylreste bzw. Alkinylreste, die über (-C(=O)-O-) an das Gerüst gebunden sind. Bevorzugt ist ($C_2$-$C_6$)- bzw. ($C_2$-$C_4$)-Alkenylcarbonyloxy. Bevorzugt ist ($C_2$-$C_6$)- bzw. ($C_2$-$C_4$)-Alkinylcarbonyloxy.

**($C_1$-$C_{10}$)-Alkylthio,** soweit nicht an anderer Stelle anders definiert: Alkylrest, der über -S- an das Gerüst gebunden ist. Bevorzugt ist ($C_1$-$C_6$)- bzw. ($C_1$-$C_4$)-Alkylthio.

Analog sind **($C_2$-$C_{10}$)-Alkenylthio** und **($C_3$-$C_{10}$)-Alkinylthio,** soweit nicht an anderer Stelle anders definiert: Alkenylreste bzw. Alkinylreste, die über -S- an das Gerüst gebunden sind. Bevorzugt ist ($C_2$-$C_6$)- bzw. ($C_2$-$C_4$)-Alkenylthio. Bevorzugt ist ($C_3$-$C_6$)- bzw. ($C_3$-$C_4$)-Alkinylthio.

**($C_1$-$C_{10}$)-Alkylsulfinyl,** soweit nicht an anderer Stelle anders definiert: Alkylrest, der über -S(=O)- an das Gerüst gebunden ist. Bevorzugt ist ($C_1$-$C_6$)- bzw. ($C_1$-$C_4$)-Alkylsulfinyl.

Analog sind **($C_2$-$C_{10}$)-Alkenylsulfinyl** und **($C_3$-$C_{10}$)-Alkinylsulfinyl,** soweit nicht an anderer Stelle anders definiert: Alkenylreste bzw. Alkinylreste, die über -S(=O)- an das Gerüst gebunden sind. Bevorzugt ist ($C_2$-$C_6$)- bzw. ($C_2$-$C_4$)-Alkenylsulfinyl. Bevorzugt ist ($C_3$-$C_6$)- bzw. ($C_3$-$C_4$)-Alkinylsulfinyl.

**($C_1$-$C_{10}$)-Alkylsulfonyl,** soweit nicht an anderer Stelle anders definiert: Alkylrest, der über $-S(=O)_2-$ an das Gerüst gebunden ist. Bevorzugt ist ($C_1$-$C_6$)- bzw. ($C_1$-$C_4$)-Alkylsulfonyl.

Analog sind **($C_2$-$C_{10}$)-Alkenylsulfonyl** und **($C_3$-$C_{10}$)-Alkinylsulfonyl,** soweit nicht an anderer Stelle anders

definiert: Alkenylreste bzw. Alkinylreste, die über -$S(=O)_2$- an das Gerüst gebunden sind. Bevorzugt ist ($C_2$-$C_6$)- bzw. ($C_2$-$C_4$)-Alkenylsulfonyl. Bevorzugt ist ($C_3$-$C_6$)- bzw. ($C_3$-$C_4$)-Alkinylsulfonyl.

($C_1$-$C_{10}$)-Halogenalkyl, ($C_2$-$C_{10}$)-Halogenalkenyl, ($C_2$-$C_{10}$)-Halogenalkinyl, ($C_1$-$C_{10}$)-Halogenalkoxy, ($C_2$-$C_{10}$)-Halogenalkenoxy, ($C_3$-$C_{10}$)-Halogenalkinoxy, ($C_1$-$C_{10}$)-Halogenalkylcarbonyl, ($C_2$-$C_{10}$)-Halogenalkenylcarbonyl, ($C_2$-$C_{10}$)-Halogenalkinylcarbonyl, ($C_1$-$C_{10}$)-Halogenalkoxycarbonyl, ($C_2$-$C_{10}$)-Halogenalkenoxycarbonyl, ($C_3$-$C_{10}$)-Halogenalkinoxycarbonyl, ($C_2$-$C_{10}$)-Halogenalkylcarbonyloxy, ($C_2$-$C_{10}$)-Halogenalkenylcarbonyloxy, ($C_2$-$C_{10}$)-Halogenalkinylcarbonyloxy, ($C_1$-$C_{10}$)-Halogenalkylthio, ($C_2$-$C_{10}$)-Halogenalkenylthio, ($C_3$-$C_{10}$)-Halogenalkinylthio, ($C_1$-$C_{10}$)-Halogenalkylsulfinyl, ($C_2$-$C_{10}$)-Halogenalkenylsulfinyl, ($C_3$-$C_{10}$)-Halogenalkinylsulfinyl, ($C_1$-$C_{10}$)-Halogenalkylsulfonyl, ($C_2$-$C_{10}$)-Halogenalkenylsulfonyl, ($C_3$-$C_{10}$)-Halogenalkinylsulfonyl sind jeweils soweit nicht anders definiert analog zu ($C_1$-$C_{10}$)-Alkyl, ($C_2$-$C_{10}$)-Alkenyl, ($C_2$-$C_{10}$)-Alkinyl, ($C_1$-$C_{10}$)-Alkoxy, ($C_2$-$C_{10}$)-Alkenoxy, ($C_3$-$C_{10}$)-Alkinoxy, ($C_1$-$C_{10}$)- Alkylcarbonyl, ($C_2$-$C_{10}$)-Alkenylcarbonyl, ($C_2$-$C_{10}$)-Alkinylcarbonyl, ($C_1$-$C_{10}$)-Alkoxycarbonyl, ($C_2$-$C_{10}$)-Alkenoxycarbonyl, ($C_3$-$C_{10}$)-Alkinoxycarbonyl, ($C_1$-$C_{10}$)-Alkylcarbonyloxy, ($C_2$-$C_{10}$)-Alkenylcarbonyloxyl, ($C_2$-$C_{10}$)-Alkinylcarbonyloxy, ($C_1$-$C_{10}$)-Alkylthio, ($C_2$-$C_{10}$)-Alkenylthio, ($C_3$-$C_{10}$)-Alkinylthio, ($C_1$-$C_{10}$)-Alkylsulfinyl, ($C_2$-$C_{10}$)-Alkenylsulfinyl, ($C_3$-$C_{10}$)-Alkinylsulfinyl, ($C_1$-$C_{10}$)-Alkylsulfonyl, ($C_3$-$C_{10}$)-Alkenylsulfonyl, ($C_3$-$C_{10}$)-Alkinylsulfonyl definiert, wobei mindestens ein Wasserstoffatom durch ein Halogenatom wie vorstehend genannt ersetzt ist. In einer Ausführungsform sind alle Wasserstoffatome durch Halogen ersetzt. Beispiele für halogenierte Strukturen sind z. B. Chlormethyl, Trichlormethyl, Fluormethyl, Chlordifluormethyl, Dichlorfluormethyl, Trifluormethyl, 2,2-Difluorethyl, Difluormethyl, Difluormethoxy, Trifluormethoxy, Difluormethylthio, Trifluormethylthio.

## Cyclische Gruppen

**3 bis 14-gliedrige cyclische Gruppe,** soweit nicht an anderer Stelle anders definiert: ($C_3$-$C_{14}$)-carbocyclische Gruppe, **3 bis 10-gliedrige** heterocyclische Gruppe, halogenierte ($C_3$-$C_{14}$)-carbocyclische Gruppe, halogenierte 3 bis 10-gliedrige heterocyclische Gruppe.

**($C_3$-$C_{14}$)-carbocyclische Gruppe,** soweit nicht an anderer Stelle anders definiert: ($C_3$-$C_{14}$)-Cycloalkyl, ($C_3$-$C_{14}$)-Cycloalkenyl, ($C_6$-$C_{14}$)-Aryl, halogeniertes ($C_3$-$C_{14}$)-Cycloalkyl, halogeniertes ($C_3$-$C_{14}$)-Cycloalkenyl, halogeniertes ($C_6$-$C_{14}$)-Aryl.

**($C_3$-$C_{14}$)-Cycloalkyl,** soweit nicht an anderer Stelle anders definiert: mono-, bi- oder tricyclische, gesättigte Kohlenwasserstoffgruppen, bevorzugt mit ($C_3$-$C_{14}$)-, ($C_3$-$C_8$)- bzw. ($C_3$-$C_6$)-Ringatomen. Ein Cycloalkyl kann auch eine spirocyclische Gruppe sein. Beispiele: Cyclopropyl, -butyl, -pentyl, -hexyl, - heptyl, Bicyclo[2.2.1]heptyl oder Adamantyl. Bevorzugt steht "Cycloalkyl" für monocyclische Gruppen aus 3, 4, 5, 6 oder 7 Ringatomen.

Analog ist **($C_3$-$C_{14}$)-Cycloalkenyl,** soweit nicht an anderer Stelle anders definiert: eine mono-, bi- oder tricyclische jedoch partiell ungesättigte Kohlenwasserstoffgruppe mit mindestens einer Doppelbindung, bevorzugt mit ($C_3$-$C_8$)- bzw. ($C_3$-$C_6$)-Ringatomen. Beispiele: Cyclopropenyl, Cyclobutenyl, Cyclopentenyl und Cyclohexenyl.

**($C_6$-$C_{14}$)-Aryl,** soweit nicht an anderer Stelle anders definiert: mono- bi- oder tricyclische Ringsystemgruppe wobei mindestens ein Cyclus aromatisch ist, bevorzugt mit ($C_6$-$C_8$)- bzw. ($C_6$)-Ringatomen. Bevorzugt ist Aryl eine aromatische $C_6$-monocyclische Ringsystemgruppe; eine bicyclische ($C_8$-$C_{14}$)-Ringsystemgruppe; oder eine tricyclische ($C_{10}$-$C_{14}$)-Ringsystemgruppe. Beispiele: Phenyl, Naphthyl, Anthryl, Phenanthryl, Tetrahydronaphthyl, Indenyl, Indanyl, Fluorenyl.

Halogenierte ($C_3$-$C_{14}$)-carbocyclische Gruppe, halogeniertes ($C_3$-$C_{14}$)-Cycloalkyl, halogeniertes ($C_3$-$C_{14}$)-Cycloalkenyl, halogeniertes ($C_6$-$C_{14}$)-Aryl sind jeweils soweit nicht anders definiert analog zu ($C_3$-$C_{14}$)-carbocyclische Gruppe, ($C_3$-$C_{14}$)-Cycloalkyl, ($C_3$-$C_{14}$)-Cycloalkenyl, ($C_6$-$C_{14}$)-Aryl definiert, wobei mindestens ein Wasserstoffatom durch ein Halogenatom wie vorstehend genannt ersetzt ist. In einer Ausführungsform sind alle Wasserstoffatome durch Halogen ersetzt. Beispiele für halogenierte Strukturen sind 3-Chlorphenyl, 2-Bromcyclopentyl.

**Heteroatom:** zum Beispiel N, O, S, P, B, Si.

**3 bis 10-gliedrige heterocyclische Gruppe,** soweit nicht an anderer Stelle anders definiert: 3 bis 9-gliedrige Heterocyclylgruppe oder 5 bis 10-gliedrige Heteroarylgruppe, halogenierte 3 bis 9-gliedrige Heterocyclylgruppe oder halogenierte 5 bis 10-gliedrige Heteroarylgruppe.

**3 bis 9-gliedriges Heterocyclyl,** soweit nicht an anderer Stelle anders definiert: 3 bis 9-gliedrige gesättigte oder partiell ungesättigte mono-, bi- oder tricyclische Ringsystemgruppe, aus C-Atomen und mindestens einem Heteroatom, vorzugsweise ausgewählt aus N, O und/oder S. Bevorzugt ist das Ringsystem ein 3 bis 6-gliedriges Ringsystem. Bevorzugt enthält das Ringsystem 1, 2, 3 oder 4 Heteroatome, besonders bevorzugt 1 oder 2 Heteroatome. Ebenfalls bevorzugt ist ein monocyclisches Ringsystem. In einer weiteren bevorzugten Ausführungsform ist ein monocyclisches Ringsystem ein partiell ungesättigtes monocyclisches Ringsystem mit einer Doppelbindung. Ein Heterocyclyl kann ein spirocyclisches System sein. Beispiele: Piperazinyl, Dihydropyridyl, Morpholinyl, etc.. Diese Definition gilt auch für Heterocyclyl als Bestandteil eines zusammengesetzten Substituenten wie z.B. 3 bis 9-gliedriges Heterocyclyl($C_1$-$C_{10}$)-alkyl,

sofern an anderer Stelle nicht definiert.

**5 bis 10-gliedriges Heteroaryl,** soweit nicht an anderer Stelle anders definiert: mono- bi- oder tricyclische 5 bis 10-gliedrige heterocyclische Gruppe aus C-Atomen und mindestens einem Heteroatom, wobei mindestens ein Cyclus aromatisch ist, vorzugsweise ausgewählt aus N, O und/oder S. Bevorzugt ist das Ringsystem ein 5 bis 6-gliedriges Ringsystem. In einer Ausführungsform ist Heteroaryl ein aromatisches monocyclisches Ringsystem aus 5 oder 6 Ringatomen. Bevorzugt ist Heteroaryl als ein aromatisches monocyclisches Ringsystem, enthaltend 1 bis 4 Heteroatome aus der Gruppe O, N, oder S. Weiterhin kann Heteroaryl ein bicyclisches Ringsystem darstellen, das aus 8 bis 14 Ringatomen besteht oder ein tricyclisches Ringsystem, das aus 13 bis 14 Ringatomen besteht. Beispiele: Furyl, Thienyl, Pyrazolyl, Imidazolyl, Triazolyl, Thiazolyl, Indolyl, Benzimidazolyl, Indazolyl, Benzofuranyl, Benzothiophenyl, Benzothiazolyl, Benzoxazolyl, Chinolinyl, Isochinolinyl. Diese Definition gilt auch für Heteroaryl als Bestandteil eines zusammengesetzten Substituenten wie z.B. 5 bis 10-gliedriges Heteroaryl($C_5$-$C_{10}$)-alkyl, sofern an anderer Stelle nicht definiert. Im folgenden sind 5- und 6-gliedrige Heteroarylgruppen näher beschrieben:

**5-gliedriges Heteroaryl,** soweit nicht an anderer Stelle anders definiert: Heteroarylgruppe enthaltend ein bis drei bzw. ein bis vier N-, O- und/oder S-Atom(e) als Ringatome. Beispiele: Furanyl, Thienyl, Oxazolyl, Thiazolyl. In einer Ausführungsform enthält eine 5-gliedrige Heteroarylgruppe neben C-Atomen ein bis vier N-Atome bzw. ein bis drei N-Atome als Ringglieder. Beispiele: Pyrrolyl, Pyrazolyl, Triazolyl, Imidazolyl. In einer weiteren Ausführungsform enthält ein 5-gliedriges Heteroaryl ein bis drei N-Atome oder ein N-Atom und ein O- oder S-Atom. Beispiele: Thiazolyl, Oxazolyl, Oxadiazolyl.

**6-gliedriges Heteroaryl,** soweit nicht an anderer Stelle anders definiert: Heteroarylgruppe enthaltend ein bis drei bzw. ein bis vier N-Atom(e) als Ringatome. In einer Ausführungsform enthält eine 6-gliedrige Heteroarylgruppe ein bis drei N-Atome. Beispiele: Pyridyl, Pyridazinyl, Pyrimidinyl, Pyrazinyl, Triazinyl, Tetrazinyl.

Halogenierte 3 bis 9-gliedrige Heterocyclylgruppe oder halogenierte 5 bis 10-gliedrige Heteroarylgruppe jeweils soweit nicht anders definiert sind analog wie 3 bis 9-gliedrige Heterocyclylgruppe oder 5 bis 10-gliedrige Heteroarylgruppe definiert, wobei mindestens ein Wasserstoffatom durch ein Halogenatom wie vorstehend genannt ersetzt ist. In einer Ausführungsform sind alle Wasserstoffatome durch Halogen ersetzt. Beispiel für halogenierte heterocyclische Strukturen: 3-Chlortetrahydrothiopyran-2-yl, 4-Chlorpyridin-2-yl.

Nicht umfasst sind solche Kombinationen, die den Naturgesetzen widersprechen und die der Fachmann daher aufgrund seines Fachwissens ausgeschlossen hätte. Beispielsweise sind Ringstrukturen mit drei oder mehreren benachbarten O-Atomen ausgeschlossen.

## Ausführungsformen der Verbindungen der Formel (I)

Dem Fachmann ist offenbar, dass alle Ausführungsformen alleine oder in Kombination vorliegen können. Insbesondere koennen die verschiedenen Restedefinitionen fuer die Verbindungen gemaess Formel (I) miteinander kombiniert werden.

Die Verbindungen der Formel (I) können gegebenenfalls in Abhängigkeit der Art der Substituenten als Salze, Tautomere, geometrische und/oder optisch aktive Isomere oder entsprechende Isomerengemische in unterschiedlicher Zusammensetzung vorliegen.

Die erfindungsgemäßen Verbindungen können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Im Folgenden werden Ausführungsformen der Verbindungen der Formel (I) näher beschrieben:

Q steht bevorzugt für folgende Strukturelemente, wobei n für jedes Q jeweils wie unten angegeben definiert ist:

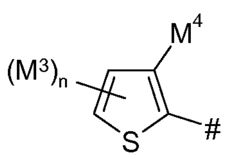

**Q¹**
n = 1-2

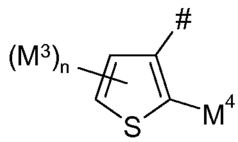

**Q²**
n =1-2

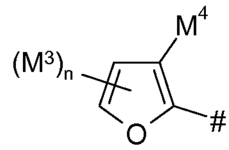

**Q³**
n = 1-2

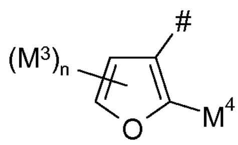

**Q⁴**
n =1-2

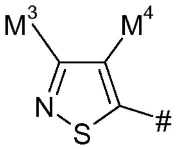

**$Q^5$**

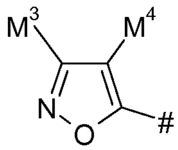

**$Q^6$**

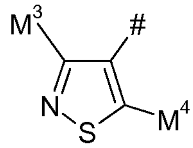

**$Q^7$**

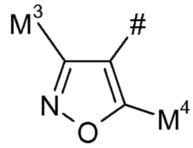

**$Q^8$**

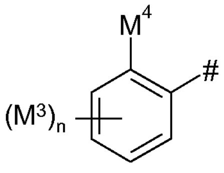

**$Q^9$**
n =1-4

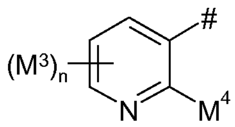

**$Q^{11}$**
n = 1-3

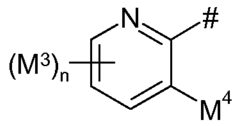

**$Q^{12}$**
n = 1-3

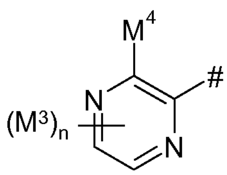

**$Q^{13}$**
n = 1-2

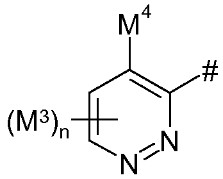

**$Q^{14}$**
n = 1-2

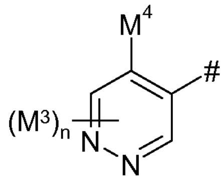

**$Q^{15}$**
n = 1-2

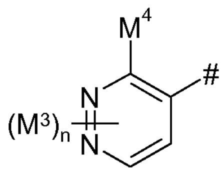

**$Q^{16}$**
n = 1-2

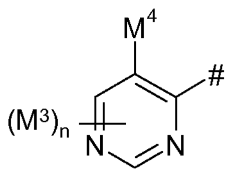

**$Q^{17}$**
n = 1-2

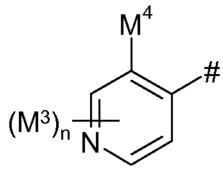

**$Q^{19}$**
n = 1-3

Q steht besonders bevorzugt für folgende Strukturelemente, wobei n für jedes Q jeweils wie unten angegeben definiert ist:

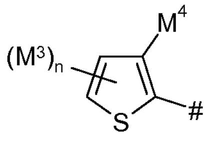

**$Q^1$**
n =1-2

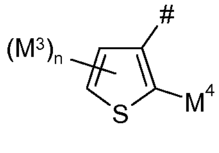

**$Q^2$**
n = 1-2

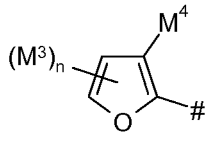

**$Q^3$**
n = 1-2

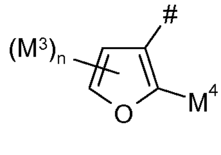

**$Q^4$**
n = 1-2

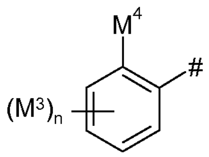

**Q**[9]
n = 1-4

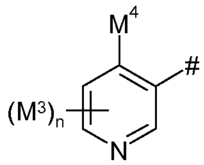

**Q**[10]
n = 1-3

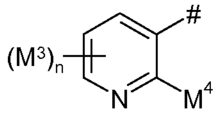

**Q**[11]
n = 1-3

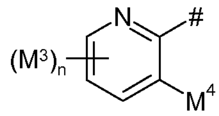

**Q**[12]
n = 1-3

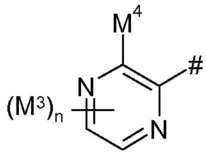

**Q**[13]
n =1-2

Q steht besonders bevorzugt für folgende Strukturelemente, wobei n für jedes Q jeweils wie unten angegeben definiert ist:

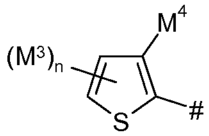

**Q**[1]
n =1-2

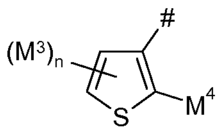

**Q**[2]
n = 1-2

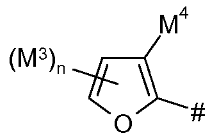

**Q**[3]
n = 1-2

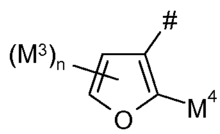

**Q**[4]
n = 1-2

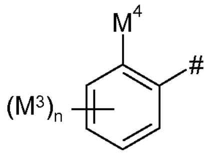

**Q**[9]
n = 1-4

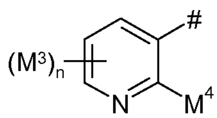

**Q**[11]
n = 1-3

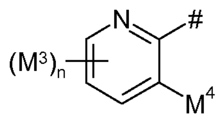

**Q**[12]
n = 1-3

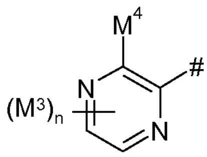

**Q**[13]
n =1-2

Q steht ganz besonders bevorzugt für folgende Strukturelemente, wobei n für jedes Q jeweils wie unten angegeben definiert ist:

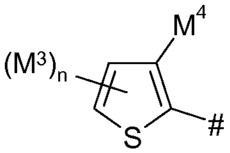

**$Q^1$**
n = 1

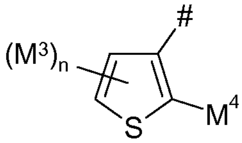

**$Q^2$**
n = 1

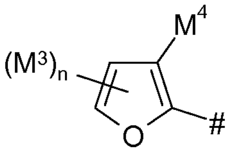

**$Q^3$**
n = 1

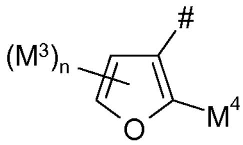

**$Q^4$**
n = 0-1

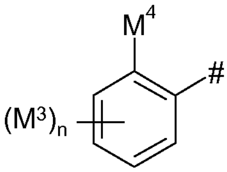

**$Q^9$**
n = 1-3

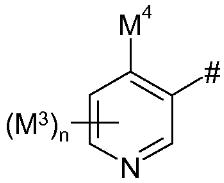

**$Q^{10}$**
n = 1-2

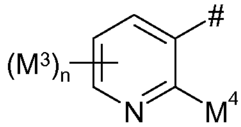

**$Q^{11}$**
n = 1-2

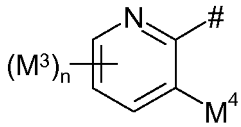

**$Q^{12}$**
n =1-2

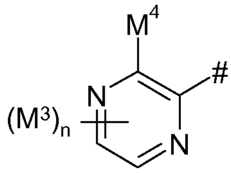

**$Q^{13}$**
n = 1

Q steht ganz besonders bevorzugt für folgende Strukturelemente, wobei n für jedes Q jeweils wie unten angegeben definiert ist:

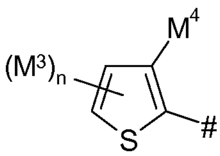

**$Q^1$**
n = 1

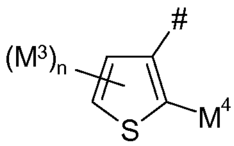

**$Q^2$**
n = 1

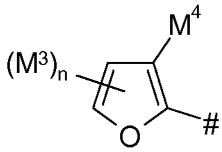

**$Q^3$**
n = 1

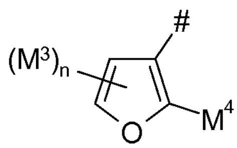

**$Q^4$**
n = 0-1

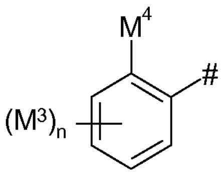

**$Q^9$**
n = 1-3

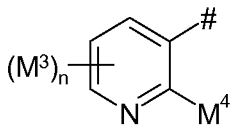

**$Q^{11}$**
n = 1-2

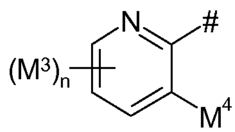

**$Q^{12}$**
n =1-2

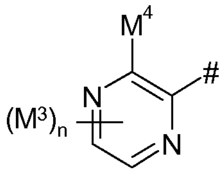

**Q**[13]
n = 1

Q steht ganz besonders bevorzugt für folgende Strukturelemente, wobei n für jedes Q jeweils wie unten angegeben definiert ist:

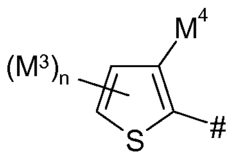

**Q**[1]
n = 1

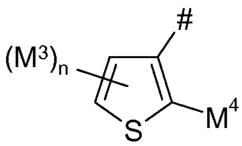

**Q**[2]
n = 1

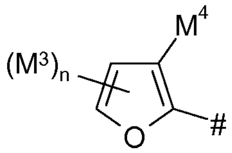

**Q**[3]
n = 1

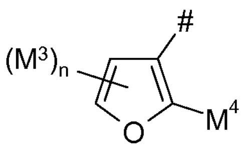

**Q**[4]
n = 0-1

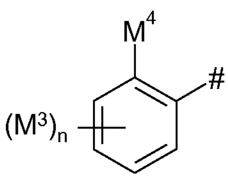

**Q**[9]
n = 1-3

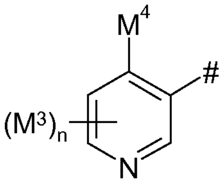

**Q**[10]
n = 1-2

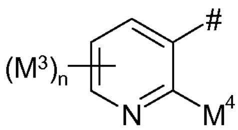

**Q**[11]
n = 1-2

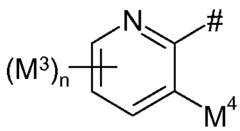

**Q**[12]
n =1-2

Q steht ganz besonders bevorzugt für folgende Strukturelemente, wobei n für jedes Q jeweils wie unten angegeben definiert ist:

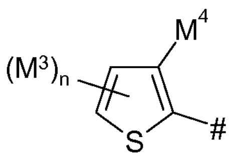

**Q**[1]
n = 1

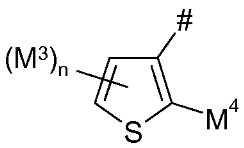

**Q**[2]
n = 1

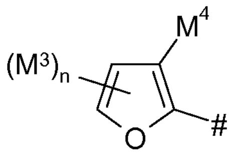

**Q**[3]
n = 1

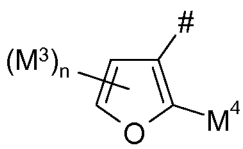

**Q**[4]
n = 0-1

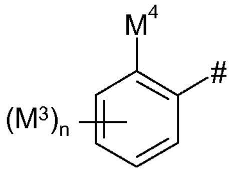

**Q9**
n = 1-3

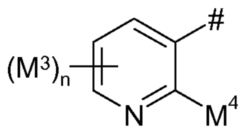

**Q11**
n = 1-2

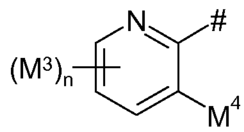

**Q12**
n =1-2

Q steht insbesonders ganz besonders bevorzugt für 2-Thienyl, 3-Fluor-2-thienyl, 3-Chlor-2-thienyl, 3,4-Dichlor-2-thienyl, 2,5-Dichlor-3-thienyl, 3,4,5-Trichlor-2-thienyl, 3-Brom-2-thienyl, 3-Iod-2-thienyl, 3-Cyano-2-thienyl, 3-Methyl-2-thienyl, 3-(Trifluormethyl)-2-thienyl, 3-Methoxy-2-thienyl, 3-Ethoxy-2-thienyl, 3-Thienyl, 2-Fluor-3-thienyl, 2-Chlor-3-thienyl, 2-Brom-3-thienyl, 2-Iod-3-thienyl, 2-Cyano-3-thienyl, 2-Methyl-3-thienyl, 2-(Trifluormethyl)-3-thienyl, 2-Methoxy-3-thienyl, 2-Ethoxy-3-thienyl, 2-Furanyl, 3-Fluor-2-furanyl, 3-Chlor-2-furanyl, 3-Brom-2-furanyl, 3-Iod-2-furanyl, 3-Cyano-2-furanyl, 3-Methyl-2-furanyl, 3-(Trifluormethyl)-2-furanyl, 3-Methoxy-2-furanyl, 3-Ethoxy-2-furanyl, 3-Furanyl, 2-Chlor-3-furanyl, 2-Brom-3-furanyl, 2-Iod-3-furanyl, 2-Cyano-3-furanyl, 2-Methyl-3-furanyl, 2-(Trifluormethyl)-3-furanyl, 2-Methoxy-3-furanyl, 2-Ethoxy-3-furanyl, 2-Methyl-phenyl, 2-Fluor-phenyl, 2,6-Difluor-phenyl, 2-Chorphenyl, 2,6-Dichlor-phenyl, 2-Brom-phenyl, 2-Iod-phenyl, 2-(Difluormethyl)-phenyl, 2-(Trifluormethyl)-phenyl, 2-(Methylsulfanyl)-phenyl, 2-(Methylsulfonyl)-phenyl, 2-(Trifluormethoxy)-phenyl, 2-(Trifluormethylsulfanyl)-phenyl, 2-(Trifluormethylsulfonyl)-phenyl, 2-Nitro-phenyl, 2-Chlor-3-pyridyl, 3-Chlor-2-pyridyl, 2-(Difluormethyl)-3-pyridyl, 2-(Trifluormethyl)-3-pyridyl, 2-(Methylsulfanyl)-3-pyridyl, 2-(Methylsulfonyl)- 3-pyridyl, 2-(Trifluormethoxy)- 3-pyridyl, 2-(Trifluormethylsulfanyl)- 3-pyridyl oder 2-(Trifluormethylsulfonyl)- 3-pyridyl;

Q steht insbesonders ganz besonders bevorzugt für 2-Thienyl, 3-Fluor-2-thienyl, 3-Chlor-2-thienyl, 3,4-Dichlor-2-thienyl, 3,4,5-Trichlor-2-thienyl, 3-Brom-2-thienyl, 3-Iod-2-thienyl, 3-Cyano-2-thienyl, 3-Methyl-2-thienyl, 3-(Trifluormethyl)-2-thienyl, 3-Methoxy-2-thienyl, 3-Ethoxy-2-thienyl, 3-Thienyl, 2-Fluor-3-thienyl, 2-Chlor-3-thienyl, 2-Brom-3-thienyl, 2-Iod-3-thienyl, 2-Cyano-3-thienyl, 2-Methyl-3-thienyl, 2-(Trifluormethyl)-3-thienyl, 2-Methoxy-3-thienyl, 2-Ethoxy-3-thienyl, 2-Furanyl, 3-Fluor-2-furanyl, 3-Chlor-2-furanyl, 3-Brom-2-furanyl, 3-Iod-2-furanyl, 3-Cyano-2-furanyl, 3-Methyl-2-furanyl, 3-(Trifluormethyl)-2-furanyl, 3-Methoxy-2-furanyl, 3-Ethoxy-2-furanyl, 3-Furanyl, 2-Chlor-3-furanyl, 2-Brom-3-furanyl, 2-Iod-3-furanyl, 2-Cyano-3-furanyl, 2-Methyl-3-furanyl, 2-(Trifluormethyl)-3-furanyl, 2-Methoxy-3-furanyl, 2-Ethoxy-3-furanyl, 2-Methyl-phenyl, 2-Fluor-phenyl, 2,6-Difluor-phenyl, 2-Chor-phenyl, 2,6-Dichlor-phenyl, 2-Brom-phenyl, 2-Iod-phenyl, 2-(Difluormethyl)-phenyl, 2-(Trifluormethyl)-phenyl, 2-Nitro-phenyl, 2-Chlor-3-pyridyl, 3-Chlor-2-pyridyl, 2-(Difluormethyl)-3-pyridyl, 2-(Trifluormethyl)-3 -pyridyl;

Y steht bevorzugt für Wasserstoff oder für gegebenenfalls ein- oder mehrfach durch $M^2$ substituiertes $(C_1$-$C_6)$-Alkyl, $(C_2$-$C_6)$-Alkenyl, $(C_2$-$C_6)$-Alkinyl, $(C_1$-$C_6)$-Halogenalkyl, $(C_2$-$C_6)$-Halogenalkenyl, $(C_2$-$C_6)$-Halogenalkinyl, $(C_1$-$C_6)$-Alkoxy, $(C_2$-$C_6)$-Alkenyloxy, $(C_3$-$C_6)$-Alkinyloxy, $(C_3$-$C_{10})$-Cycloalkyl-$(C_1$-$C_6)$-alkyl oder für eine gegebenenfalls ein- oder mehrfach durch $M^2$ substituierte 3- bis 10-gliedrige cyclische Gruppe;

Y steht bevorzugt für Wasserstoff oder für gegebenenfalls ein- oder mehrfach durch $M^2$ substituiertes $(C_1$-$C_4)$-Alkyl, $(C_2$-$C_4)$-Alkenyl, $(C_3$-$C_4)$-Alkinyl, $(C_1$-$C_4)$-Halogenalkyl, $(C_2$-$C_4)$-Halogenalkenyl, $(C_3$-$C_4)$-Halogenalkinyl, $(C_1$-$C_4)$-Alkoxy, $(C_2$-$C_4)$-Alkenyloxy, $(C_3$-$C_4)$-Alkinyloxy, $(C_3$-$C_6)$-Cycloalkyl-$(C_1$-$C_4)$-alkyl oder für eine gegebenenfalls ein- oder mehrfach durch $M^2$ substituierte $C_3$- bis $C_6$-gliedrige carbocyclische Gruppe;

Y steht besonders bevorzugt für Wasserstoff oder für gegebenenfalls ein- oder mehrfach durch $M^2$ substituiertes $(C_1$-$C_4)$-Alkyl, $(C_2$-$C_4)$-Alkenyl, $(C_3$-$C_4)$-Alkinyl, $(C_1$-$C_4)$-Halogenalkyl, $(C_2$-$C_4)$-Halogenalkenyl, $(C_1$-$C_4)$-Alkoxy, $(C_3$-$C_6)$-Cycloalkyl, $(C_3$-$C_6)$-Cycloalkyl-$(C_1$-$C_4)$-alkyl;

Y steht besonders bevorzugt für Wasserstoff, Methyl, Ethyl, n-Propyl, iso-Propyl, n-Butyl, sec-Butyl,

iso-Butyl, tert.-Butyl, Cyanomethyl, 2,2-Difluor-ethyl, 2,2,2-Trifluor-ethyl, Allyl, Butenyl, Propargyl, Butinyl, 3,3-Dichlor-prop-2-enyl, Methoxy, Ethoxy, Cyclopropylmethyl, Cyclopropyl, Cyclobutyl, Cyclopentyl oder Cyclohexyl;

Y steht ganz besonders bevorzugt für Wasserstoff, Methyl, Ethyl, n-Propyl, iso-Propyl, n-Butyl, sec-Butyl, iso-Butyl, tert.-Butyl, Methoxy, Ethoxy, Cyclopropylmethyl, Cyclopropyl, Cyclobutyl;

Y steht ganz besonders bevorzugt für Wasserstoff, Cyclopropyl;

Y steht ganz besonders bevorzugt für Wasserstoff;

W steht bevorzugt für Sauerstoff;

W steht bevorzugt für Schwefel;

$M^1$, $M^2$ und $M^3$ stehen jeweils unabhängig voneinanderbevorzugt für Wasserstoff, Halogen, Cyano, Nitro, OH, $(C_1-C_6)$-Alkyl, $(C_1-C_6)$-Halogenalkyl, $(C_1-C_6)$-Alkoxy, $(C_1-C_6)$-Halogenalkoxy, $(C_1-C_6)$-Alkylthio, $(C_1-C_6)$-Halogenalkylthio, $(C_1-C_6)$-Alkylsulfonyl, $(C_1-C_6)$-Halogenalkylsulfonyl, $(C_1-C_6)$-Alkylsulfanyl, $(C_1-C_6)$-Halogenalkylsulfanyl, $(C_3-C_{14})$-Cycloalkyl-O-, $(C_3-C_{14})$-Cycloalkenyl-O-, $(C_6-C_{14})$-Aryl-O-, halogeniertes $(C_3-C_{14})$-Cycloalkyl-O-, halogeniertes $(C_3-C_{14})$-Cycloalkenyl-O-, halogeniertes $(C_6-C_{14})$-Aryl-O- ;

$M^1$, $M^2$ und $M^3$ stehen jeweils unabhängig voneinanderbevorzugt für Wasserstoff, Halogen, Cyano, Nitro, OH, $(C_1-C_6)$-Alkyl, $(C_1-C_6)$-Halogenalkyl, $(C_1-C_6)$-Alkoxy, $(C_1-C_6)$-Halogenalkoxy, $(C_1-C_6)$-Alkylthio, $(C_1-C_6)$-Halogenalkylthio, $(C_1-C_6)$-Alkylsulfonyl, $(C_1-C_6)$-Halogenalkylsulfonyl, $(C_1-C_6)$-Alkylsulfanyl, $(C_1-C_6)$-Halogenalkylsulfanyl, $(C_3-C_{14})$-Cycloalkyl-O-, $(C_3-C_{14})$-Cycloalkenyl-O-, $(C_6-C_{14})$-Aryl-O-, halogeniertes $(C_3-C_{14})$-Cycloalkyl-O-, halogeniertes $(C_3-C_{14})$-Cycloalkenyl-O-, halogeniertes $(C_6-C_{14})$-Aryl-O-, wobei, falls Q $Q^{11}$ entspricht, $M^3$ nicht $(C_1-C_4)$-Halogenalkyl in Position 4 am Pyridyl ist;

$M^1$, $M^2$ und $M^3$ stehen jeweils unabhängig voneinander bevorzugt für Wasserstoff, Halogen, Cyano, Nitro, OH, $(C_1-C_4)$-Alkyl, $(C_1-C_4)$-Halogenalkyl, $(C_1-C_4)$-Alkoxy, $(C_1-C_4)$-Halogenalkoxy, $(C_1-C_4)$-Alkylthio, $(C_1-C_4)$-Halogenalkylthio, $(C_1-C_4)$-Alkylsulfonyl, $(C_1-C_4)$-Halogenalkylsulfonyl, $(C_1-C_4)$-Alkylsulfanyl, $(C_1-C_6)$-Halogenalkylsulfanyl, $(C_3-C_{14})$-Cycloalkyl-O-, $(C_3-C_{14})$-Cycloalkenyl-O-, $(C_6-C_{14})$-Aryl-O-, halogeniertes $(C_3-C_{14})$-Cycloalkyl-O-, halogeniertes $(C_3-C_{14})$-Cycloalkenyl-O-, halogeniertes $(C_6-C_{14})$-Aryl-O- ;

$M^1$, $M^2$ und $M^3$ stehen jeweils unabhängig voneinander bevorzugt für Wasserstoff, Halogen, Cyano, Nitro, OH, $(C_1-C_4)$-Alkyl, $(C_1-C_4)$-Halogenalkyl, $(C_1-C_4)$-Alkoxy, $(C_1-C_4)$-Halogenalkoxy, $(C_1-C_4)$-Alkylthio, $(C_1-C_4)$-Halogenalkylthio, $(C_1-C_4)$-Alkylsulfonyl, $(C_1-C_4)$-Halogenalkylsulfonyl, $(C_1-C_4)$-Alkylsulfanyl, $(C_1-C_6)$-Halogenalkylsulfanyl, $(C_3-C_{14})$-Cycloalkyl-O-, $(C_3-C_{14})$-Cycloalkenyl-O-, $(C_6-C_{14})$-Aryl-O-, halogeniertes $(C_3-C_{14})$-Cycloalkyl-O-, halogeniertes $(C_3-C_{14})$-Cycloalkenyl-O-, halogeniertes $(C_6-C_{14})$-Aryl-O-, wobei, falls Q $Q^{11}$ entspricht, $M^3$ nicht $(C_1-C_4)$-Halogenalkyl in Position 4 am Pyridyl ist;

$M^1$, $M^2$ und $M^3$ stehen jeweils unabhängig voneinander ganz besonders bevorzugt für Wasserstoff, Halogen, Cyano, Nitro, OH, $(C_1-C_4)$-Alkyl, $(C_1-C_4)$-Halogenalkyl, $(C_1-C_4)$-Alkoxy, $(C_1-C_4)$-Halogenalkoxy, $(C_1-C_4)$-Alkylthio, $(C_1-C_4)$-Halogenalkylthio, $(C_1-C_4)$-Alkylsulfonyl, $(C_1-C_4)$-Halogenalkylsulfonyl, $(C_1-C_4)$-Alkylsulfanyl, $(C_1-C_4)$-Halogenalkylsulfanyl,$(C_6-C_{14})$-Aryl-O-, halogeniertes $(C_6-C_{14})$-Aryl)-O-;

$M^1$, $M^2$ und $M^3$ stehen jeweils unabhängig voneinander ganz besonders bevorzugt für Wasserstoff, Halogen, Cyano, Nitro, OH, $(C_1-C_4)$-Alkyl, $(C_1-C_4)$-Halogenalkyl, $(C_1-C_4)$-Alkoxy, $(C_1-C_4)$-Halogenalkoxy, $(C_1-C_4)$-Alkylthio, $(C_1-C_4)$-Halogenalkylthio, $(C_1-C_4)$-Alkylsulfonyl, $(C_1-C_4)$-Halogenalkylsulfonyl, $(C_1-C_4)$-Alkylsulfanyl, $(C_1-C_4)$-Halogenalkylsulfanyl,$(C_6-C_{14})$-Aryl-O-, halogeniertes $(C_6-C_{14})$-Aryl)-O-, wobei, falls Q $Q^{11}$ entspricht, $M^3$ nicht $(C_1-C_4)$-Halogenalkyl in Position 4 am Pyridyl ist;

$M^1$, $M^2$ und $M^3$ stehen jeweils unabhängig voneinander ganz besonders bevorzugt für Wasserstoff, Halogen, Cyano, Nitro, OH, $(C_1-C_2)$-Alkyl, $(C_1-C_2)$-Halogenalkyl, $(C_1-C_2)$-Alkoxy, $(C_1-C_2)$-Halogenalkoxy, $(C_1-C_2)$-Al-

kylthio, $(C_1-C_2)$-Halogenalkylthio, $(C_1-C_2)$-Alkylsulfonyl, $(C_1-C_2)$-Halogenalkylsulfonyl, $(C_1-C_2)$-Alkylsulfanyl, $(C_1-C_2)$-Halogenalkylsulfanyl,$(C_6)$-Aryl-O-, halogeniertes $(C_6)$-Aryl)-O-;

$M^1$, $M^2$ und $M^3$ stehen jeweils unabhängig voneinander ganz besonders bevorzugt für Wasserstoff, Halogen, Cyano, Nitro, OH, $(C_1-C_2)$-Alkyl, $(C_1-C_2)$-Halogenalkyl, $(C_1-C_2)$-Alkoxy, $(C_1-C_2)$-Halogenalkoxy, $(C_1-C_2)$-Alkylthio, $(C_1-C_2)$-Halogenalkylthio, $(C_1-C_2)$-Alkylsulfonyl, $(C_1-C_2)$-Halogenalkylsulfonyl, $(C_1-C_2)$-Alkylsulfanyl, $(C_1-C_2)$-Halogenalkylsulfanyl,$(C_6)$-Aryl-O-, halogeniertes $(C_6)$-Aryl)-O-, wobei, falls Q $Q^{11}$ entspricht, $M^3$ nicht $(C_1-C_2)$-Halogenalkyl in Position 4 am Pyridyl ist;

$M^1$, $M^2$ und $M^3$ stehen jeweils unabhängig voneinander ganz besonders bevorzugt für Wasserstoff, Fluor, Brom, Chlor, Iod, Cyano, Nitro, OH, Methyl, Ethyl, n-Propyl, iso-Propyl, n-Butyl, iso-Butyl, sec.-Butyl, tert.-Butyl, Trifluormethyl, Difluormethyl, Fluormethyl, Trichlormethyl, Dichlormethyl, Chlormethyl, Methoxy, Ethoxy, iso-Propoxy, Trifluormethoxy, Difluormethoxy, Methylthio, Trifluormethylthio, Difluormethylthio, 2,2,2-Trifluorethylthio, Methylsulfonyl, Ethylsulfonyl, Trifluormethylsulfonyl, 2,2,2-Trifluorethylsulfonyl, Methylsulfanyl, Ethylsulfanyl, Trifluormethylsulfanyl, 2,2,2-Trifluorethylsulfanyl oder Phenoxy;

$M^1$, $M^2$ und $M^3$ stehen jeweils unabhängig voneinander ganz besonders bevorzugt für Wasserstoff, Fluor, Brom, Chlor, Iod, Cyano, Nitro, OH, Methyl, Ethyl, n-Propyl, iso-Propyl, n-Butyl, iso-Butyl, sec.-Butyl, tert.-Butyl, Trifluormethyl, Difluormethyl, Fluormethyl, Trichlormethyl, Dichlormethyl, Chlormethyl, Methoxy, Ethoxy, iso-Propoxy, Trifluormethoxy, Difluormethoxy, Methylthio, Trifluormethylthio, Difluormethylthio, 2,2,2-Trifluorethylthio, Methylsulfonyl, Ethylsulfonyl, Trifluormethylsulfonyl, 2,2,2-Trifluorethylsulfonyl, Methylsulfanyl, Ethylsulfanyl, Trifluormethylsulfanyl, 2,2,2-Trifluorethylsulfanyl oder Phenoxy, wobei, falls Q $Q^{11}$ entspricht, $M^3$ nicht Trifluormethyl in Position 4 am Pyridyl ist;

$M^1$, $M^2$ und $M^3$ stehen jeweils unabhängig voneinander ganz besonders bevorzugt für Wasserstoff, Fluor, Brom, Chlor, Iod, Cyano, Nitro, OH, Methyl, Ethyl, iso-Propyl, tert.-Butyl, Trifluormethyl, Difluormethyl, Methoxy, Ethoxy, iso-Propoxy, Trifluormethoxy, Difluormethoxy, Methylthio, Trifluormethylthio, Difluormethylthio, 2,2,2-Trifluorethylthio, Methylsulfonyl, Ethylsulfonyl, Trifluormethylsulfonyl, 2,2,2-Trifluorethylsulfonyl, Methylsulfanyl, Ethylsulfanyl, Trifluormethylsulfanyl, 2,2,2-Trifluorethylsulfanyl oder Phenoxy;

$M^1$, $M^2$ und $M^3$ stehen jeweils unabhängig voneinander ganz besonders bevorzugt für Wasserstoff, Fluor, Brom, Chlor, Iod, Cyano, Nitro, OH, Methyl, Ethyl, iso-Propyl, tert.-Butyl, Trifluormethyl, Difluormethyl, Methoxy, Ethoxy, iso-Propoxy, Trifluormethoxy, Difluormethoxy, Methylthio, Trifluormethylthio, Difluormethylthio, 2,2,2-Trifluorethylthio, Methylsulfonyl, Ethylsulfonyl, Trifluormethylsulfonyl, 2,2,2-Trifluorethylsulfonyl, Methylsulfanyl, Ethylsulfanyl, Trifluormethylsulfanyl, 2,2,2-Trifluorethylsulfanyl oder Phenoxy, wobei, falls Q $Q^{11}$ entspricht, $M^3$ nicht Trifluormethyl in Position 4 am Pyridyl ist;

$M^1$, $M^2$ und $M^3$ stehen jeweils unabhängig voneinander steht insbesonders ganz besonders bevorzugt für Wasserstoff, Fluor, Brom, Chlor, Iod, Cyano, Nitro, Methyl, Trifluormethyl, Difluormethyl, Methoxy, Ethoxy, iso-Propoxy oder Phenoxy;

$M^1$, $M^2$ und $M^3$ stehen jeweils unabhängig voneinander steht insbesonders ganz besonders bevorzugt für Wasserstoff, Fluor, Brom, Chlor, Iod, Cyano, Nitro, Methyl, Trifluormethyl, Difluormethyl, Methoxy, Ethoxy, iso-Propoxy oder Phenoxy, wobei, falls Q $Q^{11}$ entspricht, $M^3$ nicht Trifluormethyl in Position 4 am Pyridyl ist;

$M^1$, $M^2$ und $M^3$ stehen jeweils unabhängig voneinander steht insbesonders ganz besonders bevorzugt für Wasserstoff, Fluor, Brom, Chlor, Iod, Cyano, Nitro, Trifluormethyl, Difluormethyl;

$M^1$, $M^2$ und $M^3$ stehen jeweils unabhängig voneinander steht insbesonders ganz besonders bevorzugt für Wasserstoff, Fluor, Brom, Chlor, Iod, Cyano, Nitro, Trifluormethyl, Difluormethyl, wobei, falls Q $Q^{11}$ entspricht, $M^3$ nicht Trifluormethyl in Position 4 am Pyridyl ist;

$M^4$ steht bevorzugt für Wasserstoff, Halogen, Cyano, Nitro, OH, $(C_1-C_6)$-Alkyl, $(C_1-C_6)$-Halogenalkyl, $(C_1-C_6)$-Alkoxy, $(C_1-C_6)$-Halogenalkoxy, $(C_1-C_6)$-Alkylthio, $(C_1-C_6)$-Halogenalkylthio, $(C_1-C_6)$-Alkylsulfonyl, $(C_1-C_6)$-Halogenalkylsulfonyl, $(C_1-C_6)$-Alkylsulfanyl, $(C_1-C_6)$-Halogenalkylsulfanyl,

($C_3$-$C_{14}$)-Cycloalkyl-O-, ($C_3$-$C_{14}$)-Cycloalkenyl-O-, ($C_6$-$C_{14}$)-Aryl-O-, halogeniertes ($C_3$-$C_{14}$)-Cycloalkyl-O-, halogeniertes ($C_3$-$C_{14}$)-Cycloalkenyl-O-, halogeniertes ($C_6$-$C_{14}$)-Aryl-O- ;

$M^4$ steht bevorzugt für Wasserstoff, Halogen, Cyano, Nitro, OH, ($C_1$-$C_6$)-Alkyl, ($C_1$-$C_6$)-Halogenalkyl, ($C_1$-$C_6$)-Alkoxy, ($C_1$-$C_6$)-Halogenalkoxy, ($C_1$-$C_6$)-Alkylthio, ($C_1$-$C_6$)-Halogenalkylthio, ($C_1$-$C_6$)-Alkylsulfonyl, ($C_1$-$C_6$)-Halogenalkylsulfonyl, ($C_1$-$C_6$)-Alkylsulfanyl, ($C_1$-$C_6$)-Halogenalkylsulfanyl, ($C_3$-$C_{14}$)-Cycloalkyl-O-, ($C_3$-$C_{14}$)-Cycloalkenyl-O-, ($C_6$-$C_{14}$)-Aryl-O-, halogeniertes ($C_3$-$C_{14}$)-Cycloalkyl-O-, halogeniertes ($C_3$-$C_{14}$)-Cycloalkenyl-O-, halogeniertes ($C_6$-$C_{14}$)-Aryl-O-, wobei, falls Q $Q^{10}$ entspricht, $M^4$ nicht ($C_1$-$C_4$)-Halogenalkyl ist;

$M^4$ steht bevorzugt für Wasserstoff, Halogen, Cyano, Nitro, OH, ($C_1$-$C_4$)-Alkyl, ($C_1$-$C_4$)-Halogenalkyl, ($C_1$-$C_4$)-Alkoxy, ($C_1$-$C_4$)-Halogenalkoxy, ($C_1$-$C_4$)-Alkylthio, ($C_1$-$C_4$)-Halogenalkylthio, ($C_1$-$C_4$)-Alkylsulfonyl, ($C_1$-$C_4$)-Halogenalkylsulfonyl, ($C_1$-$C_4$)-Alkylsulfanyl, ($C_1$-$C_6$)-Halogenalkylsulfanyl, ($C_3$-$C_{14}$)-Cycloalkyl-O-, ($C_3$-$C_{14}$)-Cycloalkenyl-O-, ($C_6$-$C_{14}$)-Aryl-O-, halogeniertes ($C_3$-$C_{14}$)-Cycloalkyl-O-, halogeniertes ($C_3$-$C_{14}$)-Cycloalkenyl-O-, halogeniertes ($C_6$-$C_{14}$)-Aryl-O- ;

$M^4$ steht bevorzugt für Wasserstoff, Halogen, Cyano, Nitro, OH, ($C_1$-$C_4$)-Alkyl, ($C_1$-$C_4$)-Halogenalkyl, ($C_1$-$C_4$)-Alkoxy, ($C_1$-$C_4$)-Halogenalkoxy, ($C_1$-$C_4$)-Alkylthio, ($C_1$-$C_4$)-Halogenalkylthio, ($C_1$-$C_4$)-Alkylsulfonyl, ($C_1$-$C_4$)-Halogenalkylsulfonyl, ($C_1$-$C_4$)-Alkylsulfanyl, ($C_1$-$C_6$)-Halogenalkylsulfanyl, ($C_3$-$C_{14}$)-Cycloalkyl-O-, ($C_3$-$C_{14}$)-Cycloalkenyl-O-, ($C_6$-$C_{14}$)-Aryl-O-, halogeniertes ($C_3$-$C_{14}$)-Cycloalkyl-O-, halogeniertes ($C_3$-$C_{14}$)-Cycloalkenyl-O-, halogeniertes ($C_6$-$C_{14}$)-Aryl-O-, wobei, falls Q $Q^{10}$ entspricht, $M^4$ nicht ($C_1$-$C_4$)-Halogenalkyl ist;

$M^4$ steht ganz besonders bevorzugt für Wasserstoff, Halogen, Cyano, Nitro, OH, ($C_1$-$C_4$)-Alkyl, ($C_1$-$C_4$)-Halogenalkyl, ($C_1$-$C_4$)-Alkoxy, ($C_1$-$C_4$)-Halogenalkoxy, ($C_1$-$C_4$)-Alkylthio, ($C_1$-$C_4$)-Halogenalkylthio, ($C_1$-$C_4$)-Alkylsulfonyl, ($C_1$-$C_4$)-Halogenalkylsulfonyl, ($C_1$-$C_4$)-Alkylsulfanyl, ($C_1$-$C_4$)-Halogenalkylsulfanyl,($C_6$-$C_{14}$)-Aryl-O-, halogeniertes ($C_6$-$C_{14}$)-Aryl)-O-;

$M^4$ steht ganz besonders bevorzugt für Wasserstoff, Halogen, Cyano, Nitro, OH, ($C_1$-$C_4$)-Alkyl, ($C_1$-$C_4$)-Halogenalkyl, ($C_1$-$C_4$)-Alkoxy, ($C_1$-$C_4$)-Halogenalkoxy, ($C_1$-$C_4$)-Alkylthio, ($C_1$-$C_4$)-Halogenalkylthio, ($C_1$-$C_4$)-Alkylsulfonyl, ($C_1$-$C_4$)-Halogenalkylsulfonyl, ($C_1$-$C_4$)-Alkylsulfanyl, ($C_1$-$C_4$)-Halogenalkylsulfanyl,($C_6$-$C_{14}$)-Aryl-O-, halogeniertes ($C_6$-$C_{14}$)-Aryl)-O-, wobei, falls Q $Q^{10}$ entspricht, $M^4$ nicht ($C_1$-$C_2$)-Halogenalkyl ist;

$M^4$ steht ganz besonders bevorzugt für Wasserstoff, Halogen, Cyano, Nitro, OH, ($C_1$-$C_2$)-Alkyl, ($C_1$-$C_2$)-Halogenalkyl, ($C_1$-$C_2$)-Alkoxy, ($C_1$-$C_2$)-Halogenalkoxy, ($C_1$-$C_2$)-Alkylthio, ($C_1$-$C_2$)-Halogenalkylthio, ($C_1$-$C_2$)-Alkylsulfonyl, ($C_1$-$C_2$)-Halogenalkylsulfonyl, ($C_1$-$C_2$)-Alkylsulfanyl, ($C_1$-$C_2$)-Halogenalkylsulfanyl,($C_6$)-Aryl-O-, halogeniertes ($C_6$)-Aryl)-O-;

$M^4$ steht ganz besonders bevorzugt für Wasserstoff, Halogen, Cyano, Nitro, OH, ($C_1$-$C_2$)-Alkyl, ($C_1$-$C_2$)-Halogenalkyl, ($C_1$-$C_2$)-Alkoxy, ($C_1$-$C_2$)-Halogenalkoxy, ($C_1$-$C_2$)-Alkylthio, ($C_1$-$C_2$)-Halogenalkylthio, ($C_1$-$C_2$)-Alkylsulfonyl, ($C_1$-$C_2$)-Halogenalkylsulfonyl, ($C_1$-$C_2$)-Alkylsulfanyl, ($C_1$-$C_2$)-Halogenalkylsulfanyl,($C_6$)-Aryl-O-, halogeniertes ($C_6$)-Aryl)-O-, wobei, falls Q $Q^{10}$ entspricht, $M^4$ nicht ($C_1$-$C_2$)-Halogenalkyl ist;

$M^4$ steht ganz besonders bevorzugt für Fluor, Brom, Chlor, Iod, Cyano, Nitro, OH, Methyl, Ethyl, n-Propyl, iso-Propyl, n-Butyl, iso-Butyl, sec.-Butyl, tert.-Butyl, Trifluormethyl, Difluormethyl, Fluormethyl, Trichlormethyl, Dichlormethyl, Chlormethyl, Methoxy, Ethoxy, iso-Propoxy, Trifluormethoxy, Difluormethoxy, Methylthio, Trifluormethylthio, Difluormethylthio, 2,2,2-Trifluorethylthio, Methylsulfonyl, Ethylsulfonyl, Trifluormethylsulfonyl, 2,2,2-Trifluorethylsulfonyl, Methylsulfanyl, Ethylsulfanyl, Trifluormethylsulfanyl, 2,2,2-Trifluorethylsulfanyl oder Phenoxy;

$M^4$ steht ganz besonders bevorzugt für Wasserstoff, Fluor, Brom, Chlor, Iod, Cyano, Nitro, OH, Methyl, Ethyl, n-Propyl, iso-Propyl, n-Butyl, iso-Butyl, sec.-Butyl, tert.-Butyl, Trifluormethyl, Difluormethyl, Fluormethyl, Trichlormethyl, Dichlormethyl, Chlormethyl, Methoxy, Ethoxy, iso-Propoxy, Trifluormethoxy, Difluormethoxy, Methylthio, Trifluormethylthio, Difluormethylthio, 2,2,2-Trifluorethylthio, Methylsulfonyl, Ethylsulfonyl, Trifluormethylsulfonyl, 2,2,2-Trifluorethylsulfonyl, Methylsulfanyl, Ethylsulfanyl, Trifluormethylsulfanyl, 2,2,2-Trifluorethylsulfanyl oder Phenoxy, wobei, falls Q $Q^{10}$ entspricht, $M^4$ nicht

Trifluoromethyl ist;

$M^4$ steht ganz besonders bevorzugt für Wasserstoff, Fluor, Brom, Chlor, Iod, Cyano, Nitro, OH, Methyl, Ethyl, iso-Propyl, tert.-Butyl, Trifluormethyl, Difluormethyl, Methoxy, Ethoxy, iso-Propoxy, Trifluormethoxy, Difluormethoxy, Methylthio, Trifluormethylthio, Difluormethylthio, 2,2,2-Trifluorethylthio, Methylsulfonyl, Ethylsulfonyl, Trifluormethylsulfonyl, 2,2,2-Trifluorethylsulfonyl, Methylsulfanyl, Ethylsulfanyl, Trifluormethylsulfanyl, 2,2,2-Trifluorethylsulfanyl oder Phenoxy;

$M^4$ steht ganz besonders bevorzugt für Wasserstoff, Fluor, Brom, Chlor, Iod, Cyano, Nitro, OH, Methyl, Ethyl, iso-Propyl, tert.-Butyl, Trifluormethyl, Difluormethyl, Methoxy, Ethoxy, iso-Propoxy, Trifluormethoxy, Difluormethoxy, Methylthio, Trifluormethylthio, Difluormethylthio, 2,2,2-Trifluorethylthio, Methylsulfonyl, Ethylsulfonyl, Trifluormethylsulfonyl, 2,2,2-Trifluorethylsulfonyl, Methylsulfanyl, Ethylsulfanyl, Trifluormethylsulfanyl, 2,2,2-Trifluorethylsulfanyl oder Phenoxy, wobei, falls Q $Q^{10}$ entspricht, $M^4$ nicht Trifluoromethyl ist;

$M^4$ steht insbesonders ganz besonders bevorzugt für Fluor, Brom, Chlor, Iod, Cyano, Nitro, Methyl, Trifluormethyl, Difluormethyl, Methoxy, Trifluormethylsulfanyl, Trifluormethoxy, Difluormethoxy, Ethoxy, iso-Propoxy oder Phenoxy ;

$M^4$ steht insbesonders ganz besonders bevorzugt für Wasserstoff, Fluor, Brom, Chlor, Iod, Cyano, Nitro, Methyl, Trifluormethyl, Difluormethyl, Methoxy, Trifluormethylsulfanyl, Trifluormethoxy, Difluormethoxy, Ethoxy, iso-Propoxy oder Phenoxy, wobei, falls Q $Q^{10}$ entspricht, $M^4$ nicht Trifluoromethyl ist;

$M^4$ steht insbesonders ganz besonders bevorzugt für Fluor, Brom, Chlor, Iod, Cyano, Nitro, Trifluormethyl, Difluormethyl, Methoxy, Trifluormethylsulfanyl, Trifluormethoxy, Difluormethoxy ;

$M^4$ steht insbesonders ganz besonders bevorzugt für Wasserstoff, Fluor, Brom, Chlor, Iod, Cyano, Nitro, Trifluormethyl, Difluormethyl, Methoxy, Trifluormethylsulfanyl, Trifluormethoxy, Difluormethoxy, wobei, falls Q $Q^{10}$ entspricht, $M^4$ nicht Trifluoromethyl ist;

$M^4$ steht insbesonders ganz besonders bevorzugt für Fluor, Brom, Chlor, Iod, Cyano, Nitro, Methyl, Trifluormethyl, Difluormethyl, Methoxy, Ethoxy, iso-Propoxy oder Phenoxy;

$M^4$ steht insbesonders ganz besonders bevorzugt für Wasserstoff, Fluor, Brom, Chlor, Iod, Cyano, Nitro, Methyl, Trifluormethyl, Difluormethyl, Methoxy, Ethoxy, iso-Propoxy oder Phenoxy, wobei, falls Q $Q^{10}$ entspricht, $M^4$ nicht Trifluoromethyl ist;

k steht bevorzugt für 1 oder 2;

k steht besonders bevorzugt für 1;

$R^{21}$, $R^{22}$ steht bevorzugt für jeweils unabhängig voneinander Wasserstoff, Fluor oder gegebenenfalls ein- oder mehrfach durch $M^2$ substituiertes $(C_1$-$C_6)$-Alkyl, $(C_2$-$C_6)$-Alkenyl, $(C_2$-$C_6)$-Alkinyl, $(C_1$-$C_6)$-Halogenalkyl, $(C_2$-$C_6)$-Halogenalkenyl, $(C_2$-$C_6)$-Halogenalkinyl, $(C_1$-$C_6)$-Alkoxy, $(C_1$-$C_6)$-Halogenalkoxy, $(C_2$-$C_6)$-Alkenyloxy, $(C_3$-$C_6)$-Alkinyloxy, $(C_3$-$C_6)$-Cycloalkyl-$(C_1$-$C_6)$-alkyl oder für eine gegebenenfalls ein- oder mehrfach durch $M^2$ substituierte $(C_3$-$C_{14})$- carbocyclische Gruppe;

$R^{21}$, $R^{22}$ steht bevorzugt für jeweils unabhängig voneinander Wasserstoff, Fluor oder gegebenenfalls ein- oder mehrfach durch $M^2$ substituiertes $(C_1$-$C_4)$-Alkyl, $(C_2$-$C_4)$-Alkenyl, $(C_2$-$C_4)$-Alkinyl, $(C_1$-$C_4)$-Halogenalkyl, $(C_2$-$C_4)$-Halogenalkenyl, $(C_2$-$C_4)$-Halogenalkinyl, $(C_1$-$C_6)$-Alkoxy, $(C_1$-$C_4)$-Halogenalkoxy, $(C_2$-$C_4)$-Alkenyloxy, $(C_3$-$C_4)$-Alkinyloxy, $(C_3$-$C_4)$-Cycloalkyl-$(C_1$-$C_4)$-alkyl, $(C_3$-$C_8)$-Cycloalkyl oder halogeniertes $(C_3$-$C_8)$-Cycloalkyl;

$R^{21}$, $R^{22}$ steht bevorzugt für $C(R^{21}, R^{22})$ als spiro-$C(CH_2$-$CH_2)$;

$R^{21}$, $R^{22}$ steht besonders bevorzugt für jeweils unabhängig voneinander Wasserstoff, Fluor oder gegebenenfalls ein- oder mehrfach durch $M^2$ substituiertes $(C_1$-$C_4)$-Alkyl, $(C_2$-$C_4)$-Alkenyl, $(C_2$-$C_4)$-Alkinyl, $(C_1$-$C_4)$-Halogenalkyl, $(C_1$-$C_6)$-Alkoxy, $(C_2$-$C_4)$-Alkenyloxy, $(C_3$-$C_4)$-Alkinyloxy, $(C_3$-$C_4)$-Cycloal-

kyl-($C_1$-$C_4$)-alkyl, ($C_3$-$C_6$)-Cycloalkyl;

$R^{21}$, $R^{22}$ steht bevorzugt für jeweils unabhängig voneinander Wasserstoff, Fluor, Methyl, Ethyl, n-Propyl, iso-Propyl, tert-Butyl, Butyl, Allyl, Propargyl, Chlormethyl, Trichlormethyl, Fluormethyl, Chlordifluormethyl, Dichlorfluormethyl, Trifluormethyl, 2,2-Difluorethyl, Difluormethyl, Methoxy, Ethoxy, Allyloxy, Propargyloxy, Cyclopropylmethyl, Cyclopropyl;

$R^{21}$, $R^{22}$ steht bevorzugt für jeweils unabhängig voneinander Wasserstoff, Fluor, Methyl, Ethyl, n-Propyl, iso-Propyl, Allyl, Propargyl, Methoxy, Ethoxy, Allyloxy, Propargyloxy, Cyclopropylmethyl, Cyclopropyl;

$R^{21}$, $R^{22}$ steht ganz besonders bevorzugt für jeweils unabhängig voneinander Wasserstoff, Fluor oder ($C_1$-$C_4$)-Alkyl, ($C_1$-$C_4$)-Halogenalkyl;

$R^{21}$, $R^{22}$ steht insbesonders ganz besonders bevorzugt für jeweils unabhängig voneinander Wasserstoff, Methyl oder Ethyl;

$R^{31}$, $R^{32}$ steht bevorzugt für jeweils unabhängig voneinander Wasserstoff, Fluor oder gegebenenfalls ein- oder mehrfach durch $M^2$ substituiertes ($C_1$-$C_6$)-Alkyl, ($C_2$-$C_6$)-Alkenyl, ($C_2$-$C_6$)-Alkinyl, ($C_1$-$C_6$)-Halogenalkyl, ($C_2$-$C_6$)-Halogenalkenyl, ($C_2$-$C_6$)-Halogenalkinyl, ($C_1$-$C_6$)-Alkoxy, ($C_1$-$C_6$)-Halogenalkoxy, ($C_2$-$C_6$)-Alkenyloxy, ($C_3$-$C_6$)-Alkinyloxy, ($C_3$-$C_6$)-Cycloalkyl-($C_1$-$C_6$)-alkyl oder für eine gegebenenfalls ein- oder mehrfach durch $M^2$ substituierte ($C_3$-$C_{14}$)- carbocyclische Gruppe;

$R^{31}$, $R^{32}$ steht bevorzugt für jeweils unabhängig voneinander Wasserstoff, Fluor oder gegebenenfalls ein- oder mehrfach durch $M^2$ substituiertes ($C_1$-$C_4$)-Alkyl, ($C_2$-$C_4$)-Alkenyl, ($C_2$-$C_4$)-Alkinyl, ($C_1$-$C_4$)-Halogenalkyl, ($C_2$-$C_4$)-Halogenalkenyl, ($C_2$-$C_4$)-Halogenalkinyl, ($C_1$-$C_6$)-Alkoxy, ($C_1$-$C_4$)-Halogenalkoxy, ($C_2$-$C_4$)-Alkenyloxy, ($C_3$-$C_4$)-Alkinyloxy, ($C_3$-$C_4$)-Cycloalkyl-($C_1$-$C_4$)-alkyl oder für gegebenenfalls ein- oder mehrfach durch $M^2$ substituiertes ($C_3$-$C_8$)-Cycloalkyl oder halogeniertes ($C_3$-$C_8$)-Cycloalkyl;

$R^{31}$, $R^{32}$ steht bevorzugt für C($R^{31}$, $R^{32}$) als spiro-C($CH_2$-$CH_2$);

$R^{31}$, $R^{32}$ steht besonders bevorzugt für C($R^{31}$, $R^{32}$) als 1,1-cyclopropyl;

$R^{31}$, $R^{32}$ steht besonders bevorzugt für jeweils unabhängig voneinander Wasserstoff, Fluor oder gegebenenfalls ein- oder mehrfach durch $M^2$ substituiertes ($C_1$-$C_4$)-Alkyl, ($C_1$-$C_4$)-Halogenalkyl, ($C_2$-$C_4$)-Halogenalkenyl, ($C_2$-$C_4$)-Alkenyl, ($C_2$-$C_4$)-Alkinyl, ($C_1$-$C_6$)-Alkoxy, ($C_2$-$C_4$)-Alkenyloxy, ($C_3$-$C_4$)-Alkinyloxy, ($C_3$-$C_4$)-Cycloalkyl-($C_1$-$C_4$)-alkyl, ($C_3$-$C_6$)-Cycloalkyl;

$R^{31}$, $R^{32}$ steht besonders bevorzugt für jeweils unabhängig voneinander Wasserstoff, Methyl, Ethyl, n-Propyl, iso-Propyl, tert-Butyl, Allyl, Propargyl, Methoxy, Ethoxy, Allyloxy, Propargyloxy, Cyclopropylmethyl, Cyclopropyl;

$R^{31}$, $R^{32}$ steht ganz besonders bevorzugt für jeweils unabhängig voneinander Wasserstoff, Fluor oder ($C_1$-$C_4$)-Alkyl;

$R^{31}$, $R^{32}$ steht insbesonders ganz besonders bevorzugt für jeweils unabhängig voneinander Wasserstoff, Methyl, Ethyl, n-Propyl, iso-Propyl oder tert.-Butyl;

$M^5$ steht bevorzugt jeweils unabhängig voneinander für Halogen, Formyl, Cyano, Nitro, ($C_1$-$C_6$)-Alkyl, ($C_1$-$C_6$)-Halogenalkyl, ($C_2$-$C_6$)-Alkenyl, ($C_2$-$C_6$)-Halogenalkenyl, ($C_2$-$C_6$)-Alkinyl, ($C_2$-$C_6$)-Halogenalkinyl, ($C_1$-$C_6$)-Alkoxy, ($C_1$-$C_6$)-Halogenalkoxy, ($C_2$-$C_6$)-Alkenyloxy, ($C_2$-$C_6$)-Halogenalkenoxy, ($C_3$-$C_6$)-Alkinyloxy, ($C_3$-$C_6$)-Halogenalkinoxy, ($C_1$-$C_6$)-Alkylthio, ($C_1$-$C_6$)-Halogenalkylthio, ($C_2$-$C_6$)-Alkenylthio, ($C_2$-$C_6$)-Halogenalkenylthio, ($C_3$-$C_6$)-Alkinylthio, ($C_3$-$C_6$)-Halogenalkinylthio, ($C_1$-$C_6$)-Alkylsulfonyl, ($C_1$-$C_6$)-Halogenalkylsulfonyl, ($C_2$-$C_6$)-Alkenylsulfonyl, ($C_2$-$C_6$)-Halogenalkenylsulfonyl, ($C_3$-$C_6$)-Alkinylsulfonyl, ($C_3$-$C_6$)-Halogenalkinylsulfonyl, ($C_1$-$C_6$)-Alkylsulfanyl, ($C_1$-$C_6$)-Halogenalkylsulfanyl, ($C_2$-$C_6$)-Alkenylsulfanyl, ($C_2$-$C_6$)-Halogenalkenylsulfanyl, ($C_3$-$C_6$)-Alkinylsulfanyl, ($C_3$-$C_6$)-Halogenalkinylsulfanyl, Formyl, ($C_1$-$C_6$)-Alkylcarbonyl, ($C_1$-$C_6$)-Halogenalkylcarbonyl, ($C_2$-$C_6$)-Alkenylcarbonyl, ($C_2$-$C_6$)-Halogenalkenylcarbonyl, ($C_2$-$C_6$)-Alkinylcarbonyl, ($C_2$-$C_6$)-Halogenalkinylcarbonyl, ($C_1$-$C_6$)-Alkoxycarbonyl, ($C_1$-$C_6$)-Halogenalkoxycarbonyl,

($C_2$-$C_6$)-Alkenoxycarbonyl, ($C_2$-$C_6$)-Halogenalkenoxycarbonyl, ($C_3$-$C_6$)-Alkinoxycarbonyl, ($C_3$-$C_6$)-Halogenalkinoxycarbonyl, ($C_1$-$C_6$)-Alkylcarbonyloxy, ($C_1$-$C_6$)-Halogenalkylcarbonyloxy, ($C_2$-$C_6$)-Alkenylcarbonyloxy, ($C_2$-$C_6$)-Halogenalkenylcarbonyloxy, ($C_2$-$C_6$)-Alkinylcarbonyloxy, ($C_2$-$C_6$)-Halogenalkinylcarbonyloxy oder ($C_3$-$C_{14}$)-Cycloalkyl.

$M^5$ steht besonders bevorzugt jeweils unabhängig voneinander für Halogen, Formyl, Cyano, Nitro, ($C_1$-$C_4$)-Alkyl, ($C_1$-$C_4$)-Halogenalkyl, ($C_2$-$C_4$)-Alkenyl, ($C_2$-$C_4$)-Halogenalkenyl, ($C_2$-$C_4$)-Alkinyl, ($C_2$-$C_4$)-Halogenalkinyl, ($C_1$-$C_4$)-Alkoxy, ($C_1$-$C_4$)-Halogenalkoxy, ($C_2$-$C_4$)-Alkenyloxy, ($C_2$-$C_4$)-Halogenalkenoxy, ($C_3$-$C_4$)-Alkinyloxy, ($C_3$-$C_4$)-Halogenalkinoxy, ($C_1$-$C_4$)-Alkylthio, ($C_1$-$C_4$)-Halogenalkylthio, ($C_2$-$C_4$)-Alkenylthio, ($C_2$-$C_4$)-Halogenalkenylthio, ($C_3$-$C_4$)-Alkinylthio, ($C_3$-$C_4$)-Halogenalkinylthio, ($C_1$-$C_4$)-Alkylsulfonyl, ($C_1$-$C_4$)-Halogenalkylsulfonyl, ($C_2$-$C_4$)-Alkenylsulfonyl, ($C_2$-$C_4$)-Halogenalkenylsulfonyl, ($C_3$-$C_4$)-Alkinylsulfonyl, ($C_3$-$C_4$)-Halogenalkinylsulfonyl, ($C_1$-$C_4$)-Alkylsulfanyl, ($C_1$-$C_4$)-Halogenalkylsulfanyl, ($C_2$-$C_4$)-Alkenylsulfanyl, ($C_2$-$C_4$)-Halogenalkenylsulfanyl, ($C_3$-$C_4$)-Alkinylsulfanyl, ($C_3$-$C_4$)-Halogenalkinylsulfanyl, ($C_1$-$C_4$)-Alkylcarbonyl, ($C_1$-$C_4$)-Halogenalkylcarbonyl, ($C_2$-$C_4$)-Alkenylcarbonyl, ($C_2$-$C_4$)-Halogenalkenylcarbonyl, ($C_2$-$C_4$)-Alkinylcarbonyl, ($C_2$-$C_4$)-Halogenalkinylcarbonyl, ($C_1$-$C_4$)-Alkoxycarbonyl, ($C_1$-$C_4$)-Halogenalkoxycarbonyl, ($C_2$-$C_4$)-Alkenoxycarbonyl, ($C_2$-$C_4$)-Halogenalkenoxycarbonyl, ($C_3$-$C_4$)-Alkinoxycarbonyl, ($C_3$-$C_4$)-Halogenalkinoxycarbonyl, ($C_1$-$C_4$)-Alkylcarbonyloxy, ($C_1$-$C_4$)-Halogenalkylcarbonyloxy, ($C_2$-$C_4$)-Alkenylcarbonyloxy, ($C_2$-$C_4$)-Halogenalkenylcarbonyloxy, ($C_2$-$C_4$)-Alkinylcarbonyloxy, ($C_2$-$C_4$)-Halogenalkinylcarbonyloxy oder ($C_3$-$C_6$)-Cycloalkyl.

$M^5$ steht ganz besonders bevorzugt jeweils unabhängig voneinander für Chlor, Fluor, Formyl, Cyano, Nitro, ($C_1$-$C_4$)-Alkyl, ($C_1$-$C_4$)-Halogenalkyl, ($C_1$-$C_4$)-Alkoxy, ($C_1$-$C_4$)-Halogenalkoxy, ($C_1$-$C_4$)-Alkylthio, ($C_1$-$C_4$)-Halogenalkylthio, ($C_1$-$C_4$)-Alkylsulfonyl, ($C_1$-$C_4$)-Halogenalkylsulfonyl, ($C_1$-$C_4$)-Alkylsulfanyl, ($C_1$-$C_4$)-Halogenalkylsulfanyl, ($C_1$-$C_4$)-Alkylcarbonyl, ($C_1$-$C_4$)-Halogenalkylcarbonyl oder ($C_3$-$C_6$)-Cycloalkyl.

$M^5$ steht ganz besonders bevorzugt jeweils unabhängig voneinander für Fluor, Brom, Chlor, Iod, Cyano, Nitro, Methyl, Ethyl, iso-Propyl, tert.-Butyl, Trifluormethyl, Difluormethyl, Methoxy, Ethoxy, iso-Propoxy, Trifluormethoxy, Difluormethoxy, Methylthio, Trifluormethylthio, Difluormethylthio, 2,2,2-Trifluorethylthio, Methylsulfonyl, Ethylsulfonyl, Trifluormethylsulfonyl, 2,2,2-Trifluorethylsulfonyl, Methylsulfanyl, Ethylsulfanyl, Trifluormethylsulfanyl, 2,2,2-Trifluorethylsulfanyl, Cyclopropyl, Cyclobutyl oder Cyclopentyl.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend.

Die genannten Definitionen können untereinander in beliebiger Weise kombiniert werden. Außerdem können auch einzelne Definitionen entfallen.

Bevorzugt, besonders bevorzugt oder ganz besonders bevorzugt sind Verbindungen der Formel (I), welche jeweils die unter bevorzugt, besonders bevorzugt, ganz besonders bevorzugt oder insbesonders ganz besonders bevorzugt genannten Substituenten tragen.

Weiterhin bevorzugt sind Verbindungen der Formel (I)

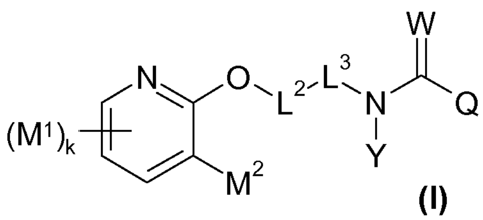

**(I)**

in welcher $M^1$, $M^2$, $M^3$, $M^4$, $M^5$, k, $L^2$, $L^3$, W und Y wie oben definiert sind und

Q bevorzugt für folgende Strukturelemente steht, wobei n für jedes Q jeweils wie unten angegeben definiert ist:

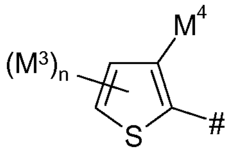

**Q**1
n = 1-2

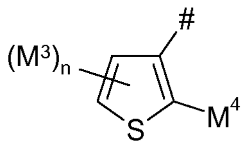

**Q**2
n = 1-2

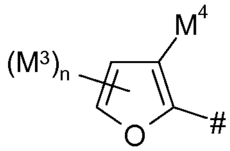

**Q**3
n =1-2

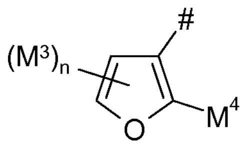

**Q**4
n =1-2

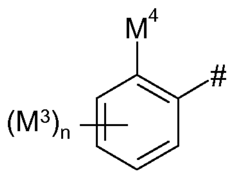

**Q**9
n =1-4

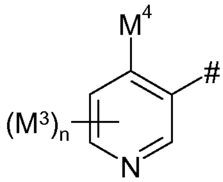

**Q**10
n = 1-3

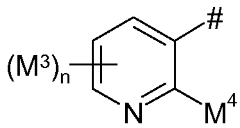

**Q**11
n = 1-3

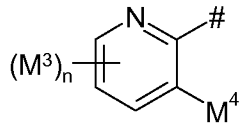

**Q**12
n = 1-3

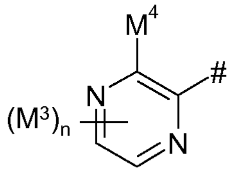

**Q**13
n = 1-2

Q bevorzugt für folgende Strukturelemente steht, wobei n für jedes Q jeweils wie unten angegeben definiert ist:

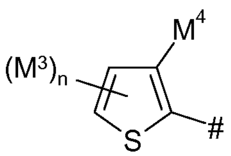

**Q**1
n = 1-2

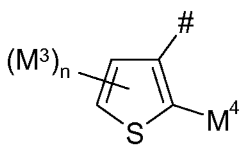

**Q**2
n = 1-2

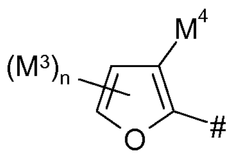

**Q**3
n =1-2

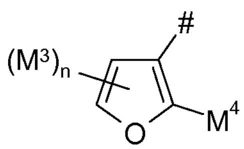

**Q**4
n =1-2

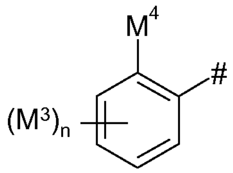

**Q**9
n =1-4

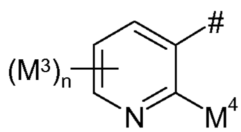

**Q**11
n = 1-3

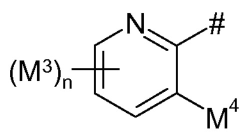

**Q**12
n = 1-3

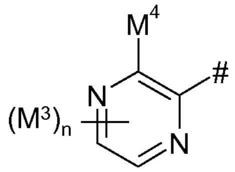

$\mathbf{Q^{13}}$
n = 1-2

Q bevorzugt für folgende Strukturelemente steht, wobei n für jedes Q jeweils wie unten angegeben definiert ist:

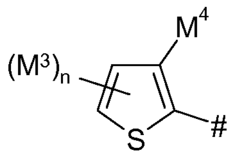

$\mathbf{Q^{1}}$
n = 1

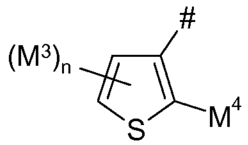

$\mathbf{Q^{2}}$
n = 1

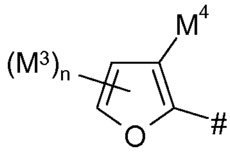

$\mathbf{Q^{3}}$
n = 1

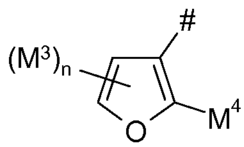

$\mathbf{Q^{4}}$
n = 0-1

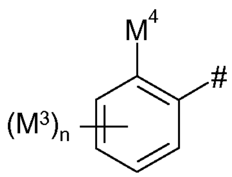

$\mathbf{Q^{9}}$
n = 1-3

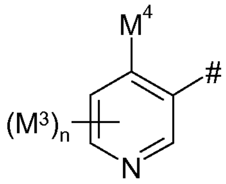

$\mathbf{Q^{10}}$
n = 1-2

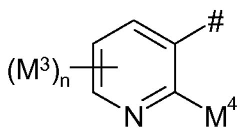

$\mathbf{Q^{11}}$
n = 1-2

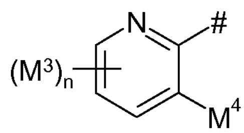

$\mathbf{Q^{12}}$
n =1-2

Q bevorzugt für folgende Strukturelemente steht, wobei n für jedes Q jeweils wie unten angegeben definiert ist:

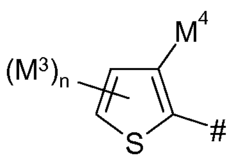

$\mathbf{Q^{1}}$
n = 1

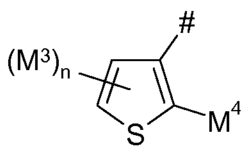

$\mathbf{Q^{2}}$
n = 1

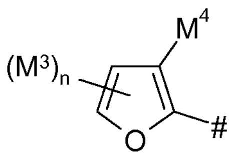

$\mathbf{Q^{3}}$
n = 1

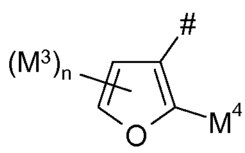

$\mathbf{Q^{4}}$
n = 0-1

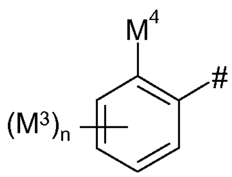

$\mathbf{Q^{9}}$
n = 1-3

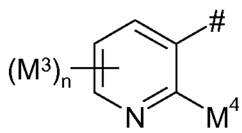

$\mathbf{Q^{11}}$
n = 1-2

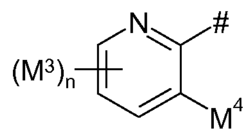

$\mathbf{Q^{12}}$
n =1-2

Q besonders bevorzugt für 2-Thienyl, 3-Fluor-2-thienyl, 3-Chlor-2-thienyl, 3,4-Dichlor-2-thienyl, 2,5-Dichlor-3-thienyl, 3,4,5-Trichlor-2-thienyl, 3-Brom-2-thienyl, 3-Iod-2-thienyl, 3-Cyano-2-thienyl, 3-Methyl-2-thienyl, 3-(Trifluormethyl)-2-thienyl, 3-Methoxy-2-thienyl, 3-Ethoxy-2-thienyl, 3-Thienyl, 2-Fluor-3-thienyl, 2-Chlor-3-thienyl, 2-Brom-3-thienyl, 2-Iod-3-thienyl, 2-Cyano-3-thienyl, 2-Methyl-3-thienyl, 2-(Trifluormethyl)-3-thienyl, 2-Methoxy-3-thienyl, 2-Ethoxy-3-thienyl, 2-Furanyl, 3-Fluor-2-furanyl, 3-Chlor-2-furanyl, 3-Brom-2-furanyl, 3-Iod-2-furanyl, 3-Cyano-2-furanyl, 3-Methyl-2-furanyl, 3-(Trifluormethyl)-2-furanyl, 3-Methoxy-2-furanyl, 3-Ethoxy-2-furanyl, 3-Furanyl, 2-Chlor-3-furanyl, 2-Brom-3-furanyl, 2-Iod-3-furanyl, 2-Cyano-3-furanyl, 2-Methyl-3-furanyl, 2-(Trifluormethyl)-3-furanyl, 2-Methoxy-3-furanyl, 2-Ethoxy-3-furanyl, 2-Methyl-phenyl, 2-Fluor-phenyl, 2,6-Difluor-phenyl, 2-Chor-phenyl, 2,6-Dichlor-phenyl, 2-Brom-phenyl, 2-Iod-phenyl, 2-(Difluormethyl)-phenyl, 2-(Trifluormethyl)-phenyl, 2-(Methylsulfanyl)-phenyl, 2-(Methylsulfonyl)-phenyl, 2-(Trifluormethoxy)-phenyl, 2-(Trifluormethylsulfanyl)-phenyl, 2-(Trifluormethylsulfonyl)-phenyl, 2-Nitro-phenyl, 2-Chlor-3-pyridyl, 3-Chlor-2-pyridyl, 2-(Difluormethyl)-3-pyridyl, 2-(Trifluormethyl)-3-pyridyl, 2-(Methylsulfanyl)-3-pyridyl, 2-(Methylsulfonyl)- 3-pyridyl, 2-(Trifluormethoxy)- 3-pyridyl, 2-(Trifluormethylsulfanyl)- 3-pyridyl oder 2-(Trifluormethylsulfonyl)- 3-pyridyl steht;

Q besonders bevorzugt für 2-Thienyl, 3-Fluor-2-thienyl, 3-Chlor-2-thienyl, 3,4-Dichlor-2-thienyl, 3,4,5-Trichlor-2-thienyl, 3-Brom-2-thienyl, 3-Iod-2-thienyl, 3-Cyano-2-thienyl, 3-Methyl-2-thienyl, 3-(Trifluormethyl)-2-thienyl, 3-Methoxy-2-thienyl, 3-Ethoxy-2-thienyl, 3-Thienyl, 2-Fluor-3-thienyl, 2-Chlor-3-thienyl, 2-Brom-3-thienyl, 2-Iod-3-thienyl, 2-Cyano-3-thienyl, 2-Methyl-3-thienyl, 2-(Trifluormethyl)-3-thienyl, 2-Methoxy-3-thienyl, 2-Ethoxy-3-thienyl, 2-Furanyl, 3-Fluor-2-furanyl, 3-Chlor-2-furanyl, 3-Brom-2-furanyl, 3-Iod-2-furanyl, 3-Cyano-2-furanyl, 3-Methyl-2-furanyl, 3-(Trifluormethyl)-2-furanyl, 3-Methoxy-2-furanyl, 3-Ethoxy-2-furanyl, 3-Furanyl, 2-Chlor-3-furanyl, 2-Brom-3-furanyl, 2-Iod-3-furanyl, 2-Cyano-3-furanyl, 2-Methyl-3-furanyl, 2-(Trifluormethyl)-3-furanyl, 2-Methoxy-3-furanyl, 2-Ethoxy-3-furanyl, 2-Methyl-phenyl, 2-Fluor-phenyl, 2,6-Difluor-phenyl, 2-Chor-phenyl, 2,6-Dichlor-phenyl, 2-Bromphenyl, 2-Iod-phenyl, 2-(Difluormethyl)-phenyl, 2-(Trifluormethyl)-phenyl, 2-Nitro-phenyl, 2-Chlor-3-pyridyl, 3-Chlor-2-pyridyl oder 2-(Trifluormethyl)-3-pyridyl steht.

Weiterhin bevorzugt sind Verbindungen der Formel (I)

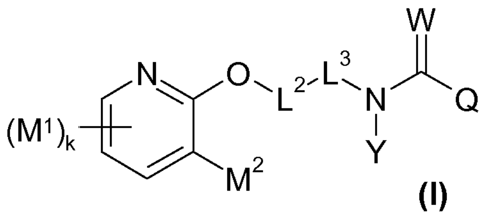

**(I)**

in welcher $M^1$, $M^2$, $M^3$, $M^4$, $M^5$, k, $L^2$, $L^3$, Q und W wie oben definiert sind und

Y bevorzugt für Wasserstoff oder für gegebenenfalls ein- oder mehrfach durch $M^2$ substituiertes $(C_1-C_4)$-Alkyl, $(C_2-C_4)$-Alkenyl, $(C_3-C_4)$-Alkinyl, $(C_1-C_4)$-Halogenalkyl, $(C_2-C_4)$-Halogenalkenyl, $(C_3-C_4)$-Halogenalkinyl, $(C_1-C_4)$-Alkoxy, $(C_2-C_4)$-Alkenyloxy, $(C_3-C_4)$-Alkinyloxy, $(C_3-C_6)$-Cycloalkyl-$(C_1-C_4)$-alkyl oder für eine gegebenenfalls substituierte $C_3$- bis $C_6$-gliedrige carbocyclische Gruppe steht;

Y besonders bevorzugt für Wasserstoff, Methyl, Ethyl, n-Propyl, iso-Propyl, n-Butyl, sec-Butyl, iso-Butyl, tert.-Butyl, Cyanomethyl, 2,2-Difluor-ethyl, 2,2,2-Trifluor-ethyl, Allyl, Butenyl, Propargyl, Butinyl, 3,3-Dichlor-prop-2-enyl, Methoxy, Ethoxy, Cyclopropylmethyl, Cyclopropyl, Cyclobutyl, Cyclopentyl oder Cyclohexyl steht;

Y ganz besonders bevorzugt für Wasserstoff steht.

Weiterhin bevorzugt sind Verbindungen der Formel (I)

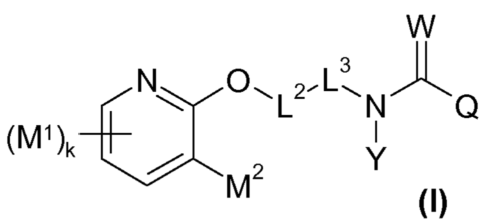

**(I)**

in welcher $M^1$, $M^2$, $M^3$, $M^4$, $M^5$, k, $L^2$, $L^3$, Q und Y wie oben definiert sind und

W bevorzugt für Sauerstoff steht.

Weiterhin bevorzugt sind Verbindungen der Formel (I)

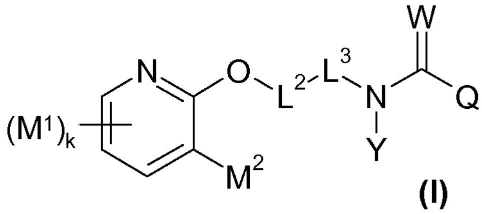

**(I)**

in welcher $M^1$, $M^2$, $M^3$, $M^4$, $M^5$, k, $L^3$, Q, W und Y wie oben definiert sind und

$L^2$ bevorzugt für $C(R^{21}, R^{22})$ steht, wobei $R^{21}$ und $R^{22}$ jeweils unabhängig voneinander für Wasserstoff, Fluor oder gegebenenfalls ein- oder mehrfach durch $M^2$ substituiertes $(C_1\text{-}C_4)$-Alkyl, $(C_3\text{-}C_4)$-Alkenyl, $(C_3\text{-}C_4)$-Alkinyl, $(C_1\text{-}C_4)$-Halogenalkyl, $(C_3\text{-}C_4)$-Halogenalkenyl, $(C_3\text{-}C_4)$-Halogenalkinyl, $(C_1\text{-}C_4)$-Alkoxy, $(C_3\text{-}C_4)$-Alkenyloxy, $(C_3\text{-}C_4)$-Alkinyloxy, $(C_3\text{-}C_6)$-Cycloalkyl-$(C_1\text{-}C_4)$-alkyl oder für eine gegebenenfalls substituierte $C_3$- bis $C_6$-gliedrige carbocyclische Gruppe steht, oder wobei $R^{21}$ und $R^{22}$ zusammen für eine gegebenenfalls substituierte spiroverknüpfte 3 bis 6-gliedrige cyclische Gruppe stehen;

$L^2$ besonders bevorzugt für $C(R^{21}, R^{22})$ steht, wobei $R^{21}$ und $R^{22}$ jeweils unabhängig voneinander für Wasserstoff, Fluor, Methyl, Ethyl, n-Propyl, iso-Propyl, Allyl, Propargyl, Methoxy, Ethoxy, Allyloxy, Propargyloxy, Cyclopropylmethyl, Cyclopropyl stehen, oder wobei $C(R^{21}, R^{22})$ für spiro-$C(CH_2\text{-}CH_2)$ steht.

Weiterhin bevorzugt sind Verbindungen der Formel (I)

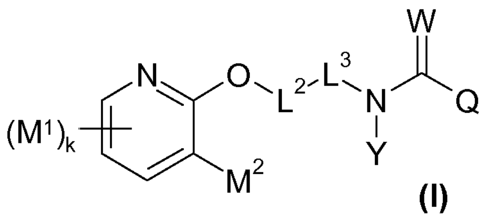

**(I)**

in welcher $M^1$, $M^2$, $M^3$, $M^4$, $M^5$, k, $L^2$, Q, W und Y wie oben definiert sind und

$L^3$ bevorzugt für $C(R^{31}, R^{23})$ steht, wobei $R^{31}$ und $R^{32}$ jeweils unabhängig voneinander für Wasserstoff, Fluor oder gegebenenfalls ein- oder mehrfach durch $M^2$ substituiertes $(C_1\text{-}C_4)$-Alkyl, $(C_3\text{-}C_4)$-Alkenyl, $(C_3\text{-}C_4)$-Alkinyl, $(C_1\text{-}C_4)$-Halogenalkyl, $(C_3\text{-}C_4)$-Halogenalkenyl, $(C_3\text{-}C_4)$-Halogenalkinyl, $(C_1\text{-}C_4)$-Alkoxy, $(C_3\text{-}C_4)$-Alkenyloxy, $(C_3\text{-}C_4)$-Alkinyloxy, $(C_3\text{-}C_6)$-Cycloalkyl-$(C_1\text{-}C_4)$-alkyl oder für eine gegebenenfalls substituierte $C_3$- bis $C_6$-gliedrige carbocyclische Gruppe stehen, oder wobei $R^{31}$ und $R^{32}$ zusammen für eine gegebenenfalls substituierte spiroverknüpfte 3 bis 6-gliedrige cyclische Gruppe stehen;

$L^3$ besonders bevorzugt für $C(R^{31}, R^{32})$ steht, wobei $R^{31}$ und $R^{32}$ jeweils unabhängig voneinander für Wasserstoff, Methyl, Ethyl, n-Propyl, iso-Propyl, Allyl, Propargyl, Methoxy, Ethoxy, Allyloxy, Propargyloxy, Cyclopropylmethyl, Cyclopropyl stehen, oder wobei $C(R^{31}, R^{32})$ für spiro-$C(CH_2\text{-}CH_2)$ steht;

sowie Salze, N-Oxide und tautomere Formen der Verbindungen der Formel (I).

Weiterhin ganz besonders bevorzugt sind Verbindungen der Formel (I)

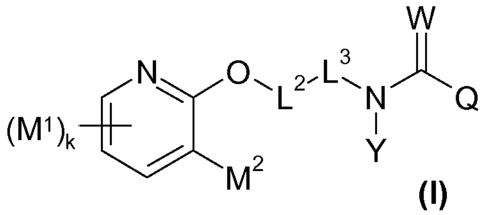

(I)

in welcher

Q für 2-Thienyl, 3-Fluor-2-thienyl, 3-Chlor-2-thienyl, 3,4-Dichlor-2-thienyl, 3,4,5-Trichlor-2-thienyl, 3-Brom-2-thienyl, 3-Iod-2-thienyl, 3-Cyano-2-thienyl, 3-Methyl-2-thienyl, 3-(Trifluormethyl)-2-thienyl, 3-Methoxy-2-thienyl, 3-Ethoxy-2-thienyl, 3-Thienyl, 2-Fluor-3-thienyl, 2-Chlor-3-thienyl, 2-Brom-3-thienyl, 2-Iod-3-thienyl, 2-Cyano-3-thienyl, 2-Methyl-3-thienyl, 2-(Trifluormethyl)-3-thienyl, 2-Methoxy-3-thienyl, 2-Ethoxy-3-thienyl, 2-Furanyl, 3-Fluor-2-furanyl, 3-Chlor-2-furanyl, 3-Brom-2-furanyl, 3-Iod-2-furanyl, 3-Cyano-2-furanyl, 3-Methyl-2-furanyl, 3-(Trifluormethyl)-2-furanyl, 3-Methoxy-2-furanyl, 3-Ethoxy-2-furanyl, 3-Furanyl, 2-Chlor-3-furanyl, 2-Brom-3-furanyl, 2-Iod-3-furanyl, 2-Cyano-3-furanyl, 2-Methyl-3-furanyl, 2-(Trifluormethyl)-3-furanyl, 2-Methoxy-3-furanyl, 2-Ethoxy-3-furanyl, 2-Methyl-phenyl, 2-Fluor-phenyl, 2,6-Difluor-phenyl, 2-Chor-phenyl, 2,6-Dichlor-phenyl, 2-Brom-phenyl, 2-Iod-phenyl, 2-(Difluormethyl)-phenyl, 2-(Trifluormethyl)-phenyl, 2-Nitro-phenyl, 2-Chlor-3-pyridyl, 3-Chlor-2-pyridyl oder 2-(Trifluormethyl)-3-pyridyl steht;

Y für Wasserstoff, Methyl, Ethyl, n-Propyl, iso-Propyl, n-Butyl, sec-Butyl, iso-Butyl, tert.-Butyl, Cyanomethyl, 2,2-Difluor-ethyl, 2,2,2-Trifluor-ethyl, Allyl, Butenyl, Propargyl, Butinyl, 3,3-Dichlor-prop-2-enyl, Methoxy, Ethoxy, Cyclopropylmethyl, Cyclopropyl, Cyclobutyl, Cyclopentyl oder Cyclohexyl steht;

W für Sauerstoff steht;

$L^2$ für $C(R^{21}, R^{22})$ steht, wobei $R^{21}$ und $R^{22}$ jeweils unabhängig voneinander für Wasserstoff, Fluor, Methyl, Ethyl, n-Propyl, iso-Propyl, Allyl, Propargyl, Methoxy, Ethoxy, Allyloxy, Propargyloxy, Cyclopropylmethyl, Cyclopropyl stehen, oder wobei $C(R^{21}, R^{22})$ für spiro-$C(CH_2\text{-}CH_2)$ steht;

$L^3$ für $C(R^{31}, R^{32})$ steht, wobei $R^{31}$ und $R^{32}$ jeweils unabhängig voneinander für Wasserstoff, Methyl, Ethyl, n-Propyl, iso-Propyl, Allyl, Propargyl, Methoxy, Ethoxy, Allyloxy, Propargyloxy, Cyclopropylmethyl, Cyclopropyl stehen, oder wobei $C(R^{31}, R^{32})$ für spiro-$C(CH_2\text{-}CH_2)$ steht;

k für 1 steht und

$M^1$ und $M^2$ jeweils unabhängig voneinander für Wasserstoff, Fluor, Brom, Chlor, Iod, Cyano, Methyl, Trifluormethyl, Difluormethyl, Methoxy, Ethoxy, iso-Propoxy oder Phenoxy stehen;

sowie Salze, N-Oxide und tautomere Formen der Verbindungen der Formel (I).

Weiterhin ganz besonders bevorzugt sind Verbindungen der Formel (I)

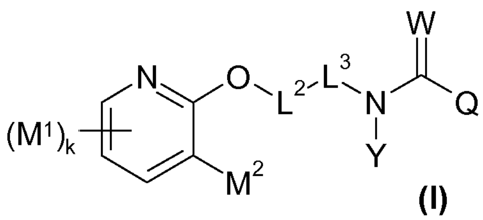

(I)

in welcher

Q für folgende Strukturelemente steht, wobei n für jedes Q jeweils wie unten angegeben definiert ist:

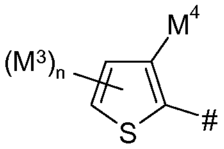

**Q¹**
n = 1-2

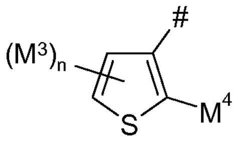

**Q²**
n =1-2

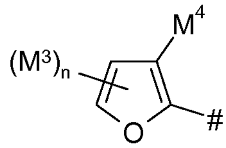

**Q³**
n = 1-2

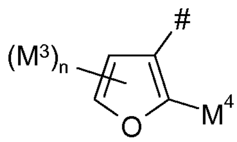

**Q⁴**
n =1-2

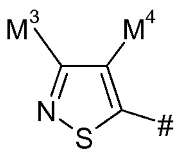

**Q⁵**

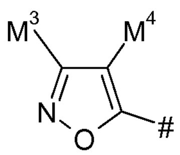

**Q⁶**

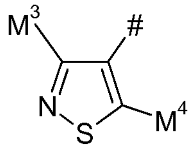

**Q⁷**

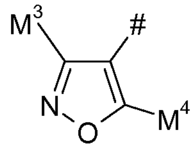

**Q⁸**

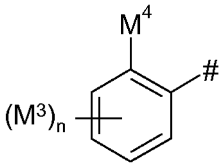

**Q⁹**
n =1-4

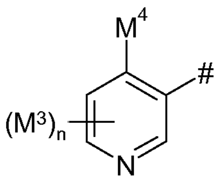

**Q¹⁰**
n =1-3

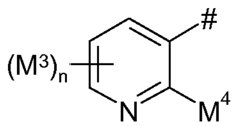

**Q¹¹**
n = 1-3

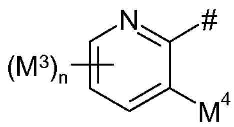

**Q¹²**
n = 1-3

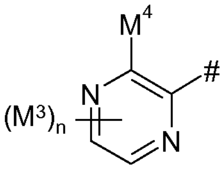

**Q¹³**
n = 1-2

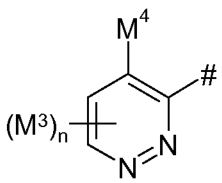

**Q¹⁴**
n = 1-2

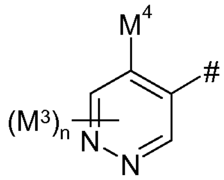

**Q¹⁵**
n = 1-2

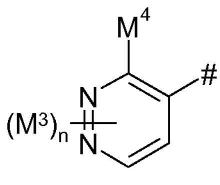

**Q¹⁶**
n = 1-2

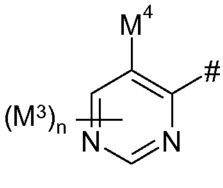

**Q¹⁷**
n = 1-2

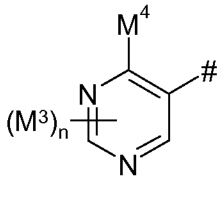

**Q¹⁸**
n = 1-2

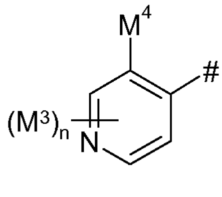

**Q¹⁹**
n = 1-3

Y für Wasserstoff oder für gegebenenfalls ein- oder mehrfach durch $M^2$ substituiertes $(C_1-C_{10})$-Alkyl, $(C_2-C_{10})$-Alkenyl, $(C_2-C_{10})$-Alkinyl, $(C_1-C_{10})$-Halogenalkyl, $(C_2-C_{10})$-Halogenalkenyl, $(C_2-C_{10})$-Halogenalkinyl, $(C_1-C_{10})$-Alkoxy, $(C_2-C_{10})$-Alkenyloxy, $(C_3-C_{10})$-Alkinyloxy, $(C_3-C_{14})$-Cycloalkyl-(Ci-

Cio)-alkyl oder für eine gegebenenfalls ein- oder mehrfach durch $M^2$ substituierte 3- bis 14-gliedrige cyclische Gruppe steht;

W für Sauerstoff oder Schwefel steht;

$L^2$ für $-C(R^{21}, R^{22})-$ steht;

$L^3$ für $-C(R^{31}, R^{32})-$ steht;

$M^1$, $M^2$ und $M^3$ jeweils unabhängig voneinander für Wasserstoff, Halogen, Cyano, Nitro, OH, $(C_1-C_{10})$-Alkyl, $(C_1-C_{10})$-Halogenalkyl, $(C_1-C_{10})$-Alkoxy, $(C_1-C_{10})$-Halogenalkoxy, $(C_1-C_{10})$-Alkylthio, $(C_1-C_{10})$- Halogenalkylthio, $(C_1-C_{10})$-Alkylsulfonyl, $(C_1-C_{10})$-Halogenalkylsulfonyl, $(C_1-C_{10})$-Alkylsulfanyl, $(C_1-C_{10})$-Halogenalkylsulfanyl, oder (3- bis 14-gliedrige cyclische Gruppe)-O-stehen, wobei, falls Q $Q^{11}$ entspricht, $M^3$ nicht $(C_1-C_4)$-Halogenalkyl in 4-Position am Pyridyl ist;

$M^4$ für Wasserstoff, Halogen, Cyano, Nitro, OH, $(C_1-C_{10})$-Alkyl, $(C_1-C_{10})$-Halogenalkyl, $(C_1-C_{10})$-Alkoxy, $(C_1-C_{10})$-Halogenalkoxy, $(C_1-C_{10})$-Alkylthio, $(C_1-C_{10})$-Halogenalkylthio, $(C_1-C_{10})$-Alkylsulfonyl, $(C_1-C_{10})$-Halogenalkylsulfonyl, $(C_1-C_{10})$-Alkylsulfanyl, $(C_1-C_{10})$-Halogenalkylsulfanyl, oder (3- bis 14-gliedrige cyclische Gruppe)-O- steht, wobei, falls Q $Q^{10}$ entspricht, $M^4$ nicht $(C_1-C_4)$-Halogenalkyl ist;

k für 1, 2 oder 3 steht;

$R^{21}$, $R^{22}$ für jeweils unabhängig voneinander Wasserstoff, Halogen oder gegebenenfalls ein- oder mehrfach durch $M^2$ substituiertes $(C_1-C_{10})$-Alkyl, $(C_2-C_{10})$-Alkenyl, $(C_2-C_{10})$-Alkinyl, $(C_1-C_{10})$-Halogenalkyl, $(C_2-C_{10})$-Halogenalkenyl, $(C_2-C_{10})$-Halogenalkinyl, $(C_1-C_{10})$-Alkoxy, $(C_1-C_{10})$-Halogenalkoxy, $(C_2-C_{10})$-Alkenyloxy, $(C_3-C_{10})$-Alkinyloxy, $(C_3-C_{14})$-Cycloalkyl-$(C_1-C_{10})$-alkyl oder für eine gegebenenfalls ein- oder mehrfach durch $M^2$ substituierte 3- bis 14-gliedrige cyclische Gruppe steht;

$R^{21}$, $R^{22}$ zusammen für eine jeweils gegebenenfalls ein- oder mehrfach durch $M^2$ substituierte spiroverknüpfte 3- bis 14-gliedrige carbo- oder 3- bis 10-gliedrige heterocyclische Gruppe stehen;

$R^{31}$, $R^{32}$ für jeweils unabhängig voneinander Wasserstoff, Halogen oder gegebenenfalls ein- oder mehrfach durch $M^2$ substituiertes $(C_1-C_{10})$-Alkyl, $(C_2-C_{10})$-Alkenyl, $(C_2-C_{10})$-Alkinyl, $(C_1-C_{10})$-Halogenalkyl, $(C_2-C_{10})$-Halogenalkenyl, $(C_2-C_{10})$-Halogenalkinyl, $(C_3-C_{14})$-Cycloalkyl-$(C_1-C_{10})$-alkyl oder für eine gegebenenfalls ein- oder mehrfach durch $M^2$ substituierte 3- bis 14-gliedrige cyclische Gruppe steht;

$R^{31}$, $R^{32}$ zusammen für eine jeweils gegebenenfalls ein- oder mehrfach durch $M^5$ substituierte spiroverknüpfte 3- bis 14-gliedrige carbo- oder 3- bis 10-gliedrige heterocyclische Gruppe stehen;

$M^5$ für jeweils unabhängig voneinander für Halogen, Formyl, Cyano, Nitro, $(C_1-C_{10})$-Alkyl, $(C_1-C_{10})$-Halogenalkyl, $(C_2-C_{10})$-Alkenyl, $(C_2-C_{10})$-Halogenalkenyl, $(C_2-C_{10})$-Alkinyl, $(C_2-C_{10})$-Halogenalkinyl, $(C_1-C_{10})$-Alkoxy, $(C_1-C_{10})$-Halogenalkoxy, $(C_2-C_{10})$-Alkenyloxy, $(C_2-C_{10})$-Halogenalkenoxy, $(C_3-C_{10})$-Alkinyloxy, $(C_3-C_{10})$-Halogenalkinoxy, $(C_1-C_{10})$-Alkylthio, $(C_1-C_{10})$-Halogenalkylthio, $(C_2-C_{10})$-Alkenylthio, $(C_2-C_{10})$-Halogenalkenylthio, $(C_3-C_{10})$-Alkinylthio, $(C_3-C_{10})$-Halogenalkinylthio, $(C_1-C_{10})$-Alkylsulfonyl, $(C_1-C_{10})$-Halogenalkylsulfonyl, $(C_2-C_{10})$-Alkenylsulfonyl, $(C_2-C_{10})$- Halogenalkenylsulfonyl, $(C_3-C_{10})$-Alkinylsulfonyl, $(C_3-C_{10})$-Halogenalkinylsulfonyl, $(C_1-C_{10})$-Alkylsulfanyl, $(C_1-C_{10})$-Halogenalkylsulfanyl, $(C_2-C_{10})$-Alkenylsulfanyl, $(C_2-C_{10})$- Halogenalkenylsulfanyl, $(C_3-C_{10})$-Alkinylsulfanyl, $(C_3-C_{10})$-Halogenalkinylsulfanyl, $(C_1-C_{10})$-Alkylcarbonyl, $(C_1-C_{10})$-Halogenalkylcarbonyl, $(C_2-C_{10})$-Alkenylcarbonyl, $(C_2-C_{10})$-Halogenalkenylcarbonyl, $(C_2-C_{10})$-Alkinylcarbonyl, $(C_2-C_{10})$-Halogenalkinylcarbonyl, $(C_1-C_{10})$-Alkoxycarbonyl, $(C_1-C_{10})$-Halogenalkoxycarbonyl, $(C_2-C_{10})$-Alkenoxycarbonyl, $(C_2-C_{10})$-Halogenalkenoxycarbonyl, $(C_3-C_{10})$-Alkinoxycarbonyl, $(C_3-C_{10})$-Halogenalkinoxycarbonyl, $(C_1-C_{10})$-Alkylcarbonyloxy, $(C_1-C_{10})$-Halogenalkylcarbonyloxy, $(C_2-C_{10})$-Alkenylcarbonyloxy, $(C_2-C_{10})$-Halogenalkenylcarbonyloxy, $(C_2-C_{10})$-Alkinylcarbonyloxy, $(C_2-C_{10})$-Halogenalkinylcarbonyloxy, 3 bis 14-gliedrige cyclische Gruppe steht;

sowie deren Salze, N-Oxide und tautomere Formen der Verbindungen der Formel (I). Gemäß Anspruch 1 sind Verbindungen der Formel (I) definiert

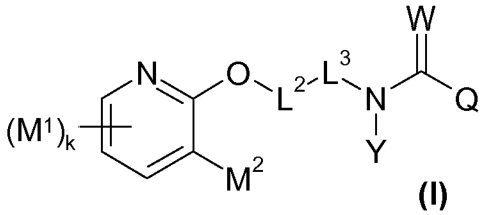

**(I)**

in welcher

Q für 2-Thienyl, 3-Fluor-2-thienyl, 3-Chlor-2-thienyl, 3,4-Dichlor-2-thienyl, 2,5-Dichlor-3-thienyl, 3,4,5-Trichlor-2-thienyl, 3-Brom-2-thienyl, 3-Iod-2-thienyl, 3-Cyano-2-thienyl, 3-Methyl-2-thienyl, 3-(Trifluormethyl)-2-thienyl, 3-Methoxy-2-thienyl, 3-Ethoxy-2-thienyl, 3-Thienyl, 2-Fluor-3-thienyl, 2-Chlor-3-thienyl, 2-Brom-3-thienyl, 2-Iod-3-thienyl, 2-Cyano-3-thienyl, 2-Methyl-3-thienyl, 2-(Trifluormethyl)-3-thienyl, 2-Methoxy-3-thienyl, 2-Ethoxy-3-thienyl, 2-Furanyl, 3-Fluor-2-furanyl, 3-Chlor-2-furanyl, 3-Brom-2-furanyl, 3-Iod-2-furanyl, 3-Cyano-2-furanyl, 3-Methyl-2-furanyl, 3-(Trifluormethyl)-2-furanyl, 3-Methoxy-2-furanyl, 3-Ethoxy-2-furanyl, 3-Furanyl, 2-Chlor-3-furanyl, 2-Brom-3-furanyl, 2-Iod-3-furanyl, 2-Cyano-3-furanyl, 2-Methyl-3-furanyl, 2-(Trifluormethyl)-3-furanyl, 2-Methoxy-3-furanyl, 2-Ethoxy-3-furanyl, 2-Methyl-phenyl, 2-Fluor-phenyl, 2,6-Difluor-phenyl, 2-Chlor-phenyl, 2,6-Dichlor-phenyl, 2-Bromphenyl, 2-Iod-phenyl, 2-(Difluormethyl)-phenyl, 2-(Trifluormethyl)-phenyl, 2-(Methylsulfanyl)-phenyl, 2-(Methylsulfonyl)-phenyl, 2-(Trifluormethoxy)-phenyl, 2-(Trifluormethylsulfanyl)-phenyl, 2-(Trifluormethylsulfonyl)-phenyl, 2-Nitrophenyl, 2-Chlor-3-pyridyl, 3-Chlor-2-pyridyl, 2-(Difluormethyl)-3-pyridyl,2-(Trifluormethyl)-3-pyridyl, 2-(Methylsulfanyl)-3-pyridyl, 2-(Methylsulfonyl)- 3-pyridyl, 2-(Trifluormethoxy)- 3-pyridyl, 2-(Trifluormethylsulfanyl)- 3-pyridyl oder 2-(Trifluormethylsulfonyl)- 3-pyridyl steht;

Y für Wasserstoff, Methyl, Ethyl, n-Propyl, iso-Propyl, n-Butyl, sec-Butyl, iso-Butyl, tert.-Butyl, Cyanomethyl, 2,2-Difluor-ethyl, 2,2,2-Trifluor-ethyl, Allyl, Butenyl, Propargyl, Butinyl, 3,3-Dichlor-prop-2-enyl, Methoxy, Ethoxy, Cyclopropylmethyl, Cyclopropyl, Cyclobutyl, Cyclopentyl oder Cyclohexyl steht;

W für Sauerstoff steht;

$L^2$ für $C(R^{21}, R^{22})$ steht, wobei $R^{21}$ und $R^{22}$ jeweils unabhängig voneinander für Wasserstoff, Fluor, Methyl, Ethyl, n-Propyl, iso-Propyl, Allyl, Propargyl, Methoxy, Ethoxy, Allyloxy, Propargyloxy, Cyclopropylmethyl, Cyclopropyl stehen, oder wobei $C(R^{21}, R^{22})$ für spiro-$C(CH_2-CH_2)$ steht;

$L^3$ für $C(R^{31}, R^{32})$ steht, wobei $R^{31}$ und $R^{32}$ jeweils unabhängig voneinander für Wasserstoff, Methyl, Ethyl, n-Propyl, iso-Propyl, Allyl, Propargyl, Methoxy, Ethoxy, Allyloxy, Propargyloxy, Cyclopropylmethyl, Cyclopropyl stehen, oder wobei $C(R^{31}, R^{32})$ für spiro-$C(CH_2-CH_2)$ steht;

k für 1 steht und

$M^1$ und $M^2$ jeweils unabhängig voneinander für Wasserstoff, Fluor, Brom, Chlor, Iod, Cyano, Methyl, Trifluormethyl, Difluormethyl, Methoxy, Ethoxy, iso-Propoxy oder Phenoxy stehen;

sowie Salze, N-Oxide und tautomere Formen der Verbindungen der Formel (I). Weiterhin ganz besonders bevorzugt sind Verbindungen der Formel (I) +

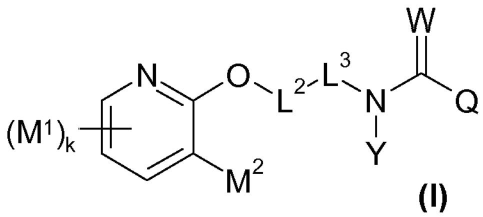

**(I)**

in welcher

Q für 2-Thienyl, 3-Fluor-2-thienyl, 3-Chlor-2-thienyl, 3,4-Dichlor-2-thienyl, 2,5-Dichlor-3-thienyl, 3,4,5-Trichlor-2-thienyl, 3-Brom-2-thienyl, 3-Iod-2-thienyl, 3-Cyano-2-thienyl, 3-Methyl-2-thienyl, 3-(Trifluormethyl)-2-thienyl, 3-Methoxy-2-thienyl, 3-Ethoxy-2-thienyl, 3-Thienyl, 2-Fluor-3-thienyl, 2-Chlor-3-thienyl, 2-Brom-3-thienyl, 2-Iod-3-thienyl, 2-Cyano-3-thienyl, 2-Methyl-3-thienyl, 2-(Trifluormethyl)-3-thienyl, 2-Methoxy-3-thienyl, 2-Ethoxy-3-thienyl, 2-Furanyl, 3-Fluor-2-furanyl, 3-Chlor-2-furanyl, 3-Brom-2-furanyl, 3-Iod-2-furanyl, 3-Cyano-2-furanyl, 3-Methyl-2-furanyl, 3-(Trifluormethyl)-2-furanyl, 3-Methoxy-2-furanyl, 3-Ethoxy-2-furanyl, 3-Furanyl, 2-Chlor-3-furanyl, 2-Brom-3-furanyl, 2-Iod-3-furanyl, 2-Cyano-3-furanyl, 2-Methyl-3-furanyl, 2-(Trifluormethyl)-3-furanyl, 2-Methoxy-3-furanyl, 2-Ethoxy-3-furanyl, 2-Methyl-phenyl, 2-Fluor-phenyl, 2,6-Difluor-phenyl, 2-Chlor-phenyl, 2,6-Dichlor-phenyl, 2-Bromphenyl, 2-Iod-phenyl, 2-(Difluormethyl)-phenyl, 2-(Trifluormethyl)-phenyl, 2-(Methylsulfanyl)-phenyl, 2-(Methylsulfonyl)-phenyl, 2-(Trifluormethoxy)-phenyl, 2-(Trifluormethylsulfanyl)-phenyl, 2-(Trifluormethylsulfonyl)-phenyl, 2-Nitrophenyl, 2-Chlor-3-pyridyl, 3-Chlor-2-pyridyl, 2-(Difluormethyl)-3-pyridyl,2-(Trifluormethyl)-3-pyridyl, 2-(Methylsulfanyl)-3-pyridyl, 2-(Methylsulfonyl)- 3-pyridyl, 2-(Trifluormethoxy)- 3-pyridyl, 2-(Trifluormethylsulfanyl)- 3-pyridyl oder 2-(Trifluormethylsulfonyl)- 3-pyridyl steht;

Y für Wasserstoff steht;

W für Sauerstoff steht;

$L^2$ für $C(R^{21}, R^{22})$ steht, wobei $R^{21}$ und $R^{22}$ jeweils unabhängig voneinander für Wasserstoff, Methyl, Cyclopropyl stehen;

$L^3$ für $C(R^{31}, R^{32})$ steht, wobei $R^{31}$ und $R^{32}$ jeweils unabhängig voneinander für Wasserstoff, Methyl, Ethyl, n-Propyl, iso-Propyl stehen, oder wobei $C(R^{31}, R^{32})$ für 1,1-Cyclopropyl steht;

k für 1 steht und

$M^1$ und $M^2$ jeweils unabhängig voneinander für Wasserstoff, Fluor, Brom, Chlor, Cyano, Trifluormethyl, Difluormethyl, oder Nitro stehen;

sowie Salze, N-Oxide und tautomere Formen der Verbindungen der Formel (I). Weiterhin beschrieben sind Verbindungen der Formel (I),

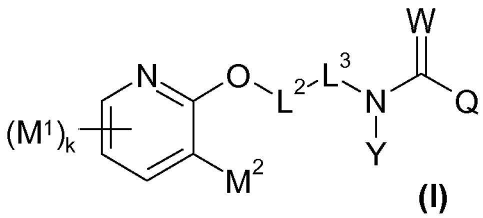

**(I)**

wobei

Q für folgende Strukturelemente steht, wobei n für jedes Q jeweils wie unten angegeben definiert ist:

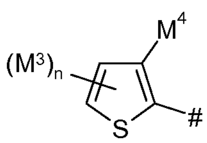

**Q¹**
n =1-2

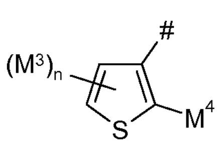

**Q²**
n = 1-2

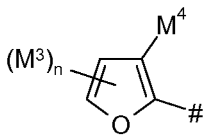

**Q³**
n = 1-2

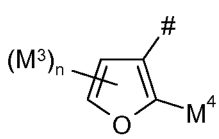

**Q⁴**
n = 1-2

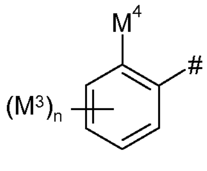

**$Q^9$**
n = 1-4

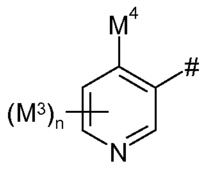

**$Q^{10}$**
n = 1-3

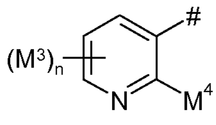

**$Q^{11}$**
n = 1-3

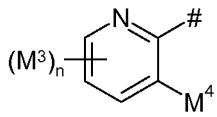

**$Q^{12}$**
n = 1-3

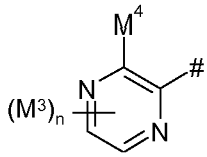

**$Q^{13}$**
n =1-2

Y für Wasserstoff oder für gegebenenfalls ein- oder mehrfach durch $M^2$ substituiertes ($C_1$-$C_6$)-Alkyl, ($C_2$-$C_6$)-Alkenyl, ($C_2$-$C_6$)-Alkinyl, ($C_1$-$C_6$)-Halogenalkyl, ($C_2$-$C_6$)-Halogenalkenyl, ($C_2$-$C_6$)-Halogenalkinyl, ($C_1$-$C_6$)-Alkoxy, ($C_2$-$C_6$)-Alkenyloxy, ($C_3$-$C_6$)-Alkinyloxy, ($C_3$-$C_{10}$)-Cycloalkyl-($C_1$-$C_6$)-alkyl oder für eine gegebenenfalls ein- oder mehrfach durch $M^2$ substituierte 3- bis 10-gliedrige cyclische Gruppe steht;

W für Sauerstoff steht;

$M^1$, $M^2$ und $M^3$, jeweils unabhängig voneinander für Wasserstoff, Halogen, Cyano, Nitro, OH, ($C_1$-$C_6$)-Alkyl, ($C_1$-$C_6$)-Halogenalkyl, ($C_1$-$C_6$)-Alkoxy, ($C_1$-$C_6$)-Halogenalkoxy, ($C_1$-$C_6$)-Alkylthio, ($C_1$-$C_6$)-Halogenalkylthio, ($C_1$-$C_6$)-Alkylsulfonyl, ($C_1$-$C_6$)-Halogenalkylsulfonyl, ($C_1$-$C_6$)-Alkylsulfanyl, ($C_1$-$C_6$)-Halogenalkylsulfanyl, ($C_3$-$C_{14}$)-Cycloalkyl-O-, ($C_3$-$C_{14}$)-Cycloalkenyl-O-, ($C_6$-$C_{14}$)-Aryl-O-, halogeniertes ($C_3$-$C_{14}$)-Cycloalkyl-O-, halogeniertes ($C_3$-$C_{14}$)-Cycloalkenyl-O-, halogeniertes ($C_6$-$C_{14}$)-Aryl)-O-stehen, wobei, falls Q $Q^{11}$ entspricht, $M^3$ nicht ($C_1$-$C_4$)-Halogenalkyl in 4-Position am Pyridyl ist;

$M^4$ für Wasserstoff, Halogen, Cyano, Nitro, OH, ($C_1$-$C_6$)-Alkyl, ($C_1$-$C_6$)-Halogenalkyl, ($C_1$-$C_6$)-Alkoxy, ($C_1$-$C_6$)-Halogenalkoxy, ($C_1$-$C_6$)-Alkylthio, ($C_1$-$C_6$)-Halogenalkylthio, ($C_1$-$C_6$)-Alkylsulfonyl, ($C_1$-$C_6$)-Halogenalkylsulfonyl, ($C_1$-$C_6$)-Alkylsulfanyl, ($C_1$-$C_6$)-Halogenalkylsulfanyl, ($C_3$-$C_{14}$)-Cycloalkyl-O-, ($C_3$-$C_{14}$)-Cycloalkenyl-O-, ($C_6$-$C_{14}$)-Aryl-O-, halogeniertes ($C_3$-$C_{14}$)-Cycloalkyl-O-, halogeniertes ($C_3$-$C_{14}$)-Cycloalkenyl-O-, halogeniertes ($C_6$-$C_{14}$)-Aryl)-O- steht, wobei, falls Q $Q^{10}$ entspricht, $M^4$ nicht ($C_1$-$C_4$)-Halogenalkyl ist;

$M^5$ jeweils unabhängig voneinander für Halogen, Formyl, Cyano, Nitro, ($C_1$-$C_6$)-Alkyl, ($C_1$-$C_6$)-Halogenalkyl, ($C_2$-$C_6$)-Alkenyl, ($C_2$-$C_6$)-Halogenalkenyl, ($C_2$-$C_6$)-Alkinyl, ($C_2$-$C_6$)-Halogenalkinyl, ($C_1$-$C_6$)-Alkoxy, ($C_1$-$C_6$)-Halogenalkoxy, ($C_{uG}$-$C_{oG}$)-Alkenyloxy, ($C_2$-$C_6$)-Halogenalkenoxy, ($C_3$-$C_6$)-Alkinyloxy, ($C_3$-$C_6$)-Halogenalkinoxy, ($C_1$-$C_6$)-Alkylthio, ($C_1$-$C_6$)-Halogenalkylthio, ($C_2$-$C_6$)-Alkenylthio, ($C_2$-$C_6$)-Halogenalkenylthio, ($C_3$-$C_6$)-Alkinylthio, ($C_3$-$C_6$)-Halogenalkinylthio, ($C_1$-$C_6$)-Alkylsulfonyl, ($C_1$-$C_6$)-Halogenalkylsulfonyl, ($C_2$-$C_6$)-Alkenylsulfonyl, ($C_2$-$C_6$)-Halogenalkenylsulfonyl, ($C_3$-$C_6$)-Alkinylsulfonyl, ($C_3$-$C_6$)-Halogenalkinylsulfonyl, ($C_1$-$C_6$)-Alkylsulfanyl, ($C_1$-$C_6$)-Halogenalkylsulfanyl, ($C_2$-$C_6$)-Alkenylsulfanyl, ($C_2$-$C_6$)-Halogenalkenylsulfanyl, ($C_3$-$C_6$)-Alkinylsulfanyl, ($C_3$-$C_6$)-Halogenalkinylsulfanyl, Formyl, ($C_1$-$C_6$)-Alkylcarbonyl, ($C_1$-$C_6$)-Halogenalkylcarbonyl, ($C_2$-$C_6$)-Alkenylcarbonyl, ($C_2$-$C_6$)-Halogenalkenylcarbonyl, ($C_2$-$C_6$)-Alkinylcarbonyl, ($C_2$-$C_6$)-Halogenalkinylcarbonyl, ($C_1$-$C_6$)-Alkoxycarbonyl, ($C_1$-$C_6$)-Halogenalkoxycarbonyl, ($C_2$-$C_6$)-Alkenoxycarbonyl, ($C_2$-$C_6$)-Halogenalkenoxycarbonyl, ($C_3$-$C_6$)-Alkinoxycarbonyl, ($C_3$-$C_6$)-Halogenalkinoxycarbonyl, ($C_1$-$C_6$)-Alkylcarbonyloxy, ($C_1$-$C_6$)-Halogenalkylcarbonyloxy, ($C_2$-$C_6$)-Alkenylcarbonyloxy, ($C_2$-$C_6$)-Halogenalkenylcarbonyloxy, ($C_2$-$C_6$)-Alkinylcarbonyloxy, ($C_2$-$C_6$)-Halogenalkinylcarbonyloxy oder ($C_3$-$C_{14}$)-Cycloalkyl steht.

k für 1 oder 2 steht;

$R^{21}$, $R^{22}$ für jeweils unabhängig voneinander Wasserstoff, Fluor oder gegebenenfalls ein- oder mehrfach durch $M^2$ substituiertes $(C_1-C_6)$-Alkyl, $(C_2-C_6)$-Alkenyl, $(C_2-C_6)$-Alkinyl, $(C_1-C_6)$-Halogenalkyl, $(C_2-C_6)$-Halogenalkenyl, $(C_2-C_6)$-Halogenalkinyl, $(C_1-C_6)$-Alkoxy, $(C_1-C_6)$-Halogenalkoxy, $(C_2-C_6)$-Alkenyloxy, $(C_3-C_6)$-Alkinyloxy, $(C_3-C_6)$-Cycloalkyl-$(C_1-C_6)$-alkyl stehen, oder für eine gegebenenfalls ein- oder mehrfach durch $M^2$ substituierte $(C_3-C_{14})$- carbocyclische Gruppe steht; oder

$R^{21}$, $R^{22}$ für $C(R^{21}, R^{22})$ als spiro-$C(CH_2-CH_2)$ steht;

$R^{31}$, $R^{32}$ für jeweils unabhängig voneinander Wasserstoff, Fluor oder gegebenenfalls ein- oder mehrfach durch $M^2$ substituiertes $(C_1-C_6)$-Alkyl, $(C_2-C_6)$-Alkenyl, $(C_2-C_6)$-Alkinyl, $(C_1-C_6)$-Halogenalkyl, $(C_2-C_6)$-Halogenalkenyl, $(C_2-C_6)$-Halogenalkinyl, $(C_1-C_6)$-Alkoxy, $(C_1-C_6)$-Halogenalkoxy, $(C_2-C_6)$-Alkenyloxy, $(C_3-C_6)$-Alkinyloxy, $(C_3-C_6)$-Cycloalkyl-$(C_1-C_6)$-alkyl stehen, oder für eine gegebenenfalls ein- oder mehrfach durch $M^2$ substituierte $(C_3-C_{14})$- carbocyclische Gruppe steht; oder

$R^{31}$, $R^{32}$ für $C(R^{31}, R^{32})$ als spiro-$C(CH_2-CH_2)$ steht;

$M^5$ jeweils unabhängig voneinander für Halogen, Formyl, Cyano, Nitro, $(C_1-C_6)$-Alkyl, $(C_1-C_6)$-Halogenalkyl, $(C_2-C_6)$-Alkenyl, $(C_2-C_6)$-Halogenalkenyl, $(C_2-C_6)$-Alkinyl, $(C_2-C_6)$-Halogenalkinyl, $(C_1-C_6)$-Alkoxy, $(C_1-C_6)$-Halogenalkoxy, $(C_{uG}-C_{oG})$-Alkenyloxy, $(C_2-C_6)$-Halogenalkenoxy, $(C_3-C_6)$-Alkinyloxy, $(C_3-C_6)$-Halogenalkinoxy, $(C_1-C_6)$-Alkylthio, $(C_1-C_6)$-Halogenalkylthio, $(C_2-C_6)$-Alkenylthio, $(C_2-C_6)$-Halogenalkenylthio, $(C_3-C_6)$-Alkinylthio, $(C_3-C_6)$-Halogenalkinylthio, $(C_1-C_6)$-Alkylsulfonyl, $(C_1-C_6)$-Halogenalkylsulfonyl, $(C_2-C_6)$-Alkenylsulfonyl, $(C_2-C_6)$-Halogenalkenylsulfonyl, $(C_3-C_6)$-Alkinylsulfonyl, $(C_3-C_6)$-Halogenalkinylsulfonyl, $(C_1-C_6)$-Alkylsulfanyl, $(C_1-C_6)$-Halogenalkylsulfanyl, $(C_2-C_6)$-Alkenylsulfanyl, $(C_2-C_6)$-Halogenalkenylsulfanyl, $(C_3-C_6)$-Alkinylsulfanyl, $(C_3-C_6)$-Halogenalkinylsulfanyl, Formyl, $(C_1-C_6)$-Alkylcarbonyl, $(C_1-C_6)$-Halogenalkylcarbonyl, $(C_2-C_6)$-Alkenylcarbonyl, $(C_2-C_6)$-Halogenalkenylcarbonyl, $(C_2-C_6)$-Alkinylcarbonyl, $(C_2-C_6)$-Halogenalkinylcarbonyl, $(C_1-C_6)$-Alkoxycarbonyl, $(C_1-C_6)$-Halogenalkoxycarbonyl, $(C_2-C_6)$-Alkenoxycarbonyl, $(C_2-C_6)$-Halogenalkenoxycarbonyl, $(C_3-C_6)$-Alkinoxycarbonyl, $(C_3-C_6)$-Halogenalkinoxycarbonyl, $(C_1-C_6)$-Alkylcarbonyloxy, $(C_1-C_6)$-Halogenalkylcarbonyloxy, $(C_2-C_6)$-Alkenylcarbonyloxy, $(C_2-C_6)$-Halogenalkenylcarbonyloxy, $(C_2-C_6)$-Alkinylcarbonyloxy, $(C_2-C_6)$-Halogenalkinylcarbonyloxy oder $(C_3-C_{14})$-Cycloalkyl steht.

Weiterhin beschrieben sind Verbindungen der Formel (I),

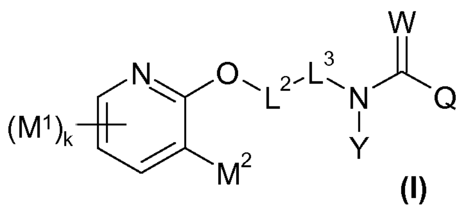

**(I)**

wobei

Q für folgende Strukturelemente steht, wobei n für jedes Q jeweils wie unten angegeben definiert ist:

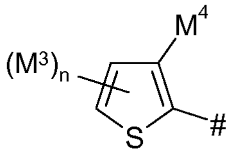

**Q¹**
n = 1

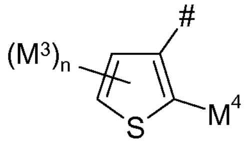

**Q²**
n = 1

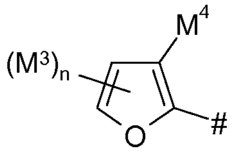

**Q³**
n = 1

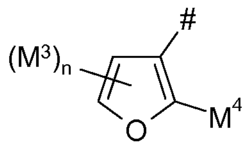

**Q⁴**
n = 0-1

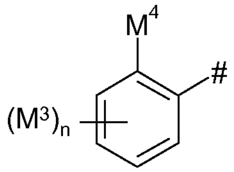

**$Q^9$**
n = 1-3

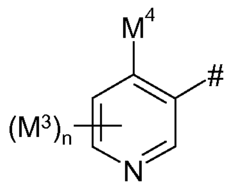

**$Q^{10}$**
n = 1-2

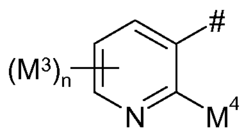

**$Q^{11}$**
n = 1-2

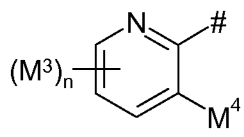

**$Q^{12}$**
n =1-2

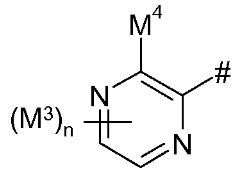

**$Q^{13}$**
n = 1

Y für Wasserstoff oder für gegebenenfalls ein- oder mehrfach durch $M^2$ substituiertes ($C_1$-$C_4$)-Alkyl, ($C_2$-$C_4$)-Alkenyl, ($C_3$-$C_4$)-Alkinyl, ($C_1$-$C_4$)-Halogenalkyl, ($C_2$-$C_4$)-Halogenalkenyl, ($C_3$-$C_4$)-Halogenalkinyl, ($C_1$-$C_4$)-Alkoxy, ($C_2$-$C_4$)-Alkenyloxy, ($C_3$-$C_4$)-Alkinyloxy, ($C_3$-$C_6$)-Cycloalkyl-($C_1$-$C_4$)-alkyl oder für eine gegebenenfalls ein- oder mehrfach durch $M^2$ substituierte $C_3$- bis $C_6$-gliedrige carbocyclische Gruppe steht;

$M^1$, $M^2$ und $M^3$ jeweils unabhängig voneinander für Wasserstoff, Halogen, Cyano, Nitro, OH, ($C_1$-$C_4$)-Alkyl, ($C_1$-$C_4$)-Halogenalkyl, ($C_1$-$C_4$)-Alkoxy, ($C_1$-$C_4$)-Halogenalkoxy, ($C_1$-$C_4$)-Alkylthio, ($C_1$-$C_4$)-Halogenalkylthio, ($C_1$-$C_4$)-Alkylsulfonyl, ($C_1$-$C_4$)-Halogenalkylsulfonyl, ($C_1$-$C_4$)-Alkylsulfanyl, ($C_1$-$C_6$)-Halogenalkylsulfanyl, ($C_3$-$C_{14}$)-Cycloalkyl-O-, ($C_3$-$C_{14}$)-Cycloalkenyl-O-, ($C_6$-$C_{14}$)-Aryl-O-, halogeniertes ($C_3$-$C_{14}$)-Cycloalkyl-O-, halogeniertes ($C_3$-$C_{14}$)-Cycloalkenyl-O-, halogeniertes ($C_6$-$C_{14}$)-Aryl)-O- stehen, wobei, falls Q $Q^{11}$ entspricht, $M^3$ nicht ($C_1$-$C_4$)-Halogenalkyl in 4-Position am Pyridyl ist;

$M^4$ für Wasserstoff, Halogen, Cyano, Nitro, OH, ($C_1$-$C_4$)-Alkyl, ($C_1$-$C_4$)-Halogenalkyl, ($C_1$-$C_4$)-Alkoxy, ($C_1$-$C_4$)-Halogenalkoxy, ($C_1$-$C_4$)-Alkylthio, ($C_1$-$C_4$)-Halogenalkylthio, ($C_1$-$C_4$)-Alkylsulfonyl, ($C_1$-$C_4$)-Halogenalkylsulfonyl, ($C_1$-$C_4$)-Alkylsulfanyl, ($C_1$-$C_6$)-Halogenalkylsulfanyl, ($C_3$-$C_{14}$)-Cycloalkyl-O-, ($C_3$-$C_{14}$)-Cycloalkenyl-O-, ($C_6$-$C_{14}$)-Aryl-O-, halogeniertes ($C_3$-$C_{14}$)-Cycloalkyl-O-, halogeniertes ($C_3$-$C_{14}$)-Cycloalkenyl-O-, halogeniertes ($C_6$-$C_{14}$)-Aryl)-O- steht, wobei, falls Q $Q^{10}$ entspricht, $M^4$ nicht ($C_1$-$C_4$)-Halogenalkyl ist;

k für 1 steht;

$R^{21}$, $R^{22}$ für jeweils unabhängig voneinander Wasserstoff, Fluor oder gegebenenfalls ein- oder mehrfach durch $M^2$ substituiertes ($C_1$-$C_4$)-Alkyl, ($C_2$-$C_4$)-Alkenyl, ($C_2$-$C_4$)-Alkinyl, ($C_1$-$C_4$)-Halogenalkyl, ($C_2$-$C_4$)-Halogenalkenyl, ($C_2$-$C_4$)-Halogenalkinyl, ($C_1$-$C_6$)-Alkoxy, ($C_1$-$C_4$)-Halogenalkoxy, ($C_2$-$C_4$)-Alkenyloxy, ($C_3$-$C_4$)-Alkinyloxy, ($C_3$-$C_4$)-Cycloalkyl-($C_1$-$C_4$)-alkyl, ($C_3$-$C_8$)-Cycloalkyl oder halogeniertes ($C_3$-$C_8$)-Cycloalkyl steht;

$R^{31}$, $R^{32}$ bevorzugt für jeweils unabhängig voneinander Wasserstoff, Fluor oder gegebenenfalls ein- oder mehrfach durch $M^2$ substituiertes ($C_1$-$C_4$)-Alkyl, ($C_2$-$C_4$)-Alkenyl, ($C_2$-$C_4$)-Alkinyl, ($C_1$-$C_4$)-Halogenalkyl, ($C_2$-$C_4$)-Halogenalkenyl, ($C_2$-$C_4$)-Halogenalkinyl, ($C_1$-$C_6$)-Alkoxy, ($C_1$-$C_4$)-Halogenalkoxy, ($C_2$-$C_4$)-Alkenyloxy, ($C_3$-$C_4$)-Alkinyloxy, ($C_3$-$C_4$)-Cycloalkyl-($C_1$-$C_4$)-alkyl oder für gegebenenfalls ein- oder mehrfach durch $M^2$ substituiertes ($C_3$-$C_8$)-Cycloalkyl oder halogeniertes ($C_3$-$C_8$)-Cycloalkyl steht;

$M^5$ jeweils unabhängig voneinander für Halogen, Formyl, Cyano, Nitro, ($C_1$-$C_4$)-Alkyl, ($C_1$-$C_4$)-Halogenal-

kyl, ($C_2$-$C_4$)-Alkenyl, ($C_2$-$C_4$)-Halogenalkenyl, ($C_2$-$C_4$)-Alkinyl, ($C_2$-$C_4$)-Halogenalkinyl, ($C_1$-$C_4$)-Alkoxy, ($C_1$-$C_4$)-Halogenalkoxy, ($C_2$-$C_4$)-Alkenyloxy, ($C_2$-$C_4$)-Halogenalkenoxy, ($C_3$-$C_4$)-Alkinyloxy, ($C_3$-$C_4$)-Halogenalkinoxy, ($C_1$-$C_4$)-Alkylthio, ($C_1$-$C_4$)-Halogenalkylthio, ($C_2$-$C_4$)-Alkenylthio, ($C_2$-$C_4$)-Halogenalkenylthio, ($C_3$-$C_4$)-Alkinylthio, ($C_3$-$C_4$)-Halogenalkinylthio, ($C_1$-$C_4$)-Alkylsulfonyl, ($C_1$-$C_4$)-Halogenalkylsulfonyl, ($C_2$-$C_4$)-Alkenylsulfonyl, ($C_2$-$C_4$)-Halogenalkenylsulfonyl, ($C_3$-$C_4$)-Alkinylsulfonyl, ($C_3$-$C_4$)-Halogenalkinylsulfonyl, ($C_1$-$C_4$)-Alkylsulfanyl, ($C_1$-$C_4$)-Halogenalkylsulfanyl, ($C_2$-$C_4$)-Alkenylsulfanyl, ($C_2$-$C_4$)-Halogenalkenylsulfanyl, ($C_3$-$C_4$)-Alkinylsulfanyl, ($C_3$-$C_4$)-Halogenalkinylsulfanyl, ($C_1$-$C_4$)-Alkylcarbonyl, ($C_1$-$C_4$)-Halogenalkylcarbonyl, ($C_2$-$C_4$)-Alkenylcarbonyl, ($C_2$-$C_4$)-Halogenalkenylcarbonyl, ($C_2$-$C_4$)-Alkinylcarbonyl, ($C_2$-$C_4$)-Halogenalkinylcarbonyl, ($C_1$-$C_4$)-Alkoxycarbonyl, ($C_1$-$C_4$)-Halogenalkoxycarbonyl, ($C_2$-$C_4$)-Alkenoxycarbonyl, ($C_2$-$C_4$)-Halogenalkenoxycarbonyl, ($C_3$-$C_4$)-Alkinoxycarbonyl, ($C_3$-$C_4$)-Halogenalkinoxycarbonyl, ($C_1$-$C_4$)-Alkylcarbonyloxy, ($C_1$-$C_4$)-Halogenalkylcarbonyloxy, ($C_2$-$C_4$)-Alkenylcarbonyloxy, ($C_2$-$C_4$)-Halogenalkenylcarbonyloxy, ($C_2$-$C_4$)-Alkinylcarbonyloxy, ($C_2$-$C_4$)-Halogenalkinylcarbonyloxy oder ($C_3$-$C_6$)-Cycloalkyl steht.

## Darstellungsverfahren A

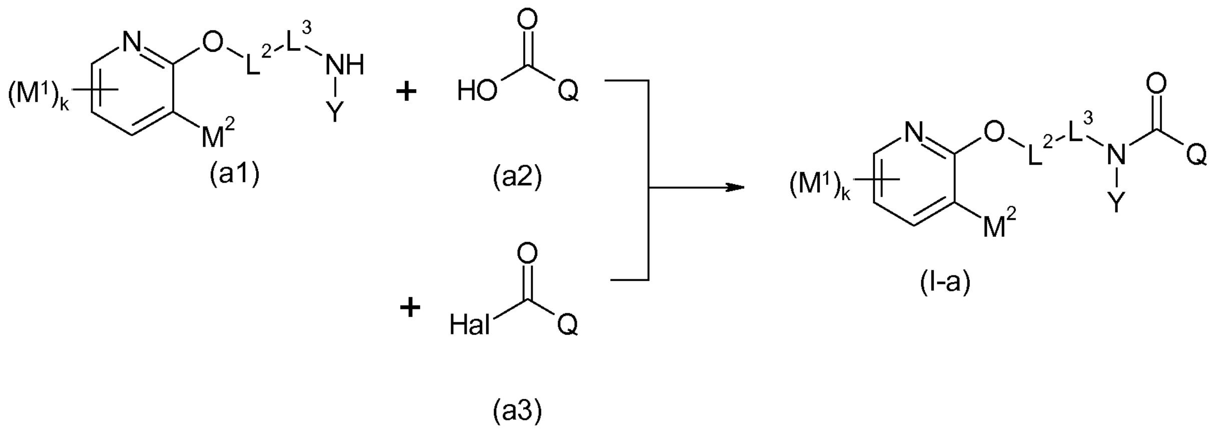

Die Reste $M^1$, $M^2$, $L^2$, $L^3$, Q und Y sowie k haben die oben beschriebenen Bedeutungen. W steht in diesem Fall für Sauerstoff.

Erfindungsgemäße Verbindungen der allgemeinen Formel (I-a) können ausgehend von Aminen gemäß Formel (a1) und Carbonsäuren gemäß Formel (a2) bzw. deren Halogenide gemäß Formel (a3) nach hinlänglich bekannten Verfahren dargestellt werden, beispielsweise wie in WO-A 2009/012998 beschrieben. Die Amine gemäß Formel (a1) und Carbonsäuren gemäß Formel (a2) sowie deren Halogenide gemäß Formel (a3) sind kommerziell verfügbar. Alternativ lassen sich die Halogenide gemäß Formel (a3) nach hinlänglich bekannten Methoden aus den Carbonsäuren gemäß Formel (a2) mit entsprechenden Halogenierungsreagenzien herstellen, beispielsweise mit Phosphorylchlorid, Phosphorylbromid, Thionylchlorid, Oxalylchlorid oder Phosgen.

Die erfindungsgemäßen Verbindungen der allgemeinen Formel (I-a) werden beim Einsatz von Carbonsäurehalogeniden der allgemeinen Struktur (a3) bevorzugt in Gegenwart eines Reaktionshilfsmittels hergestellt. Als solche kommen alle üblichen anorganischen oder organischen Basen in Frage. Hierzu gehören beispielsweise Erdalkali- oder Alkalimetallhydride, -hydroxide, -amide, alkoholate, -acetate, -carbonate oder -hydrogencarbonate, wie beispielsweise Natriumhydrid, Natriumamid, Natriummethylat, Natriumethylat, Kalium-t.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, oder Ammoniumcarbonat sowie tertiäre Amine, wie beispielsweise Trimethylamin, Triethylamin, Diisopropylethylamin, Tributylamin, N,N-Dimethylanilin, Pyridin, N-Methyl-piperidin, 4-(N,N-Dimethylamino)-pyridin, Diazabicyclooctan (DABCO), Diazabicyclononen (DBN) oder Diazabicycloundecen (DBU), insbesondere Triethylamin.

Die erfindungsgemäßen Verbindungen der allgemeinen Formel (I-a) werden beim Einsatz von Carbonsäuren der allgemeinen Struktur (a2) in Gegenwart eines Kondensationsmittels hergestellt. Die Carbonsäuren sind kommerziell verfügbar. Als Kondensationsmittel sind vor allem wasserentziehende Chemikalien geeignet. Hierzu gehören vorzugsweise Säureanhydride und Säurehalogenide, wie beispielsweise Acetanhydrid, Propionsäureanhydrid, Phosphor-(V)-oxid, Phosphorylchlorid, Phosphorylbromid, Phosportrichlorid, Phosphortribromid, Thionylchlorid, Oxalylchlorid, Phosgen, Diphosgen, Ameisensäuremethylester, Ameisensäureethylester, sowie Carbodiimide, wie beispielsweise

N,N'-Dicyclohexylcarbodiimid (DCC) oder 1-Ethyl-3-(3-dimethylaminopropyl)carbodiimid Hydrochlorid (EDC-HCl). Weitere bekannte Kondensationsreagenzien sind Triphenylphosphin/Tetrachlormethan, 4-(4,6-Dimethoxy[1.3.5]triazin-2-yl)-4-methyl-morpholiniumchlorid-Hydrat oder Hydroxybenzotriazol (HOBt). Insbesondere die Kombination von 1-Ethyl-3-(3-dimethylaminopropyl)carbodiimid Hydrochlorid (EDC-HCl) und Hydroxybenzotriazol (HOBt) sei hier aufgeführt.

Die erfindungsgemäßen Verbindungen der allgemeinen Formel (I-a) werden gegebenenfalls unter Einsatz eines oder mehrerer Verdünnungsmittel hergestellt. Als Verdünnungsmittel kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan, Tetrahydrofuran, Dioxan, Acetonitril oder Dimethylformamid.

Die Reaktionstemperaturen können bei der Durchführung des Verfahrens A-1 in einem größeren Bereich variiert werden. Im Allgemeinen arbeitet man bei Temperaturen zwischen 0 °C und 150 °C, vorzugsweise zwischen 10 °C und 120 °C.

Das Verfahren A wird im Allgemeinen unter Normaldruck durchgeführt. Es ist aber auch möglich, das Verfahren A unter erhöhtem oder verminderten Druck - im Allgemeinen zwischen 0,1 bar und 10 bar - durchzuführen.

## Darstellungsverfahren B

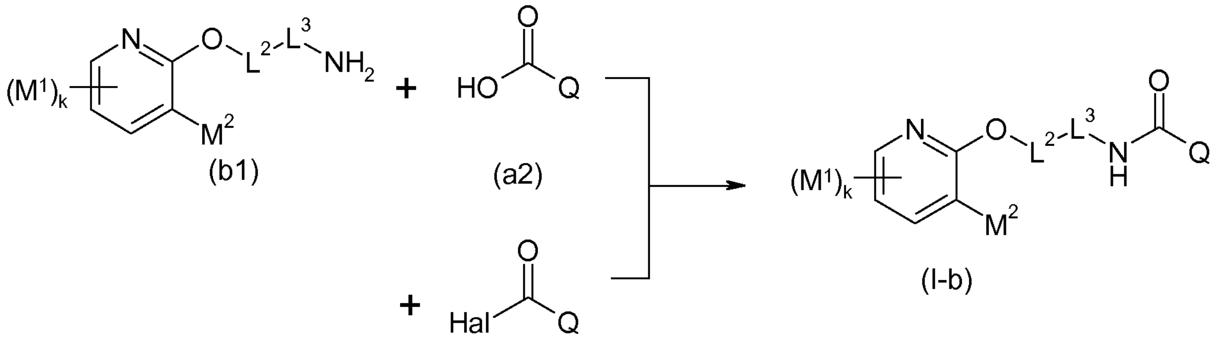

(a3)

Die Reste $M^1$, $M^2$, $L^2$, $L^3$ und Q sowie k haben die oben beschriebenen Bedeutungen. W steht in diesem Fall für Sauerstoff.

Erfindungsgemäße Verbindungen der allgemeinen Formel (I-a) können ausgehend von Aminen gemäß Formel (b1) und Carbonsäuren gemäß Formel (a2) bzw. deren Halogenide gemäß Formel (a3) nach hinlänglich bekannten Verfahren dargestellt werden, beispielsweise wie in WO-A 2009/012998 beschrieben. Die Amine gemäß Formel (b1) und Carbonsäuren gemäß Formel (a2) sowie deren Halogenide gemäß Formel (a3) sind kommerziell verfügbar. Alternativ lassen sich die Halogenide gemäß Formel (a3) nach hinlänglich bekannten Methoden aus den Carbonsäuren gemäß Formel (a2) mit entsprechenden Halogenierungsreagenzien herstellen, beispielsweise mit Phosphorylchlorid, Phosphorylbromid, Thionylchlorid, Oxalylchlorid oder Phosgen.

## Darstellungsverfahren C

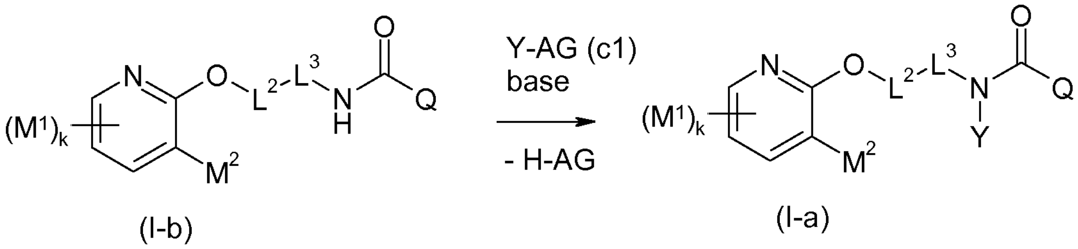

Die Reste $M^1$, $M^2$, $L^2$, $L^3$, Q und Y sowie k haben die oben beschriebenen Bedeutungen. W steht in diesem Fall für Sauerstoff und AG für Abgangsgruppe, beispielsweise Halogene oder Alkyl- bzw. Arylsulfonate wie zum Beispiel Tolylsulfonate oder Benzolsulfonate.

Erfindungsgemäße Verbindungen der allgemeinen Formel (I) bzw. deren Ausführungsform (I-a) können ausgehend von Amiden gemäß Formel (I-b) und Alkylierungsmitteln gemäß Formel (c1) nach hinlänglich bekannten Verfahren dargestellt werden, beispielsweise wie in EP2007060166 beschrieben.

## Darstellungsverfahren D

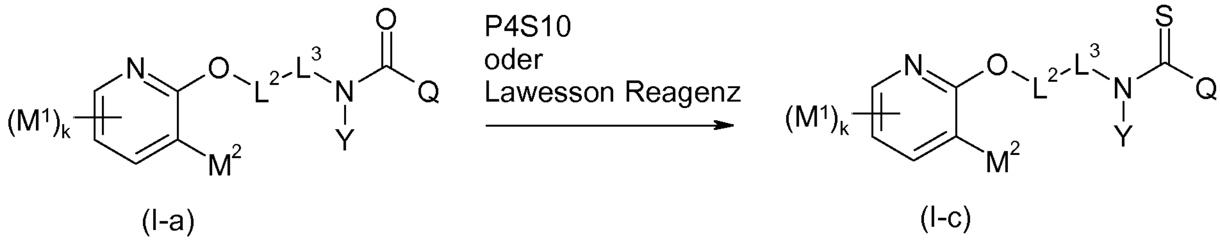


(I-a) (I-c)

Die Reste $M^1$, $M^2$, $L^2$, $L^3$, Q und Y sowie k haben die oben beschriebenen Bedeutungen. W = Sauerstoff wird direkt zu W = Schwefel transformiert.

Erfindungsgemäße Verbindungen der allgemeinen Formel (I) bzw. deren Ausführungsform (I-c) können ausgehend von Verbindungen der Formel (I-a) und geeigneten Schwefelungsreagenzien, z.B. Tetraphosphordecasulfid ("Phosphorpentasulfid") oder 2,4-Bis-[4-methoxyphenyl]-2,4-dithiono-1,2,3,4-dithiadiphosphetan ("Lawesson Reagenz"), nach hinlänglich bekannten Verfahren hergestellt werden. Verfahrensbeispiele sind u.a. bekannt aus Houben-Weyl, Methoden der Organischen Chemie, E5, 1255 (Thieme Verlag, Stuttgart, 1985).

Ein weiterer Aspekt der vorliegenden Erfindung ist ein Verfahren zum Bekämpfen von endoparasitären Schädlingen oder Nematoden das dadurch gekennzeichnet ist, dass man eine erfindungsgemäße Verbindung der Formel (I) oder (I-a) - (I-c) sowie deren Salze, N-Oxide und tautomere Formen auf die Schädlinge und/oder ihren Lebensraum einwirken lässt.

## Anthelmintische Anwendungsgebiete

Die erfindungsgemäßen Mittel eignen sich bei günstiger Warmblütertoxizität zur Bekämpfung von pathogenen Endoparasiten, die bei Menschen und in der Tierhaltung und Tierzucht bei Nutz-, Zucht-, Zoo-, Labor-, Versuchs- und Hobbytieren vorkommen. Sie können gegen alle oder einzelne Entwicklungsstadien der Schädlinge sowie gegen resistente und normal sensible Endoparasiten-Isolate eingesetzt werden. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so dass durch den Einsatz der Wirkstoffe eine wirtschaftlichere, einfachere und gesündere Tierhaltung möglich ist. Zu den pathogenen Endoparasiten zählen Helminthen wie Platyhelmintha (insbesondere Monogenea, Cestoden und Trematoden), Nematoden, Pentastoma und Acanthocephala. Als Beispiele seien genannt:

**Monogenea:** z.B.: Gyrodactylus spp., Dactylogyrus spp., Polystoma spp..

**Cestoden:** Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllida z.B.: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diochis spp., Dipylidium spp., Joyeuxiella spp., Diplopylidium spp..

**Trematoden:** Aus der Klasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp.,

Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp..

**Acanthocephala:** aus der Ordnung der Oligacanthorhynchida z.B.: *Macracanthorhynchus* spp., *Prosthenorchis* spp.; aus der Ordnung der Polymorphida zum Beispiel: *Filicollis* spp.; aus der Ordnung der Moniliformida zum Beispiel: *Moniliformis* spp.,

Aus der Ordnung der Echinorhynchida zum Beispiel *Acanthocephalus* spp., *Echinorhynchus* spp., *Leptorhynchoides* spp.

**Pentastoma:** aus der Ordnung der Porocephalida zum Beispiel *Linguatula* spp.

**Nematoden:** Aus der Ordnung der Trichinellida z.B.: Trichuris spp., Capillaria spp., Trichomosoides spp., Trichinella spp..

Aus der Ordnung der Tylenchida z.B.: Micronema spp., Strongyloides spp..

Aus der Ordnung der Rhabditina z.B.: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp.

Aus der Ordnung der Spirurida z.B.: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp.

**Acantocephala:** Aus der Ordnung der Oligacanthorhynchida z.B: Macracanthorhynchus spp., Prosthenorchis spp.; aus der Ordnung der Polymorphida z.B.: Filicollis spp.; aus der Ordnung der Moniliformida z.B.: Moniliformis spp.,

Aus der Ordnung der Echinorhynchida z.B. Acanthocephalus spp., Echinorhynchus spp., Leptorhynchoides spp.

**Pentastoma:** Aus der Ordnung der Porocephalida z.B. Linguatula spp.

Gemäß einer bevorzugten Ausfuhrungsform werden die Verbindungen der Formel (I) zur Bekämpfung von Nematoden eingesetzt. Als Nematoden seien besonders bevorzugt genannt: Trichinellida, Tylenchida, Rhabditina oder die folgenden aus der Ordnung der Spirurida: Gnathostoma spp., Physaloptera spp., Thelazia spp., Gongylonema spp., Habronema spp., Parabronema spp., Draschia spp., Dracunculus spp ..

Gegenstand einer weiteren besonders bevorzugten Ausführungsform ist die Verwendung zur Bekämpfung von Strongylida, insbesondere Haemonchus spp. (z. B. Haemonchus contortus), Trichostrongylus spp. (z. B. Trichostrongylus colubriformis), Cooperia spp., und Ostertagia spp oder Teladorsagia spp..

Gegenstand einer weiteren besonders bevorzugten Ausführungsform ist die Verwendung zur Bekämpfung von Ascaridida wie z. B. Parascaris spp.

Tiere können Fische, Reptilien, Vögel oder insbesondere Säugetiere sein.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Strauße, Fische wie Forellen, Lachse, Karpfen, Barsche, Hechte, Aale.

Zu Labor- und Versuchstieren gehören Mäuse, Ratten, Meerschweinchen, Goldhamster, Hunde und Katzen.

Zu den Hobbytieren gehören Hunde und Katzen.

Erfindungsgemäß bevorzugt ist die Anwendung bei Tieren, aber grundsätzlich kommt auch die Anwendung am Menschen in Frage. Vorzugsweise werden beim Menschen Ascaris spp., Ancylostoma spp, Necator spp., Trichuris spp., Strongyloides spp. und Enterobius spp. bekämpft.

Unter den Säugetieren werden gemäß einer Ausführungsform für die erfindungsgemäße Anwendung die Pflanzenfresser (Herbivoren) bevorzugt, also Tiere, die sich hauptsächlich von Pflanzen ernähren. Besonders bevorzugt ist die Behandlung von Wiederkäuern (wie z. B. Schafe, Ziegen, Rinder).

Als nicht wiederkäuende Pflanzenfresser, die Säugetiere sind, seien als bevorzugtes Beispiel die Pferde genannt. Dort können die obengenannten Kombinationen bevorzugt z. B. zur Bekämpfung von Strongylida oder insbesondere von Spulwürmern (Ascaridida), wie z. B. Parascaris equorum, eingesetzt werden.

Bei den Wiederkäuern können bevorzugt Strongylida, insbesondere Haemonchus spp., Trichostrongylus spp., Cooperia spp. und Ostertagia spp. bekämpft werden.

Besonders bevorzugt werden erfindungsgemäß Schafe behandelt.

Ebenfalls besonders bevorzugt werden erfindungsgemäß Rinder behandelt.

Die Anwendung der erfindungsgemäßen Wirkstoffe erfolgt im Veterinärsektor und bei der Tierhaltung in an

sich bekannter Weise direkt oder in Form von geeigneten Zubereitungen. Die Anwendung kann sowohl prophylaktisch als auch therapeutisch erfolgen.

Die Verbindungen der Formel (I) wie oben definiert koennen als Arzneimittel zur Bekämpfung von Endoparasiten bei Tieren oder Menschen verwendet werden.

Die Verbindungen der Formel (I) wie oben definiert koennen zur Herstellung eines Arzneimittels zur Bekämpfung von Endoparasiten bei Tieren oder Menschen verwendet werden.

Die Verbindungen der Formel (I) wie oben definiert koennen bei der Bekämpfung von Endoparasiten bei Tieren verwendet werden, indem die Verbindungen sowie deren Salze, N-Oxide und tautomere Formen Tieren oder Menschen prophylaktisch oder therapeutisch verabreicht werden

Es werden endoparasitizide Mittel beansprucht, die Verbindungen der Formel (I) wie oben definiert enthalten.

Es werden endoparasitizide Zusammensetzungen beschrieben, die eine oder mehrere Verbindungen der Formel (I) wie oben definiert sowie ein oder mehrere pharmazeutisch akzeptable Hilfsstoffe enthalten.

Es wird eine Methode zur Behandlung von Endoparasiten in Menschen und Tieren beschrieben, indem eine Verbindung der Formel (I) wie oben beschrieben oder eine pharmazeutische Zusammensetzung diese Verbindungen enthaltend verabreicht wird.

Es wird eine Methode zur Behandlung von Endoparasiten in Tieren beschrieben, indem eine Verbindung der Formel (I) wie oben beschrieben oder eine pharmazeutische Zusammensetzung diese Verbindungen enthaltend verabreicht wird.

Es wird eine Methode zur Behandlung von Endoparasiten in Rindern oder Schafen beschrieben, indem eine Verbindung der Formel (I) wie oben beschrieben oder eine pharmazeutische Zusammensetzung diese Verbindungen enthaltend verabreicht wird.

**Nematizide Anwendungsgebiete:**

Der Begriff "Nematizide" umfasst im vorliegenden Zusammenhang Substanzen, die geeignet sind, Nematoden, die im Boden und in Pflanzen- oder Pflanzenteilen leben und diese schädigen, zu bekämpfen.

Der Begriff "Nematoden" umfasst im vorliegenden Zusammenhang alle Arten der Ordnung Nematoda und insbesondere Arten, die Gesundheitsprobleme für Pflanzen oder für Pilze (zum Beispiel Arten der Ordnung *Aphelenchida,* Meloidogyne, *Tylenchida* und andere) oder für Menschen und Tiere (zum Beispiel Arten der Ordnungen Trichinellida, Tylenchida, Rhabditina und Spirurida) verursachen, sowie andere parasitäre Helminthen.

Der Begriff "phytopathogene Nematoden" bezieht sich im vorliegenden Zusammenhang auf Pflanzennematoden, worunter man pflanzenparasitäre Nematoden versteht, die Pflanzen schädigen. Pflanzennematoden umfassen pflanzenparasitäre Nematoden und im Boden lebende Nematoden. Zu den pflanzenparasitären Nematoden zählen, jedoch ohne Einschränkung, Ektoparasiten wie *Xiphinema spp., Longidorus spp.* und *Trichodorus spp.;* Halbparasiten wie *Tylenchulus spp.;* migratorische Endoparasiten wie *Pratylenchus spp., Radopholus spp.* und *Scutellonerna spp.;* ortsgebundene Parasiten wie *Heterodera spp., Globoderal spp.* und *Meloidogyne spp.,* sowie Stängel- und Blattendoparasiten wie *Ditylenchus spp., Aphelenchoides spp.* und *Hirshmaniella spp.*. Besonders schädliche wurzelparasitäre Bodennematoden sind zum Beispiel zystenbildende Nematoden der Gattungen *Heterodera* oder *Globodera,* und/oder Wurzelgallennematoden der Gattung *Meloidogyne.* Schädliche Arten dieser Gattungen sind zum Beispiel *Meloidogyne incognita, Heterodera glycines* (Sojabohnenzystennematode), *Globodera pallida* und *Globodera rostochiensis* (Kartoffelzystennematode), wobei diese Arten wirksam mit dem im vorliegenden Text beschriebenen Verbindungen bekämpft werden. Die Verwendung der im vorliegenden Text beschriebenen Verbindungen ist jedoch keineswegs auf diese Gattungen oder Arten beschränkt, sondern erstreckt sich in gleicher Weise auch auf andere Nematoden.

Zu den phytogenen Nematoden zählen, jedoch nicht ausschließlich, z.B. *Aglenchus agricola, Anguina tritici, Aphelenchoides arachidis, Aphelenchoides fragaria* und die Stängel- und Blattendoparasiten *Aphelenchoides spp.* im Allgemeinen, *Belonolaimus gracilis, Belonolaimus longicaudatus, Belonolaimus nortoni, Bursaphelenchus eremus, Bursaphelenchus xylophilus* und *Bursaphelenchus spp.* im Allgemeinen, *Cacopaurus pestis, Criconemella curvata, Criconemella onoensis, Criconemella ornata, Criconemella rusium, Criconemella xenoplax* (= *Mesocriconema xenoplax)* und *Criconemella spp.* im Allgemeinen, *Criconemoides ferniae, Criconemoides onoense, Criconemoides ornatum* und *Criconemoides spp.* im Allgemeinen, *Ditylenchus destructor, Ditylenchus dipsaci, Ditylenchus myceliophagus* sowie die Stängel- und Blattendoparasiten *Ditylenchus spp.* im Allgemeinen, *Dolichodorus heterocephalus, Globodera pallida (=Heterodera pallida), Globodera rostochiensis* (Kartoffelzystennematode), *Globodera solanacearum, Globodera tabacum, Globodera virginia* und die ortsgebundenen zystenbildenden Parasiten *Globodera spp.* im Allgemeinen, *Helicotylenchus digonicus, Helicotylenchus dihystera, Helicotylenchus erythrine, Helicotylenchus multicinctus, Helicotylenchus nannus, Helicotylenchus pseudorobustus* und *Helicotylenchus spp.* , im Allgemeinen, *Hemicriconemoides, Hemicycliophora arenaria, Hemicycliophora nudata, Hemicycliophora parvana, Heterodera avenae, Heterodera cruciferae, Heterodera glycines* (Sojabohnenzysten- nematode), *Heterodera oryzae, Heterodera schachtii, Heterodera zeae* und die ortsgebundenen zystenbildenden Parasiten *Heterodera spp.* im Allgemeinen, *Hirschmaniella gracilis, Hirschmaniella*

oryzae, *Hirschmaniella spinicaudata* und die Stängel- und Blattendoparasiten *Hirschmaniella spp.* im Allgemeinen, *Hoplolaimus aegyptii, Hoplolaimus californicus, Hoplolaimus columbus, Hoplolaimus galeatus, Hoplolaimus indicus, Hoplolaimus magnistylus, Hoplolaimus pararobustus, Longidorus africanus, Longidorus breviannulatus, Longidorus elongatus, Longidorus laevicapitatus, Longidorus vineacola* und die Ektoparasiten *Longidorus spp.* im Allgemeinen, *Meloidogyne acronea, Meloidogyne africana, Meloidogyne arenaria, Meloidogyne arenaria thamesi, Meloidogyne artiella, Meloidogyne chitwoodi, Meloidogyne coffeicola, Meloidogyne ethiopica, Meloidogyne exigua, Meloidogyne graminicola, Meloidogyne graminis, Meloidogyne hapla, Meloidogyne incognita, Meloidogyne incognita acrita, Meloidogyne javanica, Meloidogyne kikuyensis, Meloidogyne naasi, Meloidogyne paranaensis, Meloidogyne thamesi* und die ortsgebundenen Parasiten *Meloidogyne spp.* im Allgemeinen, *Meloinema spp., Nacobbus aberrans, Neotylenchus vigissi, Paraphelenchus pseudoparietinus, Paratrichodorus allius, Paratrichodorus lobatus, Paratrichodorus minor, Paratrichodorus nanus, Paratrichodorus porosus, Paratrichodorus teres* und *Paratrichodorus spp.* im Allgemeinen, *Paratylenchus hamatus, Paratylenchus minutus, Paratylenchus projectus* und *Paratylenchus spp.* im Allgemeinen, *Pratylenchus agilis, Pratylenchus alleni, Pratylenchus andinus, Pratylenchus brachyurus, Pratylenchus cerealis, Pratylenchus coffeae, Pratylenchus crenatus, Pratylenchus delattrei, Pratylenchus giibbicaudatus, Pratylenchus goodeyi, Pratylenchus hamatus, Pratylenchus hexincisus, Pratylenchus loosi, Pratylenchus neglectus, Pratylenchus penetrans, Pratylenchus pratensis, Pratylenchus scribneri, Pratylenchus teres, Pratylenchus thornei, Pratylenchus vulnus, Pratylenchus zeae* und die migratorischen Endoparasiten *Pratylenchus spp.* im Allgemeinen, *Pseudohalenchus minutus, Psilenchus magnidens, Psilenchus tumidus, Punctodera chalcoensis, Quinisulcius acutus, Radopholus citrophilus, Radopholus similis,* die migratorischen Endoparasiten *Radopholus spp.* im Allgemeinen, *Rotylenchulus borealis, Rotylenchulus parvus, Rotylenchulus reniformis* und *Rotylenchulus spp.* im Allgemeinen, *Rotylenchus laurentinus, Rotylenchus macrodoratus, Rotylenchus robustus, Rotylenchus uniformis* und *Rotylenchus spp.* im Allgemeinen, *Scutellonema brachyurum, Scutellonema bradys, Scutellonema clathricaudatum* und die migratorischen Endoparasiten *Scutellonema spp.* im Allgemeinen, *Subanguina radiciola, Tetylenchus nicotianae, Trichodorus cylindricus, Trichodorus minor, Trichodorus primitivus, Trichodorus proximus, Trichodorus similis, Trichodorus sparsus* und *the ectoparasites Trichodorus spp.* im Allgemeinen, *Tylenchorhynchus agri, Tylenchorhynchus brassicae, Tylenchorhynchus clarus, Tylenchorhynchus claytoni, Tylenchorhynchus digitatus, Tylenchorhynchus ebriensis, Tylenchorhynchus maximus, Tylenchorhynchus nudus, Tylenchorhynchus vulgaris und Tylenchorhynchus spp.* im Allgemeinen, *Tylenchulus semipenetrans* und die Semiparasiten *Tylenchulus spp.* im Allgemeinen, *Xiphinema americanum, Xiphinema brevicolle, Xiphinema dimorphicaudatum, Xiphinema index* und die Ektoparasiten *Xiphinema spp.* im Allgemeinen.

Zu beispielhaften Nematoden bei denen ein erfindungsgemäßes Nematizid eingesetzt werden kann, zählen, jedoch nicht ausschließlich Nematoden der Gattung *Meloidogyne* wie der Southern Root-Knot Nematode (*Meloidogyne incognita),* der Javanese Root-Knot Nematode (*Meloidogyne javanica,* der Northern Root-Knot Nematode (*Meloidogyne hapla*) und der Peanut Root-Knot Nematode (*Meloidogyne arenaria;* Nematoden der Gattung *Ditylenchus* wie das Krätzeälchen (*Ditylenchus destructor*) und das Stock- und Stängelälchen (*Ditylenchus dipsaci*); Nematoden der Gattung *Pratylenchus* wie der Cob Root-Lesion Nematode (*Pratylenchus penetrans*), der Chrysanthemum Root-Lesion Nematode (*Pratylenchus fallax*), der Kaffeewurzelnematode (*Pratylenchus coffeae*), der Teewurzelnematode (*Pratylenchus loosi*) und der Walnut Root-Lesion Nematode (*Pratylenchus vulnus*); Nematoden der Gattung *Globodera* wie das Gelbe Kartoffelzystenälchen (*Globodera rostochiensis*) und das Weiße Kartoffelzystenälchen (*Globodera pallida*); Nematoden der Gattung *Heterodera* wie der Sojabohnenzystennematode (*Heterodera glycines*) und das Rübenälchen (*Heterodera schachtii*); Nematoden der Gattung *Aphelenchoides* wie der Rice White-tip Nematode *(Aphelenchoides besseyi)*, das Chrysanthemenälchen (*Aphelenchoides ritzemabosi*) und das Erdbeerälchen (*Aphelenchoides fragariae*); Nematoden der Gattung *Aphelenchus* wie der fungivore Nematode (*Aphelenchus avenae*); Nematoden der Gattung *Radopholus,* wie der Burrowing-Nematode (*Radopholus similis*); Nematoden der Gattung *Tylenchulus* wie der Orangenwurzelnematode (*Tylenchulus semipenetrans*); Nematoden der Gattung *Rotylenchulus* wie der reniforme Nematode (*Rotylenchulus reniformis*); in Bäumen lebende Nematoden, wie der Kiefernholznematode (*Bursaphelenchus xylophilus*) und dergleichen.

Pflanzen, für die ein erfindungsgemäßes Nematizid verwendet werden kann, sind nicht besonders eingeschränkt; so können zum Beispiel Pflanzen wie Getreide (zum Beispiel Reis, Gerste, Weizen, Roggen, Hafer, Mais, Kaoliang 5 und dergleichen), Bohnen (Sojabohne, Azukibohne, Bohne, Dicke Bohne, Erbsen, Erdnüsse und dergleichen), Obstbäume/Früchte (Äpfel, Zitrusarten, Birnen, Trauben, Pfirsiche, japanische Aprikosen, Kirschen, Walnüsse, Mandeln, Bananen, Erdbeeren und dergleichen), Gemüsearten (Kohl, Tomate, Spinat, Brokkoli, Salat, Zwiebel, Hohllauch, Pfeffer und dergleichen), Hackfrüchte (Karotte, Kartoffel, Süßkartoffel, Rettich, Lotuswurzel, Steckrübe und dergleichen), pflanzliche Rohstoffe (Baumwolle, Hanf, Papiermaulbeere, Mitsumata, Raps, Rübe, Hopfen, Zuckerrohr, Zuckerrübe, Olive, Gummi, Kaffee, Tabak, Tee und dergleichen), Kürbisgewächse (Kürbis, Gurke, Wassermelone, Melone und dergleichen), Weidepflanzen (Knaulgras, Sorgum, Wiesenlieschgras, Klee, Luzerne und dergleichen), Rasengräser (Maskarenengras, Straußgras und dergleichen), Gewürzpflanzen usw. (Lavendel, Rosmarin, Thymian, Petersilie, Pfeffer, Ingwer und dergleichen) und Blumenpflanzen (Chrysantheme, Rose, Orchidee und dergleichen) erwähnt werden.

Die Verbindung(en) und Zusammensetzung(en), die die erfindungsgemäße(n) Verbindung(en) enthalten, eignet/eignen sich besonders für die Bekämpfung von Nematoden des Kaffees, die zu mindestens einer Art aus der Gruppe der pflanzenparasitären Nematoden gehören bestehend aus *Pratylenchus brachyurus, Pratylenchus coffeae, Meloidogyne exigua, Meloidogyne incognita, Meloidogyne coffeicola, Helicotylenchus spp.* sowie aus *Meloidogyne paranaensis, Rotylenchus spp., Xiphinema spp., Tylenchorhynchus spp., Scutellonema spp.*.

Die Verbindung(en) und Zusammensetzung(en), die die erfindungsgemäße(n) Verbindung(en) enthalten, eignet/eignen sich besonders für die Bekämpfung von Nematoden der Kartoffel, die zu mindestens einer Art aus der Gruppe der pflanzenparasitären Nematoden gehören bestehend aus *Pratylenchus brachyurus, Pratylenchus pratensis, Pratylenchus scribneri, Pratylenchus penetrans, Pratylenchus coffeae, Ditylenchus dipsaci* sowie aus *Pratylenchus alleni, Pratylenchus andinus, Pratylenchus cerealis, Pratylenchus crenatus, Pratylenchus hexincisus, Pratylenchus loosi, Pratylenchus neglectus, Pratylenchus teres, Pratylenchus thornei, Pratylenchus vulnus, Belonolaimus longicaudatus, Trichodorus cylindricus, Trichodorus primitivus, Trichodorus proximus, Trichodorus similis, Trichodorus sparsus, Paratrichodorus minor, Paratrichodorus allius, Paratrichodorus nanus, Paratrichodorus teres, Meloidogyne arenaria, Meloidogyne hapla, Meloidogyne thamesi, Meloidogyne incognita, Meloidogyne chitwoodi, Meloidogyne javanica, Nacobbus aberrans, Globodera rostochiensis, Globodera pallida, Ditylenchus destructor, Radopholus similis, Rotylenchulus reniformis, Neotylenchus vigissi, Paraphelenchus pseudoparietinus, Aphelenchoidesfragariae, Meloinema spp.*.

Die Verbindung(en) und Zusammensetzung(en), die die erfindungsgemäße(n) Verbindung(en) enthalten, eignet/eignen sich besonders für die Bekämpfung von Nematoden der Tomate, die zu mindestens einer Art aus der Gruppe der pflanzenparasitären Nematoden gehören bestehend aus *Meloidogyne arenaria, Meloidogyne hapla, Meloidogyne javanica, Meloidogyne incognita, Pratylenchus penetrans* und aus *Pratylenchus brachyurus, Pratylenchus coffeae, Pratylenchus scribneri, Pratylenchus vulnus, Paratrichodorus minor, Meloidogyne exigua, Nacobbus aberrans, Globodera solanacearum, Dolichodorus heterocephalus, Rotylenchulus reniformis.*

Die Verbindung(en) und Zusammensetzung(en), die die erfindungsgemäße(n) Verbindung(en) enthalten, eignet/eignen sich besonders für die Bekämpfung von Nematoden von Gurkengewächsen, die zu mindestens einer Art aus der Gruppe der pflanzenparasitären Nematoden gehören bestehend aus *Meloidogyne arenaria, Meloidogyne hapla, Meloidogyne javanica, Meloidogyne incognita, Rotylenchulus reniformis* und aus *Pratylenchus thornei.*

Die Verbindung(en) und Zusammensetzung(en), die die erfindungsgemäße(n) Verbindung(en) enthalten, eignet/eignen sich besonders für die Bekämpfung von Nematoden der Baumwolle, die zu mindestens einer Art aus der Gruppe der pflanzenparasitären Nematoden gehören bestehend aus *Belonolaimus longicaudatus, Meloidogyne incognita, Hoplolaimus columbus, Hoplolaimus galeatus, Rotylenchulus reniformis.*

Die Verbindung(en) und Zusammensetzung(en), die die erfindungsgemäße(n) Verbindung(en) enthalten, eignet/eignen sich besonders für die Bekämpfung von Nematoden des Maises, die zu mindestens einer Art aus der Gruppe der pflanzenparasitären Nematoden gehören bestehend aus insbesondere *Belonolaimus longicaudatus, Paratrichodorus minor* und aus *Pratylenchus brachyurus, Pratylenchus delattrei, Pratylenchus hexincisus, Pratylenchus penetrans, Pratylenchus zeae, (Belonolaimus gracilis), Belonolaimus nortoni, Longidorus breviannulatus, Meloidogyne arenaria, Meloidogyne arenaria thamesi, Meloidogyne graminis, Meloidogyne incognita, Meloidogyne incognita acrita, Meloidogyne javanica, Meloidogyne naasi, Heterodera avenae, Heterodera oryzae, Heterodera zeae, Punctodera chalcoensis, Ditylenchus dipsaci, Hoplolaimus aegyptii, Hoplolaimus magnistylus, Hoplolaimus galeatus, Hoplolaimus indicus, Helicotylenchus digonicus, Helicotylenchus dihystera, Helicotylenchus pseudorobustus, Xiphinema americanum, Dolichodorus heterocephalus, Criconemella ornata, Criconemella onoensis, Radopholus similis, Rotylenchulus borealis, Rotylenchulus parvus, Tylenchorhynchus agri, Tylenchorhynchus clarus, Tylenchorhynchus claytoni, Tylenchorhynchus maximus, Tylenchorhynchus nudus, Tylenchorhynchus vulgaris, Quinisulcius acutus, Paratylenchus minutus, Hemicycliophora parvana, Aglenchus agricola, Anguina tritici, Aphelenchoides arachidis, Scutellonema brachyurum, Subanguina radiciola.*

Die Verbindung(en) und Zusammensetzung(en), die die erfindungsgemäße(n) Verbindung(en) enthalten, eignet/eignen sich besonders für die Bekämpfung von Nematoden der Sojabohne, die zu mindestens einer Art aus der Gruppe der pflanzenparasitären Nematoden gehören bestehend aus insbesondere *Pratylenchus brachyurus, Pratylenchus pratensis, Pratylenchus penetrans, Pratylenchus scribneri, Belonolaimus longicaudatus, Heterodera glycines, Hoplolaimus columbus* und aus *Pratylenchus coffeae, Pratylenchus hexincisus, Pratylenchus neglectus, Pratylenchus crenatus, Pratylenchus alleni, Pratylenchus agilis, Pratylenchus zeae, Pratylenchus vulnus, (Belonolaimus gracilis), Meloidogyne arenaria, Meloidogyne incognita, Meloidogyne javanica, Meloidogyne hapla, Hoplolaimus columbus, Hoplolaimus galeatus, Rotylenchulus reniformis.*

Die Verbindung(en) und Zusammensetzung(en), die die erfindungsgemäße(n) Verbindung(en) enthalten, eignet/eignen sich besonders für die Bekämpfung von Nematoden der Sojabohne, die zu mindestens einer Art aus der Gruppe der pflanzenparasitären Nematoden gehören bestehend aus insbesondere *Pratylenchus brachyurus, Pratylenchus pratensis, Pratylenchus penetrans, Pratylenchus scribneri, Belonolaimus longicaudatus, Hoplolaimus columbus* und aus *Pratylenchus coffeae, Pratylenchus hexincisus, Pratylenchus neglectus, Pratylenchus crenatus, Pratylenchus alleni, Pratylenchus agilis, Pratylenchus zeae, Pratylenchus vulnus, (Belonolaimus gracilis), Meloidogyne arenaria, Meloidogyne incognita, Meloidogyne javanica, Meloidogyne hapla, Hoplolaimus columbus, Hoplolaimus galeatus, Rotylen-*

*chulus reniformis.*

Die Verbindung(en) und Zusammensetzung(en), die die erfindungsgemäße(n) Verbindung(en) enthalten, eignet/eignen sich besonders für die Bekämpfung von Nematoden des Tabaks, die zu mindestens einer Art aus der Gruppe der pflanzenparasitären Nematoden gehören bestehend aus insbesondere *Meloidogyne incognita, Meloidogyne javanica* und aus *Pratylenchus brachyurus, Pratylenchus pratensis, Pratylenchus hexincisus, Pratylenchus penetrans, Pratylenchus neglectus, Pratylenchus crenatus, Pratylenchus thornei, Pratylenchus vulnus, Pratylenchus zeae, Longidorus elongatu, Paratrichodorus lobatus, Trichodorus spp., Meloidogyne arenaria, Meloidogyne hapla, Globodera tabacum, Globodera solanacearum, Globodera virginiae, Ditylenchus dipsaci, Rotylenchus spp., Helicotylenchus spp., Xiphinema americanum, Criconemella spp., Rotylenchulus reniformis, Tylenchorhynchus claytoni, Paratylenchus spp., Tetylenchus nicotianae.*

Die Verbindung(en) und Zusammensetzung(en), die die erfindungsgemäße(n) Verbindung(en) enthalten, eignet/eignen sich besonders für die Bekämpfung von Nematoden von Zitrusgewächsen, die zu mindestens einer Art aus der Gruppe der pflanzenparasitären Nematoden gehören bestehend aus insbesondere *Pratylenchus coffeae* und aus *Pratylenchus brachyurus, Pratylenchus vulnus, Belonolaimus longicaudatus, Paratrichodorus minor, Paratrichodorus porosus, Trichodorus, Meloidogyne incognita, Meloidogyne incognita acrita, Meloidogyne javanica, Rotylenchus macrodoratus, Xiphinema americanum, Xiphinema brevicolle, Xiphinema index, Criconemella spp., Hemicriconemoides, (Radopholus similis), Radopholus citrophilus, Hemicycliophora arenaria, Hemicycliophora nudata, Tylenchulus semipenetrans.*

Die Verbindung(en) und Zusammensetzung(en), die die erfindungsgemäße(n) Verbindung(en) enthalten, eignet/eignen sich besonders für die Bekämpfung von Nematoden der Banane, die zu mindestens einer Art aus der Gruppe der pflanzenparasitären Nematoden gehören bestehend aus insbesondere *Pratylenchus coffeae, Radopholus similis* und aus *Pratylenchus giibbicaudatus, Pratylenchus loosi, Meloidogyne spp., Helicotylenchus multicinctus, Helicotylenchus dihystera, Rotylenchulus spp..*

Die Verbindung(en) und Zusammensetzung(en), die die erfindungsgemäße(n) Verbindung(en) enthalten, eignet/eignen sich besonders für die Bekämpfung von Nematoden der Ananas, die zu mindestens einer Art aus der Gruppe der pflanzenparasitären Nematoden gehören bestehend aus insbesondere *Pratylenchus zeae, Pratylenchus pratensis, Pratylenchus brachyurus, Pratylenchus goodeyi., Meloidogyne spp., Rotylenchulus reniformis* und aus *Longidorus elongatus, Longidorus laevicapitatus, Trichodorus primitivus, Trichodorus minor, Heterodera spp., Ditylenchus myceliophagus, Hoplolaimus californicus, Hoplolaimus pararobustus, Hoplolaimus indicus, Helicotylenchus dihystera, Helicotylenchus nannus, Helicotylenchus multicinctus, Helicotylenchus erythrine, Xiphinema dimorphicaudatum, Radopholus similis, Tylenchorhynchus digitatus, Tylenchorhynchus ebriensis, Paratylenchus minutus, Scutellonema clathricaudatum, Scutellonema bradys, Psilenchus tumidus, Psilenchus magnidens, Pseudohalenchus minutus, Criconemoides ferniae, Criconemoides onoense, Criconemoides ornatum.*

Die Verbindung(en) und Zusammensetzung(en), die die erfindungsgemäße(n) Verbindung(en) enthalten, eignet/eignen sich besonders für die Bekämpfung von Nematoden von Trauben, die zu mindestens einer Art aus der Gruppe der pflanzenparasitären Nematoden gehören bestehend aus insbesondere *Pratylenchus vulnus, Meloidogyne arenaria, Meloidogyne incognita, Meloidogyne javanica, Xiphinema americanum, Xiphinema index* und aus *Pratylenchus pratensis, Pratylenchus scribneri, Pratylenchus neglectus, Pratylenchus brachyurus, Pratylenchus thornei, Tylenchulus semipenetrans.*

Die Verbindung(en) und Zusammensetzung(en), die die erfindungsgemäße(n) Verbindung(en) enthalten, eignet/eignen sich besonders für die Bekämpfung von Nematoden von Baumkulturen - Kernobst, die zu mindestens einer Art aus der Gruppe der pflanzenparasitären Nematoden gehören bestehend aus insbesondere *Pratylenchus penetrans* und aus *Pratylenchus vulnus, Longidorus elongatus, Meloidogyne incognita, Meloidogyne hapla.*

Die Verbindung(en) und Zusammensetzung(en), die die erfindungsgemäße(n) Verbindung(en) enthalten, eignet/eignen sich besonders für die Bekämpfung von Nematoden von Baumkulturen - Steinfrüchten, die zu mindestens einer Art aus der Gruppe der pflanzenparasitären Nematoden gehören bestehend aus insbesondere *Pratylenchus penetrans, Pratylenchus vulnus, Meloidogyne arenaria, Meloidogyne hapla, Meloidogyne javanica, Meloidogyne incognita, Criconemella xenoplax* und aus *Pratylenchus brachyurus, Pratylenchus coffeae, Pratylenchus scribneri, Pratylenchus zeae, Belonolaimus longicaudatus, Helicotylenchus dihystera, Xiphinema americanum, Criconemella curvata, Tylenchorhynchus claytoni, Paratylenchus hamatus, Paratylenchus projectus, Scutellonema brachyurum, Hoplolaimus galeatus.*

Die Verbindung(en) und Zusammensetzung(en), die die erfindungsgemäße(n) Verbindung(en) enthalten, eignet/eignen sich besonders für die Bekämpfung von Nematoden von Baumkulturen - Nüssen, die zu mindestens einer Art aus der Gruppe der pflanzenparasitären Nematoden gehören bestehend aus insbesondere *Trichodorus spp., Criconemella rusium* und aus *Pratylenchus vulnus, Paratrichodorus spp., Meloidogyne incognita, Helicotylenchus spp., Tylenchorhynchus spp., Cacopaurus pestis.*

Die vorliegende Erfindung betrifft weiterhin Formulierungen und daraus bereitete Anwendungsformen als Pflanzenschutzmittel und/oder Schädlingsbekämpfungsmittel wie z. B. Drench-, Drip- und Spritzbrühen, umfassend mindestens einen der erfindungsgemäßen Wirkstoffe. Gegebenenfalls enthalten die Anwendungsformen weitere Pflanzenschutzmittel und/oder Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren erfindungsgemäßen Wirkstoffen weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Wirkstoffe mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung des Wirkstoffs oder den aus diesen Formulierungen bereiteten Anwendungsformen (wie z.B. gebrauchsfähigen Pflanzenschutzmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn einer der Wirkstoff und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Wirkstoffe mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen agrochemischer Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,001 und 98 Gew.-% Wirkstoff oder, besonders bevorzugt zwischen 0,01 und 95 Gew.-% Wirkstoff, besonders bevorzugt zwischen 0,5 und 90 Gew.-% Wirkstoff, bezogen auf das Gewicht der Formulierung.

Die Behandlung der Pflanzen und Pflanzenteile mit den erfindungsgemäßen Verbindungen, Zusammensetzungen bzw. Mitteln erfolgt direkt oder durch Einwirkung auf deren Umgebung, Lebensraum oder Lagerraum nach den üblichen Behandlungsmethoden, z.B. durch Tauchen, (Ver-) Spritzen, (Ver-)Sprühen, Berieseln, Verdampfen, Zerstäuben, Vernebeln, (Ver-)Streuen, Verschäumen, Bestreichen, Verstreichen, Injizieren, Gießen (Drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Eine bevorzugte direkte Behandlung der Pflanzen ist die Blattapplikation, d.h. erfindungsgemäße Verbindungen, Zusammensetzungen bzw. Mittel werden auf das Blattwerk aufgebracht, wobei die Behandlungsfrequenz und die Aufwandmenge auf den Befallsdruck des jeweiligen phytopathogenen Nematoden abgestimmt sein kann.

Bei systemisch wirksamen Verbindungen gelangen die erfindungsgemäßen Verbindungen, Zusammensetzungen bzw. Mittel über das Wurzelwerk in die Pflanzen. Die Behandlung der Pflanzen erfolgt dann durch Einwirkung der erfindungsgemäßen Verbindungen, Zusammensetzungen bzw. Mittel auf den Lebensraum der Pflanze. Das kann beispielsweise durch Drenchen, Einmischen in den Boden oder die Nährlösung sein, d.h. der Standort der Pflanze (z.B. Boden oder hydroponische Systeme) wird mit einer flüssigen Form der erfindungsgemäßen Wirkstoffe, Wirkstoffkombinationen bzw. Mittel getränkt, oder. die erfindungsgemäßen Verbindungen, Zusammensetzungen bzw. Mittel werden in fester Formdurch eine Bodenapplikation, (z.B. in Form eines Granulats) in den Standort der Pflanzen eingebracht. Bei Wasserreiskulturen kann das auch durch Zudosieren der Erfindung in einer festen Anwendungsform (z.B. als Granulat) in ein überflutetes Reisfeld sein.

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombina¬ion mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Bevorzugt sind Pflanzen aus der Gruppe der Nutzpflanzen, Zierpflanzen, Rasenarten, allgemein genutzte Bäume, die in öffentlichen und privaten Bereichen als Zierpflanzen Verwendungen finden, und Forstbestand. Der Forstbestand umfasst Bäume für die Herstellung von Holz, Zellstoff, Papier und Produkten, die aus Teilen der Bäume hergestellt werden.

Der Begriff Nutzpflanzen, wie hier verwendet, bezeichnet Kulturpflanzen, die als Pflanzen für die Gewinnung von Nahrungsmitteln, Futtermitteln, Treibstoffen oder für technische Zwecke eingesetzt werden.

Zu den Nutzpflanzen, die mit dem erfindungsgemäßen Verfahren verbessert werden können, zählen z.B. folgende Pflanzenarten: Turf, Reben, Getreide, beispielsweise Weizen, Gerste, Roggen, Hafer, Reis, Mais und Hirse; Rüben, beispielsweise Zuckerrüben und Futterrüben; Früchte, beispielsweise Kernobst, Steinobst und Beerenobst, beispielsweise Äpfel, Birnen, Pflaumen, Pfirsiche, Mandeln, Kirschen und Beeren, z. B. Erdbeeren, Himbeeren, Brombeeren; Hülsenfrüchte, beispielsweise Bohnen, Linsen, Erbsen und Sojabohnen; Ölkulturen, beispielsweise Raps, Senf, Mohn, Oliven, Sonnenblumen, Kokos, Castorölpflanzen, Kakaobohnen und Erdnüsse; Gurkengewächse, beispielsweise Kürbis, Gurken und Melonen; Fasergewächse, beispielsweise Baumwolle, Flachs, Hanf und Jute; Citrusfrüchte, beispielsweise Orangen, Zitronen, Pampelmusen und Mandarinen; Gemüsesorten, beispielsweise Spinat, (Kopf)-Salat, Spargel, Kohlarten, Möhren, Zwiebeln, Tomaten, Kartoffeln und Paprika; Lorbeergewächse, beispielsweise Avocado, Cinnamomum, Kampfer, oder ebenso Pflanzen wie Tabak, Nüsse, Kaffee, Aubergine, Zuckerrohr, Tee, Pfeffer, Weinreben, Hopfen, Bananen, Naturkautschukgewächse sowie Zierpflanzen, beispielsweise Blumen, Sträucher, Laubbäume und Nadelbäume wie Koniferen. Diese Aufzählung stellt keine Limitierung dar.

Als besonders geeignete Zielkulturen für die Anwendung des erfindungsgemäßen Verfahrens sind folgende Pflanzen anzusehen: Bamwolle, Aubergine, Turf, Kernobst, Steinobst, Beerenobst, Mais, Weizen, Gerste, Gurke, Tabak, Reben, Reis, Getreide, Birne, Bohnen, Sojabohnen, Raps, Tomate, Paprika, Melonen, Kohl, Kartoffel und Apfel.

Als Bäume, die entsprechend dem erfindungsgemäßen Verfahren verbessert werden können, seien beispielhaft genannt: Abies sp., Eucalyptus sp., Picea sp., Pinus sp., Aesculus sp., Platanus sp., Tilia sp., Acer sp., Tsuga sp., Fraxinus sp., Sorbus sp., Betula sp., Crataegus sp., Ulmus sp., Quercus sp., Fagus sp., Salix sp., Populus sp..

Als bevorzugte Bäume, die entsprechend dem erfindungsgemäßen Verfahren verbessert werden können, können genannt werden: Aus der Baumart Aesculus: A. hippocastanum, A. pariflora, A. carnea; aus der Baumart Platanus: P. aceriflora, P. occidentalis, P. racemosa; aus der Baumart Picea: P. abies; aus der Baumart Pinus: P. radiate, P. ponderosa, P. contorta, P. sylvestre, P. elliottii, P. montecola, P. albicaulis, P. resinosa, P. palustris, P. taeda, P. flexilis, P. jeffregi, P. baksiana, P. strobes; aus der Baumart Eucalyptus: E. grandis, E. globulus, E. camadentis, E. nitens, E. obliqua, E. regnans, E. pilularus.

Als besonders bevorzugte Bäume, die entsprechend dem erfindungsgemäßen Verfahren verbessert werden können, können genannt werden: Aus der Baumart Pinus: P. radiate, P. ponderosa, P. contorta, P. sylvestre, P. strobes; aus der Baumart Eucalyptus: E. grandis, E. globulus, E. camadentis.

Als ganz besonders bevorzugte Bäume, die entsprechend dem erfindungsgemäßen Verfahren verbessert

werden können, können genannt werden: Rosskastanie, Platanengewächse, Linde, Ahornbaum.

Die vorliegende Erfindung kann auch an beliebigen Rasenarten ("turfgrasses") durchgeführt werden, einschließlich "cool season turfgrasses" und "warm season turfgrasses". Beispiele für Rasenarten für die kalte Jahreszeit sind Blaugräser ("blue grasses"; Poa spp.), wie "Kentucky bluegrass" (Poa pratensis L.), "rough bluegrass" (Poa trivialis L.), "Canada bluegrass" (Poa compressa L.), "annual bluegrass" (Poa annua L.), "upland bluegrass" (Poa glaucantha Gaudin), "wood bluegrass" (Poa nemoralis L.) und "bulbous bluegrass" (Poa bulbosa L.); Straussgräser ("Bentgrass", Agrostis spp.), wie "creeping bentgrass" (Agrostis palustris Huds.), "colonial bentgrass" (Agrostis tenuis Sibth.), "velvet bentgrass" (Agrostis canina L.), "South German Mixed Bentgrass" (Agrostis spp. einschließlich Agrostis tenius Sibth., Agrostis canina L., und Agrostis palustris Huds.), und "redtop" (Agrostis alba L.);

Schwingel ("Fescues", Festucu spp.), wie "red fescue" (Festuca rubra L. spp. rubra), "creeping fescue" (Festuca rubra L.), "chewings fescue" (Festuca rubra commutata Gaud.), "sheep fescue" (Festuca ovina L.), "hard fescue" (Festuca longifolia Thuill.), "hair fescue" (Festucu capillata Lam.), "tall fescue" (Festuca arundinacea Schreb.) und "meadow fescue" (Festuca elanor L.);

Lolch ("ryegrasses", Lolium spp.), wie "annual ryegrass" (Lolium multiflorum Lam.), "perennial ryegrass" (Lolium perenne L.) und "italian ryegrass" (Lolium multiflorum Lam.);

und Weizengräser ("wheatgrasses", Agropyron spp..), wie "fairway wheatgrass" (Agropyron cristatum (L.) Gaertn.), "crested wheatgrass" (Agropyron desertorum (Fisch.) Schult.) und "western wheatgrass" (Agropyron smithii Rydb.).

Beispiele für weitere "cool season turfgrasses" sind "beachgrass" (Ammophila breviligulata Fern.), "smooth bromegrass" (Bromus inermis Leyss.), Schilf ("cattails") wie "Timothy" (Phleum pratense L.), "sand cattail" (Phleum subulatum L.), "orchardgrass" (Dactylis glomerata L.), "weeping alkaligrass" (Puccinellia distans (L.) Parl.) und "crested dog's-tail" (Cynosurus cristatus L.).

Beispiele für "warm season turfgrasses" sind "Bermudagrass" (Cynodon spp. L. C. Rich), "zoysiagrass" (Zoysia spp. Willd.), "St. Augustine grass" (Stenotaphrum secundatum Walt Kuntze), "centipedegrass" (Eremochloa ophiuroides Munro Hack.), "carpetgrass" (Axonopus affinis Chase), "Bahia grass" (Paspalum notatum Flügge), "Kikuyugrass" (Pennisetum clandestinum Hochst, ex Chiov.), "buffalo grass" (Buchloe dactyloids (Nutt.) Engelm.), "Blue gramma" (Bouteloua gracilis (H.B.K.) Lag. ex Griffiths), "seashore paspalum" (Paspalum vaginatum Swartz) und "sideoats grama" (Bouteloua curtipendula (Michx. Torr.). "Cool season turfgrasses" sind für die erfindungsgemäße Verwendung im Allgemeinen bevorzugt. Besonders bevorzugt sind Blaugras, Straussgras und "redtop", Schwingel und Lolch. Straussgras ist insbesondere bevorzugt.

Die folgenden Beispiele erläutern die Erfindung, ohne sie einzuschränken.

**Herstellungs beispiele:**

Beispiel 1-20

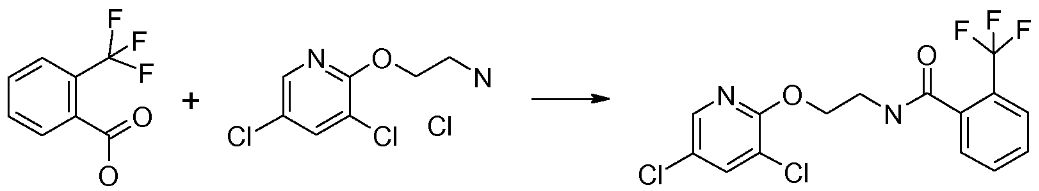

76 mg (0,4 mmol) 2-(Trifluormethyl)-benzoesäure, 107,2 mg (0,44 mmol 2-[(3,5-Dichlorpyridin-2-yl)oxy]ethanamin (Hydrochlorid), 30,6 mg (0,2 mmol) 1-Hydroxybenzotriazol (HOBt), 24,4 mg (0,02 mmol) DMAP, 76,7 mg (0,4 mmol) EDC-Hydrochlorid und 51,7 mg (0,4 mmol) Diisopropylethylamin werden in 10 ml Dichlormethan gelöst und bei Raumtemperatur für 16 h gerührt. Nach beendeter Reaktion wird mit 10 ml Wasser versetzt und die organische Phase abgetrennt, die wässrige Phase mit 5 ml Dichlormethan nachextrahiert. Die Dichlormethanphasen werden über Natriumsulfat/Kieselgelkartuschen filtriert, das Lösungsmittel wird abgedampft und der Rückstand mittels präparativer HPLC getrennt.

Ausbeute: 107 mg (70,6 % d. Th.), farbloser Feststoff.

$^1$H-NMR (d6-DMSO): δ [ppm], 8,70 (t, NH), 8,22 (s, 1H), 8,18 (d, 1H), 7,78 - 7,49 (m, 4H), 4,46 (t, 2H), 3,65 - 3,61 (q, 2H).

Nachfolgende Tabellenbeispiele sind auf gleiche Weise herstellbar.

**Tabelle 1**

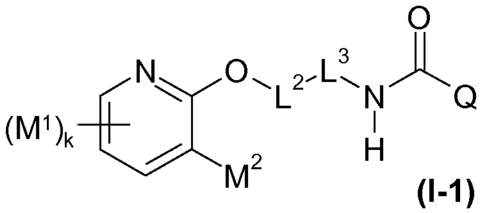

**(I-1)**

| Nummer | Q | M¹ | M² | L² | L³ |
|---|---|---|---|---|---|
| 1-1 | 2-(Trifluormethyl)-phenyl | 5-CF3 | Cl | CH2 | CH2 |
| 1-2 | 2-(Difluormethyl)-phenyl | 5-CF3 | Cl | CH2 | CH2 |
| 1-3 | 3-Chlor-2-pyridyl | 5-CF3 | Cl | CH2 | CH2 |
| 1-4 | 2-(Trifluormethyl)-phenyl | 5-CF3 | Cl | CH2 | CH(CH3) |
| 1-5 | 2-Fluor-phenyl | H | Cl | CH2 | CH2 |
| 1-6 | 2-Chlor-phenyl | H | Cl | CH2 | CH2 |
| 1-7 | 2-Brom-phenyl | H | Cl | CH2 | CH2 |
| 1-8 | 2-Iod-phenyl | H | Cl | CH2 | CH2 |
| 1-9 | 2,6-Difluor-phenyl | H | Cl | CH2 | CH2 |
| 1-10 | 2-(Trifluormethyl)-phenyl | H | Cl | CH2 | CH2 |
| 1-11 | 2-Chlor-3-pyridyl | H | Cl | CH2 | CH2 |
| 1-12 | 3-Iod-thien-2-yl | H | Cl | CH2 | CH2 |
| 1-13 | 3-Chlor-thien-2-yl | H | Cl | CH2 | CH2 |
| 1-14 | 3-Brom-furan-2-yl | H | Cl | CH2 | CH2 |
| 1-15 | 2-Fluor-phenyl | 5-Cl | Cl | CH2 | CH2 |
| 1-16 | 2-Chlor-phenyl | 5-Cl | Cl | CH2 | CH2 |
| 1-17 | 2-Brom-phenyl | 5-Cl | Cl | CH2 | CH2 |
| 1-18 | 2-Iod-phenyl | 5-Cl | Cl | CH2 | CH2 |
| 1-19 | 2,6-Difluor-phenyl | 5-Cl | Cl | CH2 | CH2 |
| 1-20 | 2-(Trifluormethyl)-phenyl | 5-Cl | Cl | CH2 | CH2 |
| 1-21 | 2-Chlor-3-pyridyl | 5-Cl | Cl | CH2 | CH2 |
| 1-22 | 3-Iod-thien-2-yl | 5-Cl | Cl | CH2 | CH2 |
| 1-23 | 3-Chlor-thien-2-yl | 5-Cl | Cl | CH2 | CH2 |
| 1-24 | 3-Brom-furan-2-yl | 5-Cl | Cl | CH2 | CH2 |
| 1-25 | 2-(Trifluormethyl)-phenyl | 5-CF3 | Cl | CH(CH3) | CH2 |
| 1-26 | 2-Chlor-phenyl | 5-CF3 | Cl | CH2 | CH2 |
| 1-27 | 2-Brom-phenyl | 5-CF3 | Cl | CH2 | CH2 |
| 1-28 | 2-Iod-phenyl | 5-CF3 | Cl | CH2 | CH2 |
| 1-29 | 2-Methyl-phenyl | 5-CF3 | Cl | CH2 | CH2 |
| 1-30 | 2-(Trifluormethyl)-phenyl | 5-CF3 | Cl | CH(cPr) | CH2 |
| 1-31 | 2-Fluor-phenyl | 5-Cl | H | CH2 | CH2 |
| 1-32 | 2-Chlor-phenyl | 5-Cl | H | CH2 | CH2 |

(fortgesetzt)

| Nummer | Q | $M^1$ | $M^2$ | $L^2$ | $L^3$ |
|---|---|---|---|---|---|
| 1-33 | 2-Brom-phenyl | 5-Cl | H | CH2 | CH2 |
| 1-34 | 2-Iod-phenyl | 5-Cl | H | CH2 | CH2 |
| 1-35 | 2,6-Difluor-phenyl | 5-Cl | H | CH2 | CH2 |
| 1-36 | 2-(Trifluormethyl)-phenyl | 5-Cl | H | CH2 | CH2 |
| 1-37 | 2-Chlor-3-pyridyl | 5-Cl | H | CH2 | CH2 |
| 1-38 | 3-Iod-thien-2-yl | 5-Cl | H | CH2 | CH2 |
| 1-39 | 3-Chlor-thien-2-yl | 5-Cl | H | CH2 | CH2 |
| 1-40 | 3-Brom-furan-2-yl | 5-Cl | H | CH2 | CH2 |
| 1-41 | 2-Fluor-phenyl | 5-CF3 | H | CH2 | CH2 |
| 1-42 | 2-Chlor-phenyl | 5-CF3 | H | CH2 | CH2 |
| 1-43 | 2-Brom-phenyl | 5-CF3 | H | CH2 | CH2 |
| 1-44 | 2-Iod-phenyl | 5-CF3 | H | CH2 | CH2 |
| 1-45 | 2,6-Difluor-phenyl | 5-CF3 | H | CH2 | CH2 |
| 1-46 | 2-(Trifluormethyl)-phenyl | 5-CF3 | H | CH2 | CH2 |
| 1-47 | 2-Chlor-3-pyridyl | 5-CF3 | H | CH2 | CH2 |
| 1-48 | 3-Iod-thien-2-yl | 5-CF3 | H | CH2 | CH2 |
| 1-49 | 3-Chlor-thien-2-yl | 5-CF3 | H | CH2 | CH2 |
| 1-50 | 3-Brom-furan-2-yl | 5-CF3 | H | CH2 | CH2 |
| 1-51 | 2-Fluor-phenyl | 5-CF3 | Cl | CH2 | CH2 |
| 1-52 | 2,6-Difluor-phenyl | 5-CF3 | Cl | CH2 | CH2 |
| 1-53 | 2-Nitro-phenyl | 5-CF3 | Cl | CH2 | CH2 |
| 1-54 | 2-Chlor-3-pyridyl | H | Cl | CH2 | CH2 |
| 1-55 | 3-Chlor-2-thienyl | H | Cl | CH2 | CH2 |
| 1-56 | 3-Brom-furan-2-yl | 5-CF3 | Cl | CH2 | CH2 |
| 1-57 | 2-(Trifluormethyl)-3-pyridyl | 5-CF3 | H | CH2 | CH2 |
| 1-58 | 2-(Trifluormethyl)-3-pyridyl | H | Cl | CH2 | CH2 |
| 1-59 | 2-(Trifluormethyl)-5-chlor-3-pyridyl | 5-CF3 | Cl | CH2 | CH2 |
| 1-60 | 2-(Trifluormethyl)-5-chlor-3-pyridyl | 5-CF3 | H | CH2 | CH2 |
| 1-61 | 2-(Trifluormethyl)-5-chlor-3-pyridyl | H | Cl | CH2 | CH2 |
| 1-62 | 2-Chlor-4-(trifluormethyl)-3-pyridyl | H | Cl | CH2 | CH2 |
| 1-63 | 2-(Trifluormethyl)-3-pyridyl | 5-CF3 | Cl | CH2 | CH2 |
| 1-64 | 2-Chlor-4-(trifluormethyl)-3-pyridyl | 5-CF3 | H | CH2 | CH2 |
| 1-65 | 2-Nitro-phenyl | H | Cl | CH2 | C(CH3)(CH2CH3) |
| 1-66 | 2-Nitro-phenyl | H | CF3 | CH2 | CH2 |
| 1-67 | 2-Nitro-phenyl | H | F | CH2 | CH2 |
| 1-68 | 2-Nitro-phenyl | H | CF3 | CH2 | C(CH3)2 |
| 1-69 | 2-Nitro-phenyl | Cl | Cl | CH2 | C(CH3)2 |
| 1-70 | 2-(Trifluormethyl)-3-pyridyl | H | F | CH2 | CH2 |

(fortgesetzt)

| Nummer | Q | $M^1$ | $M^2$ | $L^2$ | $L^3$ |
|---|---|---|---|---|---|
| 1-71 | 2-(Trifluormethyl)-3-pyridyl | 5-CF3 | H | CH2 | C(CH3)2 |
| 1-72 | 2-Nitro-phenyl | H | Br | CH2 | C(CH3)2 |
| 1-73 | 2-(Trifluormethyl)-3-pyridyl | H | Cl | CH2 | C(CH3)(CH2CH3) |
| 1-74 | 2-(Trifluormethyl)-3-pyridyl | H | CF3 | CH2 | CH2 |
| 1-75 | 2-(Trifluormethyl)-3-pyridyl | H | Br | CH2 | C(CH3)2 |
| 1-76 | 2-(Trifluormethyl)-3-pyridyl | H | CF3 | CH2 | C(CH3)2 |
| 1-77 | 2-(Trifluormethyl)-3-pyridyl | Cl | Cl | CH2 | C(CH3)2 |
| 1-78 | 2-(Trifluormethyl)-phenyl | 5-CF3 | H | CH2 | C(CH3)2 |
| 1-79 | 2-(Trifluormethyl)-phenyl | H | F | CH2 | CH2 |
| 1-80 | 2-(Trifluormethyl)-phenyl | H | Cl | CH2 | C(CH3)(CH2CH3) |
| 1-81 | 2-(Trifluormethyl)-phenyl | H | CF3 | CH2 | CH2 |
| 1-82 | 2-(Trifluormethyl)-phenyl | Cl | Cl | CH2 | C(CH3)2 |
| 1-83 | 2-(Trifluormethyl)-phenyl | H | Br | CH2 | C(CH3)2 |
| 1-84 | 2,6-Difluor-phenyl | 5-CF3 | H | CH2 | C(CH3)2 |
| 1-85 | 2,6-Difluor-phenyl | H | F | CH2 | CH2 |
| 1-86 | 2,6-Difluor-phenyl | H | CF3 | CH2 | CH2 |
| 1-87 | 2,6-Difluor-phenyl | H | Cl | CH2 | C(CH3)(CH2CH3) |
| 1-88 | 2,6-Difluor-phenyl | Cl | Cl | CH2 | C(CH3)2 |
| 1-89 | 2,6-Difluor-phenyl | H | Br | CH2 | C(CH3)2 |
| 1-90 | 2-Nitro-phenyl | 5-CF3 | H | CH2 | C(CH3)2 |
| 1-91 | 2-(Trifluormethylsulfanyl)-phenyl | 5-CF3 | Cl | CH2 | CH2 |
| 1-92 | 2-(Trifluormethylsulfanyl)-phenyl | 5-CF3 | H | CH2 | CH2 |
| 1-93 | 2-(Trifluormethylsulfanyl)-phenyl | H | CF3 | CH2 | CH2 |
| 1-94 | 2-(Trifluormethylsulfanyl)-phenyl | 5-CF3 | H | CH2 | C(CH3)2 |
| 1-95 | 2-(Trifluormethylsulfanyl)-phenyl | H | Cl | CH2 | CH2 |
| 1-96 | 2-(Trifluormethylsulfanyl)-3-pyridyl | 5-CF3 | Cl | CH2 | CH2 |
| 1-97 | 2-(Trifluormethylsulfonyl)-phenyl | 5-CF3 | Cl | CH2 | CH2 |
| 1-98 | 2-(Trifluormethylsulfonyl)-phenyl | H | Cl | CH2 | CH2 |
| 1-99 | 2-(Trifluormethylsulfonyl)-phenyl | H | CF3 | CH2 | CH2 |
| 1-100 | 2-(Trifluormethylsulfonyl)-3-pyridyl | 5-CF3 | Cl | CH2 | CH2 |
| 1-101 | 2-(Trifluormethoxy)-3-pyridyl | H | CF3 | CH2 | CH2 |
| 1-102 | 2-(Trifluormethoxy)-3-pyridyl | 5-CF3 | H | CH2 | C(CH3)2 |
| 1-103 | 2-(Trifluormethoxy)-3-pyridyl | H | Cl | CH2 | CH2 |
| 1-104 | 2-(Trifluormethoxy)-3-pyridyl | 5-CF3 | H | CH2 | CH2 |
| 1-105 | 2-(Trifluormethoxy)-3-pyridyl | 5-CF3 | Cl | CH2 | CH2 |
| 1-106 | 2-(Difluormethoxy)-3-pyridyl | 5-CF3 | Cl | CH2 | CH2 |
| 1-107 | 2-Fluor-phenyl | 4-CN | H | CH2 | CH2 |
| 1-108 | 2-Chlor-phenyl | 4-CN | H | CH2 | CH2 |

(fortgesetzt)

| Nummer | Q | $M^1$ | $M^2$ | $L^2$ | $L^3$ |
|---|---|---|---|---|---|
| 1-109 | 2-Brom-phenyl | 4-CN | H | CH2 | CH2 |
| 1-110 | 2-Iod-phenyl | 4-CN | H | CH2 | CH2 |
| 1-111 | 2,6-Difluor-phenyl | 4-CN | H | CH2 | CH2 |
| 1-112 | 2-(Trifluormethyl)-phenyl | 4-CN | H | CH2 | CH2 |
| 1-113 | 2-Chlor-3-pyridyl | 4-CN | H | CH2 | CH2 |
| 1-114 | 2-(Trifluormethyl)-3-pyridyl | 4-CN | H | CH2 | CH2 |
| 1-115 | 2-(Trifluormethoxy)-3-pyridyl | 4-CN | H | CH2 | CH2 |
| 1-116 | 2-(Trifluormethyl)-phenyl | 4-Cl | H | CH2 | CH2 |
| 1-117 | 2-(Trifluormethyl)-3-pyridyl | 4-Cl | H | CH2 | CH2 |
| 1-118 | 2,6-Difluor-phenyl | 4-Cl | H | CH2 | CH2 |
| 1-119 | 2-Iod-phenyl | 4-Cl | H | CH2 | CH2 |
| 1-120 | 2-Fluor-phenyl | 4-CF3 | H | CH2 | CH2 |
| 1-121 | 2-Chlor-phenyl | 4-CF3 | H | CH2 | CH2 |
| 1-122 | 2-Brom-phenyl | 4-CF3 | H | CH2 | CH2 |
| 1-123 | 2-Iod-phenyl | 4-CF3 | H | CH2 | CH2 |
| 1-124 | 2,6-Difluor-phenyl | 4-CF3 | H | CH2 | CH2 |
| 1-125 | 2-(Trifluormethyl)-phenyl | 4-CF3 | H | CH2 | CH2 |
| 1-126 | 2-Chlor-3-pyridyl | 4-CF3 | H | CH2 | CH2 |
| 1-127 | 2-(Trifluormethyl)-3-pyridyl | 4-CF3 | H | CH2 | CH2 |
| 1-128 | 2-(Trifluormethoxy)-3-pyridyl | 4-CF3 | H | CH2 | CH2 |
| 1-129 | 2-(Trifluormethylsulfanyl)-3-pyridyl | 4-CF3 | H | CH2 | CH2 |
| 1-130 | 2-(Trifluormethylsulfanyl)-phenyl | 4-CF3 | H | CH2 | CH2 |
| 1-131 | 2-Fluor-phenyl | 5-F | F | CH2 | CH2 |
| 1-132 | 2-Brom-phenyl | 5-F | F | CH2 | CH2 |
| 1-133 | 2,6-Difluor-phenyl | 5-F | F | CH2 | CH2 |
| 1-134 | 2-(Trifluormethyl)-phenyl | 5-F | F | CH2 | CH2 |
| 1-135 | 2-Chlor-3-pyridyl | 5-F | F | CH2 | CH2 |
| 1-136 | 2-(Trifluormethyl)-3 -pyridyl | 5-F | F | CH2 | CH2 |
| 1-137 | 2-(Trifluormethoxy)-3-pyridyl | 5-F | F | CH2 | CH2 |
| 1-138 | 2-(Trifluormethylsulfanyl)- 3 -pyridyl | 5-F | F | CH2 | CH2 |
| 1-139 | 2-(Trifluormethylsulfanyl)-phenyl | 5-F | F | CH2 | CH2 |
| 1-140 | 2,6-Bis(trifluormethyl)-phenyl | 5-F | F | CH2 | CH2 |
| 1-141 | 2-Iod-phenyl | 5-F | F | CH2 | CH2 |
| 1-142 | 2,6-Difluor-phenyl | 6-CF3 | H | CH2 | CH2 |
| 1-143 | 2-(Trifluormethylsulfanyl)-phenyl | 6-CF3 | H | CH2 | CH2 |
| 1-144 | 2-Brom-3-pyridyl | 6-CF3 | H | CH2 | CH2 |
| 1-145 | 2-(Trifluormethyl)-phenyl | 6-CF3 | H | CH2 | CH2 |
| 1-146 | 2-(Trifluormethylsulfanyl)-3-pyridyl | 6-CF3 | H | CH2 | CH2 |

(fortgesetzt)

| Nummer | Q | $M^1$ | $M^2$ | $L^2$ | $L^3$ |
|---|---|---|---|---|---|
| 1-147 | 2-(Trifluormethyl)-3-pyridyl | 6-CF3 | H | CH2 | CH2 |
| 1-148 | 2-Brom-3-pyridyl | H | NO2 | CH2 | CH2 |
| 1-149 | 2-(Trifluormethyl)-phenyl | H | NO2 | CH2 | CH2 |
| 1-150 | 2,6-Difluor-phenyl | H | NO2 | CH2 | CH2 |
| 1-151 | 2-(Trifluormethylsulfanyl)-phenyl | H | NO2 | CH2 | CH2 |
| 1-152 | 2-(Trifluormethylsulfanyl)-3-pyridyl | H | NO2 | CH2 | CH2 |
| 1-153 | 2,5-Dichlor-3-thienyl | 5-CF3 | H | CH2 | CH2 |
| 1-154 | 2,5-Dichlor-3-thienyl | 5-CF3 | Cl | CH2 | CH2 |
| 1-155 | 2-Methoxy-3-pyridyl | 5-CF3 | Cl | CH2 | CH2 |
| 1-156 | 2-Chlor-phenyl | 5-CF3 | Cl | CH(CH3) | CH2 |
| 1-157 | 2-(Difluormethyl)-phenyl | 5-CF3 | Cl | CH2 | CH(CH3) |
| 1-158 | 2-(Difluormethyl)-phenyl | 5-CF3 | Cl | CH2 | C(CH3)2 |
| 1-159 | 2-(Difluormethyl)-phenyl | 5-CF3 | Cl | CH(CH3) | CH2 |
| 1-160 | 2-Nitro-phenyl | 5-CF3 | Cl | CH2 | CH(CH3) |
| 1-161 | 2-Nitro-phenyl | 5-CF3 | Cl | CH2 | C(CH3)2 |
| 1-162 | 2-Nitro-phenyl | 5-CF3 | Cl | CH(CH3) | CH2 |
| 1-163 | 2-Fluor-phenyl | 5-CF3 | Cl | CH2 | CH(CH3) |
| 1-164 | 2-(Trifluormethyl)-phenyl | 5-CN | F | CH2 | CH2 |
| 1-165 | 2-Brom-phenyl | 5-CF3 | Cl | CH2 | 1,1-cPr |
| 1-166 | 2-(Trifluormethoxy)-3 -pyridyl | 5-CF3 | Cl | CH2 | 1,1-cPr |
| 1-167 | 2,6-Difluor-phenyl | 5-CF3 | Cl | CH2 | 1,1-cPr |
| 1-168 | 2,6-Difluor-phenyl | 5-CF3 | H | CH2 | 1,1-cPr |
| 1-169 | 2-(Trifluormethyl)-phenyl | 5-CF3 | Cl | CH2 | 1,1-cPr |
| 1-170 | 2-Iod-phenyl | 5-CF3 | Cl | CH2 | 1,1-cPr |
| 1-171 | 2-Brom-3-pyridyl | 5-CF3 | Cl | CH2 | 1,1-cPr |
| 1-172 | 2-Chlor-3-pyridyl | 5-CF3 | Cl | CH2 | 1,1-cPr |
| 1-173 | 2-(Trifluormethyl)-phenyl | 5-CF3 | H | CH2 | 1,1-cPr |
| 1-174 | 2-Iod-phenyl | 5-CF3 | H | CH2 | 1,1-cPr |
| 1-175 | 2-Brom-3-pyridyl | 5-CF3 | H | CH2 | 1,1-cPr |
| 1-176 | 2-Brom-phenyl | 5-CF3 | H | CH2 | 1,1-cPr |
| 1-177 | 2-Chlor-3-pyridyl | 5-CF3 | H | CH2 | 1,1-cPr |
| 1-178 | 2-(Trifluormethyl)-2-pyridyl | 5-CF3 | H | CH2 | 1,1-cPr |
| 1-179 | 2-(Trifluormethyl)-2-pyridyl | 5-CF3 | Cl | CH2 | 1,1-cPr |
| 1-180 | 2-(Trifluormethoxy)-3 -pyridyl | 5-CF3 | H | CH2 | 1,1-cPr |
| 1-181 | 2-Nitro-phenyl | 5-CN | H | CH2 | CH2 |
| 1-182 | 2-(Trifluormethyl)-2-pyridyl | 5-CN | F | CH2 | CH2 |
| 1-183 | 2-Nitro-phenyl | 5-CN | F | CH2 | CH2 |
| 1-184 | 2-Chlor-3-pyridyl | 5-CN | F | CH2 | CH2 |

(fortgesetzt)

| Nummer | Q | $M^1$ | $M^2$ | $L^2$ | $L^3$ |
|---|---|---|---|---|---|
| 1-185 | 2-Iod-phenyl | 5-CN | H | CH2 | CH2 |
| 1-186 | 2-Brom-3-pyridyl | 5-CN | F | CH2 | CH2 |
| 1-187 | 2-Iod-phenyl | 5-CN | F | CH2 | CH2 |
| 1-188 | 2,6-Difluor-phenyl | 5-CN | H | CH2 | CH2 |
| 1-189 | 2,6-Difluor-phenyl | 5-CN | F | CH2 | CH2 |
| 1-190 | 2-(Trifluormethoxy)-3 -pyridyl | 5-CN | H | CH2 | CH2 |
| 1-191 | 2-Brom-phenyl | 5-CN | H | CH2 | CH2 |
| 1-192 | 2-Brom-phenyl | 5-CN | F | CH2 | CH2 |
| 1-193 | 2-Fluor-phenyl | 5-CN | H | CH2 | CH2 |
| 1-194 | 2-Fluor-phenyl | 5-CN | F | CH2 | CH2 |
| 1-195 | 2-Nitro-phenyl | 5-CF3 | Cl | CH2 | 1,1-cPr |
| 1-196 | 2-Nitro-phenyl | 5-CF3 | H | CH2 | 1,1-cPr |
| 1-197 | 2-Iod-phenyl | 5-CF3 | Cl | CH(CH3) | CH2 |
| 1-198 | 2-(Trifluormethyl)-3 -pyridyl | 5-CF3 | Cl | CH2 | CH(CH3) |
| 1-199 | 2-(Trifluormethyl)-3 -pyridyl | 5-CF3 | Cl | CH2 | C(CH3)2 |
| 1-200 | 2-(Trifluormethyl)-3 -pyridyl | 5-CF3 | Cl | CH(CH3) | CH2 |
| 1-201 | 2-Brom-phenyl | 5-CF3 | Cl | CH2 | CH(CH3) |
| 1-202 | 2-Brom-phenyl | 5-CF3 | Cl | CH(CH3) | CH2 |
| 1-203 | 2-Chlor-phenyl | 5-CF3 | Cl | CH2 | CH(CH3) |
| 1-204 | 2,6-Difluor-phenyl | 5-CF3 | Cl | CH(CH3) | CH2 |
| 1-205 | 2-Chlor-3-pyridyl | 5-CF3 | Cl | CH2 | C(CH3)2 |
| 1-206 | 2-Chlor-3-pyridyl | 5-CF3 | Cl | CH(CH3) | CH2 |
| 1-207 | 2-Chlor-3-pyridyl | 5-CF3 | Cl | CH2 | CH(CH3) |
| 1-208 | 3-Chlor-2-pyridyl | 5-CF3 | Cl | CH2 | CH(CH3) |
| 1-209 | 3-Chlor-2-pyridyl | 5-CF3 | Cl | CH(CH3) | CH2 |
| 1-210 | 2-(Trifluormethylsulfanyl)- 3 -pyridyl | 5-CF3 | Cl | CH(CH3) | CH2 |
| 1-211 | 2-Iod-phenyl | 5-CF3 | Cl | CH2 | CH(CH3) |
| 1-212 | 2-Iod-phenyl | 5-CF3 | Cl | CH2 | C(CH3)2 |

## $^1$H-NMR Daten

Die Bestimmung der $^1$H-NMR-Daten erfolgte mit einem Bruker Avance 400 ausgestattet mit einem Durchflussprobenkopf (60 µl Volumen), mit Tetramethylsilan als Referenz (0.0) und den Lösungsmitteln $CD_3CN$, $CDCl_3$ oder $D_6$-DMSO.

Die NMR-Daten ausgewählter Beispiele werden entweder in klassischer Form (δ-Werte, Multiplettaufspaltung, Anzahl der H-Atome) oder als NMR-Peak-Listen aufgeführt.

NMR-Peak-Listenverfahren

Die $^1$H-NMR -Daten ausgewählter Beispiele werden in Form von $^1$H-NMR -Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:

$$\delta_1 \text{ (Intensität}_1\text{)}; \delta_2 \text{ (Intensität}_2\text{)};\ldots\ldots.; \delta_i \text{ (Intensität}_i\text{)};\ldots\ldots; \delta_n \text{ (Intensität}_n\text{)}$$

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von $^1$H-NMR -Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen $^1$H-NMR-Ausdrucken und enthalten somit gewöhnlich alle Peaks, die bei einer klassischen NMR-Interpretation aufgeführt werden.

Darüber hinaus können sie wie klassische $^1$H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von $^1$H-NMR-Peaks die gewöhnlichen Lösungsmittelpeaks, zum Beispiel Peaks von DMSO in DMSO-$D_6$ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen $^1$H-NMR-Interpretation.

Weitere Details zu $^1$H-NMR-Peaklisten können der Research Disclosure Database Number 564025 entnommen werden.

**Tabelle 2**

| |
|---|
| Verbindung Nr. 1-1, Solvent: [$CD_3CN$], Spektrometer: 399.95MHz<br>8,4284 (6,38); 8,4257 (6,49); 8,0664 (7,14); 8,0618 (6,79); 7,7463 (4,17); 7,7269 (5,38); 7,6702 (1,71); 7,6528 (4,6); 7,6515 (4,61); 7,6345 (3,86); 7,6125 (3,65); 7,5936 (4,22); 7,5758 (1,45); 7,5747 (1,44); 7,4977 (5,11); 7,4792 (4,09); 7,0749 (1,4); 4,6125 (9); 4,5988 (16); 4,5851 (9,66); 3,7802 (4,97); 3,7659 (12,33); 3,7521 (11,66); 3,7381 (4,63); 2,1732 (9,66); 2,1635 (17,44); 1,9645 (0,67); 1,9583 (0,92); 1,9526 (8,01); 1,9465 (15,25); 1,9403 (21,55); 1,9341 (14,99); 1,9279 (7,75); 1,1711 (0,37); 1,0063 (0,37); 0,008 (0,36); -0,0002 (10,83); - 0,0085 (0,43) |
| Verbindung Nr. 1-2, Solvent: [$CD_3CN$], Spektrometer: 399.95MHz<br>8,4217 (4,67); 8,4193 (5,86); 8,4166 (5,83); 8,4142 (4,68); 8,0677 (6,82); 8,0622 (6,3); 7,7497 (3,69); 7,731 (4,89); 7,6271 (1,55); 7,6207 (2,22); 7,6093 (2,32); 7,6048 (3,32); 7,5924 (1,73); 7,5854 (3,35); 7,5792 (1,49); 7,5622 (5,53); 7,5606 (5,55); 7,5519 (6,26); 7,5473 (8,57); 7,545 (9,21); 7,5332 (1,06); 7,5263 (0,43); 7,3905 (4,12); 7,2516 (8,53); 7,2134 (1,33); 7,1127 (4,22); 4,6435 (9,63); 4,63 (16); 4,6164 (10,18); 3,7901 (5,4); 3,7758 (12,66); 3,7622 (11,61); 3,7482 (4,97); 2,1411 (18,4); 2,1134 (0,34); 2,1072 (0,42); 1,964 (3,92); 1,9579 (6,31); 1,952 (26,19); 1,9458 (45,34); 1,9397 (58,55); 1,9335 (40,04); 1,9273 (20,35); 1,9144 (0,33); 1,7682 (0,34); 1,3405 (1,98); 1,285 (2,34); 1,2691 (0,66); -0,0002 (2,57) |
| Verbindung Nr. 1-3, Solvent: [CD3CN], Spektrometer: 399.95MHz<br>8,4897 (5,13); 8,4865 (4,96); 8,4783 (5,26); 8,4751 (4,92); 8,4115 (5,97); 8,4089 (5,88); 8,0542 (6,81); 8,0489 (6,42); 7,9599 (1,2); 7,8971 (5,31); 7,8939 (5,01); 7,8766 (5,82); 7,8734 (5,39); 7,4658 (5,54); 7,4543 (5,42); 7,4453 (5,07); 7,4338 (4,86); 4,6485 (9,38); 4,635 (16); 4,6214 (9,95); 3,8205 (5,16); 3,8062 (11,71); 3,7925 (11,16); 3,7783 (4,78); 2,1357 (15,92); 1,965 (2,63); 1,9588 (4,06); 1,9529 (17,77); 1,9468 (31,07); 1,9407 (40,7); 1,9345 (28,17); 1,9283 (14,43); 1,3408 (1,08); 1,2849 (1,27); 1,2691 (0,45); 1,2574 (0,75); 1,2423 (0,73); - 0,0002 (1,76) |

(fortgesetzt)

| |
|---|
| Verbindung Nr. 1-4, Solvent: [CD3CN], Spektrometer: 399.95MHz<br>8,4 (0,65); 8,3973 (0,67); 8,3819 (0,4); 8,0603 (0,75); 8,0549 (0,72); 7,4706 (0,54); 7,4573 (0,44); 7,4502 (1,05); 7,2413 (0,6); 7,2352 (0,64); 7,2201 (0,41); 7,2138 (0,39); 6,9853 (0,69); 5,7284 (1,61); 5,2623 (0,38); 4,8564 (5,11); 4,6238 (0,39); 4,6127 (0,4); 4,6012 (0,49); 4,1401 (0,39); 4,1287 (0,39); 4,1173 (0,36); 4,0177 (0,48); 2,1329 (19,61); 1,9638 (1,92); 1,9578 (3,13); 1,9519 (13,39); 1,9457 (23,31); 1,9396 (30,3); 1,9334 (20,84); 1,9272 (10,7); 1,3864 (0,7); 1,3714 (12,25); 1,3631 (0,66); 1,3571 (0,62); 1,3404 (13,77); 1,2851 (16); 1,2765 (6,8); -0,0002 (1,15) |
| Verbindung Nr. 1-5, Solvent: [DMSO], Spektrometer: 399.95MHz<br>8,4706 (2,94); 8,3172 (0,85); 8,1327 (6,06); 8,1287 (6,13); 8,1205 (6,34); 8,1165 (6,03); 7,9076 (6); 7,9036 (5,72); 7,8884 (6,41); 7,8844 (5,75); 7,6334 (2,5); 7,6292 (2,7); 7,615 (4,86); 7,6106 (5,38); 7,5955 (2,92); 7,5914 (2,69); 7,5526 (1,31); 7,5481 (1,28); 7,5392 (1,64); 7,5343 (3,16); 7,5315 (2,68); 7,5199 (2,75); 7,5137 (3,22); 7,5089 (1,79); 7,5001 (1,8); 7,4957 (1,49); 7,3031 (4,1); 7,2923 (4,87); 7,2813 (4,41); 7,2741 (9,97); 7,2547 (7,39); 7,0504 (6,17); 7,0381 (6,12); 7,0312 (5,98); 7,0189 (5,71); 4,5057 (7,56); 4,491 (16); 4,4765 (7,9); 3,685 (3,83); 3,6706 (10,58); 3,6563 (10,29); 3,6418 (3,5); 3,3281 (50,62); 3,3049 (0,37); 2,6723 (0,4); 2,5075 (47,63); 2,5032 (59,17); 2,4989 (43,36); 2,3299 (0,38); 1,3372 (1,1); 1,2499 (1,27); 1,2351 (0,56); -0,0002 (1,95) |
| Verbindung Nr. 1-6, Solvent: [DMSO], Spektrometer: 399.95MHz<br>8,6342 (1,87); 8,621 (3,44); 8,6076 (1,88); 8,1404 (6,62); 8,1363 (7,05); 8,1282 (6,96); 8,124 (7,03); 7,9088 (6,78); 7,9047 (6,8); 7,8896 (7,35); 7,8855 (6,86); 7,4934 (3,36); 7,4917 (3,43); 7,4738 (8,03); 7,4724 (7,59); 7,4541 (3,52); 7,4477 (4,06); 7,4383 (4,54); 7,4319 (6,37); 7,4284 (1,86); 7,4189 (2,93); 7,4126 (7,37); 7,4005 (10,48); 7,3951 (12,85); 7,3919 (9,09); 7,3793 (4,26); 7,3761 (4,62); 7,3729 (1,76); 7,3603 (1,31); 7,357 (1,29); 7,0511 (7,39); 7,0389 (7,21); 7,0319 (7,12); 7,0197 (6,96); 5,7569 (1,91); 4,5022 (7,34); 4,4877 (16); 4,4731 (7,74); 3,6597 (3,85); 3,6454 (10,93); 3,631 (10,59); 3,6166 (3,53); 3,3257 (36,46); 2,6712 (0,36); 2,5243 (1,33); 2,511 (22,59); 2,5066 (44,58); 2,502 (58,55); 2,4975 (42,71); 2,493 (20,81); 2,3288 (0,39); 1,3367 (0,76); 1,2496 (0,95); -0,0002 (2,47) |
| Verbindung Nr. 1-7, Solvent: [DMSO], Spektrometer: 399.95MHz<br>8,6304 (1,96); 8,6172 (3,66); 8,6035 (1,98); 8,1417 (6,77); 8,1376 (7,18); 8,1295 (7,18); 8,1254 (7,11); 7,9087 (7,11); 7,9045 (6,98); 7,8895 (7,63); 7,8853 (7,02); 7,6479 (6,53); 7,6299 (6,22); 7,6278 (6,86); 7,4449 (2,04); 7,4421 (2,13); 7,4244 (5,77); 7,4082 (6,48); 7,4053 (5,91); 7,3924 (0,37); 7,381 (5,01); 7,377 (9,95); 7,3683 (6,62); 7,3629 (6,36); 7,3575 (4,06); 7,3507 (4,86); 7,3491 (5,4); 7,3457 (3,92); 7,3441 (3,84); 7,3312 (2,95); 7,3262 (2,31); 7,0511 (7,51); 7,0389 (7,38); 7,0319 (7,26); 7,0197 (7,1); 5,7567 (0,94); 4,5031 (7,3); 4,4884 (16); 4,4738 (7,71); 3,6539 (3,83); 3,6395 (10,87); 3,6251 (10,56); 3,6106 (3,51); 3,325 (36,04); 2,6711 (0,39); 2,5242 (1,47); 2,5109 (23,79); 2,5065 (46,61); 2,5019 (60,68); 2,4974 (44,11); 2,493 (21,44); 2,3286 (0,41); 1,9892 (0,9); 1,3366 (0,75); 1,2496 (0,91); 1,175 (0,49); - 0,0002 (2,49) |
| Verbindung Nr. 1-8, Solvent: [DMSO], Spektrometer: 399.95MHz<br>8,5946 (1,92); 8,581 (3,66); 8,5673 (1,93); 8,1423 (6,72); 8,1382 (7,16); 8,1301 (7,07); 8,126 (7,09); 7,9081 (6,71); 7,904 (6,68); 7,8982 (0,58); 7,8889 (7,4); 7,8848 (7,24); 7,8803 (6,62); 7,878 (6,78); 7,8605 (6,79); 7,8582 (6,69); 7,4538 (2,91); 7,4512 (3,01); 7,4351 (6,91); 7,4324 (6,93); 7,4164 (4,56); 7,4137 (4,42); 7,3186 (6,45); 7,3145 (7,13); 7,2997 (5,2); 7,2955 (5,05); 7,1814 (3,87); 7,1772 (3,77); 7,1622 (5,67); 7,158 (5,41); 7,1432 (3,3); 7,1389 (3,06); 7,0512 (7,48); 7,039 (7,28); 7,032 (7,15); 7,0198 (7,05); 5,7563 (1,36); 4,5058 (7,03); 4,4909 (16); 4,476 (7,48); 3,6449 (3,68); 3,6303 (10,55); 3,6159 (10,24); 3,6011 (3,36); 3,3232 (54,71); 2,6752 (0,49); 2,6708 (0,64); 2,6661 (0,46); 2,524 (2,03); 2,5107 (37,32); 2,5062 (73,77); 2,5016 (96,79); 2,4971 (70,12); 2,4926 (33,56); 2,3328 (0,46); 2,3284 (0,63); 2,3237 (0,45); 1,9889 (0,55); 1,336 (0,75); 1,2496 (0,97); -0,0002 (3,84) |
| Verbindung Nr. 1-9, Solvent: [DMSO], Spektrometer: 399.95MHz<br>8,9333 (1,93); 8,9203 (3,44); 8,9072 (1,89); 8,1368 (6,83); 8,1326 (7,3); 8,1246 (7,18); 8,1204 (7,15); 7,9073 (7,02); 7,9032 (6,96); 7,8881 (7,52); 7,884 (7,03); 7,5403 (1,3); 7,5237 (2,86); 7,5191 (2,58); 7,507 (1,96); 7,5026 (5,47); 7,4981 (2,04); 7,4858 (2,68); 7,4815 (3,23); 7,4649 (1,49); 7,1826 (1,24); 7,1795 (1,66); 7,172 (8,97); 7,1527 (11,37); 7,132 (7,6); 7,1243 (1,35); 7,0531 (7,63); 7,0408 (7,39); 7,0339 (7,26); 7,0216 (7,09); 4,4754 (7,5); 4,461 (16); 4,4466 (7,91); 3,6791 (3,98); 3,6649 (11,09); 3,6506 (10,74); 3,6364 (3,61); 3,3242 (62,43); 3,31 (0,38); 2,6756 (0,45); 2,6709 (0,6); 2,6665 (0,43); 2,5241 (2,2); 2,5109 (34,82); 2,5065 (67,05); 2,502 (86,82); 2,4974 (62,84); 2,4929 (30,3); 2,3331 (0,41); 2,3287 (0,55); 2,3241 (0,41); 1,3363 (1,11); 1,2497 (1,37); -0,0002 (3,16) |
| Verbindung Nr. 1-10, Solvent: [DMSO], Spektrometer: 399.95MHz |

(fortgesetzt)

| |
|---|
| 8,7155 (1,98); 8,702 (3,69); 8,6889 (1,96); 8,144 (6,72); 8,1399 (7,07); 8,1318 (7,05); 8,1277 (7); 7,9115 (6,74); 7,9075 (6,53); 7,8924 (7,17); 7,8882 (6,63); 7,7801 (4,83); 7,7606 (6,4); 7,7362 (2,1); 7,7176 (5,47); 7,699 (4); 7,6573 (3,85); 7,6381 (4,89); 7,6192 (1,81); 7,5103 (5,82); 7,4915 (4,87); 7,0541 (7,36); 7,0419 (7,18); 7,0349 (7,05); 7,0227 (6,95); 5,757 (2,59); 4,4825 (7,25); 4,4679 (16); 4,4533 (7,67); 3,6571 (3,83); 3,6427 (10,84); 3,6283 (10,5); 3,6139 (3,46); 3,3247 (50,54); 2,6756 (0,39); 2,671 (0,52); 2,6665 (0,38); 2,5243 (1,76); |
| 2,5109 (31,68); 2,5065 (61,85); 2,502 (80,34); 2,4974 (58,04); 2,493 (27,81); 2,3332 (0,4); 2,3288 (0,51); 2,3243 (0,38); 1,9891 (0,46); 1,3366 (0,72); 1,2497 (0,91); -0,0002 (2,89) |
| Verbindung Nr. 1-11, Solvent: [DMSO], Spektrometer: 399.95MHz<br>8,823 (1,96); 8,8094 (3,58); 8,7962 (1,92); 8,4847 (0,37); 8,4708 (6,39); 8,4659 (6,69); 8,4587 (6,71); 8,4539 (6,51); 8,316 (0,5); 8,1423 (6,67); 8,1382 (7,3); 8,1301 (6,99); 8,126 (6,95); 8,1157 (0,58); 7,922 (0,37); 7,9126 (6,78); 7,9085 (6,69); 7,8934 (7,57); 7,8893 (6,78); 7,8795 (0,6); 7,8687 (6,56); 7,8638 (6,62); 7,8499 (7,54); 7,845 (7,37); 7,8247 (0,47); 7,6927 (0,37); 7,6743 (0,36); 7,521 (0,57); 7,5049 (7,6); 7,4929 (7,28); 7,4861 (6,75); 7,4741 (6,55); 7,055 (7,39); 7,0427 (7,2); 7,0358 (7,06); 7,0236 (6,87); 6,5738 (2,9); 4,5072 (7,47); 4,4929 (16); 4,4786 (7,88); 3,7336 (0,74); 3,7185 (0,47); 3,6769 (3,96); 3,6627 (11,19); 3,6485 (10,86); 3,6343 (3,62); 3,5788 (0,6); 3,5642 (0,49); 3,3225 (134,17); 2,6752 (1,15); 2,6707 (1,55); 2,6662 (1,11); 2,6616 (0,53); 2,5238 (5,54); 2,5105 (89,08); 2,5061 (173,3); 2,5016 (226,16); 2,4971 (163,99); 2,4926 (79,08); 2,3373 (0,57); 2,3329 (1,12); 2,3284 (1,49); 2,3239 (1,06); 2,3194 (0,5); 1,9888 (0,55); 1,3358 (0,52); 1,2983 (0,35); 1,2586 (0,56); 1,2493 (0,81); 1,235 (1,49); 1,1748 (0,34); -0,0002 (6,57) |
| Verbindung Nr. 1-12, Solvent: [DMSO], Spektrometer: 399.95MHz<br>8,3525 (2,3); 8,3394 (4,18); 8,3261 (2,25); 8,3159 (1,27); 8,1334 (6,69); 8,1293 (6,81); 8,1211 (7,01); 8,1171 (6,65); 7,9049 (7,12); 7,9008 (6,74); 7,8857 (7,62); 7,8816 (6,83); 7,7097 (12,31); 7,697 (12,92); 7,3366 (0,66); 7,3324 (0,66); 7,2183 (13,38); 7,2055 (12,71); 7,1815 (1,17); 7,1762 (0,71); 7,0501 (7,33); 7,0379 (7,26); 7,0309 (7,11); 7,0187 (6,86); 5,7564 (0,42); 4,5015 (7,42); 4,4868 (16); 4,4723 (7,78); 3,6655 (3,98); 3,6511 (10,96); 3,6368 (10,66); 3,6224 (3,59); 3,3221 (169,56); 2,6754 (1,52); 2,671 (2); 2,6665 (1,45); 2,5103 (126,03); 2,5064 (232,83); 2,5019 (294,1); 2,4974 (214,49); 2,3331 (1,53); 2,3286 (2); 2,3241 (1,46); 1,351 (0,78); 1,3359 (11,56); 1,2986 (1,04); 1,2587 (1,99); 1,2497 (13,56); 1,2352 (2,69); 1,1875 (0,76); 1,1478 (0,38); 0,8541 (0,41); 0,0079 (0,4); -0,0001 (8,45); -0,0084 (0,35) |
| Verbindung Nr. 1-13, Solvent: [DMSO], Spektrometer: 399.95MHz<br>8,3163 (0,6); 8,2175 (0,79); 8,204 (1,37); 8,1919 (0,76); 8,137 (0,45); 8,1268 (2,53); 8,1229 (2,55); 8,1147 (2,76); 8,1106 (2,44); 7,9085 (2,51); 7,9044 (2,34); 7,8893 (2,65); 7,8853 (2,37); 7,8425 (4,48); 7,8293 (4,62); 7,3365 (0,79); 7,333 (0,72); 7,182 (1,34); 7,1767 (0,78); 7,1477 (4,72); 7,1345 (4,52); 7,0507 (2,71); 7,0385 (2,67); 7,0315 (2,6); 7,0193 (2,49); 6,574 (2,18); 4,5135 (3,03); 4,499 (6,48); 4,4847 (3,17); 3,6937 (1,62); 3,6793 (4,63); 3,6651 (4,48); 3,6507 (1,48); 3,3228 (240,25); 2,6757 (1,66); 2,6713 (2,19); 2,6666 (1,54); 2,6624 (0,7); 2,5242 (8,37); 2,5109 (136,82); 2,5067 (256,87); 2,5022 (327,09); 2,4977 (233,46); 2,4934 (110,5); 2,3335 (1,67); 2,329 (2,19); 2,3244 (1,54); 1,3516 (0,87); 1,3362 (13,59); 1,2988 (1,08); 1,2591 (1,99); 1,2501 (16); 1,236 (2,12); 1,1878 (0,94); 1,1484 (0,41); 0,8544 (0,35); 0,0083 (0,35); 0,0003 (8,84) |
| Verbindung Nr. 1-14, Solvent: [DMSO], Spektrometer: 399.95MHz<br>8,5191 (1,8); 8,5052 (3,44); 8,4914 (1,79); 8,126 (6,24); 8,1218 (6,68); 8,1138 (6,56); 8,1096 (6,6); 7,9049 (13,44); 7,9003 (16); 7,8975 (7,67); 7,8823 (7); 7,8782 (6,46); 7,0472 (7,07); 7,035 (6,85); 7,028 (6,74); 7,0158 (6,61); 6,8501 (13,56); 6,8453 (13,35); 4,4823 (6,66); 4,4675 (15,09); 4,4527 (7,07); 3,6325 (3,5); 3,6179 (10,22); 3,6034 (9,96); 3,5887 (3,22); 3,3234 (94,86); 2,6803 (0,32); 2,6759 (0,68); 2,6714 (0,95); 2,6668 (0,69); 2,5246 (3,17); 2,5113 (54,87); 2,5069 (108,29); 2,5023 (142,67); 2,4977 (103,38); 2,4932 (49,73); 2,3382 (0,35); 2,3336 (0,71); 2,329 (0,95); 2,3244 (0,68); 2,3199 (0,33); 1,336 (2,65); 1,2585 (0,49); 1,2496 (3,32); 1,2342 (0,71); -0,0002 (4,46) |
| Verbindung Nr. 1-15, Solvent: [DMSO], Spektrometer: 399.95MHz<br>8,4628 (2,84); 8,3169 (0,37); 8,207 (11,18); 8,2011 (15,18); 8,171 (14,45); 8,1651 (10,66); 7,6256 (2,56); 7,6211 (2,97); 7,6072 (4,99); 7,6025 (5,95); 7,5925 (0,74); 7,5876 (3,14); 7,5832 (3,12); 7,5523 (1,47); 7,5477 (1,4); 7,5391 (1,65); 7,5342 (3,27); 7,5312 (2,51); 7,5294 (2,4); 7,5271 (2,07); 7,5202 (2,63); 7,5185 (2,53); 7,5161 (2,52); 7,5133 (3,53); 7,5085 (1,94); 7,4999 (1,99); 7,4953 (1,72); 7,3788 (0,82); 7,3017 (4,26); 7,2916 (4,81); 7,2892 (4,64); 7,2806 (4,16); 7,2732 (10,06); 7,2537 (8,53); 4,4986 (7,3); 4,4841 (16); 4,4697 (7,79); 3,6787 (3,6); 3,6645 (10,28); 3,6502 (10,04); 3,6359 (3,36); 3,3253 (39,68); 2,6766 (0,37); 2,6721 (0,49); 2,6674 (0,36); 2,5254 (1,53); 2,512 (28,48); 2,5076 (56,26); 2,503 (73,42); 2,4984 (53,33); 2,494 (25,81); 2,3342 (0,34); 2,3298 (0,48); 2,3253 (0,35); 2,0612 (0,52); 2,0418 (0,5); 1,3368 (1,84); 1,3181 (0,36); 1,2729 (0,36); 1,2498 (2,21); 1,1881 (0,46); -0,0002 (2,5) |

(fortgesetzt)

| |
|---|
| Verbindung Nr. 1-16, Solvent: [DMSO], Spektrometer: 399.95MHz<br>8,6294 (2,12); 8,616 (4,03); 8,6027 (2,13); 8,2196 (10,99); 8,2137 (14,01); 8,1748 (12,77); 8,169 (9,97); 7,493 (3,9); 7,4736 (8,74); 7,4722 (8,38); 7,455 (3,58); 7,4481 (4,25); 7,4397 (4,64); 7,4328 (6,58); 7,4289 (1,88); 7,4198 (2,42); 7,4129 (5,92); 7,398 (12,53); 7,3955 (11,71); 7,3923 (14,07); 7,3801 (4,62); 7,3768 (4,61); 7,3611 (1,17); 7,3579 (1,12); 5,7567 (0,94); 4,4947 (7,41); 4,4804 (16); 4,4661 (7,79); 3,6557 (3,91); 3,6415 (11,01); 3,6273 (10,66); 3,613 (3,54); 3,3238 (41,2); 2,6756 (0,44); 2,6711 (0,6); 2,6668 (0,43); 2,5243 (1,93); 2,511 (34,39); 2,5066 (67,44); 2,5021 (88); 2,4976 (64,27); 2,4932 (31,35); 2,3333 (0,4); 2,3288 (0,54); 2,3245 (0,39); 1,3364 (1,72); 1,2496 (2,1); 1,2345 (0,39); -0,0002 (2,65) |
| Verbindung Nr. 1-18, Solvent: [DMSO], Spektrometer: 399.95MHz<br>8,5901 (2,13); 8,5763 (4,19); 8,5625 (2,12); 8,2209 (12,78); 8,215 (16); 8,1723 (15,92); 8,1664 (12,64); 7,879 (6,65); 7,8767 (7,02); 7,8592 (7,27); 7,8569 (7,19); 7,4547 (3,16); 7,452 (3,27); 7,4359 (7,42); 7,4332 (7,5); 7,4172 (4,91); 7,4145 (4,78); 7,3149 (6,87); 7,3108 (7,62); 7,296 (5,6); 7,2918 (5,45); 7,1817 (4,36); 7,1775 (4,14); 7,1625 (6,05); 7,1583 (5,77); 7,1434 (3,64); 7,1392 (3,33); 5,7564 (0,34); 4,4968 (6,84); 4,4822 (15,38); 4,4677 (7,23); 3,6411 (3,61); 3,6268 (10,3); 3,6124 (9,99); 3,5979 (3,27); 3,3225 (68,56); 2,6753 (0,58); 2,6708 (0,81); 2,6662 (0,58); 2,5241 (2,63); 2,5193 (4,22); 2,5108 (45,29); 2,5063 (89,94); 2,5017 (118,6); 2,4971 (85,59); 2,4926 (40,71); 2,333 (0,57); 2,3284 (0,77); 2,3239 (0,56); 1,989 (0,48); 1,3359 (1,94); 1,2586 (0,36); 1,2495 (2,5); 1,2345 (0,4); -0,0002 (4,14) |
| Verbindung Nr. 1-19, Solvent: [DMSO], Spektrometer: 399.95MHz<br>8,9233 (1,87); 8,9098 (3,5); 8,8965 (1,86); 8,2136 (11,78); 8,2078 (15,55); 8,174 (16); 8,1681 (12,07); 7,5404 (1,33); 7,5238 (2,85); 7,5191 (2,52); 7,5071 (1,84); 7,5026 (5,49); 7,4981 (1,94); 7,4859 (2,65); 7,4815 (3,22); 7,4649 (1,51); 7,1824 (1,11); 7,1792 (1,5); 7,1717 (9,2); 7,1524 (11,24); 7,1425 (1,44); 7,1317 (7,77); 7,124 (1,31); 5,7564 (1,55); 4,4717 (6,79); 4,4575 (14,5); 4,4433 (7,24); 3,6753 (3,55); 3,6612 (9,98); 3,6471 (9,68); 3,633 (3,27); 3,3233 (75,21); 2,6756 (0,5); 2,671 (0,72); 2,6662 (0,52); 2,5243 (2,38); 2,5193 (3,74); 2,5109 (41,31); 2,5064 (82,92); 2,5019 (109,87); 2,4973 (79,77); 2,4927 (38,45); 2,3333 (0,55); 2,3286 (0,75); 2,324 (0,54); 1,3358 (0,83); 1,2495 (0,9); 0,008 (1,65); -0,0002 (47,98); -0,0085 (1,59) |
| Verbindung Nr. 1-20, Solvent: [DMSO], Spektrometer: 399.95MHz<br>8,7112 (1,85); 8,6977 (3,57); 8,6839 (1,84); 8,3161 (2,56); 8,2246 (12,63); 8,2187 (16); 8,1782 (15); 8,1723 (11,66); 7,7793 (4,55); 7,7599 (6,05); 7,7369 (1,97); 7,7195 (5,22); 7,7008 (3,79); 7,6586 (3,61); 7,6394 (4,62); 7,6204 (1,73); 7,5078 (5,53); 7,489 (4,66); 5,7564 (0,71); 4,4739 (6,53); 4,4595 (14,43); 4,4452 (6,92); 3,6534 (3,33); 3,6392 (9,44); 3,625 (9,18); 3,6107 (3,06); 3,3241 (92,23); 3,3003 (0,91); 2,6755 (0,54); 2,6711 (0,75); 2,6665 (0,55); 2,5244 (2,62); 2,5197 (4,12); 2,5111 (43,72); 2,5066 (87,05); 2,502 (114,72); 2,4974 (82,67); 2,4928 (39,27); 2,3334 (0,59); 2,3288 (0,78); 2,3242 (0,57); 1,3361 (1); 1,2496 (1,28); 0,008 (1,97); -0,0002 (55,4); -0,0086 (1,75) |
| Verbindung Nr. 1-21, Solvent: [DMSO], Spektrometer: 399.95MHz<br>8,8179 (2,16); 8,8042 (4,07); 8,7906 (2,12); 8,472 (7,01); 8,4672 (7,48); 8,46 (7,48); 8,4551 (7,33); 8,3156 (2,8); 8,2234 (12,42); 8,2175 |
| (15,92); 8,1801 (16); 8,1742 (12,38); 7,8707 (7,31); 7,8658 (7,43); 7,8519 (8,38); 7,847 (7,92); 7,5064 (8,23); 7,4943 (8,01); 7,4875 (7,51); 7,4755 (7,32); 5,7562 (0,42); 4,4992 (7,32); 4,4851 (15,51); 4,471 (7,79); 3,6736 (3,81); 3,6596 (10,75); 3,6454 (10,46); 3,6313 (3,55); 3,3229 (204,99); 3,2992 (1,58); 2,6796 (0,5); 2,6753 (1,08); 2,6707 (1,52); 2,6663 (1,08); 2,6617 (0,52); 2,524 (4,78); 2,5107 (87,35); 2,5062 (173,27); 2,5017 (227,98); 2,4971 (166,85); 2,4927 (81,56); 2,3373 (0,57); 2,333 (1,15); 2,3285 (1,56); 2,3239 (1,14); 2,3195 (0,56); 1,3357 (0,39); 1,2586 (0,34); 1,2494 (0,51); 1,234 (0,33); 0,1459 (0,36); 0,0079 (3,21); -0,0002 (90,25); -0,0085 (3,42); -0,1497 (0,4) |
| Verbindung Nr. 1-22, Solvent: [DMSO], Spektrometer: 399.95MHz<br>8,3452 (2,08); 8,3315 (3,94); 8,3172 (2,07); 8,2057 (11,24); 8,1998 (14,92); 8,1932 (1,04); 8,166 (16); 8,1601 (11,87); 7,8415 (0,35); 7,8283 (0,35); 7,7404 (0,51); 7,7306 (0,35); 7,7099 (14); 7,6972 (14,54); 7,2166 (14,91); 7,2039 (14,09); 7,1463 (0,34); 5,7569 (0,6); 4,497 (6,57); 4,4827 (14,1); 4,4684 (6,79); 3,6621 (3,5); 3,6479 (9,66); 3,6336 (9,43); 3,6193 (3,28); 3,3246 (50,2); 2,6767 (0,39); 2,6721 (0,53); 2,6674 (0,37); 2,5254 (1,83); 2,5205 (2,99); 2,512 (31,02); 2,5076 (61,25); 2,503 (80,27); 2,4984 (57,89); 2,4939 (27,62); 2,3342 (0,39); 2,3297 (0,52); 2,3251 (0,37); 2,1006 (0,5); 2,0384 (0,43); 1,3365 (1,61); 1,2991 (0,52); 1,2588 (0,78); 1,2496 (1,93); 1,1878 (0,76); 0,008 (1,41); - 0,0002 (38,07); -0,0085 (1,26) |
| Verbindung Nr. 1-23, Solvent: [DMSO], Spektrometer: 399.95MHz |

(fortgesetzt)

| |
|---|
| 8,3161 (0,9); 8,2164 (1,85); 8,1999 (12,19); 8,194 (16); 8,1717 (15,01); 8,1658 (10,05); 7,8413 (12,95); 7,8281 (13,44); 7,1462 (13,84); 7,133 (13,38); 4,5076 (6,56); 4,4933 (14,2); 4,4791 (6,96); 3,6864 (3,48); 3,6723 (9,92); 3,658 (9,64); 3,6438 (3,2); 3,324 (113,44); 3,3005 (0,34); 2,6761 (0,61); 2,6716 (0,85); 2,667 (0,64); 2,5249 (2,67); 2,5201 (3,95); 2,5115 (46,64); 2,507 (94,88); 2,5024 (126,43); 2,4979 (92,04); 2,4933 (44,39); 2,3338 (0,59); 2,3292 (0,83); 2,3246 (0,59); 1,3359 (1,85); 1,2985 (0,55); 1,2586 (0,83); 1,2495 (2,17); 1,1874 (0,8); 0,0079 (1,77); -0,0003 (54,36); -0,0086 (1,77) |
| Verbindung Nr. 1-24, Solvent: [DMSO], Spektrometer: 399.95MHz<br>8,5144 (1,98); 8,5005 (3,89); 8,4865 (1,99); 8,1974 (11,54); 8,1915 (16); 8,1641 (14,77); 8,1582 (10,52); 7,9049 (14,26); 7,9001 (14,22); 6,8501 (15,25); 6,8453 (15); 5,7572 (0,48); 4,4757 (6,7); 4,4611 (15,08); 4,4466 (7,08); 3,6273 (3,52); 3,6129 (10,25); 3,5984 (9,98); 3,5839 (3,23); 3,3252 (75,56); 2,6768 (0,48); 2,6721 (0,64); 2,6678 (0,46); 2,5256 (1,94); 2,5207 (3,12); 2,5123 (37,61); 2,5078 (75,19); 2,5032 (99,08); 2,4986 (71,38); 2,4941 (33,88); 2,3344 (0,47); 2,3299 (0,65); 2,3255 (0,48); 2,1031 (0,56); 2,038 (0,5); 1,3365 (1,43); 1,2991 (0,47); 1,2589 (0,7); 1,2498 (1,7); 0,008 (0,9); -0,0002 (26,9); -0,0085 (0,82) |
| Verbindung Nr. 1 - 25:<br>$^1$H-NMR(400,0 MHz, DMSO): δ= 8,724(1,3);8,710(2,6);8,695(1,3);8,585(0,6);8,583(0,7);8,580(0,6);8,578(0,6); 8,568(4,0);8,565(4,9);8,562(4, 8);8,560(4,0);8,408(0,5);8,402(0,4);8,371(5,5);8,366(5,2);8,324(0,7);8,321(0,7); 8,318(1,2);7,970(0,8);7,965(0,8);7,760(3,1);7,742(3,9);7,710 (1,3);7,693(3,3);7,674(2,5);7,644(2,5);7,625(3,0); 7,606(1,1);7,434(3,5);7,415(3,1);5,508(1,2);5,496(1,5);5,491(2,1);5,480(2,0);5,476(1,6);5,4 64(1,2);4,813(0,8); 4,801(0,9);3,656(0,9);3,644(1,2);3,641(1,2);3,629(1,1);3,621(1,7);3,609(2,0);3,607(2,0);3,595(1,5);3,533(1,5); 3,518(2,2); 3,502(1,8);3,483(1,3);3,468(0,9);3,399(0,4);3,325(22,2);2,526(0,9);2,521(1,4);2,512(16,1);2,508 (32,1);2,503(42,6);2,499(30,9);2,494(14,7);1 ,380(16,0);1,364(15,8);1,337(1,3);1,300(0,6);1,259(0,9);1,250 (2,0);1,232(1,2);1,073(2,8);1,058(2,8);0,008(0,8);0,000(20,8);-0,009(0,7) |
| Verbindung Nr. 1-26:<br>$^1$H-NMR(400,0 MHz, CD3CN): δ= 8,426(4,6);8,423(5,8);8,420(5,9);8,418(4,7);8,064(6,6);8,058(6,3);7,477(0,4); 7,472(0,7);7,467(0,4);7,459( 0,8);74493,0000(4,4);74485,0000(4,3);7,447(5,4);7,445(4,8);7,437(1,2);7,431(5,9); 7,427(13,3);7,423(9,8);7,421(6,9);7,416(4,5);7,403(7,3);7 ,399(4,8);7,389(1,2);7,383(3,0);7,379(2,2);7,371(1,0); 7,366(5,8);7,361(4,8);7,348(5,4);7,344(5,0);7,331(1,9);7,326(1,9);7,043(1,2);4,635(9,9 );4,622(16,0);4,608 (10,5);3,791(5,6);3,777(12,7);3,764(11,4);3,749(5,1);3,741(0,5);3,737(0,5);3,727(0,6);3,722(1,6);3,710(1,2); 3,708(1,3);3, 681(0,7);3,679(0,7);3,667(1,4);3,661(0,4);3,652(1,0);3,648(0,6);2,136(20,2);2,107(0,4);1,964(3,8); 1,958(5,7);1,952(25,1);1,946(43,8);1,940( 57,9);1,933(40,0);1,927(20,5);1,915(0,3);1,768(0,3);1,436(2,6);1,372 (1,4);1,340(0,6);1,285(1,0);1,276(2,0);1,269(2,3);1,200(0,4);1,014(0,4); 0,000(1,1) |
| Verbindung Nr. 1-27:<br>$^1$H-NMR(400,0 MHz, CD3CN): δ= 8,427(4,6);8,425(5,8);8,422(5,9);8,419(4,7);8,064(6,4);8,063(6,3);8,058(6,4); 8,057(6,0);7,619(4,0);7,618( 5,1);7,617(5,2);7,616(4,2);7,599(4,5);7,598(7,7);7,596(4,6);7,415(0,7);7,413(0,6); 7,407(0,4);7,396(5,2);7,394(6,0);7,391(6,8);7,390(7,0);7,38 2(15,8);7,380(16,0);7,372(1,5);7,371(1,3);7,346 (0,7);7,336(4,9);7,326(3,4);7,322(3,1);7,316(4,2);7,313(2,8);7,306(2,8);7,302(3,0);7,293(2,3) ;6,997(1,2);4,635 (10,0);4,622(15,9);4,608(10,7);3,785(5,6);3,770(12,2);3,757(11,3);3,747(0,8);3,743(5,2);2,134(13,5);2,107(0,3); 1,964(3,4); 1,958(4,9);1,952(22,8);1,946(40,4);1,940(53,6);1,934(37,2);1,927(19,2);1,921(0,6);1,768(0,3);1,436 (1,7);1,372(0,6);1,276(0,7);0,000(1,1) |
| Verbindung Nr. 1-28:<br>$^1$H-NMR(400,0 MHz, CD3CN): δ= 8,429(4,6);8,426(5,7);8,424(5,7);8,421(4,4);8,063(6,7);8,057(6,4);7,888(5,2); 7,885(5,2);7,868(5,5);7,865( 5,4);7,436(2,6);7,433(2,5);7,417(6,2);7,415(5,9);7,398(4,2);7,396(4,0);7,330(5,8); 7,326(6,3);7,311(4,1);7,307(4,0);7,160(3,4);7,155(3,2);7,14 1(4,8);7,136(4,5);7,121(2,9);7,117(2,6);6,958(1,3); 4,640(9,4);4,627(16,0);4,613(10,0);3,779(5,3);3,764(12,1);3,751(11,5);3,737(4,9);2,135(5 4,6);2,120(0,7);2,113 (0,9);2,107(1,2);2,101(0,9);2,095(0,4);1,971(1,0);1,964(10,7);1,958(16,3);1,952(71,6);1,946(125,5);1,940 (164,4);1,933( 113,4);1,927(58,1);1,914(0,8);1,780(0,4);1,774(0,7);1,768(1,0);1,762(0,7);1,756(0,3);1,437(1,8); 1,372(0,9);1,285(0,4);1,277(1,1);1,271(0,4); 1,222(0,4);0,000(3,2) |
| Verbindung Nr. 1-29: |

(fortgesetzt)

| |
|---|
| $^1$H-NMR(400,0 MHz, CD3CN): δ= 8,424(1,9);8,421(2,3);8,419(2,3);8,416(1,8);8,064(2,6);8,063(2,5);8,058(2,5);8,057(2,4);7,334(0,6);7,330( 0,8);7,321(1,3);7,316(2,4);7,312(1,8);7,302(2,0);7,298(2,9);7,295(2,5);7,238(1,8);7,236(2,3);7,235(1,9);7,229(0,4);7,228(0,3);7,217(2,2);7,21 6(2,2);7,199(1,6);7,197(1,6);7,181(0,6);7,179(0,7);7,178(0,6);6,889(0,4);4,636(4,1);4,631(0,6);4,622(6,4);4,609(4,3);3,773(2,3);3,769(0,4);3, 759(5,1);3,746(4,6);3,731(2,1);2,552(1,8);2,359(16,0);2,197(0,4);2,159(3,2);2,120(0,5);2,113(0,4);2,107(0,4);1,964(2,0);1,958(2,9);1,952(13, 1);1,946(23,0);1,940(30,0);1,934(20,6);1,927(10,6);1,372(0,7);1,285(0,4);1,276(0,9);0,000(0,6) |
| Verbindung Nr. 1-30:<br>$^1$H-NMR(400,0 MHz, DMSO): δ= 8,720(4,2);8,705(8,1);8,691(4,2);8,511(14,0);8,509(14,0);8,363(16,0);8,358(15,1);7,741(8,5);7,723(11,6);7 ,681(3,5);7,663(9,7);7,645(8,0);7,627(7,9);7,608(8,7);7,590(3,0);7,343(10,4);7,324(9,2);5,031(3,2);5,021(3,8);5,013(4,9);5,010(4,7);5,003(4, 8);5,000(4,9);4,992(4,1);4,982(3,3);3,839(2,9);3,829(3,4);3,824(3,3);3,814(3,3);3,804(4,1);3,794(4,2);3,789(4,3);3,779(3,5);3,558(3,5);3,543 (4,9);3,525(5,0);3,508(3,9);3,506(3,9);3,492(2,8);3,324(49,6);2,676(0,5);2,672(0,6);2,667(0,5);2,525(1,9);2,507(73,3);2,503(95,9);2,498(71,5 );2,334(0,5);2,330(0,7);2,325(0,5);1,337(4,0);1,300(2,9);1,282(1,9);1,273(2,7);1,260(7,2);1,250(8,1);1,241(5,4);1,229(3,2);1,220(2,1);1,208( 1,0);1,189(0,4);0,644(1,0);0,630(1,9);0,622(4,1);0,613(4,1);0,609(4,7);0,600(5,4);0,592(2,8);0,587(3,2);0,579(2,5);0,570(2,3);0,560(3,5);0,55 8(3,4);0,548(4,6);0,538(4,8);0,527(4,3);0,515(2,0);0,506(3,0);0,494(3,3);0,484(4,8);0,472(6,4);0,460(5,0);0,448(2,2);0,438(2,8);0,427(5,2);0,415(6,0);0,403(4,8);0,393(2,7);0,381(1,1);0,000(1,7) |
| Verbindung Nr. 1-31:<br>$^1$H-NMR(400,0 MHz, DMSO): δ= 8,489(2,3);8,316(0,4);8,209(7,6);8,203(7,6);7,823(7,0);7,816(6,7);7,801(7,2);7,794(7,0);7,615(2,4);7,611(2 ,8);7,597(4,6);7,592(5,5);7,583(0,5);7,577(3,0);7,573(3,0);7,549(1,4);7,544(1,3);7,535(1,5);7,531(3,0);7,528(2,1);7,526(2,0);7,523(1,8);7,517 (2,3);7,513(2,1);7,510(3,2);7,505(1,7);7,496(1,8);7,492(1,6);7,296(3,9);7,285(4,3);7,283(4,4);7,277(3,1);7,275(3,6);7,271(3,8);7,266(8,1);7,2 50(2,8);7,247(7,5);6,887(9,3);6,865(8,9);4,395(7,2);4,380(16,0);4,365(7,7);3,648(3,5);3,633(9,9);3,619(9,7);3,605(3,2);3,324(141,6);2,680(0, 4);2,675(0,9);2,671(1,3);2,666(0,9);2,662(0,4);2,524(3,7);2,519(5,8);2,511(70,8);2,506(143,1);2,502(189,7);2,497(136,7);2,493(64,8);2,338( 0,4);2,333(0,9);2,329(1,3);2,324(0,9);2,320(0,4);1,336(1,3);1,298(0,4);1,259(0,5);1,250(1,6);1,235(0,5);0,008(0,7);0,000(22,5);-0,009(0,7) |
| Verbindung Nr. 1-32:<br>$^1$H-NMR(400,0 MHz, DMSO): δ= 8,642(1,4);8,629(2,7);8,616(1,5);8,316(1,8);8,218(6,8);8,217(7,1);8,212(7,2);8,210(6,9);7,825(6,7);7,818(6 ,4);7,803(6,9);7,796(6,8);7,490(3,4);7,488(2,6);7,472(5,6);7,471(6,9);7,469(6,5);7,452(3,4);7,444(4,0);7,437(4,1);7,429(5,7);7,425(1,7);7,417 (2,3);7,410(3,9);7,405(0,6);7,399(2,1);7,398(2,3);7,386(16,0);7,383(8,7);7,380(6,5);7,371(4,5);7,368(4,0);7,364(0,9);7,352(0,9);7,349(0,9);6, 882(8,7);6,881(8,6);6,860(8,4);6,859(8,2);4,387(6,4);4,373(14,4);4,358(6,9);3,622(3,3);3,608(9,3);3,594(9,0);3,579(3,0);3,323(100,9);3,299( 0,6);2,680(0,4);2,675(0,7);2,671(1,0);2,666(0,7);2,662(0,3);2,524(3,2);2,519(4,9);2,511(56,4);2,506(113,3);2,502(149,6);2,497(106,9);2,493( 50,2);2,338(0,3);2,333(0,7);2,328(1,0);2,324(0,7);2,319(0,3);1,336(0,7);1,298(0,7);1,259(1,1);1,250(0,9);0,008(1,4);0,000(43,2);-0,009(1,2) |
| Verbindung Nr. 1-33:<br>$^1$H-NMR(400,0 MHz, DMSO): δ= 8,635(1,8);8,621(3,3);8,608(1,7);8,219(7,8);8,218(8,0);8,212(8,5);8,211(7,9);7,823(7,8);7,816(7,4);7,801(8 ,1);7,794(7,8);7,650(1,7);7,647(3,7);7,644(7,1);7,631(2,5);7,626(5,1);7,624(8,0);7,437(2,2);7,434(2,4);7,421(2,8);7,416(7,7);7,400(6,7);7,397 (6,2);7,364(14,5);7,361(8,0);7,346(11,0);7,343(6,4);7,341(6,3);7,328(3,3);7,323(2,4);7,181(0,8);7,176(0,5);6,888(10,7);6,887(10,5);6,875(0,4 );6,866(9,5);6,865(9,3);6,706(0,5);4,388(7,1);4,374(16,0);4,359(7,6);3,992(0,4);3,617(3,7);3,603(10,6);3,588(10,3);3,574(3,4);3,324(78,3);2, 676(0,6);2,671(0,8);2,666(0,6);2,547(0,5);2,541(0,5);2,524(2,5);2,520(3,8);2,511(41,6);2,506(83,3);2,502(109,9);2,497(78,7);2,493(36,9);2,3 33(0,5);2,329(0,7);2,324(0,5);1,354(0,8);1,336(15,6);1,299(2,0);1,259(3,1);1,250(11,9);1,235(1,1);1,225(0,4);0,008(1,1);0,000(31,1);-0,009(0,9) |
| Verbindung Nr. 1-34:<br>$^1$H-NMR(400,0 MHz, DMSO): δ= 8,592(1,9);8,578(3,7);8,565(1,9);8,218(8,1);8,217(8,4);8,212(8,6);8,211(8,1);7,875(6,5);7,873(6,7);7,856(7, 0);7,853(6,9);7,820(7,9);7,813(7,5);7,798(8,2);7,791(7,9);7,445(3,1);7,442(3,2);7,426(7,3);7,423(7,3);7,414(0,4);7,407(4,8);7,405(4,6);7,300 (6,6);7,296(7,4);7,281(5,4);7,277(5,3);7,223(0,3);7,201(0,3);7,177(4,2);7,173(4,0);7,158(6,0);7,154(5,6);7,139(3,6);7,135(3,3);6,900(10,0);6, 899(10,0);6,887(0,4);6,878(9,5);6,877(9,5);5,756(1,9);4,394(7,1);4,380(16,0);4,365(7,6);3,610(3,7);3,596(10,6);3,581(10,2);3,567(3,4);3,323 (98,2);2,675(0,6);2,671(0,8);2,666(0,6);2,524(2,8);2,519(4,4);2,511(46,5);2,506(92,7);2,502(122,4);2,497(88,1);2,493(41,7);2,446(0,3);2,333 (0,6);2,328(0,8);2,324(0,6);2,111(0,6);1,336(0,8);1,298(0,4);1,259(0,5);1,250(1,1);0,008(1,1);0,000(32,7);-0,009(1,0) |

(fortgesetzt)

| |
|---|
| Verbindung Nr. 1-35: $^{1}$H-NMR(400,0 MHz, DMSO): δ= 8,939(1,8);8,925(3,2);8,913(1,7);8,316(1,4);8,217(8,7);8,211(8,3);8,210(8,5); 7,828(8,0);7,822(7,6);7,806(8 ,3);7,800(8,0);7,540(1,5);7,523(3,1);7,518(2,7);7,506(1,9);7,502(5,9);7,497(2,0); 7,485(2,7);7,481(3,4);7,464(1,6);7,181(1,2);7,177(1,6);7,170 (9,8);7,159(1,5);7,156(1,9);7,151(11,7);7,144(2,0); 7,141(1,4);7,130(8,1);7,122(1,3);6,871(10,3);6,849(9,9);4,364(7,4);4,350(16,0);4,336(8,1) ;3,642(3,9);3,628 (10,9);3,614(10,5);3,600(3,6);3,323(331,6);3,300(0,8);2,680(0,6);2,675(1,4);2,671(1,9);2,666(1,4);2,662(0,6); 2,524(5,6);2,5 19(8,6);2,511(104,4);2,506(212,2);2,502(282,3);2,497(203,7);2,492(96,7);2,448(0,6);2,338(0,7); 2,333(1,4);2,328(1,9);2,324(1,4);2,319(0,6); 1,336(1,5);1,298(1,1);1,259(1,6);1,249(1,7);1,234(0,5);0,008(2,2); 0,000(67,9);-0,009(2,1) |
| Verbindung Nr. 1-36: $^{1}$H-NMR(400,0 MHz, DMSO): δ= 8,717(1,9);8,703(3,5);8,689(1,8);8,316(1,3);8,223(8,0);8,221(8,8);8,216(8,5); 8,215(8,6);7,830(8,0);7,823(7 ,6);7,808(8,2);7,801(8,1);7,777(4,7);7,757(6,2);7,726(2,0);7,709(5,3);7,690(4,0); 7,654(3,8);7,635(4,8);7,616(1,8);7,494(5,7);7,475(4,8);6,868 (9,8);6,867(10,3);6,846(9,5);6,845(9,9);5,757(0,6); 4,364(7,2);4,350(16,0);4,335(7,8);3,622(3,7);3,608(10,5);3,594(10,2);3,579(3,4);3,323(10 5,8);3,299(0,4);2,680 (0,3);2,675(0,7);2,671(1,0);2,666(0,7);2,662(0,3);2,524(3,3);2,519(5,1);2,511(55,6);2,506(111,9);2,502(148,2); 2,497(10 7,5);2,493(51,5);2,447(0,4);2,338(0,4);2,333(0,7);2,328(1,0);2,324(0,7);2,320(0,4);1,989(0,5);1,336 (1,0);1,249(1,3);1,235(0,5);1,175(0,3);0,0 08(1,2);0,000(35,3);-0,009(1,1) |
| Verbindung Nr. 1-37: $^{1}$H-NMR(400,0 MHz, DMSO): δ= 8,830(1,8);8,817(3,4);8,803(1,8);8,468(7,3);8,463(7,8);8,456(7,9);8,451(7,7); 8,316(0,4);8,222(8,1);8,221(8 ,9);8,216(8,7);8,214(8,8);7,873(7,7);7,868(7,9);7,855(8,8);7,850(8,3);7,830(8,4); 7,823(8,0);7,808(8,7);7,801(8,5);7,496(8,7);7,484(8,4);7,477 (8,0);7,465(7,8);6,886(10,2);6,885(10,8);6,875 (0,3);6,864(9,8);6,863(10,3);4,394(7,4);4,379(16,0);4,365(8,0);3,641(3,9);3,627(11,0);3,613(1 0,6);3,599(3,6); 3,324(172,2);2,680(0,5);2,675(1,0);2,671(1,4);2,666(1,0);2,662(0,4);2,524(4,2);2,520(6,5);2,511(74,3);2,506 (150,5);2,502(19 9,5);2,497(143,7);2,493(68,0);2,447(0,5);2,338(0,5);2,333(1,0);2,329(1,3);2,324(0,9);2,319 (0,4);1,234(0,4);0,008(1,6);0,000(48,7);-0,009(1,5) |
| Verbindung Nr. 1-38: $^{1}$H-NMR(400,0 MHz, DMSO): δ= 8,366(1,9);8,353(3,5);8,339(1,9);8,316(0,7);8,212(8,4);8,206(8,4);8,205(8,2); 7,821(8,1);7,814(7,8);7,799(8, 4);7,792(8,1);7,709(15,0);7,696(15,8);7,223(0,4);7,215(16,0);7,202(15,2);6,907 (10,1);6,906(10,3);6,885(9,6);6,883(9,8);4,397(6,9);4,382(15, 3);4,368(7,4);3,629(3,6);3,615(10,2);3,600(9,9); 3,586(3,3);3,323(178,2);2,680(0,6);2,675(1,3);2,671(1,9);2,666(1,3);2,662(0,6);2,524(5,3);2, 519(8,1);2,511 (101,6);2,506(207,1);2,502(275,5);2,497(199,2);2,493(95,0);2,338(0,6);2,333(1,3);2,329(1,8);2,324(1,3);2,319 (0,6);1,336(1,7) ;1,259(0,4);1,249(2,1);1,235(0,5);0,008(2,0);0,000(64,2);-0,009(2,0) |
| Verbindung Nr. 1-39: $^{1}$H-NMR(400,0 MHz, DMSO): δ= 8,316(0,4);8,244(1,7);8,231(3,1);8,217(1,8);8,204(8,2);8,203(8,2);8,197(8,6); 8,196(7,9);7,838(15,2);7,825( 16,0);7,821(8,7);7,814(8,0);7,799(8,6);7,792(8,3);7,145(16,0);7,132(15,5);6,891 (10,1);6,890(9,7);6,869(9,7);6,868(9,2);4,408(7,2);4,393(15, 9);4,379(7,7);3,656(3,8);3,642(11,0);3,627(10,7); 3,613(3,5);3,324(71,3);2,676(0,5);2,672(0,7);2,667(0,5);2,525(2,0);2,520(3,1);2,512(38,6);2 ,507(77,9);2,503 (103,1);2,498(73,9);2,494(34,7);2,334(0,5);2,330(0,7);2,325(0,5);1,336(2,1);1,259(0,5);1,250(2,7);0,008(0,8); 0,000(25,2);-0,009(0,7) |
| Verbindung Nr. 1-40: $^{1}$H-NMR(400,0 MHz, DMSO): δ= 8,538(1,7);8,524(3,3);8,510(1,7);8,316(0,5);8,203(7,4);8,202(7,7);8,197(8,0); 8,195(7,5);7,898(15,0);7,894( 15,2);7,814(7,6);7,808(7,3);7,792(7,8);7,786(7,7);6,886(9,3);6,885(9,1);6,864 (8,9);6,863(9,0);6,847(16,0);6,842(15,8);4,377(6,4);4,363(14,3) ;4,348(6,8);3,600(3,3);3,585(9,7);3,571(9,5); 3,556(3,1);3,324(109,0);2,676(0,7);2,672(0,9);2,667(0,7);2,525(2,7);2,520(4,2);2,512(51,4);2,50 7(104,5);2,502 (138,9);2,498(100,6);2,493(48,1);2,339(0,3);2,334(0,7);2,329(0,9);2,325(0,7);1,336(1,5);1,250(1,9);0,008(1,1); 0,000(33,2);-0,009(1,1) |
| Verbindung Nr. 1-41: |

(fortgesetzt)

| |
|---|
| $^1$H-NMR(400,0 MHz, DMSO): δ= 8,586(5,6);8,584(5,7);8,582(5,7);8,580(5,6);8,509(2,5);8,317(1,2);8,086(4,1); 8,080(4,0);8,064(4,3);8,058(4 ,2);7,611(2,5);7,606(2,9);7,592(4,8);7,588(5,7);7,579(0,6);7,573(3,2);7,569(3,2); 7,549(1,5);7,545(1,4);7,536(1,6);7,531(3,2);7,528(2,3);7,527 (2,2);7,524(1,9);7,517(2,5);7,516(2,3);7,513(2,3); 7,510(3,4);7,505(1,8);7,497(2,0);7,492(1,6);7,294(4,0);7,283(4,5);7,281(4,7);7,275(3,4);7,2 73(3,8);7,270(4,1); 7,267(5,5);7,264(8,4);7,249(3,1);7,246(7,2);7,027(7,0);7,006(6,7);4,506(7,4);4,492(16,0);4,477(7,8);3,682(3,5); 3,667(10,1 );3,653(9,8);3,639(3,3);3,325(90,3);3,301(0,4);2,676(0,6);2,672(0,8);2,667(0,6);2,525(2,7);2,520 (4,3);2,512(47,1);2,507(93,9);2,502(123,7);2 ,498(89,5);2,493(42,7);2,334(0,6);2,329(0,8);2,325(0,6);2,061 (0,4);2,041(0,3);1,336(2,0);1,259(0,4);1,250(2,5);1,235(0,7);0,008(0,5);0,000(1 5,3);-0,009(0,5) |
| Verbindung Nr. 1-42:<br>$^1$H-NMR(400,0 MHz, DMSO): δ= 8,662(1,5);8,649(2,9);8,635(1,5);8,598(5,1);8,596(5,1);8,595(5,1);8,592(5,1); 8,316(3,5);8,090(3,7);8,084(3, 7);8,068(3,9);8,062(3,8);7,490(3,9);7,488(2,7);7,471(7,3);7,470(6,9);7,452(3,4); 7,444(4,0);7,438(4,1);7,430(5,6);7,425(1,6);7,418(2,5);7,410 (3,5);7,401(0,4);7,393(2,1);7,383(16,0);7,380(9,3); 7,376(6,7);7,368(4,7);7,365(4,1);7,349(0,8);7,346(0,8);7,022(6,3);7,000(6,1);4,498(6,4);4, 484(13,8);4,470(6,8); 3,657(3,2);3,643(9,1);3,629(8,9);3,615(3,0);3,324(101,5);3,300(1,2);2,676(0,6);2,671(0,8);2,666(0,6);2,524 (2,7);2,520( 4,2);2,511(45,8);2,507(91,6);2,502(120,9);2,497(87,1);2,493(41,3);2,333(0,6);2,329(0,8);2,324 (0,6);1,336(1,3);1,250(1,6);0,008(0,5);0,000(1 4,7);-0,009(0,4) |
| Verbindung Nr. 1-43:<br>$^1$H-NMR(400,0 MHz, DMSO): δ= 8,655(2,0);8,641(3,7);8,628(1,9);8,599(6,1);8,597(6,2);8,596(6,2);8,593(6,1); 8,316(0,6);8,088(4,4);8,082(4 ,3);8,066(4,6);8,060(4,5);7,649(4,6);7,645(6,1);7,641(1,7);7,635(1,5);7,625(8,5); 7,434(2,2);7,432(2,4);7,424(0,6);7,417(3,2);7,414(6,4);7,412 (4,7);7,398(6,9);7,395(6,2);7,369(2,0);7,364(10,0); 7,360(9,9);7,356(8,0);7,347(7,1);7,342(7,9);7,337(4,5);7,329(3,5);7,324(2,3);7,028(7,4);7, 007(7,2);4,499(7,4); 4,485(16,0);4,470(7,8);3,652(3,9);3,637(10,9);3,623(10,5);3,609(3,5);3,324(96,0);2,680(0,3);2,676(0,7);2,671 (0,9);2,667 (0,6);2,524(3,0);2,520(4,8);2,511(50,5);2,507(99,8);2,502(130,7);2,497(94,0);2,493(44,5);2,333(0,6); 2,329(0,9);2,324(0,6);1,336(1,6);1,259( 0,4);1,250(2,0);1,235(0,4);0,008(0,6);0,000(16,8);-0,009(0,5) |
| Verbindung Nr. 1-44:<br>$^1$H-NMR(400,0 MHz, DMSO): δ= 8,614(2,3);8,599(10,1);8,593(7,9);8,084(4,5);8,077(4,5);8,062(4,7);8,055(4,6); 7,877(6,9);7,875(7,2);7,857( 7,6);7,855(7,4);7,443(3,3);7,440(3,4);7,424(7,7);7,421(7,7);7,405(5,1);7,403(4,9); 7,296(7,0);7,292(7,9);7,277(5,8);7,273(5,7);7,179(4,4);7,17 5(4,2);7,160(6,2);7,156(5,9);7,141(3,7);7,137(3,4); 7,041(7,6);7,019(7,3);5,757(1,7);4,504(7,3);4,489(16,0);4,475(7,8);3,645(3,9);3,631(10,9) ;3,617(10,5);3,603 (3,5);3,325(64,0);2,676(0,4);2,671(0,6);2,667(0,4);2,525(2,0);2,511(34,9);2,507(69,7);2,502(91,9);2,498(67,0); 2,493(32,3) ;2,334(0,4);2,329(0,6);2,324(0,4);1,989(0,8);1,336(1,2);1,259(0,4);1,250(1,5);1,234(0,4);1,175(0,5); 0,008(0,4);0,000(12,7);-0,009(0,4) |
| Verbindung Nr. 1-45:<br>$^1$H-NMR(400,0 MHz, DMSO): δ= 8,957(1,9);8,944(3,6);8,930(1,9);8,597(6,3);8,595(6,3);8,593(6,4);8,591(6,3); 8,317(0,6);8,094(4,6);8,087(4, 6);8,072(4,8);8,065(4,7);7,542(1,5);7,525(3,3);7,521(2,8);7,508(2,1);7,504(6,2); 7,499(2,2);7,487(2,8);7,483(3,6);7,466(1,7);7,181(1,3);7,178 (1,7);7,171(10,3);7,160(1,6);7,157(2,2);7,151 (12,5);7,145(2,2);7,141(1,5);7,131(8,6);7,123(1,5);7,012(7,8);6,990(7,5);4,476(7,8);4,462(16,0) ;4,448(8,3); 3,679(4,1);3,665(11,1);3,651(10,8);3,637(3,7);3,328(115,6);3,305(0,3);2,676(0,5);2,672(0,6);2,667(0,5);2,525 (2,0);2,520(3,2);2,5 12(35,3);2,507(70,5);2,503(92,8);2,498(66,8);2,494(31, 7);2,334(0,4);2,330(0,6);2,325(0,4); 1,337(1,9);1,259(0,4);1,250(2,4);1,235(0,4);0,008 (0,4);0,000(12,7);-0,009(0,4) |
| Verbindung Nr. 1-46:<br>$^1$H-NMR(400,0 MHz, DMSO): δ= 8,736(2,0);8,722(3,8);8,708(2,0);8,603(6,2);8,601(6,2);8,599(6,2);8,597(6,2); 8,316(0,6);8,096(4,6);8,089(4 ,5);8,074(4,7);8,067(4,7);7,777(4,9);7,758(6,5);7,725(2,0);7,707(5,5);7,688(4,2); 7,655(4,0);7,636(5,0);7,617(1,8);7,491(5,9);7,472(5,0);7,007 (7,6);6,985(7,3);4,474(7,5);4,460(16,0);4,446(7,9); 3,657(3,9);3,643(10,9);3,629(10,5);3,615(3,5);3,324(150,7);2,680(0,5);2,676(1,0);2,671(1, 3);2,666(0,9);2,662 (0,4);2,524(4,2);2,520(6,7);2,511(73,3);2,507(145,7);2,502(191,4);2,497(137,2);2,493(64,5);2,338(0,4);2,333 (0,9);2,329( 1,3);2,324(0,9);2,320(0,4);1,336(1,2);1,259(0,3);1,250(1,5);1,234(0,4);0,008(0,8);0,000(23,6);- 0,009(0,7) |
| Verbindung Nr. 1-47: |

(fortgesetzt)

| |
|---|
| $^1$H-NMR(400,0 MHz, DMSO): δ= 8,849(2,0);8,836(3,8);8,822(2,1);8,602(6,4);8,600(6,6);8,598(6,6);8,596(6,4); 8,470(7,1);8,465(7,5);8,458(7, 7);8,453(7,4);8,317(3,3);8,094(4,6);8,088(4,5);8,072(4,8);8,066(4,6);7,875(7,5); 7,870(7,5);7,856(8,5);7,851(8,0);7,495(8,3);7,483(8,1);7,477 (7,7);7,465(7,4);7,027(7,9);7,005(7,5);5,757(0,6); 4,504(7,8);4,490(16,0);4,476(8,2);3,677(4,0);3,663(11,0);3,649(10,7);3,635(3,7);3,326(74,6 );3,302(1,4);2,676 (0,5);2,672(0,7);2,667(0,5);2,525(2,5);2,512(41,5);2,507(81,4);2,503(106,1);2,498(76,9);2,494(37,0);2,334(0,5); 2,329(0,7); 2,325(0,5);1,989(0,8);1,235(0,4);1,175(0,5);0,008(0,5);0,000(13,7);-0,009(0,5) |
| Verbindung Nr. 1-48:<br>1H-NMR(400,0 MHz, DMSO): δ= 8,589(6,0);8,587(6,0);8,586(6,0);8,583(5,9);8,391(2,0);8,377(3,7);8,363(1,9); 8,317(0,8);8,084(4,5);8,077(4 ,4);8,062(4,6);8,055(4,5);7,709(15,0);7,697(15,6);7,216(16,0);7,203(15,1);7,047 (7,4);7,025(7,1);4,507(6,9);4,492(14,7);4,478(7,2);3,663(3,6) ;3,649(10,0);3,635(9,7);3,620(3,2);3,325(93,7); 2,676(0,6);2,672(0,8);2,667(0,6);2,525(2,6);2,520(4,2);2,512(45,6);2,507(90,4);2,503(118,5); 2,498(85,0);2,494 (40,0);2,334(0,6);2,329(0,8);2,325(0,6);1,336(1,2);1,259(0,3);1,250(1,5);0,008(0,6);0,000(15,6);-0,009(0,4) |
| Verbindung Nr. 1-49:<br>1H-NMR(400,0 MHz, DMSO): δ= 8,578(6,2);8,576(6,4);8,574(6,4);8,572(6,3);8,317(1,3);8,269(2,0);8,256(3,6); 8,242(1,9);8,084(4,5);8,078(4 ,6);8,062(4,8);8,056(4,6);7,839(14,0);7,826(14,5);7,144(15,0);7,131(14,5);7,031 (7,6);7,009(7,3);4,520(7,5);4,506(16,0);4,491(8,0);3,690(4,0) ;3,675(11,2);3,661(10,9);3,647(3,7);3,327(56,7); 3,304(0,3);2,677(0,4);2,673(0,5);2,668(0,4);2,526(1,7);2,513(29,5);2,508(58,8);2,504(77,8); 2,499(57,2);2,495 (28,0);2,335(0,4);2,330(0,5);2,326(0,4);1,337(1,1);1,250(1,3);0,008(0,4);0,000(10,0);-0,008(0,3) |
| Verbindung Nr. 1-50:<br>1H-NMR(400,0 MHz, DMSO): δ= 8,577(5,9);8,575(5,9);8,573(6,0);8,571(6,1);8,548(3,6);8,534(1,8);8,317(3,2); 8,077(4,2);8,071(4,1);8,055(4 ,4);8,049(4,2);7,897(15,8);7,893(15,7);7,027(7,0);7,005(6,7);6,849(16,0);6,844 (15,8);4,487(6,7);4,472(14,5);4,458(7,0);3,633(3,4);3,619(9,8) ;3,605(9,6);3,590(3,2);3,326(124,5);3,302(1,1); 2,677(0,6);2,672(0,8);2,668(0,6);2,525(2,4);2,521(3,8);2,512(45,3);2,508(91,1);2,503(120,2); 2,498(86,5);2,494 (40,9);2,334(0,6);2,330(0,8);2,325(0,6);1,336(1,0);1,250(1,3);1,235(0,3);0,008(0,5);0,000(14,6);-0,009(0,4) |
| Verbindung Nr. 1-51:<br>1H-NMR(400,0 MHz, DMSO): δ= 8,559(8,2);8,557(8,2);8,482(3,2);8,393(9,2);8,388(8,8);7,621(2,7);7,617(3,1); 7,603(5,2);7,598(6,2);7,583(3 ,2);7,579(3,2);7,552(1,5);7,548(1,4);7,539(1,7);7,534(3,4);7,531(2,7);7,520(2,8); 7,513(3,6);7,509(2,0);7,500(2,1);7,495(1,7);7,299(4,6);7,289 (5,1);7,287(4,8);7,278(4,5);7,271(11,6);7,251(8,8); 4,607(7,5);4,593(16,0);4,578(8,0);3,711(3,8);3,697(10,7);3,683(10,5);3,669(3,6);3,324(34, 2);2,677(0,4);2,672 (0,6);2,668(0,4);2,525(1,6);2,512(32,6);2,508(63,9);2,503(84,1);2,499(61,9);2,494(30,5);2,334(0,4);2,330(0,6); 2,325(0,4); 1,337(1,0);1,250(1,2);1,235(0,4);1,188(0,3);0,000(0,8) |
| Verbindung Nr. 1-52:<br>1H-NMR(400,0 MHz, DMSO): δ= 8,936(2,3);8,923(4,3);8,909(2,3);8,572(7,0);8,569(8,7);8,567(8,7);8,564(7,0); 8,397(9,7);8,391(9,1);7,541(1 ,5);7,525(3,3);7,520(2,9);7,508(2,3);7,504(6,2);7,499(2,3);7,487(3,1);7,483(3,7); 7,466(1,7);7,181(1,3);7,178(1,8);7,171(10,4);7,151(13,4);7,1 31(8,7);7,123(1,5);4,582(7,8);4,568(16,0);4,554 (8,2);3,711(4,1);3,697(11,2);3,683(10,9);3,669(3,8);3,323(39,0);2,676(0,4);2,672(0,6);2,667( 0,4);2,525(1,8); 2,512(33,8);2,507(66,3);2,503(87,0);2,498(63,3);2,494(30,3);2,334(0,4);2,329(0,6);2,325(0,4);1,990(0,5);1,337 (0,5);1,250(0, 6);0,000(0,9) |
| Verbindung Nr. 1-53:<br>1H-NMR(400,0 MHz, DMSO): δ= 8,920(2,4);8,906(4,6);8,893(2,4);8,588(6,8);8,586(8,5);8,583(8,5);8,580(6,9); 8,403(9,6);8,397(9,1);8,316(0 ,7);8,043(6,8);8,041(7,0);8,023(7,9);8,021(7,7);7,806(3,2);7,803(3,3);7,787(8,2); 7,785(8,0);7,769(5,7);7,766(5,3);7,711(5,0);7,707(5,6);7,691 (6,1);7,687(6,5);7,672(3,3);7,668(3,2);7,586(7,7); 7,582(7,6);7,567(6,7);7,563(6,2);4,591(7,4);4,577(16,0);4,562(7,8);3,687(3,9);3,673(11,0); 3,659(10,7);3,645 (3,6);3,322(159,6);2,680(0,7);2,675(1,5);2,671(2,0);2,666(1,5);2,662(0,7);2,524(6,0);2,519(9,6);2,511(113,4); 2,506(225,8); 2,502(299,5);2,497(219,4);2,493(106,4);2,337(0,7);2,333(1,5);2,328(2,0);2,324(1,5);2,320(0,7); 1,336(0,9);1,298(0,6);1,259(1,1);1,250(1,4);1 ,236(0,6);0,000(2,2) |
| Verbindung Nr. 1-54: |

(fortgesetzt)

| |
|---|
| 1H-NMR(400,0 MHz, DMSO): δ= 8,824(2,4);8,811(4,3);8,798(2,4);8,490(0,3);8,485(0,4);8,471(5,8);8,467(6,3);8,459(6,2);8,455(6,1);8,143(6 ,2);8,139(6,8);8,130(6,5);8,127(6,6);7,913(6,0);7,909(6,1);7,894(6,7);7,890(6,3);7,881(0,8);7,869(5,9);7,865(6,1);7,851(6,6);7,846(6,5);7,506 (6,3);7,493(6,3);7,487(6,0);7,475(5,4);7,055(6,1);7,043(6,2);7,036(6,1);7,024(5,7);5,757(0,6);4,508(7,7);4,494(16,0);4,479(8,2);3,735(0,6);3, 720(0,4);3,678(4,2);3,664(11,5);3,649(11,3);3,635(4,0);3,580(0,5);3,565(0,4);3,324(52,5);2,671(0,9);2,667(0,7);2,506(98,3);2,502 (126,9);2,4 98(100,6);2,333(0,6);2,329(0,9);2,325(0,7);1,235(0,5);0,000(7,9) |
| Verbindung Nr. 1-55:<br>1H-NMR(400,0 MHz, DMSO): δ= 8,220(1,8);8,207(3,2);8,194(1,8);8,128(6,9);8,124(7,2);8,116(7,3);8,112(7,1);7,909(7,4);7,905(7,3);7,890(8 ,0);7,886(7,3);7,845(13,3);7,832(13,7);7,812(0,4);7,799(0,4);7,181(0,4);7,168 (0,4);7,150(14,3);7,137(13,8);7,052(8,1);7,039(7,8);7,032(7,7); 7,020(7,6);5,760(7,2);4,517(7,5);4,502(16,0);4,488(7,9);3,698(4,0);3,683(11,3);3,669(11,0);3,655(3,7);3,329(30,0);2,527(0,9);2,514(17,0);2, 509(33,6);2,505 (43,8);2,500(31,3);2,495(14,7);1,991(1,2);1,338(1,1);1,250(1,3);1,194(0,4);1,176(0,7);1,158(0,3);0,000(4,0) |
| Verbindung Nr. 1-56:<br>1H-NMR(400,0 MHz, DMSO): δ= 8,550(6,9);8,547(8,7);8,545(9,0);8,542(8,1);8,525(4,5);8,510(2,3);8,387(9,4);8,382(8,7);7,905(15,3);7,900( 15,2);7,338(0,4);7,333(0,3);7,183(0,5);7,177(0,4);6,852(16,0);6,847(15,6);5,759 (7,4);4,583(7,3);4,569(15,7);4,555(7,4);4,057(0,4);4,039(1,1) ;4,021(1,1);4,004(0,4);3,661(3,9);3,647(11,0);3,633(10,6);3,618(3,4);3,327(50,9);2,678(0,4);2,673(0,5);2,669(0,4);2,527(2,0);2,513(31,6);2,5 09(61,7);2,504 (79,5);2,500(56,3);2,495(26,1);2,336(0,4);2,331(0,5);2,326(0,4);1,991(4,9);1,353(0,3);1,337(6,7);1,300(0,7);1,259(1,2);1,250( 8,2);1,235(1,3);1,194(1,5);1,188(0,8);1,176(2,7);1,158(1,3);0,854(0,4);0,008(0,4);0,000 (12,1);-0,009(0,3) |
| Verbindung Nr. 1-57:<br>1H-NMR(400,0 MHz, DMSO): δ= 8,915(2,2);8,901(4,3);8,888(2,2);8,795(5,6);8,786(5,4);8,783(5,5);8,608(6,6);8,606(6,8);8,605(6,8);8,602(6 ,6);8,102(4,8);8,095(4,7);8,080(5,0);8,073(4,9);7,995(4,9);7,992(5,1);7,975(6,0);7,973(5,9);7,785(5,5);7,773(5,5);7,765(4,8);7,754(4,5);7,012 (8,0);6,990(7,7);5,759(0,3);4,481(7,8);4,467(16,0);4,453(8,2);3,684(4,1);3,670(11,2);3,656(10,8);3,642(3,7);3,325(44,6);2,676(0,5);2,672(0,6 );2,667(0,5);2,525 (1,9);2,512(36,4);2,507(72,1);2,503(94,0);2,498(67,4);2,494(32,0);2,334(0,4);2,330(0,6);2,325(0,4);1,337(0,5);1,250(0,6);0 ,008(1,8);0,000(49,0);-0,009(1,6) |
| Verbindung Nr. 1-58:<br>1H-NMR(400,0 MHz, DMSO): δ= 8,897(2,1);8,884(3,9);8,870(2,0);8,814(0,3);8,799(5,0);8,797(5,1);8,788(5,1);8,785(5,0);8,147(7,1);8,143(7 ,4);8,135(7,4);8,131(7,3);7,985(4,6);7,983(4,7);7,966(5,6);7,963(5,5);7,915(7,2);7,911(7,1);7,896(7,8);7,892(7,2);7,796(5,1);7,784(5,1);7,777 (4,3);7,765(4,1);7,059(7,8);7,046(7,6);7,039(7,4);7,027(7,4);4,490(7,5);4,475(16,0);4,461(7,9);3,722(0,7);3,707(0,5);3,683(4,0);3,669(11,1); 3,655(10,7);3,640 (3,6);3,584(0,5);3,569(0,4);3,326(45,8);2,676(0,3);2,672(0,5);2,667(0,3);2,525(1,6);2,511(28,1);2,507(54,4);2,503(70,3);2, 498(50,2);2,493(23,8);2,334(0,3);2,329(0,5);2,325(0,3);1,337(0,4);1,250(0,6);0,008(1,5);0,000 (38,0);-0,009(1,2) |
| Verbindung Nr. 1-59:<br>1H-NMR(400,0 MHz, DMSO): δ= 8,989(2,6);8,975(5,2);8,961(2,6);8,898(9,5);8,893(9,6);8,591(7,6);8,588(9,5);8,585(9,5);8,583(7,8);8,418(1 0,5);8,412(9,9);8,216(10,3);8,211(10,1);7,183(0,4);5,758(3,6);4,593(7,8);4,579 (16,0);4,565(8,2);3,717(4,1);3,703(11,2);3,689(10,8);3,675(3, 8);3,323(70,1);2,680(0,4);2,676(0,8);2,672(1,0);2,667(0,7);2,663(0,4);2,525(3,0);2,520(4,7);2,512(56,7);2,507(114,4);2,503(150,8);2,498(10 8,2);2,494(51,2);2,339(0,4);2,334(0,7);2,329(1,0);2,325(0,7);2,320(0,3);1,990(0,9);1,336(4,6);1,299(0,4);1,259(0,7);1,250(5,7);1,235(0,7);1,1 93(0,3);1,188(0,4);1,175(0,6);1,149(0,4);0,146(0,3);0,008(3,0);0,000(89,8);-0,009(2,8);-0,150 (0,4) |
| Verbindung Nr. 1-60:<br>1H-NMR(400,0 MHz, DMSO): δ= 8,993(2,5);8,980(4,8);8,966(2,4);8,892(9,2);8,886(9,2);8,608(7,1);8,606(7,3);8,604(7,3);8,602(7,0);8,556(0 ,4);8,554(0,4);8,387(0,4);8,382(0,4);8,240(9,8);8,235(9,5);8,105(5,1);8,099(5,0);8,083(5,3);8,077(5,1);7,183(0,4);7,016(8,6);6,994(8,2);5,759 (2,5);4,489(8,0);4,475(16,0);4,461(8,3);4,393(0,4);4,378(0,7);4,364(0,3);3,686(4,3);3,672(11,3);3,658(10,8);3,644(3,8);3,325(36,4);2,932(0,4 );2,677(0,5);2,672 (0,7);2,668(0,5);2,526(2,4);2,512(38,3);2,508(75,4);2,503(97,8);2,499(69,8);2,494(32,7);2,335(0,5);2,330(0,6);2,325(0,5);1 ,337(5,3);1,300(0,5);1,259(0,8);1,250(6,4);1,235(0,7);1,163(0,3);0,008(2,1);0,000(57,2);-0,009(1,7) |
| Verbindung Nr. 1-61: |

(fortgesetzt)

| |
|---|
| 1H-NMR(400,0 MHz, DMSO): δ= 8,977(2,2);8,963(4,3);8,950(2,2);8,896(8,3);8,890(8,4);8,210(8,9);8,205(8,6); 8,148(7,4);8,143(7,9);8,135(7 ,9);8,131(7,9);7,922(7,6);7,918(7,6);7,903(8,3);7,899(7,7);7,064(8,3);7,052(8,1); 7,045(7,9);7,033(7,8);4,495(7,5);4,481(16,0);4,467(7,9);3,68 7(3,9);3,673(11,0);3,659(10,7);3,645(3,6);3,324 (39,5);2,676(0,5);2,672(0,6);2,667(0,4);2,525(1,9);2,512(35,5);2,507(70,8);2,503(92,7);2,498 (66,9);2,494(32,0); 2,334(0,4);2,329(0,6);2,325(0,4);1,337(1,7);1,259(0,3);1,250(2,0);1,234(0,3);0,008(1,7);0,000(49,5);-0,009(1,6) |
| Verbindung Nr. 1-62:<br>1H-NMR(400,0 MHz, DMSO): δ= 9,086(2,0);9,072(3,8);9,059(2,0);8,735(7,0);8,734(7,1);8,722(7,2);8,721(7,1); 8,627(0,3);8,555(0,4);8,386(0 ,5);8,380(0,4);8,317(0,5);8,143(7,5);8,139(8,0);8,131(7,9);8,127(7,9);8,059(0,4); 7,913(7,5);7,909(7,5);7,894(8,1);7,890(7,6);7,877(10,2);7,86 4(9,8);7,336(0,7);7,332(0,7);7,182(1,3);7,177(0,8); 7,056(8,4);7,044(8,1);7,037(7,9);7,024(7,8);4,493(6,5);4,479(12,8);4,465(6,8);4,379(0,7); 4,365(1,4);4,350(0,8); 3,709(3,9);3,695(10,5);3,681(10,2);3,667(3,6);3,383(0,4);3,368(0,5);3,322(430,7);2,928(0,5);2,913(0,9);2,898 (0,5);2,6 80(1,8);2,675(3,6);2,670(4,8);2,666(3,5);2,661(1,7);2,646(0,4);2,643(0,4);2,524(15,1);2,519(23,4); 2,510(262,3);2,506(521,1);2,501(679,5);2, 497(483,6);2,492(225,6);2,337(1,4);2,333(3,2);2,328(4,4);2,324(3,1); 2,319(1,3);1,989(1,0);1,350(0,6);1,335(12,8);1,298(1,4);1,258(2,4);1,24 9(16,0);1,235(1,9);1,192(0,5);1,187 (0,8);1,175(0,7);1, 157(0,4);1,147(0,6);1, 108(0,5);0,853(0,5);0,834(0,3);0, 146(1,5);0,008(12,8);0,000(360, 7);-0,009(11,0);-0,150(1,4) |
| Verbindung Nr. 1-63:<br>1H-NMR(400,0 MHz, DMSO): δ= 8,905(2,5);8,891(4,8);8,877(2,5);8,798(6,0);8,789(5,9);8,787(6,0);8,589(7,0); 8,586(9,0);8,584(9,1);8,581(7 ,6);8,406(10,1);8,401(9,7);8,316(0,5);7,989(5,2);7,987(5,5);7,970(6,4);7,967(6,5); 7,796(5,8);7,784(5,7);7,777(5,0);7,765(4,7);5,756(0,6);4,58 6(7,7);4,572(16,0);4,558(8,1);3,713(4,0);3,699 (11,2);3,685(10,9);3,671(3,8);3,322(125,4);2,676(1,0);2,671(1,5);2,667(1,1);2,662(0,5);2,524( 4,2);2,511(82,7); 2,507(167,5);2,502(222,2);2,498(162,6);2,493(79,6);2,338(0,6);2,333(1,1);2,329(1,5);2,324(1,1);1,235(1,9); 1,183(0,6);1,16 6(0,9);1,149(0,6);0,146(0,7);0,008(5,4);0,000(157,9);-0,008(5,7);-0,150(0,7) |
| Verbindung Nr. 1-64:<br>1H-NMR(400,0 MHz, DMSO): δ= 9,100(2,0);9,086(3,9);9,073(2,1);8,735(6,9);8,724(7,1);8,649(0,3);8,603(6,4); 8,315(1,7);8,191(2,8);8,098(4 ,3);8,092(4,4);8,076(4,5);8,070(4,6);7,879(9,9);7,866(9,6);6,991(7,2);6,969(7,0); 6,545(0,4);5,756(1,4);4,861(0,4);4,849(0,4);4,618(0,3);4,481 (6,4);4,468(12,9);4,454(7,1);4,394(0,4);4,380(0,7); 4,365(0,4);4,039(2,8);4,022(2,9);3,913(0,5);3,897(0,4);3,709(3,1);3,695(8,4);3,682(8,3);3, 668(3,1);3,324 (1015,0);2,891(0,4);2,675(5,5);2,671(7,6);2,666(5,5);2,524(21,5);2,519(35,9);2,511(443,8);2,506(887,9);2,502 (1165,7);2,497( 842,3);2,493(406,4);2,337(3,3);2,333(6,2);2,328(8,3);2,324(6,1);2,300(1,2);2,280(1,8);2,263 (1,3);2,193(1,3);2,074(5,0);1,770(0,8);1,566(1,1) ;1,235(16,0);1,179(5,5);1,165(8,6);1,148(5,1);0,871(0,7);0,854 (1,8);0,837(0,9);0,146(3,4);0,008(30,6);0,000(812,4);-0,009(26,1);-0,150(3,4) |
| Verbindung Nr. 1-65:<br>1H-NMR(400,0 MHz, DMSO): δ= 8,350(2,9);8,127(2,5);8,123(2,7);8,115(2,7);8,111(2,7);8,037(2,1);8,034(2,2); 8,016(2,5);8,014(2,4);7,912(2 ,6);7,908(2,6);7,893(2,9);7,889(2,6);7,791(1,0);7,788(1,1);7,772(2,5);7,769(2,5); 7,753(1,7);7,750(1,6);7,678(1,5);7,674(1,6);7,658(1,8);7,654 (1,9);7,639(1,1);7,635(1,0);7,522(2,3);7,519(2,3); 7,504(2,1);7,500(1,9);7,042(2,8);7,030(2,7);7,023(2,6);7,011(2,6);4,644(2,6);4,618(3,4);4,4 69(3,4);4,443(2,7); 3,326(66,8);2,671(0,3);2,541(5,5);2,524(1,0);2,520(1,5);2,511(18,3);2,506(36,9);2,502(48,5);2,497(34,5);2,493 (16,2);2,06 5(0,8);2,046(0,9);2,031(1,1);2,012(1,0);1,745(1,0);1,726(1,2);1,711(1,0);1,692(0,9);1,389(16,0); 0,960(3,7);0,941(8,3);0,922(3,4);0,000(6,8) |
| Verbindung Nr. 1-66:<br>1H-NMR(400,0 MHz, DMSO): δ= 8,870(2,0);8,857(3,8);8,843(2,0);8,466(4,2);8,463(4,3);8,454(4,3);8,451(4,2); 8,122(4,2);8,119(4,2);8,104(4 ,5);8,101(4,2);8,042(6,0);8,039(6,3);8,021(7,0);8,019(6,9);7,811(2,8);7,808(3,0); 7,792(7,2);7,789(7,2);7,773(5,0);7,770(4,6);7,710(4,4);7,706 (4,9);7,690(5,4);7,686(5,7);7,671(3,0);7,667(2,9); 7,553(6,7);7,549(6,7);7,534(5,9);7,530(5,6);7,212(3,7);7,199(3,8);7,194(3,7);7,181(3,4);4,5 55(7,0);4,540(16,0); 4,525(7,4);3,649(3,7);3,634(10,7);3,620(10,3);3,605(3,4);3,327(176,0);3,306(0,4);2,676(0,6);2,671(0,8);2,667 (0,6);2,542 (3,2);2,525(2,4);2,520(3,8);2,511(47,5);2,507(95,6);2,502(125,9);2,498(90,3);2,493(42,7);2,333(0,6); 2,329(0,8);2,324(0,6);0,008(0,5);0,000( 16,0);-0,009(0,5) |
| Verbindung Nr. 1-67: |

(fortgesetzt)

| |
|---|
| 1H-NMR(400,0 MHz, DMSO): δ= 8,946(1,7);8,932(3,3);8,918(1,7);8,041(5,8);8,038(5,9);8,021(6,8);8,018(6,5); 7,992(5,4);7,989(5,5);7,980(5 ,6);7,977(5,5);7,801(2,8);7,798(2,9);7,782(7,3);7,779(7,2);7,763(5,1);7,760(4,8); 7,706(7,5);7,702(7,9);7,686(9,0);7,683(9,1);7,678(3,9);7,675 (3,8);7,667(3,2);7,664(3,1);7,659(3,9);7,655(3,6); 7,577(6,7);7,573(6,5);7,558(5,8);7,554(5,3);7,058(3,7);7,050(4,0);7,046(3,7);7,038(6,0);7,0 30(3,5);7,026(3,5); 7,018(3,3);4,497(7,3);4,482(16,0);4,468(7,7);3,662(3,8);3,648(10,8);3,633(10,5);3,619(3,4);3,327(150,7);2,676 (0,5);2,671 (0,7);2,666(0,5);2,541(3,7);2,524(2,2);2,520(3,5);2,511(43,2);2,507(86,8);2,502(114,0);2,497(81,4); 2,493(38,3);2,333(0,5);2,329(0,7);2,324( 0,5);0,008(0,5);0,000(13,8);-0,009(0,4) |
| Verbindung Nr. 1-68:<br>1H-NMR(400,0 MHz, DMSO): δ= 8,461(1,4);8,450(0,8);8,447(0,8);8,437(0,8);8,434(0,7);8,123(0,7);8,120(0,7); 8,104(0,8);8,101(0,7);8,058(1 ,0);8,056(1,1);8,038(1,2);8,035(1,2);7,795(0,5);7,792(0,5);7,776(1,2);7,774(1,2); 7,758(0,8);7,755(0,8);7,679(0,7);7,676(0,8);7,659(0,9);7,656 (0,9);7,640(0,5);7,637(0,5);7,478(1,1);7,474(1,1); 7,459(1,0);7,455(1,0);7,199(0,6);7,186(0,6);7,181(0,6);7,168(0,6);4,588(5,1);3,326(26,3);2, 542(1,4);2,525(0,5); 2,520(0,7);2,511(9,1);2,507(18,5);2,502(24,4);2,498(17,6);2,493(8,5);1,431(16,0);0,000(2,8) |
| Verbindung Nr. 1-69:<br>1H-NMR(400,0 MHz, DMSO): δ= 8,472(1,5);8,212(2,0);8,206(2,9);8,181(2,8);8,175(2,0);8,051(1,1);8,048(1,1); 8,031(1,2);8,028(1,2);7,793(0 ,5);7,790(0,5);7,774(1,3);7,771(1,2);7,755(0,8);7,752(0,8);7,679(0,7);7,676(0,8); 7,659(0,9);7,656(0,9);7,640(0,5);7,637(0,5);7,513(1,2);7,510 (1,2);7,494(1,0);7,491(1,0);4,517(5,2);3,324(19,4); 2,541(4,0);2,524(0,5);2,519(0,7);2,511(8,9);2,507(17,9);2,502(23,5);2,497(16,9);2,493(8,0 );1,442(16,0);0,000 (3,6) |
| Verbindung Nr. 1-70:<br>1H-NMR(400,0 MHz, DMSO): δ= 8,925(1,9);8,912(3,6);8,898(1,9);8,794(4,7);8,792(5,0);8,783(4,9);8,780(4,9); 7,990(5,8);7,987(6,2);7,978(1 0,2);7,974(10,6);7,957(5,5);7,955(5,4);7,787(5,1);7,775(5,0);7,767(4,3);7,756 (4,2);7,707(3,5);7,703(3,5);7,687(3,9);7,683(3,8);7,679(3,7);7,6 75(3,5);7,659(3,8);7,656(3,6);7,058(3,7);7,050 (4,0);7,046(3,8);7,038(6,3);7,030(3,6);7,026(3,6);7,018(3,3);4,490(7,7);4,476(16,0);4,462(8,2) ;3,686(4,0);3,672 (11,2);3,658(10,8);3,644(3,7);3,326(124,5);2,676(0,6);2,671(0,8);2,667(0,6);2,541(2,6);2,524(2,4);2,520(3,9); 2,511(48,8);2, 507(97,3);2,502(127,0);2,498(90,6);2,493(43,0);2,333(0,6);2,329(0,8);2,324(0,6);0,008(0,7); 0,000(19,8);-0,009(0,6) |
| Verbindung Nr. 1-71:<br>1H-NMR(400,0 MHz, DMSO): δ= 8,768(0,8);8,759(0,8);8,757(0,8);8,591(1,0);8,589(1,0);8,587(1,0);8,465(1,4); 8,099(0,7);8,092(0,7);8,077(0 ,7);8,070(0,7);7,915(0,7);7,912(0,7);7,895(0,9);7,893(0,9);7,757(0,8);7,745(0,8); 7,738(0,7);7,726(0,6);7,037(1,2);7,015(1,1);4,525(5,1);3,324 (31,1);2,541(0,6);2,524(0,8);2,519(1,3);2,511 (15,9);2,506(32,0);2,502(42,0);2,497(30,2);2,493(14,4);1,432(16,0);0,000(7,6) |
| Verbindung Nr. 1-72:<br>1H-NMR(400,0 MHz, DMSO): δ= 8,470(1,4);8,163(1,2);8,159(1,3);8,151(1,2);8,146(1,3);8,056(1,9);8,052(2,2); 8,037(1,6);8,033(2,2);7,794(0 ,5);7,791(0,5);7,775(1,2);7,772(1,2);7,756(0,8);7,753(0,8);7,680(0,7);7,676(0,8); 7,660(0,9);7,657(0,9);7,641(0,5);7,637(0,5);7,541(1,2);7,538 (1,1);7,522(1,0);7,519(0,9);6,973(1,3);6,961(1,2); 6,954(1,2);6,942(1,2);4,504(5,2);3,325(11,7);2,541(2,3);2,524(0,4);2,511(6,6);2,507(13,0); 2,502(16,9);2,497 (12,0);2,493(5,7);1,455(16,0);0,000(3,1) |
| Verbindung Nr. 1-73:<br>1H-NMR(400,0 MHz, DMSO): δ= 8,771(1,8);8,761(1,7);8,759(1,7);8,355(2,9);8,131(2,4);8,127(2,6);8,119(2,6); 8,114(2,6);7,917(2,6);7,913(2 ,6);7,898(4,2);7,894(3,3);7,880(2,0);7,878(2,0);7,778(1,8);7,766(1,8);7,759(1,4); 7,747(1,3);7,047(2,7);7,035(2,6);7,028(2,5);7,015(2,5);4,649 (2,6);4,623(3,3);4,466(3,4);4,440(2,7);3,325(54,4); 2,671(0,3);2,541(5,1);2,524(1,0);2,520(1,7);2,511(20,2);2,507(40,3);2,502(52,9);2,497(38, 3);2,493(18,6);2,329 (0,3);2,046(0,8);2,027(1,0);2,012(1,1);1,993(1,0);1,752(1,0);1,733(1,2);1,717(1,0);1,699(0,9);1,385(16,0);0,939 (3,7);0,9 20(8,2);0,902(3,4);0,000(7,9) |
| Verbindung Nr. 1-74:<br>1H-NMR(400,0 MHz, DMSO): δ= 8,841(5,4);8,828(3,0);8,800(6,9);8,798(6,8);8,788(6,5);8,464(6,4);8,451(6,2); 8,123(6,3);8,105(6,3);7,941(5 ,9);7,921(7,3);7,801(5,8);7,789(6,3);7,782(4,5);7,770(3,9);7,212(4,7);7,199(5,8); 7,181(3,8);4,553(8,8);4,539(16,0);4,525(7,8);3,671(5,3);3,65 7(12,1);3,643(11,2);3,629(3,7);3,332(71,8);3,326 (160,7);2,676(1,0);2,671(1,1);2,541(7,2);2,540(7,3);2,507(156,5);2,502(171,5);2,498(118,5) ;2,333(1,0);2,329 (1,1);1,236(0,4);0,005(5,5);0,000(14,7);-0,002(13,0);-0,008(0,7) |
| Verbindung Nr. 1-75: |

(fortgesetzt)

| |
|---|
| 1H-NMR(400,0 MHz, DMSO): δ= 8,773(0,8);8,771(0,9);8,762(0,9);8,759(0,9);8,501(1,5);8,164(1,3);8,160(1,5); 8,152(1,4);8,147(1,4);8,061(1 ,4);8,057(1,4);8,042(1,6);8,038(1,4);7,923(0,7);7,920(0,8);7,904(1,0);7,901(0,9); 7,774(0,9);7,762(0,9);7,754(0,7);7,743(0,7);6,977(1,5);6,964 (1,4);6,957(1,4);6,945(1,4);4,503(5,3);3,326(16,5); 2,542(2,0);2,525(0,3);2,520(0,5);2,512(5,6);2,507(11,1);2,502(14,5);2,498(10,3);2,493(4,8 );1,450(16,0);0,000 (2,5) |
| Verbindung Nr. 1-76:<br>1H-NMR(400,0 MHz, DMSO): δ= 8,772(0,8);8,770(0,8);8,761(0,8);8,758(0,8);8,496(1,4);8,449(0,7);8,447(0,7); 8,437(0,7);8,434(0,7);8,126(0 ,7);8,123(0,7);8,108(0,8);8,105(0,7);7,859(0,6);7,856(0,7);7,839(1,0);7,836(1,0); 7,776(0,9);7,764(0,9);7,756(0,6);7,745(0,6);7,202(0,6);7,189 (0,6);7,184(0,6);7,171(0,6);4,580(5,1);3,324(20,8); 2,542(1,5);2,525(0,4);2,520(0,7);2,511(8,9);2,507(18,1);2,502(23,9);2,498(17,0);2,493(8,0 );1,429(16,0);0,000 (4,8) |
| Verbindung Nr. 1-77:<br>1H-NMR(400,0 MHz, DMSO): δ= 8,773(0,8);8,771(0,9);8,761(0,8);8,759(0,8);8,501(1,5);8,216(2,2);8,210(3,4); 8,189(3,3);8,183(2,2);7,904(0 ,7);7,902(0,7);7,885(1,0);7,882(0,9);7,773(0,9);7,762(0,9);7,754(0,7);7,742(0,7); 4,514(5,2);3,326(29,4);2,542(4,1);2,525(0,4);2,520(0,7);2,51 1(8,7);2,507(17,6);2,502(23,2);2,498(16,5);2,493 (7,8);1,437(16,0);0,000(3,5) |
| Verbindung Nr. 1-78:<br>1H-NMR(400,0 MHz, DMSO): δ= 8,586(1,0);8,584(1,0);8,582(1,0);8,580(1,0);8,293(1,4);8,092(0,7);8,085(0,7); 8,069(0,7);8,063(0,7);7,745(0 ,8);7,725(1,0);7,700(0,3);7,682(0,9);7,663(0,7);7,626(0,6);7,607(0,8);7,431(0,9); 7,413(0,8);7,032(1,2);7,010(1,2);4,534(5,1);3,325(15,9);2,52 5(0,4);2,520(0,7);2,511(7,9);2,507(15,9);2,502 (20,8);2,498(14,8);2,493(6,9);1,423(16,0);0,000(4,0) |
| Verbindung Nr. 1-79:<br>1H-NMR(400,0 MHz, DMSO): δ= 8,745(1,8);8,732(3,4);8,718(1,8);7,987(5,5);7,983(5,7);7,974(5,8);7,971(5,7); 7,776(4,6);7,756(6,2);7,729(2 ,0);7,711(5,4);7,702(3,8);7,698(3,9);7,693(4,1);7,682(3,9);7,678(3,8);7,674(3,7); 7,670(3,5);7,655(7,3);7,651(6,1);7,635(4,7);7,616(1,7);7,490 (5,6);7,471(4,7);7,053(3,6);7,045(3,9);7,041(3,6); 7,033(6,1);7,025(3,4);7,021(3,4);7,013(3,2);4,483(7,4);4,469(16,0);4,454(7,8);3,659(3,8);3, 645(10,8);3,631 (10,5);3,617(3,5);3,325(120,8);2,675(0,6);2,671(0,8);2,667(0,6);2,541(1,7);2,524(2,6);2,511(48,6);2,506(96,6); 2,502(126,3); 2,497(90,6);2,493(43,0);2,469(0,4);2,333(0,6);2,328(0,8);2,324(0,6);1,235(0,3);0,008(0,6);0,000 (19,7);-0,009(0,6) |
| Verbindung Nr. 1-80:<br>1H-NMR(400,0 MHz, DMSO): δ= 8,184(2,8);8,126(2,6);8,122(2,8);8,114(2,8);8,110(2,8);7,913(2,7);7,908(2,7); 7,893(3,0);7,889(2,7);7,748(1 ,6);7,728(2,2);7,715(0,7);7,697(1,9);7,678(1,3);7,628(1,3);7,609(1,7);7,590(0,6); 7,472(2,0);7,454(1,6);7,040(2,9);7,028(2,8);7,021(2,7);7,009 (2,8);4,665(2,6);4,639(3,3);4,457(3,4);4,431(2,8); 3,325(36,5);2,541(0,5);2,524(0,7);2,520(1,2);2,511(15,1);2,506(30,5);2,502(40,1);2,497(28, 5);2,493(13,4); 2,063(0,8);2,044(1,0);2,028(1,1);2,010(1,0);1,740(1,0);1,721(1,2);1,705(1,0);1,687(0,9);1,373(16,0);0,932(3,7); 0,914(8,3);0,8 95(3,4);0,000(7,0) |
| Verbindung Nr. 1-81:<br>1H-NMR(400,0 MHz, DMSO): δ= 8,671(2,0);8,658(3,8);8,644(2,0);8,462(4,4);8,459(4,5);8,449(4,6);8,447(4,4); 8,121(4,4);8,118(4,4);8,102(4 ,7);8,099(4,5);7,780(5,0);7,761(6,6);7,738(2,2);7,720(5,6);7,701(4,0);7,658(3,9); 7,639(5,0);7,620(1,9);7,477(5,9);7,458(5,1);7,208(3,8);7,196 (3,9);7,191(3,9);7,178(3,6);4,547(7,2);4,533(16,0); 4,518(7,6);3,648(3,8);3,634(10,8);3,620(10,4);3,605(3,5);3,326(87,0);3,308(0,4);2,676(0,5 );2,671(0,7);2,667 (0,5);2,542(1,4);2,525(2,1);2,511(42,1);2,507(84,0);2,502(109,9);2,498(79,3);2,493(38,1);2,333(0,5);2,329(0,7); 2,324(0,5); 1,235(0,4);0,008(0,6);0,000(16,6);-0,009(0,5) |
| Verbindung Nr. 1-82:<br>1H-NMR(400,0 MHz, DMSO): δ= 8,327(1,4);8,210(2,1);8,204(3,2);8,183(3,0);8,177(2,0);7,746(0,8);7,726(1,1); 7,709(0,4);7,692(0,9);7,673(0 ,7);7,627(0,6);7,608(0,8);7,448(1,0);7,429(0,8);4,521(5,3);3,325(16,6);2,541(0,3); 2,524(0,4);2,520(0,6);2,511(7,6);2,507(15,3);2,502(20,1);2, 497(14,3);2,493(6,7);1,428(16,0);0,000(3,8) |
| Verbindung Nr. 1-83: |

(fortgesetzt)

| |
|---|
| 1H-NMR(400,0 MHz, DMSO): δ= 8,320(1,4);8,159(1,2);8,155(1,3);8,147(1,3);8,143(1,3);8,057(1,2);8,053(1,2); 8,038(1,3);8,034(1,2);7,748(0 ,9);7,728(1,1);7,710(0,4);7,692(1,0);7,673(0,7);7,627(0,7);7,608(0,9);7,482(1,0); 7,464(0,9);6,971(1,3);6,958(1,3);6,951(1,2);6,939(1,2);4,508 (5,3);3,326(13,1);2,511(5,5);2,507(11,0);2,502 (14,5);2,497(10,5);2,493(5,1);1,441(16,0);0,000(2,3) |
| Verbindung Nr. 1-84:<br>1H-NMR(400,0 MHz, DMSO): δ= 8,582(1,0);8,581(1,0);8,578(0,9);8,536(1,2);8,087(0,7);8,081(0,7);8,065(0,7); 8,059(0,7);7,484(0,4);7,480(0 ,4);7,463(0,8);7,446(0,4);7,442(0,5);7,132(1,4);7,113(1,6);7,111 (1,5);7,092(1,2); 7,008(1,1);6,987(1,1);4,521(4,9);3,327(219,1);3,299(0,5);2,6 75(0,5);2,671(0,7);2,666(0,5);2,541(0,6);2,524 (2,2);2,519(3,5);2,511(42,7);2,506(85,4);2,502(112,0);2,497(80,2);2,493(37,9);2,333(0,5);2,32 8(0,7);2,324(0,5); 1,425(16,0);0,000(8,3) |
| Verbindung Nr. 1-85:<br>1H-NMR(400,0 MHz, DMSO): δ= 8,965(1,8);8,953(3,2);8,940(1,8);7,982(5,6);7,978(5,9);7,970(5,8);7,966(5,8); 7,702(3,2);7,698(3,2);7,682(3 ,6);7,678(3,5);7,674(3,5);7,670(3,2);7,654(3,5);7,651(3,2);7,539(1,3);7,523(2,7); 7,518(2,6);7,506(1,8);7,501(5,3);7,497(1,9);7,485(2,6);7,480 (3,1);7,464(1,4);7,180(1,1);7,177(1,5);7,169(8,8); 7,150(11,5);7,129(7,3);7,122(1,3);7,054(3,4);7,046(3,7);7,042(3,5);7,034(6,0);7,026(3,3);7, 022(3,2);7,014(2,9); 4,480(7,7);4,466(16,0);4,451(8,1);3,681(4,0);3,667(11,1);3,653(10,7);3,639(3,6);3,329(240,2);3,304(0,5);2,676 (0,7);2,67 1(0,9);2,667(0,6);2,541(1,8);2,524(3,2);2,511(54,5);2,507(106,2);2,502(137,7);2,498(99,8);2,493 (48,5);2,338(0,3);2,333(0,7);2,329(0,9);2,32 5(0,6);0,000(8,6) |
| Verbindung Nr. 1-86:<br>1H-NMR(400,0 MHz, DMSO): δ= 8,869(2,7);8,856(3,9);8,843(2,0);8,452(5,2);8,449(5,3);8,440(4,7);8,437(4,2); 8,115(4,8);8,112(4,6);8,096(4 ,8);8,093(4,2);7,537(1,5);7,520(3,3);7,515(2,9);7,503(2,7);7,499(5,8);7,494(2,3); 7,482(2,9);7,478(3,3);7,461(1,5);7,206(4,3);7,205(4,4);7,192 (4,9);7,187(4,7);7,175(6,2);7,168(9,8);7,154(4,4); 7,148(11,8);7,138(2,3);7,128(7,7);7,120(1,3);4,544(8,5);4,529(16,0);4,515(7,6);3,669(5,0); 3,655(11,7);3,641 (10,6);3,627(3,5);3,335(68,8);3,328(394,1);3,292(0,5);3,288(0,4);2,680(0,7);2,676(1,0);2,671(1,3);2,667(0,9); 2,541(5,2);2, 511(109,3);2,507(171,1);2,502(200,6);2,497(137,2);2,493(62,8);2,334(1,1);2,329(1,3);2,324(0,9); 1,235(0,4);0,008(1,3);0,005(1,4);0,000(12, 1);-0,008(0,4) |
| Verbindung Nr. 1-87:<br>1H-NMR(400,0 MHz, DMSO): δ= 8,402(2,5);8,124(2,5);8,119(2,6);8,111(2,6);8,107(2,6);7,903(2,5);7,899(2,5); 7,884(2,7);7,880(2,5);7,499(0 ,4);7,482(0,9);7,478(0,8);7,466(0,6);7,461(1,8);7,457(0,6);7,445(0,9);7,440(1,0); 7,424(0,5);7,145(0,3);7,142(0,5);7,134(2,9);7,115(3,4);7,113 (3,2);7,095(2,4);7,086(0,4);7,040(2,7);7,028(2,6); 7,021(2,5);7,009(2,6);4,648(2,6);4,622(3,3);4,438(3,4);4,412(2,8);3,328(77,8);2,541(0,7);2, 524(1,0);2,520(1,6); 2,511(18,3);2,507(36,3);2,502(47,2);2,497(33,7);2,493(15,8);2,081(0,8);2,063(1,0);2,047(1,1);2,028(0,9);1,737 (1,0);1,71 8(1,2);1,702(1,0);1,684(0,9);1,368(16,0);0,932(3,7);0,914(8,3);0,895(3,4);0,000(5,1) |
| Verbindung Nr. 1-88:<br>1H-NMR(400,0 MHz, DMSO): δ= 8,554(1,5);8,203(1,8);8,197(2,6);8,174(2,4);8,168(1,7);7,481(0,5);7,477(0,4); 7,460(0,9);7,443(0,4);7,439(0 ,5);7,131(1,4);7,112(1,9);7,092(1,2);4,514(5,3);3,325(25,8);2,541(0,4);2,524(0,4); 2,507(18,6);2,502(24,1);2,498(17,6);1,433(16,0);0,000(3,2) |
| Verbindung Nr. 1-89:<br>1H-NMR(400,0 MHz, DMSO): δ= 8,560(1,2);8,157(1,1);8,153(1,3);8,145(1,2);8,141(1,3);8,049(1,2);8,045(1,2); 8,030(1,3);8,025(1,2);7,483(0 ,5);7,479(0,4);7,462(0,9);7,458(0,3);7,445(0,4);7,441(0,5);7,134(1,4);7,115(1,7); 7,113(1,6);7,094(1,2);6,970(1,3);6,958(1,2);6,951(1,2);6,939 (1,2);4,486(5,3);3,324(14,2);2,541(0,3);2,524(0,3); 2,511(6,2);2,507(12,3);2,502(16,2);2,497(11,7);2,493(5,6);1,451(16,0);0,000(2,8) |
| Verbindung Nr. 1-90:<br>1H-NMR(400,0 MHz, DMSO): δ= 8,590(1,0);8,588(1,0);8,586(1,0);8,584(1,0);8,441(1,4);8,091(0,7);8,085(0,7); 8,069(0,7);8,062(0,7);8,047(1 ,0);8,044(1,1);8,026(1,2);8,024(1,1);7,779(0,5);7,776(0,5);7,761(1,2);7,758(1,2); 7,742(0,8);7,739(0,8);7,678(0,7);7,674(0,8);7,658(0,9);7,654 (0,9);7,639(0,5);7,635(0,5);7,514(1,1);7,510(1,1); 7,495(1,0);7,491(0,9);7,055(1,2);7,033(1,2);4,526(5,0);3,326(24,9);2,541(1,7);2,524(0,5);2, 520(0,7);2,511(8,9); 2,507(17,9);2,502(23,3);2,497(16,6);2,493(7,8);1,437(16,0);0,000(3,1) |
| Verbindung Nr. 1-91: |

(fortgesetzt)

| |
|---|
| 1H-NMR(400,0 MHz, DMSO): δ= 8,817(1,2);8,803(2,3);8,790(1,2);8,569(3,4);8,566(4,3);8,563(4,3);8,561(3,5); 8,392(4,8);8,387(4,5);7,730(1 ,6);7,722(1,9);7,717(1,5);7,713(1,7);7,606(2,0);7,593(6,0);7,590(10,3);7,587(8,2); 7,582(16,0);7,578(3,1);5,757(0,6);4,617(3,6);4,603(7,2);4,5 90(3,8);3,711(1,9);3,697(5,1);3,683(4,9);3,669(1,8); 3,324(10,6);2,525(0,8);2,521(1,3);2,512(16,6);2,508(33,5);2,503(44,1);2,498(31,6);2,494 (15,0);0,008(1,1); 0,000(32,2);-0,009(1,0) |
| Verbindung Nr. 1-92:<br>1H-NMR(400,0 MHz, DMSO): δ= 8,820(1,2);8,807(2,2);8,793(1,2);8,589(3,5);8,587(3,7);8,585(3,7);8,583(3,5); 8,317(0,5);8,088(2,4);8,082(2 ,4);8,066(2,5);8,060(2,5);7,733(1,4);7,730(1,4);7,725(1,9);7,713(2,0);7,605(0,9); 7,600(2,7);7,590(4,8);7,584(8,4);7,577(16,0);7,569(3,1);7,00 0(4,2);6,979(4,0);4,512(3,9);4,498(8,0);4,484(4,2); 3,678(2,0);3,664(5,5);3,650(5,4);3,636(1,9);3,322(23,3);2,676(0,4);2,671(0,6);2,666(0,4); 2,525(1,8);2,511 (33,9);2,507(67,2);2,502(87,4);2,498(63,5);2,493(31,0);2,333(0,4);2,329(0,6);2,324(0,4);0,008(1,9);0,000 (49,4);-0,009(1,7) |
| Verbindung Nr. 1-93:<br>1H-NMR(400,0 MHz, DMSO): δ= 8,766(1,8);8,753(3,6);8,740(1,9);8,454(3,9);8,451(4,0);8,441(4,1);8,438(3,9); 8,317(0,5);8,112(3,9);8,109(3 ,9);8,093(4,2);8,090(3,9);7,735(2,4);7,727(2,8);7,724(3,0);7,714(3,2);7,610(1,3); 7,602(7,2);7,593(8,2);7,587(9,5);7,579(16,0);7,572(9,7);7,56 4(3,2);7,558(2,5);7,548(1,2);7,201(3,4);7,189(3,6); 7,184(3,5);7,171(3,2);4,576(6,3);4,562(13,4);4,547(6,6);3,674(3,3);3,660(9,3);3,646(9,1); 3,632(3,0);3,323 (91,2);2,675(1,1);2,671(1,4);2,666(1,0);2,662(0,5);2,524(4,7);2,511(85,1);2,506(166,1);2,502(214,9);2,497 (153,7);2,493(72, 8);2,333(1,0);2,328(1,4);2,324(1,0);2,319(0,5);1,989(0,4);0,146(0,4);0,008(3,3);0,000(82,1);- 0,009(2,5) |
| Verbindung Nr. 1-94:<br>1H-NMR(400,0 MHz, DMSO): δ= 8,574(1,2);8,570(1,1);8,303(1,6);8,075(0,8);8,069(0,8);8,053(0,8);8,047(0,8); 7,710(0,6);7,689(0,8);7,576(0 ,9);7,570(1,3);7,561(2,4);7,554(1,4);7,546(1,0);7,533(0,3);7,515(1,3);7,508(0,8); 7,502(0,5);7,498(0,5);7,492(0,6);7,006(1,3);6,984(1,2);4,561 (5,2);3,324(5,8);2,511(6,4);2,507(12,2);2,502 (15,7);2,498(11,4);2,494(5,6);1,450(16,0);1,398(3,3);0,000(6,2) |
| Verbindung Nr. 1-95:<br>1H-NMR(400,0 MHz, DMSO): δ= 8,804(1,2);8,791(2,3);8,777(1,2);8,134(4,5);8,130(4,7);8,122(4,7);8,118(4,6); 7,904(4,5);7,900(4,4);7,885(4 ,8);7,881(4,4);7,737(1,4);7,729(1,8);7,723(1,9);7,721(2,0);7,719(1,8);7,715(1,7); 7,621(0,6);7,609(0,8);7,606(0,8);7,593(16,0);7,586(13,2);7,5 71(1,1);7,046(4,9);7,034(4,8);7,027(4,6);7,015 (4,6);4,510(4,4);4,496(9,4);4,482(4,6);3,679(2,3);3,665(6,5);3,651(6,3);3,637(2,1);3,323(40,4) ;2,675(0,5);2,671 (0,6);2,666(0,4);2,524(2,4);2,511(38,4);2,506(74,4);2,502(95,6);2,497(67,7);2,493(31,6);2,333(0,5);2,329(0,6); 2,324(0,4);1, 989(0,7);1,175(0,4);0,008(1,6);0,000(39,7);-0,009(1,2) |
| Verbindung Nr. 1-96:<br>1H-NMR(400,0 MHz, DMSO): δ= 8,538(0,4);8,535(0,4);8,371(0,5);8,366(0,5);5,751(16,0);4,635(0,4);4,621(0,7); 4,607(0,4);3,724(0,5);3,710( 0,5);3,335(3,4);2,513(1,6);2,509(2,0);2,505(1,5);0,000(1,3) |
| Verbindung Nr. 1-97:<br>1H-NMR(400,0 MHz, DMSO): δ= 9,238(0,4);8,863(2,6);8,849(5,2);8,835(2,6);8,592(7,2);8,590(8,9);8,587(8,8); 8,585(7,2);8,536(0,8);8,533(0 ,7);8,531(0,6);8,401(10,2);8,396(9,5);8,382(0,9);8,377(0,8);8,317(0,4);8,168(0,6); 8,151(7,0);8,131(7,3);8,110(0,6);8,093(0,6);8,056(3,6);8,05 3(3,6);8,037(8,3);8,034(8,0);8,018(5,1);8,015(4,7); 7,944(0,3);7,941(0,3);7,925(0,6);7,906(0,4);7,903(0,4);7,874(4,9);7,871(5,1);7,854(6,9);7, 852(7,0);7,843(0,8); 7,835(3,7);7,832(3,7);7,827(0,8);7,824(0,7);7,679(7,7);7,677(7,8);7,660(7,3);7,657(6,7);5,757(10,6);4,627(0,6); 4,613(1,3 );4,599(0,8);4,582(7,3);4,568(16,0);4,554(7,6);3,741(0,7);3,727(0,7);3,713(0,4);3,696(3,9);3,682 (10,9);3,667(10,6);3,653(3,5);3,323(68,5);2, 681(0,4);2,676(0,9);2,671(1,2);2,667(0,8);2,662(0,4);2,525(4,0); 2,511(71,2);2,507(138,1);2,502(177,3);2,498(125,9);2,493(59,1);2,338(0,5); 2,334(0,9);2,329(1,2);2,325(0,9); 1,236(1,3);1,175(0,6);1,167(0,6);1,151(0,4);0,146(0,5);0,008(5,4);0,000(130,5);-0,009(4,2);-0,150(0,5) |
| Verbindung Nr. 1-98: |

(fortgesetzt)

| |
|---|
| 1H-NMR(400,0 MHz, DMSO): δ= 8,846(2,2);8,832(4,3);8,818(2,2);8,316(0,5);8,150(11,7);8,145(8,7);8,137(9,0); 8,133(13,3);8,055(3,3);8,05 2(3,4);8,036(7,4);8,033(7,1);8,017(4,6);8,014(4,2);7,988(0,4);7,914(7,7);7,909(7,8); 7,904(1,0);7,894(8,4);7,890(7,9);7,871(4,4);7,868(4,7);7, 852(6,0);7,849(6,2);7,832(3,3);7,829(3,1);7,692(0,3); 7,677(6,8);7,674(6,8);7,658(6,3);7,655(5,9);7,548(0,3);7,057(8,1);7,045(7,7);7,038(7,6) ;7,026(7,5);4,485(7,2); 4,470(16,0);4,455(7,5);3,664(3,8);3,650(10,7);3,635(10,4);3,620(3,4);3,324(63,7);2,680(0,5);2,676(1,0);2,671 (1,4);2,6 66(1,0);2,662(0,5);2,620(0,6);2,524(6,6);2,519(10,3);2,511(83,4);2,507(163,8);2,502(213,8);2,497 (154,7);2,493(74,7);2,338(0,5);2,333(1,1); 2,329(1,4);2,324(1,1);1,754(2,5);1,448(0,6);1,236(1,7);1,182(0,5); 1,166(0,7);1,150(0,4);0,008(2,2);0,000(61,0);-0,009(2,1) |
| Verbindung Nr. 1-99:<br>1H-NMR(400,0 MHz, DMSO): δ= 8,806(2,6);8,793(5,0);8,779(2,5);8,465(5,3);8,455(5,2);8,155(6,4);8,135(7,5); 8,124(5,3);8,121(5,2);8,105(5 ,3);8,073(0,3);8,060(3,3);8,058(3,4);8,041(7,4);8,040(7,2);8,022(4,4);7,904(0,4); 7,899(0,5);7,895(0,4);7,873(4,4);7,871(4,5);7,852(7,0);7,835 (3,2);7,832(3,1);7,630(7,2);7,612(6,7);7,548(0,3); 7,213(4,3);7,200(4,4);7,194(4,5);7,182(4,0);5,757(3,6);4,547(7,3);4,532(16,0);4,517(7,6);4, 039(0,6);4,023(0,6); 3,651(4,0);3,636(11,0);3,622(10,7);3,607(3,6);3,324(28,5);2,676(0,8);2,672(1,0);2,667(0,8);2,507(118,7);2,503 (153,3);2, 498(114,7);2,334(0,7);2,329(1,0);2,325(0,8);1,755(0,6);1,236(3,0);1,183(1,1);1,166(1,7);1,150(0,9); 0,854(0,3);0,008(0,3);0,000(7,2) |
| Verbindung Nr. 1-100:<br>1H-NMR(400,0 MHz, DMSO): δ= 9,119(1,5);9,105(2,9);9,092(1,4);8,953(0,3);8,938(4,9);8,934(5,2);8,927(5,2); 8,923(5,0);8,590(4,3);8,587(5 ,3);8,584(5,3);8,582(4,2);8,400(6,0);8,394(5,6);8,316(0,5);8,231(4,0);8,227(4,1); 8,211(5,0);8,207(4,7);8,031(5,6);8,019(5,3);8,011(4,6);7,999 (4,5);5,756(8,6);4,609(0,7);4,599(4,5);4,584(9,4); 4,570(4,5);3,830(0,4);3,751(0,7);3,730(2,9);3,716(7,0);3,702(6,9);3,688(2,8);3,654(0,6);3,6 40(0,5);2,680(0,4); 2,676(0,9);2,671(1,3);2,666(0,9);2,662(0,4);2,524(4,4);2,520(6,9);2,511(70,2);2,507(140,4);2,502(184,6);2,497 (132,5);2,4 93(63,3);2,338(0,4);2,333(0,9);2,329(1,2);2,324(0,8);2,320(0,4);1,754(16,0);0,000(2,7) |
| Verbindung Nr. 1-101:<br>1H-NMR(400,0 MHz, DMSO): δ= 8,694(2,2);8,681(4,2);8,668(2,2);8,449(4,9);8,447(5,1);8,437(5,3);8,431(8,8); 8,426(7,3);8,418(7,1);8,413(7 ,0);8,316(0,6);8,110(4,9);8,107(4,9);8,092(5,1);8,089(4,9);8,028(7,0);8,023(7,1); 8,009(7,8);8,004(7,3);7,519(7,6);7,507(7,4);7,501(7,2);7,488 (6,9);7,201(4,1);7,189(4,3);7,183(4,2);7,170(3,8); 4,557(7,7);4,543(16,0);4,529(8,1);3,687(4,2);3,673(11,6);3,659(11,3);3,645(3,8);3,324(243, 0);2,680(0,6);2,676 (1,2);2,671(1,7);2,667(1,2);2,662(0,6);2,542(51,7);2,525(5,7);2,511(100,0);2,507(200,6);2,502(262,8);2,498 (188,1);2,493 (89,3);2,338(0,6);2,333(1,2);2,329(1,6);2,324(1,2);1,259(0,4);1,235(0,8);0,008(0,5);0,000(14,2);- 0,009(0,4) |
| Verbindung Nr. 1-102:<br>1H-NMR(400,0 MHz, DMSO): δ= 8,577(1,0);8,576(1,0);8,573(1,0);8,402(1,0);8,397(1,1);8,389(1,1);8,385(1,1); 8,357(1,4);8,093(0,7);8,087(0 ,7);8,071(0,7);8,065(0,7);7,989(1,0);7,985(1,1);7,971(1,2);7,966(1,1);7,480(1,1); 7,468(1,1);7,461(1,1);7,449(1,1);7,004(1,2);6,982(1,1);4,529 (5,0);3,324(24,6);2,542(5,2);2,524(0,7);2,511 (13,9);2,507(28,0);2,502(36,8);2,497(26,3);2,493(12,5);1,438(16,0);0,000(2,3) |
| Verbindung Nr. 1-103:<br>1H-NMR(400,0 MHz, DMSO): δ= 8,773(1,9);8,760(3,5);8,746(1,8);8,433(6,3);8,428(6,7);8,421(6,7);8,416(6,6); 8,316(0,4);8,133(7,4);8,129(7 ,9);8,121(7,8);8,117(7,8);8,059(6,9);8,054(7,0);8,040(7,8);8,035(7,2);7,907(7,4); 7,903(7,4);7,888(8,1);7,884(7,4);7,518(7,2);7,506(7,1);7,499 (6,8);7,487(6,7);7,050(8,2);7,038(8,0);7,031(7,8); 7,019(7,8);4,494(7,5);4,480(16,0);4,465(8,0);3,687(4,0);3,673(11,2);3,659(10,8);3,644(3,6) ;3,324(129,8);2,680 (0,4);2,676(0,9);2,671(1,3);2,667(0,9);2,662(0,4);2,541(46,6);2,525(3,8);2,520(6,1);2,511(74,8);2,507(149,0); 2,502(193, 7);2,498(136,5);2,493(63,3);2,338(0,4);2,333(0,9);2,329(1,2);2,324(0,9);2,320(0,4);1,235(0,5);0,008 (0,5);0,000(14,0);-0,009(0,4) |
| Verbindung Nr. 1-104:<br>1H-NMR(400,0 MHz, DMSO): δ= 8,809(2,2);8,796(4,1);8,782(2,1);8,592(6,7);8,590(6,9);8,588(6,9);8,586(6,8); 8,432(7,0);8,427(7,6);8,420(7 ,5);8,415(7,4);8,316(0,4);8,096(4,8);8,090(4,7);8,074(5,2);8,067(5,4);8,063(8,2); 8,058(7,9);8,044(8,6);8,040(8,0);7,509(8,1);7,497(7,9);7,491 (7,7);7,478(7,5);7,002(8,2);6,980(7,9);4,498(7,9); 4,484(16,0);4,470(8,4);3,683(4,2);3,669(11,5);3,655(11,1);3,642(3,8);3,325(136,0);2,681(0, 4);2,676(0,9);2,671 (1,3);2,667(0,9);2,662(0,4);2,542(23,5);2,525(4,1);2,520(6,3);2,511(73,5);2,507(148,4);2,502(194,7);2,498 (139,1);2,493( 65,7);2,338(0,4);2,334(0,9);2,329(1,2);2,325(0,9);2,320(0,4);1,235(0,5);0,008(0,4);0,000(13,1);- 0,009(0,4) |

(fortgesetzt)

| |
|---|
| Verbindung Nr. 1-105:<br>1H-NMR(400,0 MHz, DMSO): δ= 8,782(2,5);8,769(4,9);8,755(2,5);8,570(7,3);8,567(9,1);8,564(9,1);8,562(7,6); 8,434(7,2);8,430(7,7);8,422(7 ,7);8,417(7,6);8,398(11,0);8,393(10,3);8,317(0,4);8,061(7,6);8,056(7,7);8,042 (8,4);8,037(7,9);7,518(8,1);7,505(7,9);7,499(7,7);7,487(7,4);4,5 97(7,9);4,584(16,0);4,570(8,3);3,717(4,2);3,703 (11,5);3,689(11,1);3,675(3,9);3,326(202,6);2,676(0,8);2,672(1,1);2,667(0,8);2,542(27,8);2,52 5(3,5);2,512(67,5); 2,507(133,9);2,503(174,5);2,498(124,5);2,494(59,0);2,339(0,4);2,334(0,8);2,330(1,1);2,325(0,8);1,235(0,5); 0,000(8,4) |
| Verbindung Nr. 1-106:<br>1H-NMR(400,0 MHz, DMSO): δ= 8,971(2,6);8,957(4,7);8,943(2,4);8,784(7,0);8,780(7,4);8,772(7,3);8,768(7,1); 8,576(7,6);8,574(9,4);8,571(9 ,4);8,568(7,6);8,414(10,6);8,408(10,0);8,317(0,4);7,984(5,1);7,982(5,4);7,965 (5,9);7,963(6,1);7,674(5,1);7,662(5,0);7,654(4,6);7,643(4,3);7,2 96(5,6);7,161(12,7);7,025(6,2);6,574(0,4);5,757 (2,1);4,621(8,0);4,607(16,0);4,594(8,2);3,728(4,2);3,714(11,3);3,700(10,9);3,686(3,8);3,324( 109,5);2,891(3,6); 2,731(2,9);2,680(0,4);2,676(0,8);2,671(1,2);2,667(0,8);2,662(0,4);2,542(0,8);2,525(3,7);2,511(67,8);2,507 (134,9);2,502(17 5,9);2,498(124,2);2,493(57,6);2,338(0,4);2,334(0,8);2,329(1,1);2,325(0,8);2,320(0,4);1,754 (2,5);1,336(0,3);1,235(0,3);0,146(0,5);0,008(5,2); 0,000(129,8);-0,009(3,9);-0,150(0,5) |
| Verbindung Nr. 1-107:<br>1H-NMR(400,0 MHz, DMSO): δ= 8,499(2,8);8,408(8,2);8,406(8,8);8,395(8,4);8,393(9,0);7,620(2,6);7,616(3,0); 7,602(4,9);7,597(5,9);7,587(0 ,7);7,582(3,1);7,578(3,2);7,552(1,5);7,547(1,4);7,538(1,7);7,533(3,3);7,531(2,5); 7,529(2,5);7,526(2,2);7,518(2,6);7,516(2,5);7,513(3,6);7,508 (2,0);7,499(2,0);7,495(1,7);7,422(7,5);7,419(8,5); 7,409(7,0);7,406(8,3);7,379(9,3);7,377(13,0);7,298(4,3);7,288(4,6);7,286(4,8);7,277(4,2);7, 273(4,4);7,269(9,8); 7,250(8,5);5,756(0,6);4,470(7,3);4,456(16,0);4,441(7,8);3,665(3,7);3,651(10,4);3,637(10,2);3,622(3,4);3,324 (42,2);2,676 (0,4);2,671(0,6);2,667(0,4);2,542(0,4);2,525(1,7);2,511(33,7);2,507(67,4);2,502(88,5);2,498(63,5); 2,493(30,5);2,334(0,4);2,329(0,6);2,325(0, 4);0,146(0,3);0,008(3,3);0,000(81,7);-0,009(3,0);-0,150(0,4) |
| Verbindung Nr. 1-108:<br>1H-NMR(400,0 MHz, DMSO): δ= 8,651(2,0);8,638(3,9);8,624(2,0);8,418(8,5);8,416(9,4);8,405(8,8);8,403(9,7); 7,492(4,2);7,491(3,6);7,474(8 ,6);7,472(8,5);7,471(8,2);7,454(3,9);7,447(4,7);7,439(5,0);7,432(7,1);7,426(9,3); 7,423(9,8);7,420(3,6);7,413(11,7);7,410(10,6);7,403(3,4);7,3 90(16,0);7,386(12,2);7,375(6,1);7,371(14,5);7,369 (15,1);7,366(10,1);7,356(1,4);7,353(1,2);4,462(7,3);4,448(16,0);4,433(7,8);3,639(3,9);3,62 5(11,0);3,611(10,7); 3,597(3,5);3,322(36,0);3,061(0,3);2,675(0,5);2,671(0,7);2,666(0,5);2,541(0,5);2,524(2,2);2,511(42,8);2,506 (87,0);2,502( 115,4);2,497(83,3);2,493(40,4);2,333(0,5);2,328(0,7);2,324(0,5);0,146(0,4);0,008(4,2);0,000 (110,6);-0,009(4,3);-0,150(0,5) |
| Verbindung Nr. 1-109:<br>1H-NMR(400,0 MHz, DMSO): δ= 8,642(2,1);8,629(3,9);8,615(2,1);8,418(8,8);8,416(8,9);8,405(9,1);8,403(9,2); 7,654(1,1);7,650(4,0);7,647(8 ,2);7,632(3,3);7,627(7,3);7,440(2,2);7,437(2,5);7,425(10,2);7,422(10,5);7,419 (9,1);7,412(7,9);7,409(9,2);7,403(7,0);7,400(6,5);7,375(13,9);7, 373(10,3);7,368(14,5);7,366(15,7);7,351(8,5); 7,348(9,2);7,331(3,1);7,326(2,5);5,756(0,4);4,462(7,3);4,448(16,0);4,434(7,7);3,634(3,8);3,620 (10,9);3,606 (10,6);3,591(3,5);3,323(40,1);2,676(0,4);2,671(0,6);2,667(0,4);2,541(0,4);2,524(1,7);2,511(34,3);2,506(69,7); 2,502(92,0);2,497( 65,7);2,493(31,3);2,333(0,4);2,329(0,6);2,324(0,4);1,989(1,2);1,193(0,3);1,175(0,7);1,157 (0,3);0,146(0,4);0,008(3,2);0,000(90,1);-0,009(3,1);-0,150(0,4) |
| Verbindung Nr. 1-110:<br>1H-NMR(400,0 MHz, DMSO): δ= 8,595(2,3);8,582(4,5);8,568(2,3);8,416(9,5);8,403(9,6);7,875(7,5);7,857(7,6); 7,855(7,7);7,447(3,1);7,445(3 ,3);7,428(7,7);7,426(8,7);7,423(9,3);7,420(9,5);7,410(11,8);7,407(13,3);7,382 (14,3);7,304(6,9);7,300(7,8);7,285(5,6);7,281(5,7);7,179(3,9);7, 175(3,9);7,160(6,4);7,156(6,2);7,141(3,4);7,137 (3,2);5,756(2,0);4,467(7,3);4,453(16,0);4,438(7,8);3,627(3,9);3,613(11,1);3,599(10,7);3,584( 3,6);3,322(46,0); 2,675(0,5);2,671(0,7);2,666(0,5);2,541(0,4);2,524(2,1);2,510(40,7);2,506(81,8);2,502(108,2);2,497(78,5);2,493 (38,3);2,333( 0,5);2,328(0,7);2,324(0,5);1,989(0,7);1,175(0,4);0,146(0,5);0,008(3,8);0,000(101,6);-0,008(4,1);- 0,150(0,5) |
| Verbindung Nr. 1-111: |

(fortgesetzt)

| |
|---|
| 1H-NMR(400,0 MHz, DMSO): δ= 8,951(2,1);8,938(3,9);8,925(2,2);8,416(9,6);8,403(9,9);7,543(1,3);7,526(2,9); 7,522(2,8);7,509(2,0);7,505(5 ,7);7,501(2,2);7,488(2,9);7,484(3,4);7,467(1,6);7,431(7,7);7,428(8,1);7,418(7,6); 7,415(7,9);7,351(13,8);7,181(1,5);7,173(9,5);7,154(13,0);7,1 33(8,1);7,125(1,6);4,443(7,5);4,429(16,0);4,414 (8,1);3,661(4,0);3,647(11,2);3,633(10,9);3,619(3,8);3,324(33,6);2,676(0,5);2,672(0,6);2,667( 0,4);2,525(1,8); 2,511(39,1);2,507(78,0);2,502(101,7);2,498(73,3);2,494(35,8);2,334(0,5);2,329(0,7);2,325(0,5);1,259(0,8);1,175 (0,3);0,146(0 ,4);0,008(3,4);0,000(84,4);-0,008(3,3);-0,149(0,4) |
| Verbindung Nr. 1-112:<br>1H-NMR(400,0 MHz, DMSO): δ= 8,731(2,1);8,718(4,1);8,704(2,2);8,423(9,3);8,422(9,0);8,410(9,5);8,409(9,3); 7,779(5,1);7,760(6,8);7,731(2 ,2);7,713(5,8);7,695(4,3);7,658(4,1);7,639(5,2);7,620(1,9);7,501(6,2);7,482(5,3); 7,430(8,4);7,427(9,0);7,417(8,1);7,414(8,7);7,351(10,0);7,34 9(13,6);7,346(9,0);4,440(7,3);4,426(16,0);4,412 (7,8);3,640(3,9);3,626(10,9);3,611(10,6);3,597(3,6);3,325(31,0);2,676(0,4);2,671(0,5);2,667( 0,4);2,525(1,6); 2,511(30,2);2,507(60,5);2,502(79,3);2,498(56,5);2,493(27,2);2,334(0,4);2,329(0,5);2,325(0,4);1,989(0,9);1,234 (0,4);1,193(0, 4);1,175(0,8);1,171(0,9);1,153(1,6);1,135(0,8);0,146(0,3);0,008(3,2);0,000(79,0);-0,009(2,8);- 0,150(0,3) |
| Verbindung Nr.1-113:<br>1H-NMR(400,0 MHz,DMSO):δ= 8,841(2,2);8,827(4,2);8,814(2,3);8,471(7,0);8,466(7,9);8,459(7,6);8,454(7,7); 8,444(0,4);8,431(0,5);8,420(9 ,8);8,419(8,9);8,408(10,2);8,406(9,0);8,266(0,4);8,260(0,3);7,878(7,3);7,873(7,4); 7,859(8,3);7,855(7,9);7,831(0,9);7,811(0,9);7,499(8,1);7,48 7(7,9);7,481(7,7);7,469(7,4);7,431(8,4);7,428(8,8); 7,418(8,0);7,415(8,7);7,400(0,6);7,376(11,3);7,374(13,9);7,371(8,5);7,163(0,7);5,756(3,1) ;4,519(0,6);4,506 (0,8);4,493(0,7);4,468(7,6);4,454(16,0);4,440(8,2);4,056(0,4);4,038(1,1);4,020(1,1);4,003(0,4);3,659(4,1);3,645 (11,4);3,631 (11,1);3,617(3,9);3,325(26,4);3,241(0,4);3,236(0,7);3,223(1,1);3,209(0,6);2,989(1,8);2,676(0,5); 2,672(0,6);2,667(0,5);2,542(0,5);2,511(39,2) ;2,507(76,4);2,503(98,5);2,498(70,6);2,494(34,6);2,334(0,5);2,329 (0,6);2,325(0,5);1,989(4,7);1,236(0,5);1,193(2,2);1,175(3,5);1,158(1,6);1,1 48(0,5);1,130(0,5);0,146(0,4);0,008 (3,6);0,000(87,7);-0,009(3,6);-0,150(0,4) |
| Verbindung Nr. 1-114:<br>1H-NMR(400,0 MHz, DMSO): δ= 8,908(2,2);8,894(4,2);8,881(2,2);8,798(5,2);8,796(5,5);8,786(5,4);8,784(5,5); 8,427(9,2);8,425(9,3);8,414(9 ,4);8,412(9,5);7,995(4,8);7,993(5,0);7,976(5,9);7,973(5,8);7,806(0,5);7,788(5,8); 7,777(5,5);7,769(4,7);7,757(4,5);7,436(8,4);7,433(9,0);7,423 (8,1);7,420(8,7);7,379(0,4);7,353(13,9);7,167(0,3); 5,756(2,8);4,446(7,5);4,432(16,0);4,418(8,0);4,038(0,6);4,020(0,7);3,664(4,0);3,650(11,1) ;3,636(10,7);3,622 (3,6);3,324(45,1);2,956(0,7);2,676(0,6);2,671(0,8);2,667(0,6);2,541(0,5);2,525(2,3);2,511(48,8);2,507(99,3); 2,502(131,2); 2,498(94,1);2,493(45,2);2,334(0,6);2,329(0,8);2,324(0,6);1,989(2,8);1,235(0,5);1,193(0,8);1,175 (1,5);1,157(0,8);0,146(0,6);0,008(4,7);0,000( 132,3);-0,009(4,9);-0,150(0,6) |
| Verbindung Nr. 1-115:<br>1H-NMR(400,0MHz,DMSO): δ= 8,803(2,3);8,789(4,4);8,776(2,3);8,434(6,8);8,429(7,5);8,422(7,5);8,416(12,0); 8,414(11,0);8,402(9,3);8,40 1(10,5);8,390(0,4);8,065(7,2);8,060(7,4);8,046(8,0);8,041(7,6);7,514(7,6);7,502 (7,5);7,495(7,3);7,483(7,0);7,430(8,6);7,427(9,3);7,417(8,3); 7,414(9,0);7,338(14,3);7,336(10,1);5,757(0,4); 4,460(7,7);4,446(16,0);4,432(8,2);3,668(4,2);3,654(11,5);3,640(11,1);3,626(3,8);3,324(30,4);2 ,948(0,6);2,676 (0,4);2,672(0,6);2,667(0,4);2,542(0,4);2,525(1,7);2,512(34,9);2,507(71,1);2,503(94,4);2,498(68,2);2,494(32,9); 2,334(0,4);2,3 30(0,6);2,325(0,5);1,989(1,2);1,193(0,4);1,176(0,7);1,158(0,3);0,146(0,4);0,008(3,6);0,000 (99,9);-0,009(3,8);-0,150(0,4) |
| Verbindung Nr. 1-116:<br>1H-NMR(400,0 MHz, DMSO): δ= 8,713(2,0);8,700(3,8);8,686(2,1);8,176(11,3);8,162(11,6);7,779(4,9);7,759(6,6); 7,729(2,1);7,712(5,6);7,69 3(4,2);7,656(4,0);7,637(5,1);7,618(1,9);7,497(6,0);7,479(5,1);7,134(7,9);7,129(8,3); 7,120(7,7);7,115(8,1);6,944(12,0);6,940(11,4);5,756(4,0) ;4,406(7,3);4,392(16,0);4,377(7,9);3,622(3,9);3,608 (11,0);3,593(10,7);3,579(3,6);3,324(13,7);2,671(0,4);2,525(1,2);2,511(23,1);2,507(46,7); 2,502(61,5);2,498 (44,3);2,493(21,5);2,329(0,4);1,989(0,6);1,175(0,3);0,008(2,5);0,000(63,8);-0,009(2,3) |
| Verbindung Nr. 1-117: |

(fortgesetzt)

| |
|---|
| 1H-NMR(400,0 MHz, DMSO): δ= 8,921(0,4);8,890(2,1);8,877(3,9);8,863(2,1);8,795(5,4);8,784(5,4);8,180(11,1);8,171(1,9);8,166(11,4);8,15 7(1,4);7,990(4,8);7,971(5,7);7,788(5,1);7,776(5,1);7,768(4,5);7,756(4,2);7,505(0,5);7,175(0,9);7,155(1,3);7,140(6,8);7,135(8,4);7,126(6,7);7, 121(7,5);6,948(11,9);6,944(11,7);5,757(2,7);4,413 (7,5);4,399(16,0);4,391(4,0);4,385(8,2);4,057(0,4);4,039(1,2);4,021(1,2);4,003(0,4);3,647( 4,1);3,633(11,4);3,619(11,1);3,605(3,8);3,325(17,2);2,945(0,5);2,672(0,4);2,525(1,4);2,512(27,6);2,507(54,5);2,503(71,2);2,498 (51,7);2,494( 25,7);2,334(0,3);2,330(0,5);1,990(5,1);1,236(0,4);1,193(1,4);1,175(2,7);1,158(1,3);0,146(0,3);0,008(3,1);0,000(73,3);-0,009(3,3);-0,150(0,3) |
| Verbindung Nr. 1-118:<br>1H-NMR(400,0 MHz, DMSO): δ= 8,935(1,9);8,921(3,5);8,908(2,0);8,171(11,1);8,157(11,4);7,542(1,3);7,525(2,9);7,521(2,7);7,509(2,0);7,50 4(5,6);7,500(2,2);7,487(2,8);7,483(3,4);7,466(1,5);7,185(1,1);7,182(1,6);7,174(9,4);7,155(12,3);7,135(13,9);7,131(9,7);7,126(2,5);7,121(7,9) ;7,117(8,0);6,946(11,9);6,941(11,4);5,757(6,9);4,405 (7,6);4,391(16,0);4,377(8,1);3,644(4,0);3,630(11,2);3,616(10,9);3,602(3,7);3,325(30,6); 2,671(0,4);2,524(1,3);2,511(23,2);2,507(46,6);2,502(61,3);2,498(44,3);2,493(21,6);2,329(0,4);1,989(1,0);1,235(0,3);1,175(0,5);0,008 (2,3);0, 000(57,9);-0,009(2,3) |
| Verbindung Nr. 1-119:<br>1H-NMR(400,0 MHz, DMSO): δ= 8,587(2,1);8,573(4,1);8,559(2,2);8,171(11,2);8,158(11,5);7,875(7,2);7,856(7,5);7,447(2,8);7,445(3,1);7,42 8(6,8);7,426(7,3);7,409(4,4);7,407(4,6);7,303(6,4);7,299(7,3);7,284(5,3);7,280(5,3);7,179(3,6);7,175(3,6);7,160(6,0);7,156(5,8);7,141(3,2);7, 137(3,1);7,128(7,3);7,123(7,8);7,114(7,0);7,109(7,5);6,980(12,1);6,976(11,6);5,756(2,6);4,434(7,3);4,420(16,0);4,405(7,7);3,609(3,9);3,595( 11,0);3,580(10,7);3,566 (3,5);3,323(16,0);2,671(0,4);2,510(24,4);2,506(48,8);2,502(64,2);2,497(46,6);2,493(22,8);2,329(0,4);1,989(1,2);1,19 3(0,3);1,175(0,6);1,157(0,3);0,008(2,4);0,000(60,9);-0,008(2,3) |
| Verbindung Nr. 1-120:<br>1H-NMR(400,0 MHz, DMSO): δ= 8,501(2,9);8,442(7,0);8,429(7,1);7,620(2,5);7,616(3,0);7,602(4,9);7,597(6,0);7,588(0,8);7,582(3,1);7,578(3 ,3);7,551(1,5);7,546(1,5);7,538(1,7);7,533(3,2);7,530(2,6);7,528(2,5);7,526(2,3);7,519(2,6);7,517(2,6);7,515(2,6);7,512(3,6);7,507(2,0);7,499 (2,0);7,494(1,7);7,341(5,3);7,338(5,6);7,327(5,3);7,325(5,4);7,298(4,3);7,287(4,6);7,285(4,9);7,277(4,3);7,273(4,5);7,268(9,2);7,250(8,0);7,1 74(10,0);5,758(0,4);4,501(7,4);4,487(16,0);4,472(7,9);3,680(3,8);3,666(10,5);3,652(10,3);3,638(3,5);3,326(12,2);2,673(0,4);2,513 (20,6);2,50 9(41,4);2,504(54,7);2,499(40,4);2,495(20,3);2,331(0,3);0,008(2,3);0,000(53,7);-0,008(2,6) |
| Verbindung Nr. 1-121:<br>1H-NMR(400,0 MHz, DMSO): δ= 8,652(1,9);8,638(3,6);8,625(1,9);8,451(7,1);8,438(7,3);7,492(3,9);7,473(8,4);7,471(8,1);7,453(4,0);7,446(4 ,6);7,438(4,9);7,431(6,7);7,427(2,1);7,418(2,4);7,411(5,0);7,403(3,0);7,390(12,6);7,389(11,5);7,385(14,5);7,373(4,9);7,370(5,0);7,366(1,6);7, 354(1,5);7,351(1,6);7,344(5,4);7,342(5,6);7,331 (5,3);7,329(5,3);7,158(9,9);5,757(1,2);4,493(7,5);4,479(16,0);4,464(7,8);4,039(0,3);4,021(0,3 );3,654(4,0);3,640 (11,1);3,626(10,7);3,612(3,6);3,324(21,5);2,676(0,3);2,672(0,5);2,667(0,3);2,525(1,4);2,512(27,9);2,507(56,3);2,503(74,0); 2,498(52,7);2,493(25,1);2,334(0,3);2,329(0,5);1,989(1,4);1,193(0,5);1,176(0,8);1,158(0,4);0,146 (0,3);0,008(3,1);0,000(81,5);-0,009(2,9);-0,150(0,3) |
| Verbindung Nr. 1-122:<br>1H-NMR(400,0 MHz, DMSO): δ= 8,643(2,1);8,629(4,0);8,616(2,2);8,451(7,3);8,438(7,5);7,646(7,7);7,626(7,4);7,438(2,0);7,435(2,3);7,422(2 ,6);7,416(7,4);7,401(6,2);7,398(6,2);7,365(15,8);7,346(12,7);7,330(8,4);7,165 (10,7);5,756(0,5);4,492(7,4);4,478(16,0);4,463(7,9);3,647(4,0); 3,633(11,2);3,618(10,8);3,604(3,7);3,322(31,3);2,675(0,5);2,671(0,7);2,666(0,6);2,541(0,6);2,510(45,2);2,506(89,9);2,502(118,8);2,497(87,2 );2,493(43,7);2,333(0,6);2,328(0,8);2,324(0,6);1,989(0,5);0,146(0,5);0,008(4,4);0,000(105,6);-0,009(4,6);-0,150(0,5) |
| Verbindung Nr. 1-123:<br>1H-NMR(400,0 MHz, DMSO): δ= 8,602(2,2);8,588(4,2);8,575(2,2);8,452(7,4);8,439(7,6);7,876(7,2);7,858(7,5);7,856(7,4);7,446(3,1);7,444(3 ,1);7,428(7,3);7,425(7,2);7,409(4,7);7,407(4,5);7,343(5,8);7,340(6,0);7,329(5,6);7,327(5,7);7,306(6,7);7,302(7,6);7,287(5,5);7,283(5,5);7,180 (13,5);7,177(13,1);7,161(6,2);7,157(5,9);7,142 (3,3);7,138(3,1);5,756(2,2);4,498(7,3);4,483(16,0);4,469(7,8);3,641(3,9);3,626(11,0);3,612(10, 7);3,598(3,6);3,323(26,8);2,676(0,5);2,671(0,6);2,667(0,5);2,542(0,4);2,524(1,8);2,511(38,2);2,507(76,3);2,502(99,9);2,498 (72,4);2,494(35,8 );2,334(0,5);2,329(0,6);2,325(0,5);1,989(1,2);1,193(0,3);1,175(0,7);1,158(0,3);0,146(0,4);0,008(3,4);0,000(88,4);-0,008(3,9);-0,150(0,4) |
| Verbindung Nr. 1-124: |

(fortgesetzt)

| |
|---|
| 1H-NMR(400,0 MHz, DMSO): δ= 8,944(1,2);8,931(2,2);8,917(1,2);8,451(4,6);8,437(4,7);7,542(0,9);7,525(1,9); 7,521(1,7);7,509(1,2);7,504(3 ,7);7,500(1,3);7,487(1,8);7,483(2,2);7,466(1,0);7,351(3,5);7,348(3,6);7,337(3,4); 7,335(3,4);7,184(0,7);7,181(1,0);7,173(6,2);7,154(7,8);7,143 (6,8);7,142(7,0);7,133(5,6);7,125(1,0);5,757(16,0); 4,470(4,8);4,456(10,0);4,442(5,1);3,676(2,5);3,662(7,0);3,648(6,7);3,634(2,3);3,325(19,0) ;2,525(0,7);2,521 (1,1);2,512(16,2);2,508(33,4);2,503(44,5);2,498(31,8);2,494(15,1);0,008(1,6);0,000(47,6);-0,009(1,6) |
| Verbindung Nr. 1-125:<br>1H-NMR(400,0 MHz, DMSO): δ= 8,723(1,7);8,710(3,1);8,696(1,6);8,456(5,9);8,442(6,0);7,778(4,1);7,759(5,4); 7,729(1,7);7,711(4,6);7,692(3 ,4);7,656(3,3);7,637(4,1);7,618(1,5);7,502(4,9);7,483(4,1);7,347(4,7);7,345(4,6); 7,334(4,4);7,332(4,3);7,177(0,5);7,136(8,0);7,135(8,3);5,757 (16,0);4,485(0,8);4,470(6,0);4,456(12,0);4,442 (5,9);4,057(0,7);4,039(2,0);4,021(2,1);4,004(0,7);3,655(3,1);3,641(8,6);3,627(8,3);3,613(3,0); 3,326(13,8);2,512 (19,3);2,508(36,6);2,503(46,5);2,499(32,6);2,494(15,2); 1,990(8,9);1,194(2,4);1,176(4,7);1,158(2,3);0,008(2,2); 0,000(46,1); -0,009(1,5) |
| Verbindung Nr. 1-126:<br>1H-NMR(400,0 MHz, DMSO): δ= 8,836(1,2);8,822(2,3);8,809(1,2);8,472(4,8);8,467(5,2);8,460(5,7);8,455(9,4); 8,442(4,7);7,883(4,9);7,878(5 ,1);7,864(5,7);7,859(5,3);7,500(5,5);7,488(5,3);7,481(5,1);7,469(5,0);7,350(3,4); 7,348(3,5);7,337(3,3);7,334(3,3);7,164(5,9);7,163(6,5);7,161 (5,7);5,758(16,0);4,500(4,7);4,486(9,6);4,472(4,9); 4,040(0,8);4,022(0,8);3,676(2,5);3,662(6,8);3,647(6,6);3,633(2,2);3,327(9,4);2,967(1,1);2, 526(0,6);2,513(10,6); 2,509(21,4);2,504(28,1);2,499(19,9);2,495(9,3);1,990(3,4);1,194(0,9);1,176(1,9);1,159(0,9);0,008(1,3);0,000 (34,2);-0,009(1,1) |
| Verbindung Nr. 1-127:<br>1H-NMR(400,0 MHz, DMSO): δ= 8,897(2,3);8,883(4,3);8,870(2,2);8,796(5,8);8,784(5,8);8,460(7,9);8,446(8,0); 7,999(5,2);7,982(6,2);7,979(6 ,2);7,788(5,5);7,776(5,5);7,769(4,8);7,757(4,5);7,354(6,1);7,352(6,3);7,341(5,9); 7,339(6,0);7,139(11,3);5,758(1,0);4,477(7,7);4,463(16,0);4,4 49(8,1);4,039(0,5);4,021(0,5);3,680(4,1);3,666 (11,4);3,652(11,0);3,638(3,8);3,326(18,5);2,673(0,4);2,525(1,3);2,512(25,8);2,508(51,0);2,503 (66,6);2,499 (47,8);2,495(23,1);2,330(0,4);1,990(2,2);1,194(0,6);1,176(1,2);1,158(0,6);0,008(2,5);0,000(60,7);-0,008(2,2) |
| Verbindung Nr. 1-128:<br>1H-NMR(400,0 MHz, DMSO): δ= 8,792(2,3);8,778(4,4);8,765(2,4);8,448(8,1);8,434(13,1);8,429(7,5);8,421(7,0); 8,416(6,8);8,071(6,7);8,066( 6,8);8,052(7,4);8,047(7,0);7,514(7,1);7,502(7,0);7,495(6,8);7,483(6,5);7,345(6,4); 7,334(6,2);7,332(6,2);7,125(11,5);5,759(2,4);4,491(7,9);4,4 77(16,0);4,463(8,4);4,058(0,5);4,040(1,4);4,022 (1,4);4,005(0,5);3,684(4,3);3,670(11,6);3,656(11,2);3,642(3,9);3,328(15,8);2,674(0,3);2,527( 1,0);2,513(21,5); 2,509(42,8);2,505(56,0);2,500(40,6);2,332(0,3);1,991(5,9);1,194(1,6);1,177(3,1);1,159(1,5);0,008(2,1);0,000 (53,5);-0,008(2,3) |
| Verbindung Nr. 1-129:<br>1H-NMR(400,0 MHz, DMSO): δ= 9,132(2,4);9,118(4,6);9,105(2,5);8,685(6,4);8,681(6,9);8,674(6,9);8,669(6,8); 8,436(8,3);8,423(8,5);8,201(6 ,1);8,196(6,4);8,181(6,7);8,177(6,6);7,514(7,3);7,502(7,3);7,494(7,1);7,482(6,9); 7,341(6,3);7,339(6,6);7,328(6,2);7,326(6,4);7,164(11,8);5,75 9(3,8);4,525(7,9);4,511(16,0);4,497(8,4);4,041 (0,5);4,023(0,6);3,700(4,2);3,686(11,3);3,672(11,1);3,658(4,0);3,331(23,2);2,676(0,4);2,529(1, 1);2,516(21,1); 2,511(42,8);2,507(56,8);2,502(41,6);2,498(20,8);2,333(0,4);1,992(2,3);1,196(0,7);1,178(1,3);1,160(0,6);0,008 (2,2);0,000(57,4 );-0,009(2,4) |
| Verbindung Nr. 1-130:<br>1H-NMR(400,0 MHz, DMSO): δ= 8,801(1,3);8,787(2,5);8,773(1,3);8,446(4,3);8,433(4,4);7,736(1,2);7,730(1,9); 7,724(1,9);7,722(1,9);7,718(1 ,7);7,715(1,9);7,606(1,6);7,593(9,3);7,590(13,6);7,583(16,0);7,339(3,3);7,336 (3,3);7,325(3,2);7,323(3,2);7,117(6,1);5,758(1,4);4,502(4,1);4,4 89(8,2);4,475(4,3);3,682(2,2);3,668(5,9);3,654 (5,7);3,640(2,0);3,327(9,1);2,526(0,5);2,513(10,4);2,509(21,2);2,504(28,1);2,500(20,3);2,495( 9,7);0,008(1,1); 0,000(29,8);-0,009(1,1) |
| Verbindung Nr. 1-131:<br>1H-NMR(400,0 MHz, DMSO): δ= 8,502(2,8);8,050(10,2);8,043(11,5);7,982(3,1);7,976(2,5);7,961(3,4);7,956(4,9); 7,950(2,7);7,936(3,0);7,92 9(2,6);7,616(2,6);7,612(3,0);7,598(4,9);7,593(5,7);7,578(3,0);7,574(2,8);7,551(1,4); 7,546(1,4);7,538(1,7);7,533(3,2);7,530(2,6);7,519(2,7);7, 512(3,3);7,507(1,8);7,498(1,9);7,494(1,6);7,298(4,2); 7,289(4,8);7,287(4,3);7,277(4,3);7,270(11,9);7,250(7,9);4,495(7,6);4,480(16,0);4,466(7, 9);3,677(3,9);3,662 (10,8);3,648(10,4);3,634(3,5);3,326(13,9);2,513(20,4);2,508(38,3);2,504(48,2);2,499(33,7);2,495(15,6);0,008 (2,3);0,000( 42,3);-0,009(1,3) |

(fortgesetzt)

| |
|---|
| Verbindung Nr. 1-132: 1H-NMR(400,0 MHz, DMSO): δ= 8,658(2,0);8,644(3,7);8,631(2,0);8,063(10,6);8,056(12,0);7,984(3,1);7,978(2,6); 7,964(3,3);7,958(4,9);7,95 2(2,9);7,938(3,1);7,931(2,6);7,648(4,0);7,645(6,3);7,632(2,0);7,625(8,3);7,443(2,1); 7,440(2,3);7,426(2,9);7,422(6,8);7,406(6,2);7,403(5,7);7, 365(13,6);7,361(7,4);7,347(10,4);7,343(5,9);7,330 (3,2);7,326(2,4);7,180(0,4);4,488(7,6);4,473(16,0);4,459(7,8);3,646(4,0);3,632(11,1);3,618 (10,7);3,604(3,6); 3,324(21,5);2,676(0,3);2,671(0,5);2,667(0,3);2,541(0,3);2,524(1,6);2,511(28,2);2,507(55,8);2,502(72,8);2,498 (52,3);2,493( 25,2);2,333(0,3);2,329(0,5);2,324(0,3);1,989(0,3);0,008(2,9);0,000(70,2);-0,009(2,5) |
| Verbindung Nr. 1-133: 1H-NMR(400,0 MHz, DMSO): δ= 8,953(2,0);8,940(3,5);8,926(1,9);8,056(11,1);8,050(12,7);7,985(3,4);7,979(2,8); 7,965(3,6);7,959(5,2);7,95 3(3,1);7,939(3,3);7,932(2,8);7,540(1,3);7,523(2,9);7,519(2,7);7,506(1,9);7,502(5,6); 7,498(2,1);7,485(2,8);7,481(3,4);7,464(1,6);7,177(1,7);7, 169(9,4);7,150(12,4);7,129(7,9);7,121(1,4);5,757(1,1); 4,468(7,8);4,454(16,0);4,440(8,2);4,266(0,4);4,039(0,7);4,021(0,7);3,675(4,1);3,661(1 1,3);3,647(10,9);3,633 (3,7);3,327(21,7);2,950(2,7);2,525(0,9);2,512(18,8);2,507(37,8);2,503(49,4);2,498(35,3);2,494(16,8);2,330(0,3); 1,990( 2,8);1,194(0,8);1,176(1,5);1,158(0,7);0,008(1,7);0,000(45,8);-0,008(1,6) |
| Verbindung Nr. 1-134: 1H-NMR(400,0 MHz, DMSO): δ= 8,738(1,0);8,724(1,9);8,711(1,0);8,063(5,9);8,057(6,7);7,983(1,9);7,976(1,6); 7,962(2,0);7,957(2,6);7,951(1 ,7);7,936(1,9);7,930(1,7);7,775(2,5);7,756(3,3);7,732(1,1);7,715(2,8);7,696(2,0); 7,655(2,0);7,636(2,5);7,618(0,9);7,495(3,0);7,476(2,5);5,755 (16,0);4,467(3,7);4,453(7,9);4,439(3,9);4,039(0,7); 4,021(0,7);3,655(2,0);3,641(5,4);3,627(5,2);3,612(1,8);3,333(54,2);2,946(1,7);2,526(0,5); 2,521(0,7);2,512(8,3); 2,508(17,0);2,503(22,4);2,499(16,0);2,494(7,5);1,990(3,3);1,194(0,9);1,176(1,8);1,158(0,9);0,008(1,0);0,000 (27,6);-0,009(0,9) |
| Verbindung Nr. 1-135: 1H-NMR(400,0 MHz, DMSO): δ= 8,850(2,0);8,836(3,7);8,823(2,0);8,470(6,9);8,465(7,5);8,458(7,4);8,453(7,3); 8,064(11,7);8,058(13,4);7,98 9(3,6);7,982(3,0);7,968(3,8);7,962(5,4);7,956(3,3);7,942(3,6);7,936(3,1);7,866 (7,1);7,861(7,3);7,847(8,1);7,842(7,8);7,501(8,1);7,489(7,9);7, 482(7,3);7,470(7,3);5,755(7,4);4,493(7,7);4,479 (16,0);4,465(8,1);3,672(4,1);3,658(11,3);3,644(11,0);3,630(3,7);3,333(284,4);2,676(0,4);2,67 2(0,6);2,667(0,4); 2,525(1,6);2,512(33,4);2,507(68,4);2,503(90,6);2,498(65,1);2,494(31,0);2,334(0,4);2,330(0,6);2,325(0,4);0,146 (0,4);0,008( 3,0);0,000(88,2);-0,009(3,1);-0,150(0,4) |
| Verbindung Nr. 1-136: 1H-NMR(400,0 MHz, DMSO): δ= 5,753(16,0);3,322(1,6);2,945(0,5);2,511(1,2);2,507(2,4);2,503(3,1);2,498(2,2); 2,494(1,1);1,989(0,5);0,000( 3,2) |
| Verbindung Nr. 1-137: 1H-NMR(400,0 MHz, DMSO): δ= 8,806(1,9);8,792(3,6);8,779(1,9);8,433(5,5);8,428(6,0);8,421(5,9);8,416(5,9); 8,059(6,5);8,054(16,0);8,047( 12,3);8,040(6,8);8,035(6,3);7,985(3,3);7,978(2,7);7,964(3,5);7,959(5,0);7,952 (2,9);7,938(3,2);7,932(2,7);7,516(6,2);7,503(6,1);7,497(5,9);7,4 85(5,7);5,757(2,0);4,484(6,7);4,470(13,5);4,456 (7,1);3,683(3,7);3,669(9,8);3,655(9,4);3,641(3,3);3,330(70,2);2,526(1,0);2,513(18,9);2,509(3 7,5);2,504(48,9); 2,500(34,9);2,495(16,6);1,236(0,3);0,008(1,9);0,000(45,9);-0,008(1,5) |
| Verbindung Nr. 1-138: 1H-NMR(400,0 MHz, DMSO): δ= 9,160(1,0);9,146(1,8);9,133(1,0);8,685(2,7);8,680(2,9);8,673(2,9);8,669(2,8); 8,193(2,6);8,189(2,6);8,173(2 ,8);8,169(2,7);8,041(5,2);8,035(6,1);7,981(1,6);7,975(1,3);7,961(1,7);7,955(2,5); 7,949(1,4);7,935(1,6);7,928(1,3);7,516(3,2);7,504(3,1);7,496 (3,1);7,484(3,0);5,757(16,0);4,516(3,3);4,502(6,7); 4,488(3,5);4,040(0,9);4,022(1,0);3,697(1,7);3,683(4,7);3,669(4,6);3,655(1,6);3,361(0,3);3, 338(83,1);3,317(0,4); 2,528(0,5);2,515(11,0);2,510(22,1);2,506(29,0);2,501(20,8);2,497(10,0);1,991(4,1);1,195(1,1);1,177(2,1);1,159 (1,1);0,0 08(1,1);0,000(29,4);-0,008(1,1) |
| Verbindung Nr. 1-139: 1H-NMR(400,0 MHz, DMSO): δ= 8,822(1,4);8,809(2,6);8,796(1,4);8,052(7,0);8,045(8,0);7,977(2,0);7,971(1,7); 7,956(2,1);7,951(3,2);7,945(1 ,9);7,931(2,0);7,924(1,7);7,731(2,0);7,722(2,5);7,714(2,2);7,614(0,4);7,604(3,0); 7,589(11,5);7,581(16,0);7,574(4,1);5,757(1,8);4,498(4,8);4,4 84(9,7);4,470(5,0);3,677(2,6);3,663(6,9);3,649 (6,7);3,635(2,3);3,331(55,9);2,526(0,7);2,512(13,9);2,508(27,6);2,504(36,1);2,499(26,0);2,495 (12,7);0,008(1,5); 0,000(34,7);-0,008(1,3) |
| Verbindung Nr. 1-140: |

(fortgesetzt)

| |
|---|
| 1H-NMR(400,0 MHz, DMSO): δ= 9,000(2,0);8,987(3,7);8,973(1,8);8,417(0,4);8,404(0,4);8,334(2,4);8,316(4,0); 8,109(13,6);8,089(16,0);8,06 4(10,8);8,057(11,9);7,985(3,4);7,979(2,9);7,965(3,7);7,959(4,9);7,953(3,0);7,939 (3,4);7,932(2,8);7,868(3,2);7,848(4,9);7,828(2,3);7,421(0,6) ;7,407(0,5);7,391(0,3);7,383(0,5);4,453(0,9);4,437 (6,8);4,424(13,4);4,410(7,0);3,802(0,5);3,786(0,5);3,664(3,5);3,651(9,4);3,637(9,0);3,623( 3,4);3,598(0,5);3,440 (0,4);3,406(0,5);3,371(0,6);3,321(428,7);3,249(0,3);2,715(0,3);2,703(0,4);2,679(2,4);2,675(4,8);2,671(6,6); 2,666(4,7);2, 662(2,4);2,631(0,5);2,607(0,7);2,602(0,7);2,541(4,6);2,524(20,4);2,510(385,3);2,506(769,5);2,502 (1008,5);2,497(720,5);2,493(346,0);2,337( 2,1);2,333(4,5);2,328(6,3);2,324(4,5);1,808(0,7);1,351(0,9);1,335 (0,8);1,298(2,2);1,259(3,6);1,234(5,8);1,187(0,7);1,158(0,4);1,139(0,4);1,11 7(0,4);1,096(0,5);1,078(0,8);1,060 (0,4);0,884(1,4);0,868(1,8);0,854(1,2);0,836(0,8);0,807(0,4);0,146(3,7);0,008(33,1);0,000(862,3);-0,008(32,6);-0,039(0,6);-0,150(3,8) |
| Verbindung Nr. 1-141:<br>1H-NMR(400,0 MHz, DMSO): δ= 8,623(2,1);8,610(4,0);8,596(2,1);8,062(11,2);8,055(12,7);7,981(3,3);7,975(2,8); 7,961(3,5);7,955(5,2);7,94 9(3,0);7,935(3,3);7,928(2,9);7,875(7,1);7,857(7,2);7,855(7,3);7,451(2,9);7,449(3,1); 7,433(6,9);7,430(7,2);7,414(4,4);7,412(4,4);7,302(6,5);7, 298(7,4);7,283(5,4);7,279(5,4);7,180(3,7);7,176(3,7); 7,161(6,0);7,157(5,8);7,142(3,2);7,138(3,0);5,756(1,9);4,492(7,3);4,477(16,0);4,463(7,7 );3,639(3,8);3,624 (10,9);3,610(10,6);3,596(3,5);3,323(14,9);2,671(0,4);2,524(1,2);2,511(25,4);2,506(51,0);2,502(67,1);2,497 (48,5);2,493(23, 6);2,328(0,4);1,989(1,1);1,175(0,6);1,157(0,3);0,008(2,3);0,000(59,5);-0,008(2,4) |
| Verbindung Nr. 1-142:<br>1H-NMR(400,0 MHz, DMSO): δ= 8,949(2,2);8,936(3,9);8,923(2,1);7,999(3,8);7,979(6,8);7,960(4,4);7,540(1,3); 7,523(3,1);7,519(2,8);7,502(1 4,9);7,484(11,5);7,464(1,6);7,176(1,8);7,169(9,4);7,149(13,6);7,142(10,4);7,129 (8,3);7,120(9,1);4,419(7,9);4,405(16,0);4,391(8,3);3,680(4,2) ;3,666(11,4);3,652(11,0);3,638(3,8);3,327(169,3); 3,299(0,4);2,676(0,6);2,672(0,8);2,667(0,6);2,542(3,8);2,511(57,4);2,507(108,4);2,503(136 ,8);2,498(97,7); 2,334(0,7);2,329(0,9);2,325(0,7);2,075(0,4);1,235(0,4);0,000(9,3);-0,009(0,4) |
| Verbindung Nr. 1-143:<br>1H-NMR(400,0 MHz, DMSO): δ= 8,819(1,3);8,805(2,4);8,792(1,3);7,992(2,2);7,972(3,8);7,952(2,5);7,724(2,0); 7,717(2,0);7,712(1,8);7,710(1 ,9);7,602(1,2);7,586(14,2);7,579(16,0);7,565(0,7);7,490(5,6);7,471(5,2);7,129 (4,7);7,107(4,5);4,453(4,3);4,440(8,8);4,426(4,6);3,680(2,3);3,6 66(6,2);3,652(6,0);3,639(2,1);3,328(154,4); 2,676(0,5);2,672(0,7);2,667(0,5);2,542(2,4);2,525(2,3);2,511(44,4);2,507(87,7);2,502(113,5);2,49 8(81,1);2,494 (39,0);2,334(0,5);2,329(0,7);2,324(0,5);0,008(0,4);0,000(9,0) |
| Verbindung Nr. 1-144:<br>1H-NMR(400,0 MHz, DMSO): δ= 8,840(2,2);8,827(4,2);8,813(2,2);8,430(7,0);8,425(7,6);8,418(7,5);8,413(7,4); 7,998(3,8);7,978(6,8);7,958(4 ,3);7,806(7,0);7,802(7,2);7,788(8,3);7,783(7,9);7,511(8,2);7,500(15,2);7,493(7,8); 7,481(12,5);7,162(8,4);7,141(8,0);4,451(7,9);4,437(16,0);4, 423(8,4);3,671(4,2);3,657(11,4);3,643(11,0);3,630 (3,8);3,326(142,2);2,676(0,7);2,671(0,9);2,667(0,7);2,542(31,9);2,511(58,1);2,507(114,8); 2,502(148,9);2,498 (107,2);2,494(52,0);2,334(0,7);2,329(0,9);2,325(0,7);2,075(0,4);1,235(0,4);0,008(0,6);0,000(15,9);-0,008(0,6) |
| Verbindung Nr. 1-145:<br>1H-NMR(400,0 MHz, DMSO): δ= 8,731(2,2);8,718(4,1);8,704(2,3);8,317(0,6);7,999(3,8);7,979(6,7);7,959(4,4); 7,773(5,4);7,754(7,2);7,723(2 ,2);7,704(6,0);7,686(4,5);7,652(4,4);7,633(5,4);7,614(2,0);7,499(16,0);7,480 (14,4);7,134(8,2);7,113(7,8);4,426(7,6);4,412(15,9);4,398(8,2);3, 658(4,0);3,644(11,1);3,630(10,8);3,616(3,8); 3,327(113,5);2,676(0,6);2,671(0,8);2,667(0,6);2,542(4,4);2,511(53,9);2,507(104,2);2,503(134,5) ;2,498(97,8); 2,334(0,6);2,329(0,8);2,325(0,6);1,236(0,3);0,008(0,5);0,000(12,9);-0,008(0,6) |
| Verbindung Nr. 1-146:<br>1H-NMR(400,0 MHz, DMSO): δ= 9,149(2,5);9,136(4,5);9,123(2,4);8,680(6,6);8,676(6,5);8,668(6,8);8,664(6,2); 8,317(0,6);8,207(6,2);8,203(5 ,8);8,188(6,8);8,184(5,9);7,994(4,1);7,974(7,2);7,954(4,6);7,504(7,2);7,493(14,8); 7,484(7,2);7,475(11,4);7,157(8,6);7,136(8,1);4,475(8,0);4,4 62(16,0);4,448(8,2);3,702(4,2);3,688(11,2);3,674 (10,7);3,661(3,7);3,328(157,2);2,677(0,8);2,673(1,0);2,668(0,7);2,543(8,2);2,508(135,6);2,5 04(167,2);2,499 (116,5);2,335(0,8);2,330(1,0);2,326(0,8);1,235(0,4);0,008(0,7);0,000(14,2);-0,009(0,5) |
| Verbindung Nr. 1-147: |

(fortgesetzt)

| |
|---|
| 1H-NMR(400,0 MHz, DMSO): δ= 8,907(2,3);8,893(4,2);8,879(2,2);8,790(5,8);8,781(5,6);8,778(5,6);8,316(0,6); 8,003(4,3);7,996(5,5);7,994(5 ,7);7,984(7,6);7,977(6,7);7,974(6,5);7,964(4,6);7,780(5,6);7,768(5,5);7,761(4,8); 7,749(4,6);7,504(10,1);7,486(9,4);7,139(8,6);7,117(8,1);4,43 1(8,0);4,418(16,0);4,404(8,5);3,682(4,3);3,668 (11,4);3,655(11,0);3,641(3,8);3,326(229,8);2,676(0,9);2,671(1,2);2,667(0,9);2,542(45,6);2,525 (4,3);2,511(74,3); 2,507(147,4);2,502(192,3);2,498(137,6);2,493(66,2);2,334(0,9);2,329(1,2);2,325(0,9);2,075(0,4);1,235(0,5); 0,008(0,7);0,00 0(19,1);-0,009(0,7) |
| Verbindung Nr. 1-148:<br>1H-NMR(400,0 MHz, DMSO): δ= 15,589(0,8);8,801(2,5);8,790(4,5);8,775(2,2);8,519(7,0);8,514(7,6);8,506(7,1); 8,502(7,3);8,450(7,1);8,446( 6,6);8,431(12,2);8,426(13,0);8,420(7,6);8,415(7,0);8,315(3,1);7,779(6,4);7,774 (6,9);7,760(8,1);7,755(7,2);7,524(7,7);7,512(7,2);7,505(7,2);7, 493(6,6);7,272(7,1);7,260(7,2);7,252(7,0);7,240 (7,2);5,752(0,7);4,628(7,5);4,615(16,0);4,601(8,1);4,337(0,8);3,672(4,1);3,657(11,2);3,643(1 0,9);3,629(4,0); 3,589(0,8);3,487(0,9);3,441(0,9);3,412(1,4);3,400(1,3);3,388(1,9);3,326(2283,0);3,297(4,0);3,286(2,9);3,256 (1,6);3,212(0,8); 2,778(0,9);2,675(8,1);2,671(10,5);2,666(8,4);2,589(1,8);2,541(87,6);2,524(41,6);2,510(692,7); 2,506(1353,2);2,502(1751,1);2,497(1269,6);2, 493(625,3);2,462(4,7);2,432(2,0);2,375(1,1);2,333(7,9);2,328 (10,5);2,324(8,0);2,074(4,1);1,298(1,2);1,289(0,9);1,275(0,9);1,259(1,4);1,235( 3,7);0,854(0,8);0,000(129,6);- 0,008(5,4);-0,017(1,0);-0,148(0,9);-2,639(0,7);-3,149(0,7);-3,644(0,7) |
| Verbindung Nr. 1-149:<br>1H-NMR(400,0 MHz, DMSO): δ= 8,691(2,3);8,678(4,0);8,664(2,2);8,518(6,7);8,514(7,7);8,506(7,2);8,502(7,6); 8,450(7,4);8,445(6,8);8,430(7 ,8);8,425(6,8);8,315(1,0);7,771(5,4);7,752(7,1);7,731(2,3);7,712(6,0);7,694(4,4); 7,653(4,2);7,634(5,3);7,615(2,0);7,493(6,3);7,474(5,3);7,271 (7,6);7,259(7,3);7,251(7,3);7,239(7,1);4,603(7,6); 4,589(16,0);4,575(8,0);3,657(3,9);3,642(11,2);3,628(11,0);3,614(3,7);3,359(0,9);3,324(442, 5);3,280(0,4);2,675 (2,3);2,671(3,1);2,666(2,2);2,662(1,1);2,541(36,7);2,524(10,2);2,510(197,3);2,506(393,3);2,502(514,3);2,497 (372,4);2,49 3(183,9);2,435(0,6);2,426(0,7);2,404(0,4);2,367(0,5);2,333(2,4);2,328(3,3);2,324(2,5);2,289(0,3); 2,074(0,3);1,299(0,3);1,258(0,4);1,236(1,2) ;0,008(1,9);0,000(57,7);-0,009(2,5) |
| Verbindung Nr. 1-150:<br>1H-NMR(400,0 MHz, DMSO): δ= 8,903(2,4);8,891(4,0);8,878(2,2);8,508(6,2);8,504(6,6);8,495(6,4);8,492(6,4); 8,446(6,6);8,442(5,6);8,427(6 ,8);8,422(5,7);8,315(0,6);7,533(1,2);7,517(2,9);7,513(2,7);7,496(5,3);7,479(2,9); 7,475(3,1);7,458(1,5);7,269(6,3);7,257(6,3);7,249(6,0);7,237 (5,8);7,163(9,1);7,143(13,3);7,123(7,5);7,114(1,5); 4,602(8,0);4,588(16,0);4,574(8,1);3,677(4,3);3,663(11,6);3,649(11,2);3,635(3,9);3,415(0,4 );3,327(605,6);3,265 (0,5);2,670(2,5);2,591(0,4);2,583(0,5);2,541(44,0);2,506(318,2);2,502(391,3);2,498(280,0);2,329(2,3);2,074 (1,1);1,258( 0,4);1,236(0,9);0,000(14,9) |
| Verbindung Nr. 1-151:<br>1H-NMR(400,0 MHz, DMSO): δ= 8,787(1,5);8,773(2,9);8,759(1,5);8,507(4,5);8,502(5,4);8,495(4,9);8,490(5,2); 8,447(5,1);8,443(4,6);8,427(5 ,4);8,423(4,6);8,315(0,4);7,729(2,0);7,717(2,8);7,708(2,6);7,599(4,7);7,589(6,6); 7,585(8,6);7,576(16,0);7,568(3,9);7,550(0,6);7,264(5,1);7,25 2(5,1);7,244(5,0);7,232(4,9);4,631(5,1);4,618 (10,1);4,604(5,2);3,678(2,7);3,664(7,2);3,650(7,0);3,636(2,5);3,406(0,3);3,383(0,7);3,330(412,2 );3,294(0,5); 2,676(1,0);2,671(1,3);2,667(1,0);2,541(20,0);2,524(6,4);2,511(89,8);2,507(173,2);2,502(222,8);2,498(158,9); 2,493(76,6);2,334( 1,1);2,329(1,4);2,325(1,0);2,074(0,6);1,236(0,5);0,008(0,3);0,000(7,9);-0,008(0,3) |
| Verbindung Nr. 1-152:<br>1H-NMR(400,0 MHz, DMSO): δ= 9,110(2,4);9,097(4,6);9,084(2,4);8,680(6,3);8,676(6,7);8,668(6,7);8,664(6,4); 8,493(6,8);8,489(8,2);8,481(7 ,1);8,476(7,9);8,451(7,8);8,447(6,7);8,431(8,2);8,427(6,8);8,315(0,9);8,162(6,1); 8,158(6,2);8,143(6,5);8,138(6,3);7,515(7,0);7,503(7,1);7,495 (6,8);7,483(6,6);7,266(7,7);7,254(7,5);7,246(7,3); 7,234(7,1);4,648(7,9);4,634(16,0);4,620(8,2);3,697(4,1);3,683(11,3);3,670(11,0);3,655(3,9) ;3,593(0,3);3,533 (0,4);3,495(0,4);3,457(0,5);3,439(0,6);3,424(0,7);3,383(1,3);3,329(739,4);3,280(0,9);3,260(0,3);3,247(0,4); 2,995(0,9);2,711 (0,4);2,676(2,6);2,671(3,5);2,667(2,6);2,630(0,5);2,610(0,5);2,598(0,6);2,542(40,2);2,511 (208,0);2,507(408,4);2,502(532,2);2,498(387,2);2,4 57(0,7);2,421(0,3);2,333(2,2);2,329(3,1);2,074(1,8);1,259 (0,4);1,235(1,3);1,117(0,4);1,100(0,7);0,008(1,3);0,000(36,6);-0,008(1,6) |
| Verbindung Nr. 1-153:<br>1H-NMR(400,0 MHz, DMSO): δ= 8,582(2,8);8,580(2,8);8,579(2,8);8,576(2,8);8,551(0,9);8,538(1,6);8,524(0,9); 8,086(2,0);8,080(1,9);8,064(2 ,1);8,057(2,0);7,309(16,0);7,027(3,5);7,006(3,3);4,484(3,4);4,470(7,3);4,456(3,6); 3,627(1,8);3,613(5,1);3,599(4,9);3,584(1,7);3,324(24,1);2,5 26(0,7);2,521(1,0);2,512(14,6);2,508(30,1);2,503 (39,8);2,499(28,0);2,494(12,9);1,990(0,5);0,008(2,0);0,000(60,1);-0,009(1,8) |

(fortgesetzt)

| |
|---|
| Verbindung Nr. 1-154: 1H-NMR(400,0 MHz, DMSO): δ= 8,559(3,2);8,556(3,9);8,553(3,9);8,551(3,1);8,532(1,0);8,518(1,9);8,505(1,0); 8,397(4,3);8,392(4,0);8,315(0 ,6);7,284(16,0);6,573(0,8);4,581(3,3);4,567(7,0);4,553(3,5);3,653(1,8);3,639(4,9); 3,625(4,8);3,611(1,6);3,321(97,7);2,946(0,4);2,676(0,7);2,6 71(1,0);2,666(0,7);2,662(0,4);2,541(0,5);2,524(2,6); 2,520(4,1);2,511(60,2);2,507(122,2);2,502(160,0);2,497(112,7);2,493(52,1);2,333(0,8);2, 329(1,0);2,324(0,7); 2,320(0,3);0,146(0,8);0,020(0,4);0,008(6,4);0,000(192,0);-0,009(6,4);-0,014(0,5);-0,150(0,8) |
| Verbindung Nr. 1-155: 1H-NMR(400,0 MHz, DMSO): δ= 8,554(2,1);8,552(2,1);8,468(0,5);8,454(1,0);8,441(0,5);8,403(2,4);8,398(2,1); 8,312(1,5);8,307(1,6);8,300(1 ,6);8,295(1,6);8,163(1,6);8,158(1,6);8,144(1,7);8,139(1,5);7,142(1,6);7,130(1,6); 7,124(1,6);7,112(1,5);4,613(1,7);4,599(3,6);4,585(1,8);3,951 (16,0);3,761(1,0);3,747(2,6);3,733(2,5);3,718(0,9); 3,325(2,3);2,509(12,7);2,505(16,2);2,501(11,7);0,000(3,3) |
| Verbindung Nr. 1-156: 1H-NMR(601,6 MHz, DMSO): δ= 8,640(1,4);8,630(2,7);8,621(1,4);8,555(4,4);8,553(4,5);8,365(5,0);8,361(5,0); 7,465(2,9);7,452(5,3);7,431(2 ,1);7,428(2,3);7,419(3,0);7,416(3,4);7,406(1,7);7,403(1,9);7,370(1,6);7,368(1,6); 7,358(3,8);7,356(4,1);7,346(2,7);7,344(3,0);7,338(5,0);7,334 (5,0);7,325(2,2);7,322(1,7);5,756(2,1);5,526(1,3); 5,515(2,1);5,508(2,0);5,505(1,7);5,497(1,3);4,036(0,5);4,024(0,5);3,638(1,0);3,629(1,4);3,6 21(1,2);3,615(1,7); 3,606(2,1);3,598(1,5);3,528(1,5);3,517(2,4);3,507(1,9);3,494(1,7);3,484(1,1);3,320(31,5);2,523(0,5);2,520(0,6); 2,517(0,7) ;2,508(16,7);2,505(36,1);2,502(50,9);2,499(37,9);2,496(18,7);2,386(0,3);1,989(1,9);1,507(0,4);1,496 (0,4);1,388(15,9);1,378(16,0);1,256(0,4) ;1,245(0,4);1,187(0,5);1,176(1,0);1,164(0,5);0,005(0,3);0,000(10,2);- 0,006(0,4) |
| Verbindung Nr. 1-157: 1H-NMR(601,6 MHz, DMSO): δ= 8,666(2,7);8,653(2,7);8,571(3,6);8,569(4,5);8,567(4,5);8,566(3,7);8,405(5,2); 8,401(5,1);7,707(2,0);7,704(2 ,5);7,693(3,2);7,639(1,0);7,636(1,2);7,627(3,1);7,624(3,3);7,615(4,6);7,612(3,0); 7,604(2,8);7,594(1,0);7,545(2,9);7,533(2,2);7,332(2,0);7,240 (4,4);7,148(2,3);6,574(0,3);5,756(1,4);4,584(2,4); 4,575(3,0);4,566(3,1);4,558(3,2);4,471(0,6);4,458(1,3);4,449(1,3);4,447(1,5);4,438(1,2);4,4 34(0,9);4,425(0,7); 4,383(3,6);4,370(2,6);4,365(3,2);4,353(2,4);3,323(20,3);2,524(0,4);2,521(0,4);2,518(0,4);2,509(11,9);2,506 (26,4);2,503(3 7,5);2,500(27,2);2,497(12,9);1,990(0,5);1,268(16,0);1,256(16,0);1,176(0,3);0,000(10,1);-0,006 (0,4) |
| Verbindung Nr. 1-158: 1H-NMR(601,6 MHz, DMSO): δ= 8,557(1,2);8,556(1,2);8,405(1,4);8,401(1,4);8,384(1,6);7,658(0,5);7,654(0,6); 7,643(0,9);7,590(0,9);7,587(1 ,4);7,580(1,5);7,575(1,4);7,572(0,9);7,499(0,7);7,489(0,6);7,484(0,5);7,252(0,6); 7,159(1,3);7,067(0,6);4,664(5,0);3,320(14,3);2,508(6,3);2,50 5(14,4);2,502(20,8);2,499(16,1);2,496(8,3);1,467 (16,0);0,000(3,8) |
| Verbindung Nr. 1-159: 1H-NMR(601,6 MHz, DMSO): δ= 8,809(1,3);8,799(2,6);8,790(1,4);8,533(4,2);8,531(4,2);8,372(5,1);8,368(5,0); 7,693(2,7);7,681(3,6);7,624(1 ,6);7,613(3,3);7,611(3,4);7,601(2,2);7,598(2,1);7,589(2,0);7,577(3,0);7,564(1,3); 7,481(3,2);7,468(2,6);7,317(2,0);7,224(4,5);7,132(2,2);5,755 (1,8);5,555(1,2);5,548(1,4);5,544(2,0);5,537(1,9); 5,533(1,5);5,526(1,2);4,035(0,7);4,024(0,6);3,676(1,0);3,667(1,3);3,659(1,2);3,653(1,6);3,6 44(1,8);3,636(1,4); 3,534(1,4);3,523(2,1);3,512(1,9);3,500(1,7);3,489(1,2);3,323(137,5);2,614(0,5);2,611(0,4);2,523(0,7);2,520 (0,8);2,517(0,8 );2,508(24,2);2,505(53,8);2,502(76,3);2,499(55,6);2,496(26,5);2,386(0,5);1,989(2,8);1,496(0,5); 1,485(0,6);1,381(15,9);1,371(16,0);1,307(0,4 );1,266(0,4);1,187(0,8);1,175(1,5);1,164(0,8);0,005(0,4);0,000 (15,6);-0,006(0,6) |
| Verbindung Nr. 1-160: |

(fortgesetzt)

| |
|---|
| 1H-NMR(601,6 MHz, DMSO): δ= 8,814(2,0);8,801(2,0);8,765(1,4);8,752(1,4);8,639(2,8);8,635(4,2);8,632(2,9); 8,555(1,9);8,553(2,2);8,551(2 ,3);8,545(2,7);8,544(3,2);8,542(3,2);8,393(1,8);8,391(2,0);8,389(1,8);8,387(1,9); 8,379(7,5);8,375(7,6);8,327(0,7);8,323(5,6);8,319(1,8);8,315 (0,6);8,311(1,7);8,308(6,2);8,304(0,8);8,265(1,8); 8,263(2,4);8,261(1,8);8,252(2,0);8,250(2,5);8,248(2,0);8,051(0,8);8,047(6,1);8,044(1,8);8,0 36(1,7);8,033(5,7); 8,029(0,7);7,790(2,9);7,776(4,9);7,763(2,7);5,755(10,7);4,613(1,8);4,604(2,2);4,595(2,3);4,592(1,5);4,587(2,6); 4,584(1,8) ;4,575(1,5);4,566(1,7);4,523(0,6);4,511(1,3);4,499(1,8);4,489(1,5);4,478(1,2);4,467(0,5);4,445(2,8); 4,433(1,9);4,428(2,5);4,423(2,1);4,415(1, 9);4,410(1,4);4,406(1,7);4,393(1,2);4,047(0,4);4,036(1,5);4,024(1,5); 4,012(0,5);3,324(302,6);2,618(0,6);2,615(0,9);2,612(0,6);2,542(0,4);2,5 24(1,4);2,521(1,7);2,518(1,5);2,509 (44,1);2,506(99,7);2,503(143,2);2,500(102,5);2,497(47,9);2,390(0,7);2,387(1,0);2,384(0,7);1,989(6,7);1,3 82 (0,5);1,371(0,5);1,303(11,8);1,292(16,0);1,281(8,4);1,270(0,7);1,259(0,6);1,235(0,4);1,188(2,0);1,176(3,8); 1,171(0,8);1,164(1,9);1,160(0,8 );1,069(0,4);1,059(0,4);0,005(1,0);0,000(37,9);-0,006(1,3) |
| Verbindung Nr. 1-161:<br>1H-NMR(601,6 MHz, DMSO): δ= 8,572(1,0);8,571(1,3);8,569(1,3);8,497(1,6);8,401(1,4);8,397(1,4);8,046(1,1); 8,045(1,1);8,033(1,2);8,031(1 ,1);7,782(0,6);7,780(0,5);7,770(1,3);7,768(1,2);7,757(0,8);7,755(0,7);7,673(0,7); 7,671(0,7);7,660(0,9);7,659(0,9);7,647(0,6);7,645(0,6);7,506 (1,1);7,504(1,1);7,493(1,1);7,491(1,0);4,626(5,0); 3,324(19,6);3,323(25,1);2,508(6,9);2,505(15,3);2,502(21,4);2,499(15,7);2,496(7,6);1,989(0, 6);1,461(16,0); 1,176(0,3);0,000(3,2) |
| Verbindung Nr. 1-162:<br>1H-NMR(601,6 MHz, DMSO): δ= 8,887(1,3);8,877(2,7);8,868(1,4);8,565(3,7);8,563(4,5);8,561(4,4);8,367(5,0); 8,363(5,0);8,322(0,9);8,024(3 ,8);8,022(3,9);8,010(4,2);8,009(4,1);7,968(1,1);7,965(1,1);7,774(1,9);7,772(1,9); 7,762(4,5);7,760(4,3);7,749(2,8);7,747(2,7);7,690(2,6);7,687 (2,8);7,676(3,4);7,675(3,5);7,664(1,9);7,661(1,9); 7,513(3,9);7,511(4,0);7,500(3,7);7,498(3,5);7,008(0,5);5,755(3,4);5,504(1,3);5,494(2,2);5,4 86(2,1);5,475(1,3); 4,809(1,2);4,801(1,3);3,862(0,4);3,636(1,0);3,627(1,4);3,618(1,2);3,613(1,6);3,604(2,0);3,595(1,5);3,530(1,4); 3,519(2,4); 3,509(1,8);3,496(1,6);3,486(1,0);3,424(0,3);3,411(0,4);3,402(0,6);3,393(0,4);3,340(0,5);3,325(62,3); 3,324(62,7);3,297(0,3);2,615(0,4);2,524( 0,6);2,521(0,7);2,518(0,7);2,509(19,3);2,506(42,9);2,503(60,5);2,500 (44,5);2,497(21,3);2,387(0,4);1,989(0,5);1,391(15,9);1,380(16,0);1,134( 0,8);1,090(0,6);1,079(0,6);1,070(4,1); 1,059(4,0);0,000(10,8);-0,006(0,4) |
| Verbindung Nr. 1-163:<br>1H-NMR(601,6 MHz, DMSO): δ= 8,564(4,0);8,562(4,9);8,560(4,6);8,390(5,2);8,387(4,7);8,358(2,1);8,345(2,2); 7,558(1,5);7,555(1,7);7,546(2 ,8);7,543(3,2);7,533(1,8);7,530(1,9);7,525(1,0);7,520(1,1);7,516(2,0);7,507(1,6); 7,503(1,9);7,499(1,0);7,494(1,1);7,491(0,9);7,278(2,3);7,272 (3,1);7,271(2,7);7,264(2,8);7,260(7,6);7,247(5,1); 5,754(0,5);4,545(2,3);4,537(2,9);4,528(3,0);4,519(3,4);4,470(0,7);4,459(1,4);4,447(1,6);4,4 37(1,3);4,426(0,8); 4,401(3,9);4,389(2,5);4,384(3,2);4,372(2,2);3,336(138,9);3,335(159,7);2,615(0,3);2,525(0,5);2,522(0,6);2,518 (0,6);2,507( 37,8);2,504(51,7);2,501(37,0);2,498(17,3);2,388(0,3);1,261(16,0);1,250(15,9);0,000(9,5);-0,006 (0,4) |
| Verbindung Nr. 1-164:<br>1H-NMR(400,0 MHz, DMSO): δ= 8,738(2,5);8,725(4,8);8,711(2,5);8,541(10,8);8,534(12,7);8,454(6,5);8,446(5,5); 8,434(6,6);8,427(5,4);7,78 2(5,9);7,763(7,9);7,735(2,5);7,717(6,7);7,698(5,0);7,661(4,8);7,642(6,0);7,623(2,2); 7,527(7,1);7,508(5,9);6,574(0,3);5,756(3,2);4,533(7,6);4, 519(16,0);4,505(8,0);3,657(4,0);3,643(11,3);3,629 (10,9);3,615(3,8);3,324(52,7);2,676(0,5);2,671(0,6);2,667(0,5);2,541(0,4);2,524(1,6);2,507 (74,8);2,502(93,3); 2,498(67,0);2,333(0,5);2,329(0,6);2,325(0,5);1,185(0,6);1,167(0,9);1,150(0,5);0,008(2,1);0,000(52,2);-0,008 (2,4) |
| Verbindung Nr. 1-165:<br>1H-NMR(400,0 MHz, DMSO): δ= 8,812(5,7);8,558(4,4);8,556(4,4);8,392(5,2);8,386(4,8);7,610(3,5);7,608(3,5); 7,591(4,2);7,588(4,0);7,407(1 ,2);7,404(1,3);7,389(3,6);7,386(3,7);7,370(3,2);7,367(3,0);7,344(2,3);7,339(3,0); 7,325(2,9);7,320(3,7);7,306(1,5);7,301(1,6);7,294(4,5);7,290 (3,5);7,276(3,0);7,271(2,5);4,673(16,0);3,388(0,4); 3,369(0,4);3,356(1,0);3,354(1,1);3,326(471,3);3,310(1,4);3,306(1,4);2,995(0,3);2,675(1,3) ;2,671(1,8);2,666 (1,3);2,558(0,4);2,541(4,2);2,524(5,1);2,510(110,1);2,506(215,5);2,502(278,7);2,497(198,1);2,493(93,6);2,332 (1,2);2,328(1 ,6);2,324(1,2);2,075(0,4);1,258(0,4);1,235(1,1);1,013(2,0);0,999(5,3);0,994(6,3);0,983(3,1);0,945 (1,0);0,907(3,1);0,896(6,0);0,891(5,4);0,877 (1,8);0,008(2,4);0,000(62,1);-0,009(2,2) |
| Verbindung Nr. 1-166: |

(fortgesetzt)

| |
|---|
| 1H-NMR(400,0 MHz, DMSO): δ= 8,972(5,8);8,528(3,8);8,526(4,7);8,523(4,6);8,520(3,7);8,405(3,8);8,401(4,1);8,393(5,1);8,389(8,9);8,384(5 ,0);7,986(4,1);7,981(4,1);7,967(4,6);7,962(4,2);7,483(4,3);7,470(4,2);7,464(4,0);7,452(3,9);4,651(16,0);4,446(0,4);3,421(0,4);3,412(0,5);3,40 6(0,5);3,384(1,3);3,369(1,3);3,336(1222,1);3,311(3,4);3,300(1,6);3,294(1,2);3,270(0,4);3,263(0,3);2,995(0,4);2,865(0,3);2,681(0,6);2,676(1,4 );2,672(1,9);2,667(1,3);2,542(8,1);2,525(4,2);2,520(7,5);2,512(111,5);2,507(226,5);2,503(296,2);2,498(208,2);2,493(96,0);2,468(1,2);2,338( 0,7);2,334(1,3);2,329(1,9);2,325(1,3);2,320(0,6);2,290(0,4);1,461(0,3);1,258(0,4);1,235(1,0);1,039(2,2);1,026(5,9);1,020(6,4);1,009(3,0);0,97 0(0,4);0,936(0,4);0,897(2,9);0,885(6,0);0,880(5,9);0,867(2,1);0,853(0,4);0,000(7,1) |
| Verbindung Nr. 1-167:<br>1H-NMR(400,0 MHz, DMSO): δ= 9,107(5,3);8,532(4,5);8,530(4,5);8,393(5,0);8,388(4,7);7,512(0,7);7,495(1,5);7,491(1,4);7,474(2,9);7,457(1 ,5);7,453(1,7);7,436(0,8);7,139(0,8);7,132(4,8);7,112(6,6);7,092(4,1);7,084(0,7);4,657(16,0);3,394(0,5);3,371(0,7);3,328(506,9);2,675(1,2);2, 671(1,6);2,666(1,2);2,567(0,4);2,564(0,4);2,541(2,8);2,524(3,8);2,510(97,9);2,506(195,1);2,502(254,0);2,497(182,4);2,493(87,6);2,333(1,2); 2,329(1,6);2,324(1,1);1,472(0,4);1,235(0,7);1,038(2,2);1,024(6,3);1,020(6,7);1,008(2,8);0,863(2,7);0,850(6,3);0,846(6,5);0,832(2,0);0,008(0, 9);0,000(28,2);-0,008(1,0) |
| Verbindung Nr. 1-168:<br>1H-NMR(400,0 MHz, DMSO): δ= 9,105(5,0);8,548(3,6);8,545(3,5);8,302(2,1);8,065(2,4);8,059(2,4);8,043(2,5);8,037(2,5);7,507(0,7);7,490(1 ,5);7,486(1,4);7,474(1,1);7,469(2,9);7,465(1,1);7,452(1,5);7,448(1,7);7,431(0,8);7,125(0,8);7,117(4,8);7,098(6,2);7,077(4,1);7,069(0,7);6,998 (4,1);6,976(4,0);4,532(16,0);3,446(0,5);3,414(0,7);3,399(1,0);3,348(594,6);3,304(0,9);2,680(0,4);2,675(0,6);2,671(0,4);2,545(4,3);2,528(1,6) ;2,515(36,3);2,511(73,3);2,506(96,2);2,502(68,7);2,497(32,7);2,337(0,4);2,333(0,6);2,328(0,4);1,498(0,9);1,236(0,9);1,184(1,3);1,001(2,1);0, 987(5,9);0,983(6,4);0,971(2,9);0,932(0,3);0,896(0,3);0,856(3,0);0,844(6,0);0,840(6,0);0,826(1,9);0,791(0,5);0,000(8,2) |
| Verbindung Nr. 1-169:<br>1H-NMR(400,0 MHz, DMSO): δ= 8,907(5,8);8,566(3,7);8,563(4,6);8,561(4,5);8,558(3,7);8,401(5,4);8,396(4,9);7,746(2,8);7,727(3,8);7,700(1 ,2);7,682(3,2);7,663(2,4);7,633(2,4);7,615(2,8);7,596(1,0);7,413(3,4);7,394(3,0);4,648(16,0);3,412(0,3);3,400(0,4);3,391(0,6);3,380(0,7);3,37 2(1,1);3,353(4,5);3,334(727,4);3,312(2,0);3,292(0,6);2,676(0,9);2,671(1,2);2,667(0,8);2,542(2,4);2,511(75,0);2,507(146,0);2,502(188,2);2,49 8(133,0);2,493(62,3);2,471(0,6);2,338(0,5);2,333(0,9);2,329(1,2);2,325(0,9);1,234(0,5);1,010(2,2);0,997(5,9);0,992(6,5);0,980(3,0);0,942(0,4 );0,874(3,0);0,863(6,2);0,858(6,0);0,844(2,0);0,008(0,7);0,000(16,6);-0,008(0,5) |
| Verbindung Nr. 1-170:<br>1H-NMR(400,0 MHz, DMSO): δ= 8,778(6,4);8,557(4,5);8,554(4,7);8,552(4,2);8,383(4,8);8,378(4,6);7,836(4,3);7,818(4,4);7,817(4,4);7,417(1 ,9);7,415(2,0);7,398(4,4);7,396(4,4);7,380(2,7);7,377(2,7);7,224(3,4);7,220(4,0);7,205(3,0);7,202(3,1);7,158(2,5);7,154(2,3);7,139(3,8);7,135 (3,4);7,120(2,1);7,116(1,9);4,668(16,0);3,383(158,4);3,377(132,9);3,372(120,8);3,364(158,6);2,678(0,3);2,674(0,5);2,669(0,3);2,544(0,9);2,5 27(1,0);2,514(27,3);2,509(55,4);2,505(72,5);2,500(51,2);2,496(23,7);2,336(0,4);2,331(0,5);2,327(0,3);1,234(0,5);1,018(1,9);0,998(6,6);0,988 (3,4);0,971(0,9);0,952(1,0);0,934(3,4);0,924(6,2);0,904(1,8);0,000(3,8) |
| Verbindung Nr. 1-171:<br>1H-NMR(400,0 MHz, DMSO): δ= 8,993(6,1);8,566(4,2);8,564(5,0);8,561(4,9);8,558(3,8);8,412(3,8);8,407(4,0);8,400(4,7);8,395(9,0);8,389(5 ,3);7,731(3,7);7,726(3,8);7,712(4,5);7,708(4,2);7,492(4,1);7,480(4,0);7,474(3,6);7,462(3,5);4,667(16,0);3,550(0,4);3,469(0,4);3,448(0,4);3,43 4(0,4);3,424(0,7);3,413(0,7);3,407(0,6);3,393(1,5);3,388(1,8);3,382(2,3);3,366(5,2);3,341(1438,6);3,302(1,4);3,296(1,2);3,290(0,9);3,277(0,9 );3,262(0,6);3,251(0,4);3,241(0,5);3,237(0,4);3,226(0,4);2,677(1,3);2,672(1,8);2,667(1,3);2,542(9,2);2,525(4,5);2,511(119,1);2,507(227,9);2, 503(287,3);2,498(204,3);2,334(1,3);2,330(1,8);2,325(1,3);1,489(0,7);1,236(0,7);1,036(2,0);1,017(6,7);1,006(3,1);0,967(0,6);0,923(3,0);0,911 (6,4);0,907(6,1);0,893(1,9);0,000(2,2) |
| Verbindung Nr. 1-172: |

(fortgesetzt)

| |
|---|
| 1H-NMR(400,0 MHz, DMSO): δ= 8,995(5,5);8,557(4,4);8,554(4,4);8,446(3,8);8,442(4,1);8,434(4,1);8,430(4,0); 8,397(5,0);8,392(4,7);7,802(3 ,9);7,798(4,0);7,784(4,5);7,779(4,2);7,471(4,4);7,459(4,2);7,452(4,0);7,440(3,9); 4,670(16,0);3,652(0,4);3,634(0,4);3,616(0,5);3,583(0,5);3,57 2(0,4);3,558(0,5);3,549(0,5);3,533(0,5);3,523(0,6); 3,515(0,6);3,497(0,7);3,483(0,5);3,472(1,1);3,439(1,0);3,424(1,9);3,407(2,4);3,375(8,2);3, 342(2847,4);3,299 (2,3);3,288(1,9);3,273(1,3);3,252(1,0);3,236(0,8);3,198(0,4);2,676(2,8);2,672(3,7);2,667(2,7);2,663(1,4);2,586 (0,4);2,581( 0,4);2,542(8,8);2,525(10,4);2,511(222,0);2,507(449,0);2,503(589,2);2,498(417,5);2,494(194,9); 2,454(0,5);2,395(0,4);2,334(2,7);2,329(3,6);2 ,325(2,6);2,291(0,7);2,074(1,4);1,474(0,5);1,298(0,5);1,258(0,6); 1,235(1,4);1,035(2,2);1,021(5,5);1,016(6,4);1,004(3,0);0,965(0,5);0,947(0,4 );0,910(2,9);0,898(6,0);0,894(5,7); 0,880(1,9);0,000(1,5) |
| Verbindung Nr. 1-173:<br>1H-NMR(400,0 MHz, DMSO): δ= 8,898(5,6);8,581(3,9);8,580(4,0);8,578(3,9);8,087(2,5);8,081(2,5);8,065(2,6); 8,059(2,6);7,742(2,8);7,723(3 ,7);7,683(1,1);7,665(3,1);7,647(2,5);7,627(2,5);7,609(2,8);7,590(1,0);7,380(3,4); 7,362(3,0);7,009(4,3);6,987(4,2);4,528(16,0);3,330(82,6);2,5 42(7,0);2,525(0,7);2,512(18,6);2,507(38,0);2,503 (50,2);2,498(37,0);0,976(2,0);0,961(5,7);0,957(6,7);0,946(3,1);0,908(0,5);0,900(0,5);0,862( 3,0);0,850(6,2); 0,846(6,0);0,832(1,8);0,000(7,2) |
| Verbindung Nr. 1-174:<br>1H-NMR(400,0 MHz, DMSO): δ= 8,766(6,4);8,573(4,0);8,073(2,7);8,066(2,6);8,051(2,8);8,044(2,8);7,835(4,3); 7,815(4,5);7,403(1,9);7,401(1 ,9);7,384(4,3);7,382(4,2);7,365(2,7);7,363(2,5);7,194(3,3);7,191(4,0);7,175(3,0); 7,172(3,2);7,154(2,5);7,150(2,1);7,135(3,8);7,131(3,3);7,116 (2,0);7,112(1,7);7,028(4,6);7,006(4,4);4,554(16,0); 3,385(73,7);3,379(75,1);3,368(67,2);3,363(84,2);2,545(1,3);2,528(0,5);2,514(13,2);2,510( 26,7);2,506(34,9); 2,501(24,8);2,497(11,8);0,984(1,9);0,963(6,6);0,954(4,5);0,921(4,5);0,911(6,2);0,891(1,8);0,000(5,0) |
| Verbindung Nr. 1-175:<br>1H-NMR(400,0 MHz, DMSO): δ= 8,983(5,4);8,580(3,5);8,578(3,5);8,576(3,5);8,407(3,7);8,402(4,0);8,395(4,0); 8,390(3,9);8,079(2,5);8,072(2 ,4);8,057(2,6);8,050(2,5);7,721(3,6);7,716(3,8);7,702(4,4);7,697(4,2);7,477(4,3); 7,465(4,1);7,458(3,8);7,446(3,7);7,020(4,1);6,998(4,0);4,548 (16,0);3,384(0,8);3,376(0,8);3,370(0,8);3,337 (566,0);3,308(1,4);2,676(0,6);2,672(0,9);2,667(0,6);2,542(6,4);2,525(2,0);2,511(50,6);2,507(10 2,5);2,502 (134,5);2,498(95,2);2,493(44,5);2,334(0,6);2,329(0,8);2,325(0,6);1,235(0,4);1,000(1,9);0,981(6,0);0,970(3,2); 0,944(0,6);0,933(0,7) ;0,907(3,1);0,896(5,7);0,877(1,7);0,000(2,9) |
| Verbindung Nr. 1-176:<br>1H-NMR(400,0 MHz, DMSO): δ= 8,805(5,4);8,577(3,5);8,576(3,5);8,574(3,5);8,572(3,5);8,077(2,5);8,070(2,4); 8,055(2,6);8,048(2,5);7,606(3 ,4);7,603(3,3);7,587(4,2);7,584(3,9);7,394(1,2);7,391(1,3);7,376(3,6);7,373(3,7); 7,357(3,4);7,354(3,0);7,339(2,5);7,334(3,2);7,320(3,0);7,315 (3,6);7,301(1,4);7,296(1,3);7,267(4,2);7,262(3,8); 7,249(3,0);7,244(2,7);7,019(4,2);6,997(4,0);4,557(16,0);3,332(74,8);2,542(1,2);2,525(0,5); 2,512(13,4);2,508 (27,2);2,503(35,5);2,498(25,2);2,494(11,8);1,235(0,4);0,980(1,8);0,960(6,0);0,950(3,3);0,931(0,8);0,914(0,8); 0,894(3,2);0, 884(5,7);0,864(1,7);0,000(5,9) |
| Verbindung Nr. 1-177:<br>1H-NMR(400,0 MHz, DMSO): δ= 8,991(5,5);8,577(3,6);8,573(3,5);8,442(3,7);8,437(3,9);8,430(3,9);8,425(3,8); 8,082(2,6);8,076(2,5);8,060(2 ,6);8,054(2,5);7,791(3,8);7,787(3,9);7,773(4,5);7,768(4,2);7,458(4,2);7,445(4,0); 7,439(3,8);7,427(3,7);7,016(4,2);6,994(4,0);4,553(16,0);3,38 4(0,4);3,374(0,6);3,339(372,1);3,307(0,6);3,299 (0,4);2,677(0,4);2,672(0,5);2,668(0,4);2,542(6,4);2,525(1,3);2,512(29,7);2,508(59,0);2,503(7 6,5);2,499(54,1); 2,494(25,3);2,334(0,4);2,330(0,5);2,325(0,4);1,001(2,0);0,986(5,2);0,982(6,2);0,971(3,1);0,933(0,9);0,895(3,0); 0,884(5,9);0, 879(5,2);0,865(1,7);0,000(6,4) |
| Verbindung Nr. 1-178:<br>1H-NMR(400,0 MHz, DMSO): δ= 9,074(5,5);8,770(3,0);8,761(2,9);8,759(2,9);8,588(3,7);8,586(3,7);8,584(3,6); 8,092(2,6);8,086(2,5);8,070(2 ,6);8,064(2,5);7,898(2,5);7,895(2,6);7,878(3,3);7,876(3,2);7,747(3,0);7,735(2,9); 7,728(2,4);7,716(2,3);7,009(4,2);6,987(4,1);4,522(16,0);3,45 6(0,4);3,440(0,5);3,425(0,5);3,412(0,9);3,394(1,4); 3,347(833,5);3,309(1,5);3,292(0,5);3,287(0,5);3,275(0,3);2,677(0,8);2,672(1,0);2,668(0,7) ;2,543(5,1);2,526 (2,5);2,512(61,4);2,508(121,1);2,503(156,1);2,499(110,0);2,494(51,1);2,335(0,7);2,330(1,0);2,325(0,7);1,234 (0,4);0,999(2, 1);0,985(5,6);0,981(6,3);0,969(3,0);0,931(0,5);0,918(0,4);0,880(3,0);0,869(6,0);0,864(5,6);0,850 (1,8);0,000(1,3) |
| Verbindung Nr. 1-179: |

(fortgesetzt)

| |
|---|
| 1H-NMR(400,0 MHz, DMSO): δ= 9,080(5,7);8,776(3,0);8,766(3,1);8,571(4,5);8,568(4,6);8,404(5,3);8,399(4,8);7,908(2,7);7,891(3,4);7,764(3 ,0);7,752(3,0);7,745(2,4);7,733(2,4);4,641(16,0);3,644(0,4);3,620(0,3);3,604(0,4);3,591(0,3);3,575(0,4);3,560(0,5);3,542(0,6);3,496(0,5);3,47 9(0,5);3,446(1,0);3,409(2,6);3,390(3,6);3,344(2699,8);3,302(3,8);3,293(2,7);3,268(1,0);3,262(1,2);3,250(0,8);3,237(0,6);3,219(0,9);3,215(0,9 );3,200(0,5);3,174(0,5);3,163(0,5);2,676(2,3);2,672(3,2);2,667(2,4);2,570(0,5);2,542(4,5);2,525(8,7);2,512(198,0);2,507(396,8);2,503(515,9); 2,498(365,8);2,494(171,0);2,334(2,4);2,329(3,2);2,325(2,4);2,292(0,7);2,074(0,6);1,298(0,4);1,259(0,5);1,235(1,2);1,034(2,2);1,021(6,0);1,0 16(6,5);1,004(3,0);0,964(0,4);0,934(0,3);0,894(2,9);0,882(6,2);0,878(6,0);0,864(1,9);0,000(5,9) |
| Verbindung Nr. 1-180:<br>1H-NMR(400,0 MHz, DMSO): δ= 8,984(5,7);8,553(3,9);8,551(3,9);8,549(3,7);8,406(3,5);8,401(3,7);8,393(3,7);8,389(3,6);8,080(2,6);8,074(2 ,5);8,058(2,7);8,052(2,6);7,981(3,7);7,976(3,7);7,962(4,1);7,957(3,8);7,474(3,8);7,462(3,7);7,455(3,6);7,443(3,5);6,990(4,4);6,968(4,2);4,536 (16,0);3,343(215,2);3,317(0,7);2,998(0,4);2,544(12,6);2,513(18,9);2,509(37,2);2,505(48,2);2,500(34,4);2,496(16,3);1,236(0,4);1,007(2,2);0,9 93(5,9);0,988(6,6);0,977(3,0);0,938(0,4);0,918(0,4);0,879(3,0);0,868(6,2);0,863(6,0);0,849(1,9);0,000(2,2) |
| Verbindung Nr. 1-181:<br>1H-NMR(400,0 MHz, DMSO): δ= 8,928(2,3);8,915(4,4);8,902(2,3);8,718(8,4);8,717(8,9);8,713(9,4);8,711(8,5);8,315(0,7);8,181(7,4);8,175(7 ,5);8,159(7,6);8,153(7,7);8,040(6,7);8,037(6,6);8,020(7,7);8,017(7,3);7,800(3,1);7,797(3,1);7,781(8,0);7,778(7,5);7,762(5,6);7,759(5,0);7,707 (4,8);7,703(5,3);7,687(6,1);7,683(6,2);7,668(3,1);7,664(3,0);7,583(7,6);7,580(7,4);7,564(6,5);7,561(6,0);7,019(10,0);7,018(9,5);6,998(9,6);6, 996(9,1);4,486(7,5);4,472(16,0);4,458(8,0);3,651(4,0);3,637(11,1);3,623(10,8);3,609(3,7);3,404(0,4);3,332(826,1);3,281(0,4);2,676(1,5);2,67 1(2,0);2,667(1,6);2,542(12,8);2,525(4,9);2,511(122,7);2,507(243,2);2,502(316,1);2,498(234,6);2,494(118,7);2,334(1,5);2,329(2,0);2,325(1,5) ;2,291(0,5);2,074(0,8);1,259(0,4);1,235(1,4);0,008(0,7);0,000(19,8) |
| Verbindung Nr. 1-182:<br>1H-NMR(400,0 MHz, DMSO): δ= 8,917(2,4);8,903(4,7);8,889(2,4);8,799(6,0);8,790(5,9);8,788(5,9);8,545(11,7);8,537(13,7);8,458(7,9);8,45 0(6,4);8,438(8,0);8,431(6,3);8,315(0,9);7,997(5,5);7,980(6,4);7,978(6,4);7,798(5,7);7,786(5,6);7,778(4,8);7,767(4,6);4,537(7,7);4,523(16,0); 4,509(8,1);3,682(4,1);3,668(11,2);3,654(10,9);3,640(3,8);3,396(0,3);3,329(720,4);2,680(0,7);2,676(1,6);2,671(2,2);2,667(1,6);2,542(7,1);2,5 25(5,1);2,511(125,0);2,507(254,4);2,502(334,0);2,498(237,7);2,493(112,0);2,338(0,7);2,334(1,5);2,329(2,1);2,324(1,5);2,290(0,5);2,074(0,4) ;1,259(0,4);1,236(1,6);0,008(0,8);0,000(24,2);-0,008(0,8) |
| Verbindung Nr. 1-183:<br>1H-NMR(400,0 MHz, DMSO): δ= 8,933(2,2);8,919(4,4);8,905(2,2);8,543(11,5);8,535(13,4);8,454(7,3);8,447(6,0);8,434(7,3);8,427(5,9);8,31 5(0,8);8,047(6,7);8,044(6,8);8,026(7,8);8,024(7,5);7,808(3,0);7,805(3,1);7,789(7,8);7,786(7,6);7,770(5,5);7,767(5,0);7,712(4,7);7,708(5,3);7, 692(5,8);7,689(6,2);7,673(3,1);7,669(3,0);7,602(7,6);7,598(7,4);7,583(6,5);7,579(6,0);4,543(7,3);4,529(16,0);4,515(7,7);3,657(3,9);3,643(11, 0);3,629(10,7);3,615(3,6);3,402(0,4);3,394(0,5);3,383(0,8);3,331(1042,8);3,299(1,1);2,680(0,7);2,676(1,6);2,671(2,2);2,667(1,6);2,662(0,7);2 ,542(9,6);2,525(4,8);2,520(7,5);2,511(124,6);2,507(255,7);2,502(336,6);2,498(238,0);2,493(110,9);2,338(0,7);2,334(1,6);2,329(2,2);2,324(1, 5);2,320(0,7);2,291(0,5);2,074(0,6);1,259(0,4);1,235(1,5);0,008(0,7);0,000(23,4);-0,009(0,7) |
| Verbindung Nr. 1-184:<br>1H-NMR(400,0 MHz, DMSO): δ= 8,844(2,4);8,831(4,5);8,817(2,3);8,540(12,1);8,532(13,7);8,475(7,8);8,470(8,5);8,463(8,6);8,458(9,2);8,45 5(9,3);8,447(6,7);8,435(8,3);8,427(6,4);8,316(0,7);7,881(8,1);7,876(8,3);7,863(9,3);7,858(8,8);7,508(9,0);7,496(8,7);7,489(8,3);7,477(8,1);4, 557(7,9);4,543(16,0);4,529(8,3);3,676(4,2);3,662(11,5);3,648(11,1);3,635(3,8);3,328(618,8);2,995(0,6);2,676(1,5);2,671(2,0);2,667(1,5);2,66 2(0,7);2,541(21,5);2,524(5,3);2,511(119,4);2,507(237,8);2,502(310,4);2,497(223,1);2,493(106,2);2,333(1,4);2,329(2,0);2,324(1,4);2,290(0,5) ;2,075(0,8);1,235(1,5);0,008(1,2);0,000(32,7);-0,008(1,1) |
| Verbindung Nr. 1-185: |

(fortgesetzt)

| |
|---|
| 1H-NMR(400,0 MHz, DMSO): δ= 8,709(8,8);8,704(8,8);8,703(8,5);8,615(2,2);8,601(4,3);8,588(2,2);8,168(7,6); 8,162(7,4);8,146(7,8);8,140(7 ,6);7,874(6,9);7,872(7,4);7,854(7,5);7,852(7,5);7,446(3,2);7,443(3,3);7,427(7,5); 7,425(7,5);7,408(4,8);7,406(4,7);7,300(7,0);7,296(7,8);7,281 (5,7);7,277(5,6);7,178(4,1);7,174(3,9);7,159(6,3); 7,155(6,0);7,140(3,5);7,136(3,2);7,029(9,9);7,028(9,9);7,007(9,5);7,006(9,6);4,499(7,4);4,4 84(16,0);4,470(7,8); 3,637(3,9);3,623(10,9);3,609(10,6);3,595(3,5);3,329(226,1);2,676(0,6);2,671(0,7);2,667(0,6);2,541(5,4);2,524 (2,0);2,511 (45,1);2,507(89,9);2,502(117,1);2,498(83,5);2,493(39,5);2,333(0,5);2,329(0,7);2,324(0,5);1,235(1,0); 0,008(0,4);0,000(12,4);-0,008(0,4) |
| Verbindung Nr. 1-186:<br>1H-NMR(400,0 MHz, DMSO): δ= 8,848(2,4);8,834(4,5);8,820(2,3);8,542(11,2);8,535(12,8);8,454(8,0);8,446(6,8); 8,439(8,5);8,434(16,0);8,4 27(12,5);8,422(8,5);8,316(0,8);7,809(7,7);7,804(7,8);7,790(9,2);7,785(8,5);7,527 (8,8);7,515(8,6);7,509(7,8);7,497(7,8);4,558(7,5);4,544(15,3 );4,530(7,8);3,671(4,0);3,657(10,8);3,643(10,4); 3,629(3,6);3,386(0,4);3,328(689,4);2,995(0,9);2,676(1,7);2,671(2,2);2,667(1,6);2,541(25,8);2 ,524(5,9);2,511 (137,1);2,507(269,1);2,502(344,8);2,497(243,6);2,493(113,3);2,338(0,8);2,333(1,7);2,329(2,2);2,324(1,6);2,290 (0,5);2,075(0, 8);1,259(0,4);1,236(1,8);0,008(1,3);0,000(34,5);-0,008(1,1) |
| Verbindung Nr. 1-187:<br>1H-NMR(400,0 MHz, DMSO): δ= 8,622(2,4);8,608(4,7);8,595(2,3);8,537(12,1);8,529(13,9);8,445(8,4);8,438(6,8); 8,425(8,6);8,418(6,7);8,31 5(0,3);7,880(7,5);7,878(7,7);7,860(8,2);7,858(7,9);7,454(3,4);7,451(3,4);7,435(8,2); 7,432(7,9);7,416(5,5);7,414(5,2);7,332(7,6);7,328(8,3);7, 313(5,9);7,309(5,6);7,185(4,5);7,180(4,3);7,166(6,5); 7,161(6,1);7,146(3,9);7,142(3,6);4,556(7,4);4,541(16,0);4,527(7,7);3,644(3,9);3,630(11, 0);3,616(10,7);3,601 (3,6);3,358(0,4);3,329(249,2);3,303(0,4);2,676(0,5);2,671(0,7);2,667(0,5);2,541(5,8);2,524(1,9);2,511(43,3); 2,507(86,2); 2,502(111,9);2,498(78,6);2,493(36,5);2,333(0,5);2,329(0,7);2,324(0,5);1,235(1,0);0,008(0,7);0,000 (19,4);-0,009(0,6) |
| Verbindung Nr. 1-188:<br>1H-NMR(400,0 MHz, DMSO): δ= 8,958(2,0);8,945(3,7);8,932(2,0);8,708(8,9);8,706(9,2);8,702(9,4);8,700(8,6); 8,315(0,6);8,177(8,6);8,171(8 ,3);8,155(8,9);8,149(8,7);7,541(1,5);7,524(3,2);7,520(2,7);7,507(2,1);7,503(6,0); 7,498(2,1);7,486(2,8);7,482(3,5);7,465(1,6);7,181(1,2);7,178 (1,6);7,170(10,1);7,151(12,7);7,130(8,4);7,122 (1,4);6,999(10,5);6,997(10,1);6,977(10,2);6,975(9,9);4,474(7,8);4,460(16,0);4,446(8,3);3,670( 4,0);3,656(11,1); 3,642(10,7);3,628(3,7);3,330(653,3);3,293(0,4);2,995(0,6);2,680(0,6);2,676(1,2);2,671(1,6);2,667(1,1);2,662 (0,5);2,542(78, 4);2,525(3,8);2,520(6,1);2,511(90,9);2,507(183,7);2,502(239,2);2,498(167,5);2,493(77,4);2,338 (0,5);2,334(1,1);2,329(1,5);2,324(1,1);2,320( 0,5);2,290(0,4);2,075(0,5);1,235(1,2);0,008(0,5);0,000(16,2);- 0,009(0,5) |
| Verbindung Nr. 1-189:<br>1H-NMR(400,0 MHz, DMSO): δ= 8,949(2,2);8,935(4,1);8,922(2,1);8,526(12,0);8,519(14,2);8,451(8,4);8,443(6,6); 8,431(8,4);8,423(6,5);8,31 5(2,0);7,541(1,5);7,524(3,3);7,520(2,8);7,508(2,2);7,503(6,1);7,499(2,1);7,487(3,1); 7,482(3,5);7,466(1,6);7,180(1,7);7,172(10,4);7,153(13,2) ;7,132(8,5);7,124(1,4);4,533(7,7);4,519(16,0);4,505 (8,1);3,679(4,1);3,665(11,3);3,651(10,9);3,637(3,8);3,489(0,4);3,463(0,3);3,445(0,5);3,43 4(0,5);3,405(0,8); 3,385(1,2);3,378(1,4);3,359(3,9);3,329(2501,7);3,298(2,1);3,285(1,2);3,277(1,0);3,258(0,5);2,995(0,5);2,680 (1,9);2,676(4,1 );2,671(5,5);2,666(3,9);2,662(1,8);2,541(39,3);2,524(13,6);2,511(329,8);2,507(660,9);2,502 (858,1);2,497(603,0);2,493(279,9);2,368(0,3);2,3 38(1,9);2,333(4,1);2,329(5,5);2,324(3,9);2,320(1,8);2,290(1,0); 2,074(1,7);1,298(0,7);1,259(1,0);1,236(4,2);0,854(0,4);0,008(1,7);0,000(54,8) ;-0,008(1,7) |
| Verbindung Nr. 1-190:<br>1H-NMR(400,0 MHz, DMSO): δ= 8,816(2,5);8,803(4,6);8,789(2,4);8,707(10,1);8,702(8,7);8,701(9,4);8,433(6,6); 8,428(7,0);8,420(6,9);8,416( 6,7);8,316(0,4);8,181(7,7);8,175(7,2);8,159(7,9);8,153(7,5);8,062(7,0);8,058(6,8); 8,044(7,7);8,039(7,0);7,512(7,1);7,500(7,2);7,494(6,8);7,48 1(6,5);6,988(10,9);6,966(10,5);4,491(8,0);4,477 (16,0);4,463(8,3);3,676(4,3);3,662(11,5);3,648(11,1);3,634(3,9);3,330(651,8);3,294(0,5);2,67 6(1,1);2,671(1,5); 2,667(1,1);2,542(5,6);2,511(95,1);2,507(179,8);2,502(229,1);2,498(163,7);2,494(78,1);2,333(1,1);2,329(1,4); 2,325(1,1);2,0 75(0,4);1,235(1,1);0,000(14,3);-0,008(0,5) |
| Verbindung Nr. 1-191: |

(fortgesetzt)

| |
|---|
| 1H-NMR(400,0 MHz, DMSO): δ= 8,711(8,6);8,705(8,7);8,658(2,1);8,644(4,0);8,630(2,1);8,172(6,3);8,166(6,1); 8,150(6,5);8,144(6,3);7,643(6 ,6);7,631(2,1);7,624(8,1);7,438(2,0);7,435(2,1);7,422(2,5);7,417(7,1);7,401(6,1); 7,399(5,7);7,364(15,1);7,347(11,2);7,342(6,4);7,329(2,8);7,3 24(2,0);7,017(10,0);6,995(9,6);4,494(7,4);4,480 (16,0);4,465(7,9);3,644(3,9);3,630(10,9);3,616(10,6);3,601(3,6);3,367(0,3);3,329(251,6);3,30 6(0,6);2,675(0,7); 2,671(0,9);2,667(0,6);2,541(1,9);2,524(2,1);2,511(53,3);2,506(105,4);2,502(136,7);2,498(97,4);2,333(0,6);2,329 (0,9);2,324 (0,6);2,075(0,5);1,235(1,0);0,008(0,4);0,000(12,2);-0,009(0,4) |
| Verbindung Nr. 1-192:<br>1H-NMR(400,0 MHz, DMSO): δ= 8,653(2,3);8,640(4,4);8,626(2,2);8,539(12,2);8,531(14,0);8,450(8,5);8,443(6,9); 8,431(8,7);8,423(6,8);8,31 6(0,4);7,652(6,6);7,650(7,4);7,633(7,5);7,630(8,1);7,444(2,1);7,442(2,3);7,425(5,9); 7,423(6,1);7,408(8,3);7,405(8,2);7,394(6,9);7,389(11,8); 7,375(4,9);7,371(8,3);7,366(4,4);7,354(4,5);7,352(6,0); 7,349(4,0);7,346(4,6);7,334(3,5);7,329(3,0);4,553(7,5);4,538(16,0);4,524(7,9);3,652(3 ,9);3,638(11,1);3,624 (10,8);3,610(3,7);3,326(190,5);2,676(0,6);2,671(0,9);2,666(0,7);2,541(4,6);2,524(2,3);2,519(3,7);2,511(52,2); 2,506(106 ,9);2,502(141,0);2,497(99,8);2,493(46,4);2,333(0,7);2,329(0,9);2,324(0,7);2,320(0,3);1,235(1,0); 0,008(1,0);0,000(28,8);-0,009(0,9) |
| Verbindung Nr. 1-193:<br>1H-NMR(400,0 MHz, DMSO): δ= 8,700(7,9);8,699(7,9);8,694(8,1);8,512(2,9);8,316(0,4);8,171(6,9);8,165(6,5); 8,150(7,1);8,144(6,7);7,611(2 ,6);7,607(2,9);7,593(4,9);7,588(5,8);7,574(3,1);7,569(3,0);7,550(1,5);7,545(1,4); 7,536(1,7);7,532(3,2);7,529(2,5);7,527(2,3);7,524(2,0);7,518 (2,6);7,516(2,5);7,514(2,5);7,511(3,4);7,506(1,8); 7,497(1,9);7,493(1,6);7,295(4,1);7,285(4,8);7,283(4,6);7,274(4,2);7,267(10,2);7,247(8,1);7, 017(9,3);7,016(8,8); 6,995(8,9);6,994(8,5);4,503(7,5);4,488(16,0);4,474(7,8);3,673(3,7);3,659(10,4);3,645(10,1);3,631(3,4);3,329 (341,2);2,99 6(0,4);2,676(0,9);2,671(1,2);2,667(0,9);2,662(0,4);2,542(12,7);2,524(4,2);2,511(78,2);2,507(149,2); 2,502(189,1);2,498(132,4);2,493(61,2);2, 334(0,9);2,329(1,2);2,325(0,9);1,236(1,0);0,008(0,6);0,000(14,0);- 0,008(0,4) |
| Verbindung Nr. 1-194:<br>1H-NMR(400,0 MHz, DMSO): δ= 8,519(11,1);8,511(14,1);8,495(3,0);8,446(7,3);8,438(5,7);8,426(7,3);8,418(5,7); 8,316(0,9);7,629(2,7);7,62 4(3,1);7,611(5,4);7,606(6,1);7,591(3,4);7,587(3,3);7,552(1,5);7,547(1,4);7,538(1,7); 7,534(3,4);7,531(2,5);7,529(2,4);7,526(2,1);7,520(2,7);7, 516(2,6);7,513(3,6);7,508(1,9);7,499(2,1);7,495(1,7); 7,301(4,3);7,289(4,9);7,286(5,1);7,280(4,2);7,276(4,6);7,273(5,4);7,270(8,9);7,255(3,4) ;7,251(6,1);4,556(7,5); 4,542(16,0);4,528(7,8);3,675(3,7);3,661(10,6);3,647(10,3);3,633(3,5);3,387(0,3);3,328(788,4);3,291(0,4);2,676 (1,9);2, 671(2,6);2,667(1,8);2,662(0,9);2,541(27,0);2,525(6,3);2,511(150,6);2,507(301,8);2,502(391,8);2,498 (275,7);2,493(127,8);2,338(0,9);2,333(1 ,8);2,329(2,5);2,324(1,8);2,290(0,5);2,075(0,5);1,259(0,5);1,235(2,2); 0,008(1,1);0,000(34,2);-0,008(1,1) |
| Verbindung Nr. 1-195:<br>1H-NMR(400,0 MHz, DMSO): δ= 9,049(6,1);8,562(4,7);8,559(4,7);8,392(5,3);8,387(5,0);8,034(3,7);8,033(3,8); 8,014(4,2);8,012(4,1);7,778(1 ,6);7,775(1,6);7,759(4,1);7,757(3,9);7,740(2,8);7,738(2,6);7,686(2,4);7,683(2,6); 7,666(3,3);7,663(3,4);7,647(1,6);7,644(1,5);7,498(3,9);7,495 (3,9);7,479(3,5);7,476(3,3);5,757(0,5);4,646(16,0); 3,324(40,8);2,672(0,4);2,507(50,0);2,503(64,4);2,498(46,4);2,329(0,4);1,022(2,0);1,007(5, 9);1,003(6,7);0,992 (3,1);0,953(0,5);0,946(0,5);0,908(3,1);0,896(6,4);0,892(6,0);0,878(1,9);0,146(0,4);0,008(3,2);0,000(73,2);-0,009 (2,6);-0,150(0,3) |
| Verbindung Nr. 1-196:<br>1H-NMR(400,0 MHz, DMSO): δ= 9,048(5,6);8,579(3,8);8,083(2,5);8,076(2,5);8,061(2,6);8,054(2,6);8,027(3,4); 8,025(3,6);8,007(3,9);8,005(3 ,9);7,761(1,4);7,758(1,5);7,742(3,7);7,740(3,8);7,723(2,6);7,721(2,5);7,680(2,3); 7,677(2,6);7,661(3,1);7,657(3,3);7,641(1,4);7,638(1,4);7,479 (3,7);7,475(3,7);7,460(3,3);7,456(3,2);7,020(4,2); 6,998(4,0);5,757(2,1);4,529(16,0);3,323(46,0);2,675(0,4);2,671(0,5);2,667(0,4);2,524(1,4); 2,511(32,5);2,506 (64,3);2,502(83,7);2,498(60,2);2,493(29,2);2,333(0,4);2,329(0,5);2,324(0,4);0,987(1,9);0,967(6,4);0,957(3,1); 0,928(0,7);0, 920(0,7);0,891(3,1);0,880(6,0);0,861(1,7);0,146(0,5);0,008(3,8);0,000(96,3);-0,008(3,6);-0,150 (0,5) |
| Verbindung Nr. 1-197: |

(fortgesetzt)

| |
|---|
| 1H-NMR(400,0 MHz, DMSO): δ= 8,616(1,4);8,602(2,9);8,588(2,1);8,569(4,1);8,567(4,9);8,564(4,9);8,489(0,4); 8,472(0,4);8,410(0,9);8,405(0 ,8);8,375(5,6);8,369(5,4);7,879(0,8);7,867(4,4);7,862(1,0);7,849(4,6);7,436(2,1); 7,433(2,0);7,417(4,5);7,415(4,3);7,398(2,7);7,396(2,6);7,298 (0,6);7,294(0,7);7,279(0,5);7,275(0,5);7,245(3,9); 7,241(4,5);7,226(3,5);7,222(3,5);7,186(0,5);7,182(0,5);7,176(2,5);7,172(2,3);7,167(0,8);7,1 63(0,8);7,157(3,8); 7,153(3,5);7,144(0,5);7,138(2,0);7,133(1,8);5,534(1,3);5,518(2,2);5,506(2,1);5,490(1,3);4,522(0,4);4,509(0,5); 4,399(0,6); 4,383(0,6);3,640(0,9);3,626(1,3);3,613(1,1);3,606(1,8);3,592(2,4);3,579(1,5);3,531(1,6);3,516(2,6); 3,500(1,9);3,481(1,6);3,466(1,0);3,355(56 1,6);3,291(0,4);2,683(0,4);2,679(0,6);2,674(0,4);2,549(45,2);2,532 (1,8);2,519(36,8);2,514(74,7);2,510(98,4);2,505(70,9);2,501(34,6);2,341(0 ,5);2,336(0,6);2,332(0,5);1,417 (16,0);1,401(15,9);1,278(2,0);1,262(2,2);1,242(1,4);0,860(0,4) |
| Verbindung Nr. 1-198:<br>1H-NMR(400,0 MHz, DMSO): δ= 8,805(4,3);8,792(5,9);8,770(3,2);8,592(6,1);8,589(5,9);8,421(6,6);8,416(6,0); 7,969(3,6);7,950(4,5);7,805(3 ,6);7,793(3,6);7,786(3,0);7,774(2,8);4,541(1,9);4,530(3,1);4,517(3,3);4,506(3,0); 4,453(0,9);4,436(1,8);4,419(2,0);4,407(1,6);4,400(1,6);4,390 (5,1);4,373(1,8);4,366(3,5);4,348(1,9);3,405(0,5); 3,352(368,4);2,683(0,4);2,679(0,5);2,674(0,4);2,549(30,3);2,514(66,3);2,510(82,9);2,505(5 9,5);2,341(0,4); 2,336(0,5);2,332(0,4);1,269(16,0);1,253(15,8) |
| Verbindung Nr. 1-199:<br>1H-NMR(400,0 MHz, DMSO): δ= 8,773(1,0);8,763(1,0);8,577(1,5);8,575(1,5);8,533(1,7);8,417(1,6);8,412(1,5); 7,905(0,9);7,887(1,1);7,773(0 ,9);7,762(0,9);7,754(0,7);7,742(0,7);4,622(5,2);3,345(93,4);2,543(10,8);2,508 (19,0);2,504(24,0);2,499(17,7);1,457(16,0) |
| Verbindung Nr. 1-200:<br>1H-NMR(400,0 MHz, DMSO): δ= 8,882(1,4);8,868(2,7);8,854(1,4);8,785(3,6);8,775(3,3);8,773(3,3);8,575(4,0); 8,572(5,0);8,569(5,0);8,567(4 ,1);8,380(5,6);8,375(5,3);7,930(2,8);7,928(2,9);7,911(3,6);7,909(3,6);7,777(3,2); 7,765(3,2);7,757(2,6);7,745(2,5);5,499(1,2);5,487(1,5);5,482 (2,1);5,471(2,0);5,466(1,5);5,455(1,2);3,697(1,0); 3,685(1,3);3,682(1,3);3,671(1,1);3,662(1,6);3,650(1,9);3,647(1,9);3,636(1,4);3,545(1,5);3,5 29(2,2);3,513(1,8); 3,494(1,5);3,479(1,0);3,405(0,4);3,346(331,6);3,302(0,5);2,673(0,4);2,543(38,4);2,526(1,2);2,513(26,8);2,509 (54,2);2,504 (71,0);2,499(50,8);2,495(24,1);2,335(0,3);2,331(0,4);2,326(0,3);1,384(16,0);1,368(15,8);1,264(0,7); 1,248(0,7);1,235(0,4) |
| Verbindung Nr. 1-201:<br>1H-NMR(400,0 MHz, DMSO): δ= 8,581(5,6);8,578(5,6);8,524(2,8);8,506(2,8);8,406(6,3);8,401(5,9);7,648(3,5); 7,645(4,1);7,637(1,2);7,626(5 ,8);7,444(1,6);7,441(1,6);7,424(4,4);7,407(4,0);7,404(3,8);7,369(1,8);7,364(4,7); 7,357(5,4);7,352(6,2);7,347(5,1);7,338(3,6);7,333(3,4);7,330 (2,5);7,325(1,3);5,325(0,3);4,531(1,4);4,521(3,1); 4,508(3,3);4,497(2,1);4,485(0,5);4,443(0,8);4,426(1,6);4,408(1,9);4,392(4,4);4,369(3,8);4,3 52(1,7);3,343 (544,6);2,676(0,6);2,672(0,8);2,668(0,6);2,542(6,7);2,507(97,0);2,503(125,3);2,499(90,2);2,334(0,6);2,330(0,8); 2,325(0,6);2,00 9(0,6);1,990(0,6);1,974(0,3);1,261(15,2);1,245(16,0);0,870(0,3);0,854(0,8);0,837(0,3);0,000(0,4) |
| Verbindung Nr. 1-202:<br>1H-NMR(400,0 MHz, DMSO): δ= 8,649(1,4);8,635(2,8);8,621(1,4);8,578(1,0);8,562(4,9);8,559(5,0);8,526(0,4); 8,507(0,4);8,406(0,9);8,401(0 ,9);8,371(5,6);8,366(5,4);7,649(0,5);7,646(0,7);7,628(4,8);7,610(4,4);7,608(4,6); 7,422(1,8);7,419(1,6);7,403(4,3);7,400(4,2);7,385(3,5);7,382 (3,3);7,369(0,4);7,365(0,8);7,356(3,0);7,351(3,9); 7,337(3,3);7,332(4,3);7,318(1,6);7,313(1,9);7,308(4,8);7,304(3,9);7,290(3,3);7,285(2,8);5,5 31(1,3);5,514(2,2); 5,503(2,1);5,499(1,7);5,487(1,2);4,521(0,4);4,509(0,5);4,393(0,6);4,370(0,5);3,645(0,9);3,632(1,3);3,618(1,1); 3,610(1,8); 3,596(2,3);3,584(1,5);3,529(1,6);3,514(2,6);3,498(2,0);3,479(1,6);3,464(1,0);3,398(0,5);3,379(0,8); 3,345(337,4);2,677(0,3);2,673(0,5);2,668( 0,4);2,543(31,5);2,526(1,3);2,512(28,4);2,508(56,9);2,504(74,8); 2,499(54,4);2,331(0,5);1,398(16,0);1,382(15,8);1,262(2,2);1,246(2,3);1,236( 0,7);0,000(0,3) |
| Verbindung Nr. 1-203:<br>1H-NMR(400,0 MHz, DMSO): δ= 8,585(5,7);8,583(5,6);8,536(2,9);8,517(2,8);8,412(6,1);8,407(5,8);7,496(3,1); 7,477(7,1);7,458(2,2);7,448(3 ,6);7,447(3,5);7,436(3,8);7,428(2,5);7,416(2,5);7,408(1,0);7,389(12,5);7,379(8,5); 4,546(1,8);4,535(3,0);4,522(3,2);4,510(2,9);4,459(0,9);4,44 1(1,7);4,424(1,9);4,411(1,5);4,405(1,4);4,395(4,9); 4,377(1,7);4,370(3,6);4,352(1,9);3,446(0,4);3,354(733,6);2,683(0,7);2,679(0,9);2,674(0,7) ;2,549(10,5);2,514 (108,5);2,510(140,4);2,505(101,7);2,341(0,7);2,337(0,9);2,332(0,6);1,263(16,0);1,247(16,0) |
| Verbindung Nr. 1-204: |

(fortgesetzt)

| |
|---|
| 1H-NMR(400,0 MHz, DMSO): δ= 8,921(1,3);8,907(2,6);8,893(1,3);8,829(0,4);8,810(0,4);8,571(0,9);8,569(0,9); 8,549(4,9);8,546(4,8);8,402(0 ,9);8,397(0,9);8,370(5,5);8,365(5,1);7,527(0,8);7,520(0,4);7,510(1,7);7,506(1,6); 7,498(0,8);7,493(1,4);7,489(3,2);7,472(1,7);7,468(1,9);7,451 (0,8);7,166(1,5);7,154(5,5);7,145(1,8);7,135(7,5); 7,114(4,5);7,106(0,8);5,499(1,3);5,488(1,6);5,483(2,2);5,472(2,1);5,468(1,6);5,456(1,2);4,5 02(0,4);4,489(0,4); 4,478(0,5);4,384(0,7);4,360(0,5);3,663(0,8);3,650(1,2);3,637(1,0);3,628(1,8);3,615(2,5);3,602(1,6);3,577(1,7); 3,562(2,9); 3,546(2,0);3,527(1,4);3,511(0,8);3,403(0,3);3,347(326,8);3,313(0,7);2,673(0,4);2,543(35,5);2,526 (1,1);2,513(25,5);2,508(50,5);2,504(65,2);2 ,500(46,3);2,495(21,9);2,331(0,4);1,373(16,0);1,357(15,8);1,257 (2,7);1,241(3,1);0,000(0,8) |
| Verbindung Nr. 1-205:<br>1H-NMR(400,0 MHz, DMSO): δ= 8,574(1,4);8,571(1,4);8,462(1,6);8,441(1,2);8,436(1,2);8,429(1,2);8,424(1,2); 8,411(1,5);8,406(1,4);7,796(1 ,1);7,791(1,1);7,777(1,2);7,772(1,2);7,477(1,2);7,465(1,2);7,458(1,1);7,446(1,0); 4,646(5,1);3,344(120,2);2,543(11,1);2,512(11,4);2,508(21,7) ;2,503(27,7);2,499(19,9);2,495(9,5);1,466(16,0); 1,235(0,5);0,000(0,5) |
| Verbindung Nr. 1-206:<br>1H-NMR(400,0 MHz, DMSO): δ= 8,820(1,4);8,806(2,7);8,792(1,4);8,581(0,6);8,578(0,6);8,576(0,6);8,566(4,2); 8,563(5,0);8,561(4,9);8,471(0 ,5);8,466(0,6);8,458(4,4);8,454(4,6);8,446(4,4);8,442(4,2);8,410(0,6);8,404(0,5); 8,377(5,6);8,371(5,2);7,851(0,4);7,846(0,4);7,832(0,5);7,828 (0,6);7,820(4,0);7,815(4,0);7,801(4,6);7,796(4,3); 7,506(0,5);7,493(0,6);7,487(4,8);7,475(4,7);7,468(4,1);7,456(3,9);5,528(1,2);5,516(1,5);5,5 11(2,1);5,500(2,0); 5,495(1,6);5,484(1,3);4,389(0,4);3,683(0,9);3,671(1,3);3,668(1,3);3,657(1,1);3,648(1,7);3,636(2,0);3,634(2,1); 3,622(1,5); 3,548(1,6);3,533(2,4);3,516(2,0);3,497(1,5);3,482(1,0);3,428(0,4);3,396(0,6);3,347(399,8);3,229 (0,4);2,677(0,4);2,673(0,5);2,668(0,4);2,543( 38,1);2,526(1,3);2,513(32,9);2,508(64,6);2,504(83,0);2,499(59,5); 2,495(28,4);2,335(0,4);2,331(0,5);2,326(0,4);1,396(16,0);1,381(15,8);1,27 1(1,4);1,255(1,4);1,235(0,5);0,000 (1,3) |
| Verbindung Nr. 1-207:<br>1H-NMR(400,0 MHz, DMSO): δ= 8,722(3,2);8,705(3,1);8,572(7,1);8,570(6,9);8,541(6,0);8,538(5,9);8,530(6,3); 8,526(5,8);8,396(7,5);8,391(7 ,1);8,026(5,7);8,023(5,3);8,006(6,3);8,003(5,6);7,548(5,9);7,537(5,7);7,528(5,4); 7,516(5,3);4,529(0,5);4,508(3,3);4,496(3,5);4,475(5,0);4,465 (3,6);4,457(3,3);4,450(6,5);4,434(4,1);4,424(2,0); 4,409(0,6);3,352(404,1);3,289(0,3);2,678(0,4);2,674(0,5);2,669(0,4);2,544(33,5);2,527(1,4) ;2,509(64,5);2,505 (83,3);2,500(59,9);2,336(0,4);2,331(0,5);2,327(0,4);1,280(16,0);1,265(14,8);1,235(0,8);0,000(1,0) |
| Verbindung Nr. 1-208:<br>1H-NMR(400,0 MHz, DMSO): δ= 8,566(1,3);8,563(1,3);8,497(1,2);8,494(1,3);8,486(1,3);8,482(1,3);8,433(1,5); 8,399(1,5);8,393(1,4);7,987(1 ,2);7,984(1,2);7,967(1,3);7,964(1,3);7,506(1,3);7,494(1,2);7,485(1,2);7,474(1,2); 4,659(5,2);3,352(83,7);2,544(5,7);2,514(5,7);2,509(11,5);2,5 05(15,1);2,500(10,8);2,496(5,1);1,485(16,0) |
| Verbindung Nr. 1-209:<br>1H-NMR(400,0 MHz, DMSO): δ= 8,820(1,3);8,806(2,5);8,791(1,3);8,724(0,3);8,573(0,8);8,570(0,8);8,542(5,4); 8,539(5,3);8,530(1,1);8,527(1 ,0);8,519(4,4);8,515(4,6);8,507(4,6);8,504(4,5);8,397(0,8);8,392(0,8);8,364(5,3); 8,358(5,0);8,027(0,7);8,024(0,7);8,012(4,4);8,009(4,6);8,003 (1,0);7,991(4,9);7,988(4,6);7,548(0,8);7,534(4,9); 7,528(0,9);7,522(4,6);7,514(4,4);7,502(4,3);5,539(1,3);5,523(2,2);5,511(2,0);5,507(1,7);5,4 95(1,2);4,508(0,3); 4,496(0,4);4,475(0,5);4,465(0,3);4,457(0,3);4,450(0,6);4,435(0,4);3,658(0,6);3,645(0,9);3,631(0,8);3,623(2,1); 3,610(2,9); 3,601(2,5);3,597(2,3);3,586(3,4);3,570(2,1);3,551(1,1);3,535(0,7);3,348(352,4);3,309(0,5);2,674 (0,4);2,544(27,8);2,527(1,4);2,513(25,6);2,5 09(51,2);2,504(66,7);2,500(47,2);2,495(22,2);2,331(0,4);1,385 (16,0);1,369(15,8);1,280(1,6);1,265(1,5);1,234(0,4);0,000(0,8) |
| Verbindung Nr. 1-210:<br>1H-NMR(400,0 MHz, DMSO): δ= 9,084(1,5);9,070(3,0);9,056(1,5);8,909(0,4);8,891(0,4);8,679(0,6);8,675(0,7); 8,663(4,1);8,660(4,0);8,652(3 ,8);8,648(3,8);8,545(0,7);8,494(5,1);8,492(5,2);8,383(0,8);8,378(0,8);8,353(6,0); 8,348(5,6);8,157(0,5);8,153(0,5);8,137(0,6);8,133(0,5);8,085 (3,5);8,081(3,6);8,066(3,8);8,062(3,7);7,513(0,6); 7,501(0,6);7,493(0,6);7,484(4,0);7,472(3,8);7,464(3,8);7,453(3,6);5,569(1,2);5,558(1,4);5,5 52(2,0);5,542(1,9); 5,536(1,5);5,525(1,2);4,594(0,4);4,579(0,4);4,568(0,4);4,408(0,5);4,383(0,4);3,681(0,9);3,668(1,3);3,656(1,1); 3,646(1,8); 3,633(2,3);3,621(1,5);3,571(1,6);3,555(2,4);3,538(2,3);3,519(1,4);3,503(1,0);3,415(0,6);3,347 (1079,8);3,283(0,8);3,275(0,6);3,263(0,5);3,25 4(0,4);3,240(0,3);2,713(0,4);2,677(0,9);2,673(1,2);2,668(0,9); 2,543(81,4);2,525(3,3);2,508(151,9);2,504(198,3);2,499(144,5);2,369(0,4);2,33 5(1,0);2,330(1,3);2,326(1,0); 1,387(16,0);1,371(15,9);1,290(2,1);1,274(2,0);1,259(0,4);1,235(1,3);0,000(0,5) |

(fortgesetzt)

| |
|---|
| Verbindung Nr. 1-211: 1H-NMR(400,0 MHz, DMSO): δ= 8,579(6,8);8,577(6,8);8,481(3,3);8,463(3,2);8,404(7,9);8,399(7,4);7,874(5,9); 7,872(6,1);7,854(6,3);7,852(6 ,3);7,452(2,6);7,449(2,7);7,433(6,1);7,431(6,1);7,414(3,8);7,412(3,8);7,291(5,5); 7,287(6,1);7,272(4,6);7,268(4,6);7,179(3,2);7,174(3,2);7,159 (5,1);7,155(4,8);7,140(2,7);7,136(2,5);4,535(0,7); 4,522(0,9);4,513(4,2);4,501(4,7);4,491(1,3);4,479(1,5);4,430(1,0);4,413(2,1);4,393(6,0);4,3 76(5,8);4,359(1,8); 3,417(0,3);3,331(964,4);3,293(0,7);3,283(0,4);2,995(1,1);2,675(1,7);2,671(2,3);2,667(1,7);2,541(68,8);2,524 (6,2);2,511(1 38,8);2,506(282,7);2,502(373,8);2,497(269,1);2,493(129,8);2,367(0,4);2,338(0,9);2,333(1,7);2,329 (2,4);2,324(1,7);1,298(0,4);1,271(16,0);1, 255(15,9);1,235(2,2);0,000(9,3);-0,009(0,3) |
| Verbindung Nr. 1-212: 1H-NMR(400,0 MHz, DMSO): δ= 8,567(1,4);8,565(1,4);8,399(1,6);8,394(1,5);8,243(1,7);7,846(1,2);7,844(1,3); 7,827(1,3);7,825(1,3);7,427(0 ,6);7,424(0,6);7,408(1,3);7,406(1,3);7,390(0,8);7,387(0,8);7,254(1,2);7,250(1,3); 7,235(1,0);7,231(1,0);7,153(0,7);7,149(0,7);7,134(1,1);7,130 (1,0);7,115(0,6);7,111(0,6);4,646(5,2);3,337(21,6); 2,543(5,1);2,513(3,3);2,508(6,7);2,504(8,8);2,499(6,4);2,495(3,1);1,474(16,0) |

## Biologische Beispiele

## Beispiel 1

**Cooperia curticei - Test (COOPCU)**

Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit "Ringerlösung" auf die gewünschte Konzentration.
Gefäße mit der Wirkstoffzubereitung der gewünschten Konzentration werden mit ca. 40 Nematodenlarven (*Cooperia curticei*) besetzt.
Nach 5 Tagen wird die Abtötung in % bestimmt. Dabei bedeutet 100 %, dass alle Larven abgetötet wurden; 0 % bedeutet, dass keine der Larven abgetötet wurde.
Bei diesem Test zeigen z.B. die folgenden Verbindungen der Herstellungsbeispiele eine Wirkung von 90% bei einer Aufwandmenge von 100ppm: 1-36.
Bei diesem Test zeigen z.B. die folgenden Verbindungen der Herstellungsbeispiele eine Wirkung von 80% bei einer Aufwandmenge von 100ppm: 1-35, 1-45.
Bei diesem Test zeigen z.B. die folgenden Verbindungen der Herstellungsbeispiele eine Wirkung von 100% bei einer Aufwandmenge von 20ppm: 1-1, 1-4, 1-28, 1-168, 1-170, 1-173.
Bei diesem Test zeigen z.B. die folgenden Verbindungen der Herstellungsbeispiele eine Wirkung von 90% bei einer Aufwandmenge von 20ppm: 1-25, 1-27, 1-82, 1-164, 1-176, 1-178.
Bei diesem Test zeigen z.B. die folgenden Verbindungen der Herstellungsbeispiele eine Wirkung von 80% bei einer Aufwandmenge von 20ppm: 1-26, 1-29, 1-48, 1-63, 1-78, 1-155, 1-169.

## Haemonchus contortus - Test (HAEMCO)

Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit "Ringerlösung" auf die gewünschte Konzentration.
Gefäße mit der Wirkstoffzubereitung der gewünschten Konzentration werden mit ca 40 Larven des Roten Magenwurmes (*Haemonchus contortus*) besetzt.
Nach 5 Tagen wird die Abtötung in % bestimmt. Dabei bedeutet 100 %, dass alle Larven abgetötet wurden; 0 % bedeutet, dass keine der Larven abgetötet wurde.
Bei diesem Test zeigen z.B. die folgenden Verbindungen der Herstellungsbeispiele eine Wirkung von 100%

bei einer Aufwandmenge von 20ppm: 1-1, 1-2, 1-16, 1-18, 1-20, 1-22, 1-25, 1-26, 1-27, 1-28, 1-44, 1-46, 1-53, 1-155, 1-170, 1-173.

Bei diesem Test zeigen z.B. die folgenden Verbindungen der Herstellungsbeispiele eine Wirkung von 90% bei einer Aufwandmenge von 20ppm: 1-4, 1-19, 1-34, 1-43, 1-45, 1-52, 1-63, 1-82, 1-164, 1-196, 1-168, 1-176.

Bei diesem Test zeigen z.B. die folgenden Verbindungen der Herstellungsbeispiele eine Wirkung von 80% bei einer Aufwandmenge von 20ppm: 1-15, 1-23, 1-29, 1-33, 1-35, 1-36, 1-42, 1-57, 1-78, 1-167, 1-169, 1-195.

**Meloidogyne incognita- Test (MELGIN)**

Lösungsmittel: 125,0 Gewichtsteile Aceton

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, einer Ei-Larven-Suspension des südlichen Wurzelgallenälchens (*Meloidogyne incognita*) und Salatsamen gefüllt. Die Salatsamen keimen und die Pflänzchen entwickeln sich. An den Wurzeln entwickeln sich die Gallen.

Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigen z. B. die folgenden Verbindungen der Herstellungsbeispiele eine Wirkung von 100% bei einer Aufwandmenge von 20ppm: 1-164, 1-170.

Bei diesem Test zeigen z. B. die folgenden Verbindungen der Herstellungsbeispiele eine Wirkung von 90% bei einer Aufwandmenge von 20ppm: 1-49, 1-63, 1-133, 1-134, 1-141, 1-177, 1-189.

Bei diesem Test zeigen z. B. die folgenden Verbindungen der Herstellungsbeispiele eine Wirkung von 80% bei einer Aufwandmenge von 20ppm: 1-18.

**Beispiel 2**

**Haemonchus contortus - Test (HAEMCO)**

Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit "Ringerlösung" auf die gewünschte Konzentration. Gefäße mit der Wirkstoffzubereitung der gewünschten Konzentration werden mit ca. 40 Larven des Roten Magenwurmes (*Haemonchus contortus*) besetzt.

Nach 5 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z. B. die folgenden Verbindungen der Herstellungsbeispiele überlegene Wirksamkeit gegenüber dem Stand der Technik: siehe Tabelle

**Cooperia curticei - Test (COOPCU)**

Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit "Ringerlösung" auf die gewünschte Konzentration. Gefäße mit der Wirkstoffzubereitung der gewünschten Konzentration werden mit ca. 40 Nematodenlarven (*Cooperia curticei*) besetzt.

Nach 5 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z. B. die folgenden Verbindungen der Herstellungsbeispiele überlegene Wirksamkeit gegenüber dem Stand der Technik: siehe Tabelle

**Tabelle 3**

| Substanz | Struktur | Tierart | Konzentration | % Wirkung dat |
|---|---|---|---|---|
| 1.239<br>Bekannt aus WO2009/0 12998 | 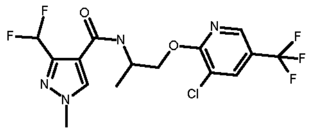 | HAEMCO<br>COOPCU | 20 ppm<br>20 ppm | 0 5 dat<br>0 5 dat |
| 4-2<br>Bekannt aus WO2012/118139 | 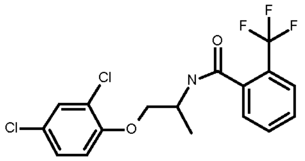 | COOPCU | 20 ppm | 0 5 dat |
| 1-4<br>aus Tabelle 1 | 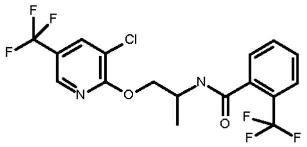 | HAEMCO<br>COOPCU | 20 ppm<br>20 ppm | 90 5 dat<br>100 5 dat |

**Patentansprüche**

1. Verbindungen der Formel (I),

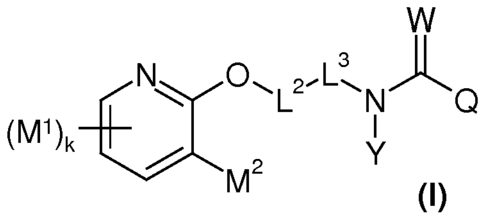

(I)

wobei

Q für 2-Thienyl, 3-Fluor-2-thienyl, 3-Chlor-2-thienyl, 3,4-Dichlor-2-thienyl, 2,5-Dichlor-3-thienyl, 3,4,5-Trichlor-2-thienyl, 3-Brom-2-thienyl, 3-Iod-2-thienyl, 3-Cyano-2-thienyl, 3-Methyl-2-thienyl, 3-(Trifluormethyl)-2-thienyl, 3-Methoxy-2-thienyl, 3-Ethoxy-2-thienyl, 3-Thienyl, 2-Fluor-3-thienyl, 2-Chlor-3-thienyl, 2-Brom-3-thienyl, 2-Iod-3-thienyl, 2-Cyano-3-thienyl, 2-Methyl-3-thienyl, 2-(Trifluormethyl)-3-thienyl, 2-Methoxy-3-thienyl, 2-Ethoxy-3-thienyl, 2-Furanyl, 3-Fluor-2-furanyl, 3-Chlor-2-furanyl, 3-Brom-2-furanyl, 3-Iod-2-furanyl, 3-Cyano-2-furanyl, 3-Methyl-2-furanyl, 3-(Trifluormethyl)-2-furanyl, 3-Methoxy-2-furanyl, 3-Ethoxy-2-furanyl, 3-Furanyl, 2-Chlor-3-furanyl, 2-Brom-3-furanyl, 2-Iod-3-furanyl, 2-Cyano-3-furanyl, 2-Methyl-3-furanyl, 2-(Trifluormethyl)-3-furanyl, 2-Methoxy-3-furanyl, 2-Ethoxy-3-furanyl, 2-Methyl-phenyl , 2-Fluorphenyl, 2,6-Difluor-phenyl, 2-Chlor-phenyl, 2,6-Dichlor-phenyl, 2-Brom-phenyl, 2-Iod-phenyl, 2-(Difluormethyl)-phenyl, 2-(Trifluormethyl)-phenyl, 2-(Methylsulfanyl)-phenyl, 2-(Methylsulfonyl)-phenyl, 2-(Trifluormethoxy)-phenyl, 2-(Trifluormethylsulfanyl)-phenyl, 2-(Trifluormethylsulfonyl)-phenyl, 2-Nitro-phenyl, 2-Chlor-3-pyridyl, 3-Chlor-2-pyridyl, 2-(Difluormethyl)-3-pyridyl,2-(Trifluormethyl)-3-pyridyl, 2-(Methylsulfanyl)-3-pyridyl, 2-(Methylsulfonyl)- 3-pyridyl, 2-(Trifluormethoxy)-3-pyridyl, 2-(Trifluormethylsulfanyl)-3-pyridyl oder 2-(Trifluormethylsulfonyl)- 3-pyridyl steht;
Y für Wasserstoff, Methyl, Ethyl, n-Propyl, iso-Propyl, n-Butyl, sec-Butyl, iso-Butyl, tert.-Butyl, Cyanomethyl, 2,2-Difluor-ethyl, 2,2,2-Trifluor-ethyl, Allyl, Butenyl, Propargyl, Butinyl, 3,3-Dichlor-prop-2-enyl, Methoxy, Ethoxy, Cyclopropylmethyl, Cyclopropyl, Cyclobutyl, Cyclopentyl oder Cyclohexyl steht;
W für Sauerstoff steht;
$L^2$ für $C(R^{21}, R^{22})$ steht, wobei $R^{21}$ und $R^{22}$ jeweils unabhängig voneinander für Wasserstoff, Fluor, Methyl, Ethyl, n-Propyl, iso-Propyl, Allyl, Propargyl, Methoxy, Ethoxy, Allyloxy, Propargyloxy, Cyclopropylmethyl, Cyclopropyl stehen, oder wobei $C(R^{21}, R^{22})$ für spiro-$C(CH_2\text{-}CH_2)$ steht;

$L^3$ für $C(R^{31}, R^{32})$ steht, wobei $R^{31}$ und $R^{32}$ jeweils unabhängig voneinander für Wasserstoff, Methyl, Ethyl, n-Propyl, iso-Propyl, Allyl, Propargyl, Methoxy, Ethoxy, Allyloxy, Propargyloxy, Cyclopropylmethyl, Cyclopropyl stehen, oder wobei $C(R^{31}, R^{32})$ für spiro-$C(CH_2\text{-}CH_2)$ steht;
k für 1 steht und
$M^1$ und $M^2$ jeweils unabhängig voneinander für Wasserstoff, Fluor, Brom, Chlor, Iod, Cyano, Methyl, Trifluormethyl, Difluormethyl, Methoxy, Ethoxy, iso-Propoxy oder Phenoxy stehen;

sowie Salze, N-Oxide und tautomere Formen der Verbindungen der Formel (I).

**2.** Verbindungen der Formel (I) gemäß Anspruch 1, wobei

Q für 2-Thienyl, 3-Fluor-2-thienyl, 3-Chlor-2-thienyl, 3,4-Dichlor-2-thienyl, 2,5-Dichlor-3-thienyl, 3,4,5-Trichlor-2-thienyl, 3-Brom-2-thienyl, 3-Iod-2-thienyl, 3-Cyano-2-thienyl, 3-Methyl-2-thienyl, 3-(Trifluormethyl)-2-thienyl, 3-Methoxy-2-thienyl, 3-Ethoxy-2-thienyl, 3-Thienyl, 2-Fluor-3-thienyl, 2-Chlor-3-thienyl, 2-Brom-3-thienyl, 2-Iod-3-thienyl, 2-Cyano-3-thienyl, 2-Methyl-3-thienyl, 2-(Trifluormethyl)-3-thienyl, 2-Methoxy-3-thienyl, 2-Ethoxy-3-thienyl, 2-Furanyl, 3-Fluor-2-furanyl, 3-Chlor-2-furanyl, 3-Brom-2-furanyl, 3-Iod-2-furanyl, 3-Cyano-2-furanyl, 3-Methyl-2-furanyl, 3-(Trifluormethyl)-2-furanyl, 3-Methoxy-2-furanyl, 3-Ethoxy-2-furanyl, 3-Furanyl, 2-Chlor-3-furanyl, 2-Brom-3-furanyl, 2-Iod-3-furanyl, 2-Cyano-3-furanyl, 2-Methyl-3-furanyl, 2-(Trifluormethyl)-3-furanyl, 2-Methoxy-3-furanyl, 2-Ethoxy-3-furanyl, 2-Methyl-phenyl , 2-Fluorphenyl, 2,6-Difluor-phenyl, 2-Chlor-phenyl, 2,6-Dichlor-phenyl, 2-Brom-phenyl, 2-Iod-phenyl, 2-(Difluormethyl)-phenyl, 2-(Trifluormethyl)-phenyl, 2-(Methylsulfanyl)-phenyl, 2-(Methylsulfonyl)-phenyl, 2-(Trifluormethoxy)-phenyl, 2-(Trifluormethylsulfanyl)-phenyl, 2-(Trifluormethylsulfonyl)-phenyl, 2-Nitro-phenyl, 2-Chlor-3-pyridyl, 3-Chlor-2-pyridyl, 2-(Difluormethyl)-3-pyridyl,2-(Trifluormethyl)-3-pyridyl, 2-(Methylsulfanyl)-3-pyridyl, 2-(Methylsulfonyl)- 3-pyridyl, 2-(Trifluormethoxy)-3-pyridyl, 2-(Trifluormethylsulfanyl)- 3-pyridyl oder 2-(Trifluormethylsulfonyl)- 3-pyridyl steht;
Y für Wasserstoff steht;
W für Sauerstoff steht;
$L^2$ für $C(R^{21}, R^{22})$ steht, wobei $R^{21}$ und $R^{22}$ jeweils unabhängig voneinander für Wasserstoff, Methyl, Cyclopropyl stehen;
$L^3$ für $C(R^{31}, R^{32})$ steht, wobei $R^{31}$ und $R^{32}$ jeweils unabhängig voneinander für Wasserstoff, Methyl, Ethyl, n-Propyl, iso-Propyl stehen, oder wobei $C(R^{31}, R^{32})$ für 1,1-Cyclopropyl steht;
k für 1 steht und
$M^1$ und $M^2$ jeweils unabhängig voneinander für Wasserstoff, Fluor, Brom, Chlor, Cyano, Trifluormethyl, Difluormethyl, oder Nitro stehen;

sowie Salze, N-Oxide und tautomere Formen der Verbindungen der Formel (I).

**3.** Verfahren zur Herstellung der Verbindungen der Formel (I) gemäß einem der Ansprüche 1 bis 2, wobei in einem ersten Schritt in Prozess A eine Verbindung der Formel (a1),

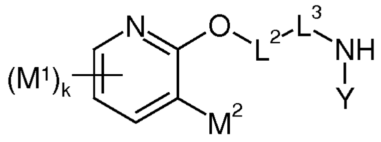

(a1)

wobei die Reste $M^1$, $M^2$, $L^2$, $L^3$, und Y sowie k die in Anspruch 1 angegebene Bedeutung haben,
mit einer Verbindung der Formel (a2) in Gegenwart eines Kondensationsmittels oder einer Verbindung der Formel (a3),

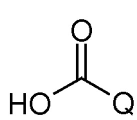

(a2)

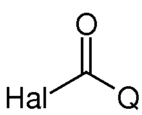

(a3)

wobei Hal für Fluor, Chlor oder Brom steht und Q die in Anspruch 1 angegebene Bedeutung hat, zu einer Verbindung der Formel (I-a)

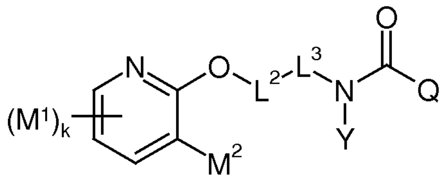

(I-a)

umgesetzt werden;
und in einem zweiten, optionalen Schritt in Prozess D, falls W in einer Verbindung gemäß Formel (I) für Schwefel steht, eine Verbindung der Formel (I-a)

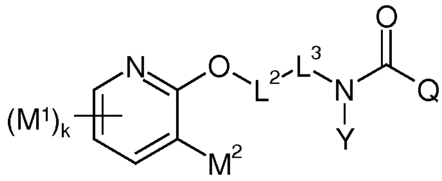

(I-a)

wobei $M^1$, $M^2$, $L^2$, $L^3$, Q und Y sowie k die in Anspruch 1 angegebene Bedeutung haben,
in Gegenwart von $P_4S_{10}$ oder Lawesson Reagenz zu einer Verbindung der Formel (I-c) umgesetzt wird.

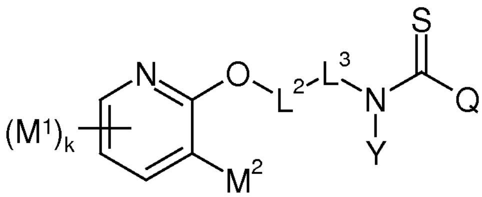

(I-c)

**4.** Verfahren zur Herstellung der Verbindungen der Formel (I) gemäß einem der Ansprüche 1 bis 2, wobei in einem ersten Schritt in Prozess B eine Verbindung der Formel (b1)

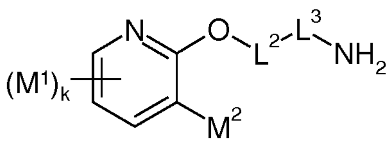

(b1)

wobei die Reste $M^1$, $M^2$, $L^2$, $L^3$ sowie k die in Anspruch 1 angegebene Bedeutung haben,
mit einer Verbindung der Formel (a2) oder einer Verbindung der Formel (a3) wie in Prozess A in Anspruch 4. beschrieben,
wobei Hal für Fluor, Chlor oder Brom steht und Q die in Anspruch 1 angegebene Bedeutung hat,
zu einer Verbindung der Formel (I-b) umgesetzt werden;

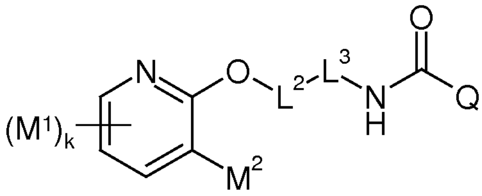

(I-b)

und

in einem zweiten, optionalen Schritt in Prozess B, falls Y in Formel (I) nicht für Wasserstoff steht, die Verbindung (I-b),

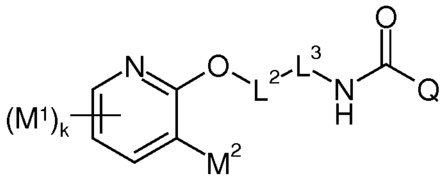

(I-b)

wobei die Reste $M^1$, $M^2$, $L^2$, $L^3$ und Q sowie k die in Anspruch 1 angegebene Bedeutung haben, mit einer Verbindung der Formel (c1)

Y-AG (c1),

wobei AG für eine Abgangsgruppe steht,

zu einer Verbindung der Formel (I-a),

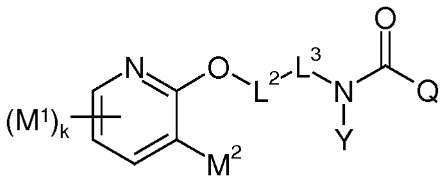

(I-a) ,

wobei $M^1$, $M^2$, $L^2$, $L^3$, Q und Y sowie k die in Anspruch 1 angegebene Bedeutung haben, umgesetzt wird; und

in einem dritten, optionalen Schritt in Prozess D, falls W in Formel (I) für Schwefel steht,

eine Verbindung der Formel (I-a),

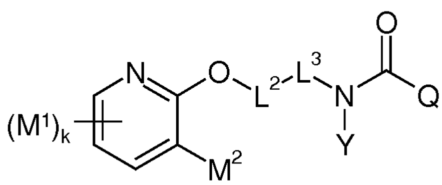

(I-a)

wobei $M^1$, $M^2$, $L^2$, $L^3$, Q und Y sowie k die in Anspruch 1 angegebene Bedeutung haben,

in Gegenwart von $P_4S_{10}$ oder Lawesson Reagenz zu einer Verbindung der Formel (I-c) umgesetzt werden.

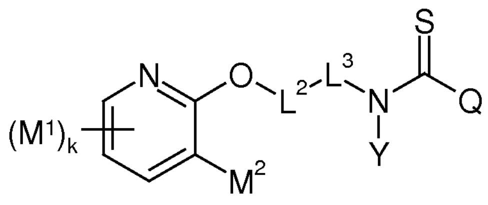

(I-c)

5. Verbindungen der Formel (I) gemäß einem der Ansprüche 1 bis 2 zur Verwendung als Arzneimittel zur Bekämpfung von Endoparasiten bei Tieren oder Menschen.

6. Verbindungen der Formel (I) gemäß einem der Ansprüche 1 bis 2 zur Verwendung bei der Bekämpfung von Endoparasiten bei Tieren oder Menschen

7. Verwendung von Verbindungen gemäß einem der Ansprüche 1 bis 2 zur Herstellung von Arzneimitteln zur Bekämpfung von Endoparasiten bei Tieren oder Menschen.

8. Verbindungen gemäß einem der Ansprüche 1 bis 2 zur Verwendung bei der Bekämpfung von Endoparasiten bei Tieren, **dadurch gekennzeichnet, dass** Verbindungen gemäß einem der Ansprüche 1 bis 3 sowie deren Salze, N-Oxide und tautomere Formen Tieren oder Menschen prophylaktisch oder therapeutisch verabreicht werden

9. Endoparasitizide Mittel enthaltend Verbindungen der Formel (I) gemäß einem der Ansprüche 1 bis 2.

10. Verwendung der Verbindungen gemäß einem der Ansprüche 1 bis 2 zur Bekämpfung von phytopathogenen Nematoden

11. Methode zur Bekämpfung von phytopathogenen Nematoden, **dadurch gekennzeichnet, dass** Verbindungen der Formel (I) gemäß einem der Ansprüche 1 bis 2 in Kontakt mit der Pflanze oder Pflanzenteilen, dem Boden, in dem die Pflanze wächst oder Pflanzenvermehrungsmaterial gebracht wird.

12. Nematizide oder endoparasitizide Zusammensetzung enthaltend eine oder mehrere Verbindungen der Formel (I) gemäß einem der Ansprüche 1 bis 2 sowie ein oder mehrere Hilfsstoffe.

**Claims**

1. Compounds of the formula (I)

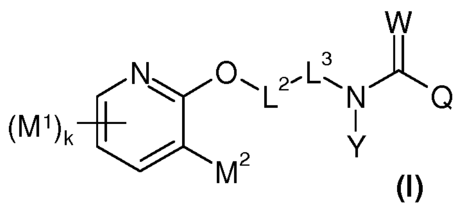

**(I)**

where

Q represents 2-thienyl, 3-fluoro-2-thienyl, 3-chloro-2-thienyl, 3,4-dichloro-2-thienyl, 2,5-dichloro-3-thienyl, 3,4,5-trichloro-2-thienyl, 3-bromo-2-thienyl, 3-iodo-2-thienyl, 3-cyano-2-thienyl, 3-methyl-2-thienyl, 3-(trifluoromethyl)-2-thienyl, 3-methoxy-2-thienyl, 3-ethoxy-2-thienyl, 3-thienyl, 2-fluoro-3-thienyl, 2-chloro-3-thienyl, 2-bromo-3-thienyl, 2-iodo-3-thienyl, 2-cyano-3-thienyl, 2-methyl-3-thienyl, 2-(trifluoromethyl)-3-thienyl, 2-methoxy-3-thienyl, 2-ethoxy-3-thienyl, 2-furanyl, 3-fluoro-2-furanyl, 3-chloro-2-furanyl, 3-bromo-2-furanyl, 3-iodo-2-furanyl, 3-cyano-2-furanyl, 3-methyl-2-furanyl, 3-(trifluoromethyl)-2-furanyl, 3-methoxy-2-furanyl, 3-ethoxy-2-furanyl, 3-furanyl, 2-chloro-3-furanyl, 2-bromo-3-furanyl, 2-iodo-3-furanyl, 2-cyano-3-furanyl, 2-methyl-3-furanyl, 2-(trifluoromethyl)-3-furanyl, 2-methoxy-3-furanyl, 2-ethoxy-3-furanyl, 2-methylphenyl , 2-fluorophenyl, 2,6-difluorophenyl, 2-chlorophenyl, 2,6-dichlorophenyl, 2-bromophenyl, 2-iodophenyl, 2-(difluoromethyl)phenyl, 2-(trifluoromethyl)phenyl, 2-(methylsulfanyl)phenyl, 2-(methylsulfonyl)phenyl, 2-(trifluoromethoxy)phenyl, 2-(trifluoromethylsulfanyl)phenyl, 2-(trifluoromethylsulfonyl)-phenyl, 2-nitrophenyl, 2-chloro-3-pyridyl, 3-chloro-2-pyridyl, 2-(difluoromethyl)-3-pyridyl, 2-(trifluoromethyl)-3-pyridyl, 2-(methylsulfanyl)-3-pyridyl, 2-(methylsulfonyl)-3-pyridyl, 2-(trifluoromethoxy)-3-pyridyl, 2-(trifluoromethylsulfanyl)-3-pyridyl or 2-(trifluoromethylsulfonyl)-3-pyridyl;
Y represents hydrogen, methyl, ethyl, n-propyl, isopropyl, n-butyl, sec-butyl, isobutyl, tert-butyl, cyanomethyl, 2,2-difluoroethyl, 2,2,2-trifluoroethyl, allyl, butenyl, propargyl, butynyl, 3,3-dichloroprop-2-enyl, methoxy, ethoxy, cyclopropylmethyl, cyclopropyl, cyclobutyl, cyclopentyl or cyclohexyl;
W represents oxygen;
$L^2$ represents C($R^{21}$, $R^{22}$), where R21 and R22 each independently of one another represent hydrogen, fluorine, methyl, ethyl, n-propyl, isopropyl, allyl, propargyl, methoxy, ethoxy, allyloxy, propargyloxy, cyclopropylmethyl, cyclopropyl, or where C(R21, R22) represents spiro-C(CH2-CH2);
$L^3$ represents C ($R^{31}$, $R^{32}$), where $R^{31}$ and $R^{32}$ each independently of one another represent hydrogen, methyl, ethyl, n-propyl, isopropyl, allyl, propargyl, methoxy, ethoxy, allyloxy, propargyloxy, cyclopropylmethyl, cyclopropyl, or where C ($R^{31}$, $R^{32}$) represents spiro-C ($CH_2$-$CH_2$) ;
k represents 1 and
$M^1$ and $M^2$ each independently of one another represent hydrogen, fluorine, bromine, chlorine, iodine, cyano, methyl, trifluoromethyl, difluoromethyl, methoxy, ethoxy, isopropoxy or phenoxy;

and salts, N-oxides and tautomeric forms of the compounds of the formula (I).

**2.** Compounds of the formula (I) according to Claim 1 where

Q represents 2-thienyl, 3-fluoro-2-thienyl, 3-chloro-2-thienyl, 3,4-dichloro-2-thienyl, 2,5-dichloro-3-thienyl, 3,4,5-trichloro-2-thienyl, 3-bromo-2-thienyl, 3-iodo-2-thienyl, 3-cyano-2-thienyl, 3-methyl-2-thienyl, 3-(trifluoromethyl)-2-thienyl, 3-methoxy-2-thienyl, 3-ethoxy-2-thienyl, 3-thienyl, 2-fluoro-3-thienyl, 2-chloro-3-thienyl, 2-bromo-3-thienyl, 2-iodo-3-thienyl, 2-cyano-3-thienyl, 2-methyl-3-thienyl, 2-(trifluoromethyl)-3-thienyl, 2-methoxy-3-thienyl, 2-ethoxy-3-thienyl, 2-furanyl, 3-fluoro-2-furanyl, 3-chloro-2-furanyl, 3-bromo-2-furanyl, 3-iodo-2-furanyl, 3-cyano-2-furanyl, 3-methyl-2-furanyl, 3-(trifluoromethyl)-2-furanyl, 3-methoxy-2-furanyl, 3-ethoxy-2-furanyl, 3-furanyl, 2-chloro-3-furanyl, 2-bromo-3-furanyl, 2-iodo-3-furanyl, 2-cyano-3-furanyl, 2-methyl-3-furanyl, 2-(trifluoromethyl)-3-furanyl, 2-methoxy-3-furanyl, 2-ethoxy-3-furanyl, 2-methylphenyl , 2-fluorophenyl, 2,6-difluorophenyl, 2-chlorophenyl, 2,6-dichlorophenyl, 2-bromophenyl, 2-iodophenyl, 2-(difluoromethyl)phenyl, 2-(trifluoromethyl)phenyl, 2-(methylsulphanyl)phenyl, 2-(methylsulphonyl)phenyl, 2-(trifluoromethoxy)phenyl, 2-(trifluoromethylsulphanyl)phenyl, 2-(trifluoromethylsulphonyl)-phenyl, 2-nitrophenyl, 2-chloro-3-pyridyl, 3-chloro-2-pyridyl, 2-(difluoromethyl)-3-pyridyl, 2-(trifluoromethyl)-3-pyridyl, 2-(methylsulphanyl)-3-pyridyl, 2-(methylsulphonyl)-3-pyridyl, 2-(trifluoromethoxy)-3-pyridyl, 2-(trifluoromethylsulphanyl)-3-pyridyl or 2-(trifluoromethylsulphonyl)-3-pyridyl;
Y represents hydrogen;
W represents oxygen;
$L^2$ represents C ($R^{21}$, $R^{22}$), where $R^{21}$ and $R^{22}$ each independently of one another represent hydrogen, methyl, cyclopropyl;
$L^3$ represents C ($R^{31}$, $R^{32}$), where $R^{31}$ and $R^{32}$ each independently of one another represent hydrogen, methyl, ethyl, n-propyl isopropyl, or where C($R^{31}$, $R^{32}$) represents 1,1-cyclopropyl;
k represents 1 and
$M^1$ and $M^2$ each independently of one another represent hydrogen, fluorine, bromine, chlorine, cyano, trifluoromethyl, difluoromethyl, or nitro;

and salts, N-oxides and tautomeric forms of the compounds of the formula (I).

**3.** Process for preparing the compounds of the formula (I) according to any of Claims 1 and 2, where
in a first step in process A a compound of the formula (a1),

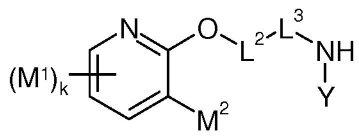

(a1)

where the radicals $M^1$, $M^2$, $L^2$, $L^3$, and Y and k have the meaning given in Claim 1, is reacted with a compound of the formula (a2) in the presence of a condensing agent or a compound of the formula (a3),

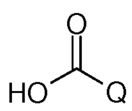

(a2)

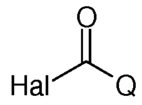

(a3)

where Hal represents fluorine, chlorine or bromine and
Q has the meaning given in Claim 1,
to give a compound of the formula (I-a)

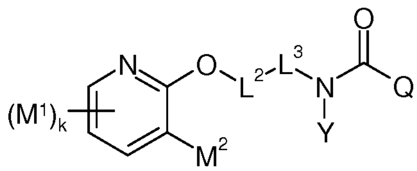

(I-a)

and in a second optional step in process D, if W in a compound of the formula (I) represents sulfur, a compound of the formula (I-a),

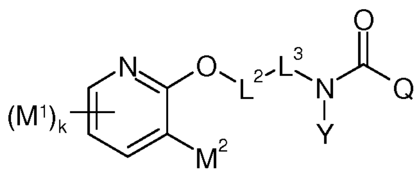

(I-a)

where $M^1$, $M^2$, $L^2$, $L^3$, Q and Y and k have the meaning given in Claim 1, is reacted in the presence of $P_4S_{10}$ or Lawesson's reagent to give a compound of the formula (I-c).

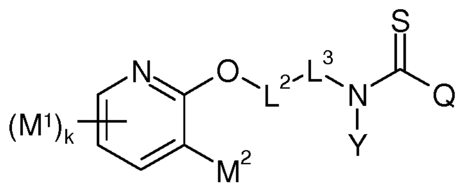

(I-c)

**4.** Process for preparing the compounds of the formula (I) according to any of Claims 1 and 2, where

in a first step in process B a compound of the formula (b1),

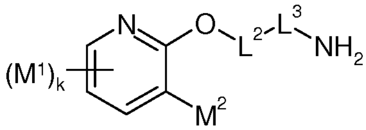

(b1)

where the radicals $M^1$, $M^2$, $L^2$, $L^3$ and k have the meaning given in Claim 1, is reacted with a compound of the formula (a2) or a compound of the formula (a3) as described in process A in Claim 4, where Hal represents fluorine, chlorine or bromine and Q has the meaning given in Claim 1, to give a compound of the formula (I-b);

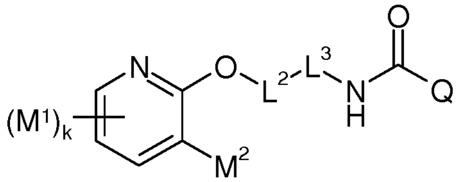

(I-b)

and in a second optional step in process B, if Y in formula (I) does not represent hydrogen, the compound (I-b),

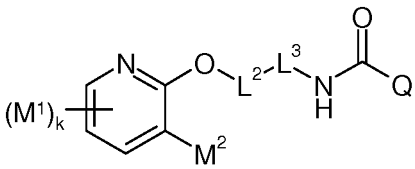

(I-b)

where the radicals $M^1$, $M^2$, $L^2$, $L^3$ and Q and k have the meaning given in Claim 1, is reacted with a compound of the formula (c1)

Y-AG (c1)

where AGrepresents a leaving group, to give a compound of the formula (I-a)

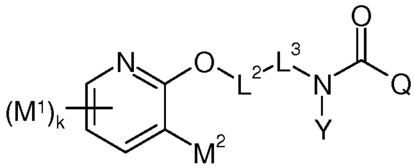

(I-a) ,

where $M^1$, $M^2$, $L^2$, $L^3$, Q and Y and k have the meaning given in Claim 1; and in a third optional step in process D, if W in formula (I) represents sulfur, a compound of the formula (I-a),

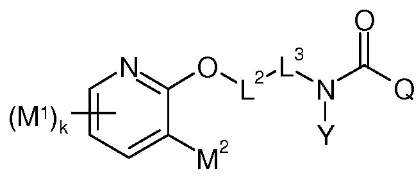

(I-a)

where $M^1$, $M^2$, $L^2$, $L^3$, Q and Y and k have the meaning given in Claim 1, is reacted in the presence of $P_4S_{10}$ or Lawesson's reagent to give a compound of the formula (I-c).

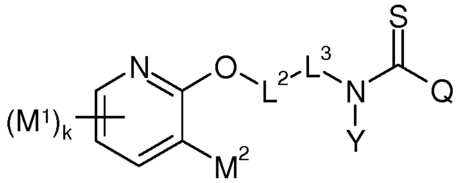

(I-c)

5. Compounds of the formula (I) according to any of Claims 1 and 2 for use as medicaments for controlling endoparasites in animals or humans.

6. Compounds of the formula (I) according to any of Claims 1 and 2 for use in the control of endoparasites in animals or humans.

7. Use of compounds according to any of Claims 1 and 2 for preparing medicaments for controlling endoparasites in animals or humans.

8. Compounds according to any of Claims 1 and 2 for use in the control of endoparasites in animals, **characterized in that** compounds according to any of Claims 1 to 3 and their salts, N-oxides and tautomeric forms are administered prophylactically or therapeutically to animals or humans.

9. Endoparasiticidal compositions comprising compounds of the formula (I) according to any of Claims 1 and 2.

10. Use of the compounds according to any of Claims 1 and 2 for controlling phytopathogenic nematodes.

11. Method for controlling phytopathogenic nematodes, **characterized in that** compounds of the formula (I) according to any of Claims 1 and 2 are brought into contact with the plant or plant parts, the soil in which the plant grows or plant propagation material.

12. Nematicidal or endoparasiticidal composition comprising one or more compounds of the formula (I) according to any of Claims 1 and 2 and one or more auxiliaries.

## Revendications

1. Composés de formule (I) dans laquelle

Q représente 2-thiényle, 3-fluoro-2-thiényle, 3-chloro-2-thiényle, 3,4-dichloro-2-thiényle, 2,5-dichloro-3-thiényle, 3,4,5-trichloro-2-thiényle, 3-bromo-2-thiényle, 3-iodo-2-thiényle, 3-cyano-2-thiényle, 3-méthyl-2-thiényle, 3-(trifluorométhyl)-2-thiényle, 3-méthoxy-2-thiényle, 3-éthoxy-2-thiényle, 3-thiényle, 2-fluoro-3-thiényle, 2-chloro-3-thiényle, 2-bromo-3-thiényle, 2-iodo-3-thiényle, 2-cyano-3-thiényle, 2-méthyl-3-thiényle, 2-(trifluorométhyl)-3-thiényle, 2-méthoxy-3-thiényle, 2-éthoxy-3-thiényle, 2-furanyle, 3-fluoro-2-furanyle, 3-chloro-2-furanyle, 3-bromo-2-furanyle, 3-iodo-2-furanyle, 3-cyano-2-furanyle, 3-méthyl-2-furanyle, 3-(trifluorométhyl)-2-furanyle, 3-méthoxy-2-furanyle, 3-éthoxy-2-furanyle, 3-furanyle, 2-chloro-3-furanyle, 2-bromo-3-furanyle, 2-iodo-3-furanyle, 2-cyano-3-furanyle, 2-méthyl-3-furanyle, 2-(trifluorométhyl)-3-furanyle, 2-méthoxy-3-furanyle, 2-éthoxy-3-furanyle, 2-méthyl-phényle, 2-fluoro-phényle, 2,6-difluoro-phényle, 2-chloro-phényle, 2,6-dichloro-phényle, 2-bromo-phényle, 2-iodo-phényle, 2-(difluorométhyl)-phényle, 2-(trifluorométhyl)-phényle, 2-(méthylsulfanyl)-phényle, 2-(méthylsulfonyl)-phényle, 2-(trifluorométhoxy)-phényle, 2-(trifluorométhylsulfanyl)-phényle, 2-(trifluorométhylsulfonyl)-phényle, 2-nitro-phényle, 2-chloro-3-pyridyle, 3-chloro-2-pyridyle, 2-(difluorométhyl)-3-pyridyle, 2-(trifluorométhyl)-3-pyridyle, 2-(méthylsulfanyl)-3-pyridyle, 2-(méthylsulfonyl)-3-pyridyle, 2-(trifluorométhoxy)-3-pyridyle, 2-(trifluorométhylsulfanyl)-3-pyridyle ou 2-(trifluorométhylsulfonyl)-3-pyridyle ;
Y représente hydrogène, méthyle, éthyle, n-propyle, iso-propyle, n-butyle, sec-butyle, iso-butyle, tert.-butyle, cyanométhyle, 2,2-difluoro-éthyle, 2,2,2-trifluoro-éthyle, allyle, butényle, propargyle, butynyle, 3,3-dichloro-prop-2-ényle, méthoxy, éthoxy, cyclopropylméthyle, cyclopropyle, cyclobutyle, cyclopentyle ou cyclohexyle ;
W représente oxygène ;
$L^2$ représente $C(R^{21}, R^{22})$, $R^{21}$ et $R^{22}$ représentant chacun indépendamment l'un de l'autre hydrogène, fluor, méthyle, éthyle, n-propyle, iso-propyle, allyle, propargyle, méthoxy, éthoxy, allyloxy, propargyloxy, cyclopropylméthyle, cyclopropyle, ou C $(R^{21}, R^{22})$ représente spiro-$C(CH_2\text{-}CH_2)$ ;
$L^3$ représente $C(R^{31}, R^{32})$, $R^{31}$ et $R^{32}$ représentant chacun indépendamment l'un de l'autre hydrogène, méthyle, éthyle, n-propyle, iso-propyle, allyle, propargyle, méthoxy, éthoxy, allyloxy, propargyloxy, cyclopropylméthyle, cyclopropyle, ou C $(R^{31}, R^{32})$ représente spiro-$C(CH_2\text{-}CH_2)$ ;
k représente 1 et
$M^1$ et $M^2$ représentent chacun indépendamment l'un de l'autre hydrogène, fluor, brome, chlore, iode, cyano, méthyle, trifluorométhyle, difluorométhyle, méthoxy, éthoxy, iso-propoxy ou phénoxy ;
ainsi que les sels, les N-oxydes et les formes tautomères des composés de formule (I).

**2.** Composés de formule (I) selon la revendication 1, dans lesquels
Q représente 2-thiényle, 3-fluoro-2-thiényle, 3-chloro-2-thiényle, 3,4-dichloro-2-thiényle, 2,5-dichloro-3-thiényle, 3,4,5-trichloro-2-thiényle, 3-bromo-2-thiényle, 3-iodo-2-thiényle, 3-cyano-2-thiényle, 3-méthyl-2-thiényle, 3-(trifluorométhyl)-2-thiényle, 3-méthoxy-2-thiényle, 3-éthoxy-2-thiényle, 3-thiényle, 2-fluoro-3-thiényle, 2-chloro-3-thiényle, 2-bromo-3-thiényle, 2-iodo-3-thiényle, 2-cyano-3-thiényle, 2-méthyl-3-thiényle, 2-(trifluorométhyl)-3-thiényle, 2-méthoxy-3-thiényle, 2-éthoxy-3-thiényle, 2-furanyle, 3-fluoro-2-furanyle, 3-chloro-2-furanyle, 3-bromo-2-furanyle, 3-iodo-2-furanyle, 3-cyano-2-furanyle, 3-méthyl-2-furanyle, 3-(trifluorométhyl)-2-furanyle, 3-méthoxy-2-furanyle, 3-éthoxy-2-furanyle, 3-furanyle, 2-chloro-3-furanyle, 2-bromo-3-furanyle, 2-iodo-3-furanyle, 2-cyano-3-furanyle, 2-méthyl-3-furanyle, 2-(trifluorométhyl)-3-furanyle, 2-méthoxy-3-furanyle, 2-éthoxy-3-furanyle, 2-méthyl-phényle, 2-fluoro-phényle, 2,6-difluoro-phényle, 2-chloro-phényle, 2,6-dichloro-phényle, 2-bromo-phényle, 2-iodo-phényle, 2-(difluorométhyl)-phényle, 2-(trifluorométhyl)-phényle, 2-(méthylsulfanyl)-phényle, 2-(méthylsulfonyl)-phényle, 2-(trifluorométhoxy)-phényle, 2-(trifluorométhylsulfanyl)-phényle, 2-(trifluorométhylsulfonyl)-phényle, 2-nitro-phényle, 2-chloro-3-pyridyle, 3-chloro-2-pyridyle, 2-(difluorométhyl)-3-pyridyle, 2-(trifluorométhyl)-3-pyridyle, 2-(méthylsulfanyl)-3-pyridyle, 2-(méthylsulfonyl)-3-pyridyle, 2-(trifluorométhoxy)-3-pyridyle, 2-(trifluorométhylsulfanyl)-3-pyridyle ou 2-(trifluorométhylsulfonyl)-3-pyridyle ;
Y représente hydrogène ;
W représente oxygène ;
$L^2$ représente $C(R^{21}, R^{22})$, $R^{21}$ et $R^{22}$ représentant chacun indépendamment l'un de l'autre hydrogène, méthyle, cyclopropyle ;
$L^3$ représente $C(R^{31}, R^{32})$, $R^{31}$ et $R^{32}$ représentant chacun indépendamment l'un de l'autre hydrogène, méthyle, éthyle, n-propyle, iso-propyle, ou $C(R^{31}, R^{32})$ représente 1,1-cyclopropyle ;
k représente 1 et
$M^1$ et $M^2$ représentent chacun indépendamment l'un de l'autre hydrogène, fluor, brome, chlore, cyano, trifluorométhyle, difluorométhyle ou nitro ;
ainsi que les sels, les N-oxydes et les formes tautomères des composés de formule (I).

**3.** Procédé de fabrication des composés de formule (I) selon l'une quelconque des revendications 1 à 2, dans lequel dans une première étape, dans le procédé A, un composé de formule (a1)
dans laquelle les radicaux $M^1$, $M^2$, $L^2$, $L^3$ et Y, ainsi que k ont la signification indiquée dans la revendication 1, est mis en réaction avec un composé de formule (a2) en présence d'un agent de condensation ou avec un composé de formule (a3)
dans laquelle Hal représente fluor, chlore ou brome, et Q a la signification indiquée dans la revendication 1, pour former un composé de formule (I-a)
et dans une deuxième étape optionnelle, dans le procédé D, lorsque W dans un composé selon la formule (I) représente le soufre,
un composé de formule (I-a)
dans laquelle $M^1$, $M^2$, $L^2$, $L^3$, Q et Y, ainsi que k ont la signification indiquée dans la revendication 1,

est mis en réaction en présence de $P_4S_{10}$ ou du réactif de Lawesson pour former un composé de formule (I-c)

4. Procédé de fabrication des composés de formule (I) selon l'une quelconque des revendications 1 à 2, dans lequel dans une première étape, dans le procédé B, un composé de formule (b1)
dans laquelle les radicaux $M^1$, $M^2$, $L^2$, $L^3$, ainsi que k ont la signification indiquée dans la revendication 1, est mis en réaction avec un composé de formule (a2) ou un composé de formule (a3) tel que décrit dans le procédé A dans la revendication 4,
Hal représentant fluor, chlore ou brome, et Q ayant la signification indiquée dans la revendication 1, pour former un composé de formule (I-b) ;
et
dans une deuxième étape optionnelle, dans le procédé B, lorsqu'Y dans la formule (I) ne représente pas l'hydrogène, le composé (I-b)
dans laquelle les radicaux $M^1$, $M^2$, $L^2$, $L^3$ et Q, ainsi que k ont la signification indiquée dans la revendication 1, est mis en réaction avec un composé de formule (c1)

Y-AG (c1)

dans laquelle AG représente un groupe partant,
pour former un composé de formule (I-a)
dans laquelle $M^1$, $M^2$, $L^2$, $L^3$, Q et Y, ainsi que k ont la signification indiquée dans la revendication 1 ; et dans une troisième étape optionnelle, dans le procédé D, lorsque W dans la formule (I) représente le soufre, un composé de formule (I-a)
dans laquelle $M^1$, $M^2$, $L^2$, $L^3$, Q et Y, ainsi que k ont la signification indiquée dans la revendication 1,
est mis en réaction en présence de $P_4S_{10}$ ou du réactif de Lawesson pour former un composé de formule (I-c)

5. Composés de formule (I) selon l'une quelconque des revendications 1 à 2, destinés à une utilisation en tant que médicament pour lutter contre des endoparasites chez des animaux ou des hommes.

6. Composés de formule (I) selon l'une quelconque des revendications 1 à 2, destinés à une utilisation pour lutter contre des endoparasites chez des animaux ou des hommes.

7. Utilisation de composés selon l'une quelconque des revendications 1 à 2 pour la fabrication de médicaments pour lutter contre des endoparasites chez des animaux ou des hommes.

8. Composés selon l'une quelconque des revendications 1 à 2, destinés à une utilisation pour lutter contre des endoparasites chez des animaux, **caractérisés en ce que** des composés selon l'une quelconque des revendications 1 à 3, ainsi que leurs sels, leurs N-oxydes et leurs formes tautomères sont administrés à titre prophylactique ou thérapeutique à des animaux ou des hommes.

9. Agents endoparasiticides contenant des composés de formule (I) selon l'une quelconque des revendications 1 à 2.

10. Utilisation des composés selon l'une quelconque des revendications 1 à 2 pour lutter contre des nématodes phytopathogènes.

11. Méthode de lutte contre des nématodes phytopathogènes, **caractérisée en ce que** des composés de formule (I) selon l'une quelconque des revendications 1 à 2 sont mis en contact avec la plante ou les parties de la plante, le sol dans lequel la plante pousse ou le matériel de reproduction de la plante.

12. Composition nématicide ou endoparasiticide contenant un ou plusieurs composés de formule (I) selon l'une quelconque des revendications 1 à 2, ainsi qu'un ou plusieurs adjuvants.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2001060783 A
- WO 199924413 A
- WO 2006108791 A
- WO 2006108792 A
- WO 2007060162 A
- WO 2007060164 A
- WO 2007060166 A
- WO 2008101976 A
- WO 2008148570 A
- WO 2010063700 A
- WO 2011128989 A
- WO 2004016088 A
- WO 2004074280 A
- WO 2005014545 A
- WO 2005058828 A
- WO 2005058833 A
- WO 2005085238 A
- WO 2009012998 A
- WO 2011151370 A
- WO 2007108483 A
- EP 1997800 A
- WO 2008126922 A
- WO 2012118139 A
- DE 10307845 A1
- WO 2013064460 A
- WO 2013064461 A
- WO 2013076230 A
- EP 2007060166 A


### In der Beschreibung aufgeführte Nicht-Patentliteratur


- **HOUBEN-WEYL.** Methoden der Organischen Chemie. Thieme Verlag, 1985, vol. E5, 1255
- **BAUR et al.** *Pesticide Science,* 1997, vol. 51, 131-152